(19) Europäisches Patentamt
European Patent Office
Office européen des brevets

(11) EP 4 782 440 A1

# (12) EUROPEAN PATENT APPLICATION
published in accordance with Art. 153(4) EPC

(43) Date of publication:
**29.07.2026 Bulletin 2026/31**

(21) Application number: **24884841.8**

(22) Date of filing: **30.10.2024**

(51) International Patent Classification (IPC):
***C07D 401/12*** (2006.01) ***C07D 405/12*** (2006.01)
***A61K 51/04*** (2006.01) ***A61P 35/00*** (2006.01)

(52) Cooperative Patent Classification (CPC):
**A61K 31/555; A61K 38/05; A61K 38/06; A61K 49/10; A61K 51/04; A61P 35/00; C07D 401/12; C07D 401/14; C07D 405/12; C07D 413/12; C07K 5/06078; C07K 5/06104; C07K 5/0804; C07K 5/0812; C07K 5/0819;** (Cont.)

(86) International application number:
**PCT/CN2024/128619**

(87) International publication number:
**WO 2025/092841 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 CN 202311428064**

(71) Applicant: **VITSGEN THERAPEUTICS INC.**
**Zaozhuang, Shandong 277000 (CN)**

(72) Inventors:
- **WEI, Yanjun**
  **Zaozhuang, Shandong 277000 (CN)**
- **JIN, Haihong**
  **Zaozhuang, Shandong 277000 (CN)**
- **SU, Xiaobo**
  **Zaozhuang, Shandong 277000 (CN)**
- **GE, Chengzhen**
  **Zaozhuang, Shandong 277000 (CN)**
- **CAO, Jinsong**
  **Zaozhuang, Shandong 277000 (CN)**
- **HOU, Shimeng**
  **Zaozhuang, Shandong 277000 (CN)**

(74) Representative: **Canzler & Bergmeier Patentanwälte Partnerschaft mbB**
**Despag-Straße 6**
**85055 Ingolstadt (DE)**

## (54) PSMA COMPOUND, AND PHARMACEUTICAL COMPOSITION CONTAINING SAME AND USE THEREOF

(57) Disclosed in the present invention is a compound represented by formula (I), or a pharmaceutically acceptable salt, ester or solvate thereof. Such a PSMA compound has higher tumor uptake and/or lower or substantially equivalent kidney uptake, can improve the drug targeting effect and/or reduce the kidney injury risk, and is conducive to better meeting the medication needs of patients.

EP 4 782 440 A1

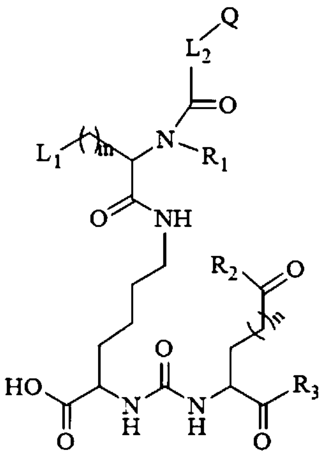

Formula I

(52) Cooperative Patent Classification (CPC): (Cont.)
A61K 51/00

## Description

### FIELD OF THE DISCLOSURE

The present disclosure belongs to the field of radiopharmaceuticals, specifically relates to a compound capable of binding to Prostate-Specific Membrane Antigen (PSMA), a pharmaceutical composition containing the same, and use thereof.

### BACKGROUND OF THE DISCLOSURE

Prostate cancer (PCa) is the second most common cancer among men, after lung cancer. Prostate cancer has an incidence rate in developed countries of 37.5 per 100,000 people, and in developing countries of 11.3 per 100,000 people. Prostate cancer has a mortality rate in developed countries of 8.1 per 100,000 people, and in developing countries of 5.9 per 100,000 people. Currently, approximately 10 million men are diagnosed with prostate cancer. Prostate cancer causes more than 400,000 deaths worldwide per year, and this number is expected to reach more than 800,000 per year by 2040.

PSMA (Prostate-Specific Membrane Antigen) is a type II transmembrane glycoprotein, also known as glutamate carboxypeptidase II and folate hydrolase 1, having folate hydrolase and N-acetyl-$\alpha$-linked acidic dipeptidase activities. The PSMA protein consists of 750 amino acids across three domains: an intracellular domain of 19 amino acids, a transmembrane domain of 24 amino acids, and an extracellular domain of 707 amino acids. PSMA is expressed at a very low level in normal prostate tissue and non-prostate tissues (e.g., liver, kidney, small intestine, and the like), but in prostate cancer tissue, it is expressed at a level that is 100 to 1000 times higher than that in normal prostate tissue. Furthermore, the expression level of PSMA is highly correlated with the invasiveness of prostate cancer (PCa), and thus is a specific molecular marker for prostate cancer. Simultaneously, the larger extracellular domain of PSMA facilitates the design of targeting molecules; therefore, PSMA is a suitable therapeutic and diagnostic target for imaging diagnosis and targeted radionuclide therapy of prostate cancer and the metastases thereof.

The application of PSMA in diagnostic imaging mainly includes the manufacture and application of targeted imaging agents based on PSMA targets. Diagnostic techniques for prostate cancer, including imaging diagnostic methods such as SPECT (single-photon emission computed tomography) and PET (positron emission tomography), can utilize radioactive isotope-labeled PSMA-targeted polypeptide substances. An early PSMA-targeted imaging agent was ProstaScint (also known as 111-In-capromab pentetide), a mouse monoclonal antibody (7E11) linked to 111-In, which could be used for SPECT imaging. However, ProstaScint can only bind to intracellular epitopes of PSMA, and therefore cannot bind to living tumor cells, limiting its clinical performance. $^{68}$Ga-labeled PSMA ligands, such as $^{68}$Ga-PSMA-11, are widely used as therapeutic and diagnostic agents targeting PSMA. Correspondingly, $^{18}$F-labeled therapeutic and diagnostic agents, such as $^{18}$F-DCFBC, $^{18}$F-DCFPyL, and $^{18}$F-PSMA-1007, have shown a higher sensitivity and diagnostic accuracy in PSMA-based prostate cancer imaging applications compared to the aforementioned $^{68}$Ga-labeled diagnostic agents.

Currently, although numerous imaging agents and inhibitors targeting PSMA have been developed and applied, the diagnosis, management, and/or treatment of prostate cancer remain highly challenging. For example, achieving efficient and accurate diagnosis or treatment while minimizing the adverse effects of medications on the kidneys or other organs of the patient is crucial. There is still a need to develop novel diagnostic or imaging agents that can target PCa tumor cells with a high selectivity and exhibit favorable pharmacokinetic properties and/or other effects, so as to proceed rapid and non-invasive tumor visualization and treatment and to achieve early diagnosis and/or treatment of PCa.

### DESCRIPTION OF THE DISCLOSURE

The purpose of the disclosure is to provide a new type of PSMA compounds. These PSMA compounds exhibit a higher tumor uptake and/or a lower or substantially equivalent renal uptake, which can improve drug targeting effect and/or reduce the risk of kidney damage, thereby better meeting the medication needs of the patients.

The disclosure provides a compound of Formula I or pharmaceutically acceptable salt, ester or solvate thereof,

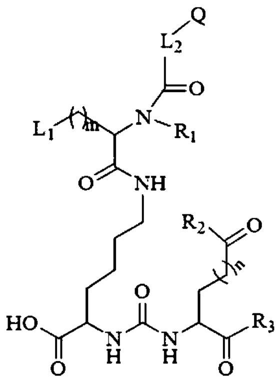

Formula I

wherein,

Q is

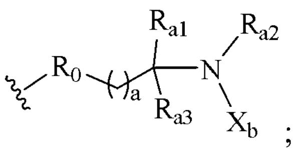

$R_0$ is selected from carbonyl or methylene;
$X_b$ is a chelating agent or

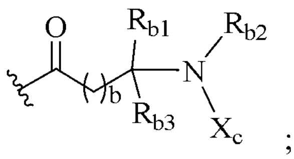

$X_c$ is a chelating agent or

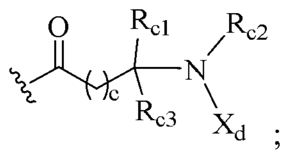

$X_d$ is a chelating agent or

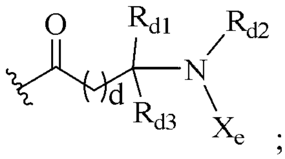

$X_e$ is a chelating agent or

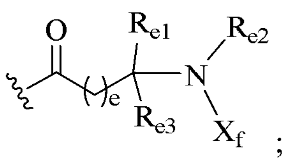

$X_f$ is a chelating agent or

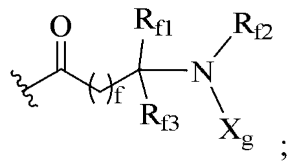

$X_g$ is a chelating agent or

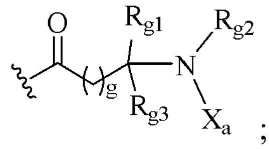

$X_a$ is a chelating agent;

$R_{a1}$, $R_{a1}$, $R_{c1}$, $R_{d1}$, $R_{c1}$, $R_{f1}$ and $R_{g1}$ are all $R_{y1}$ ($R_{a1}$, $R_{b1}$, $R_{c1}$, $R_{a1}$, $R_{c1}$, $R_{f1}$ and $R_{g1}$ can be the same or different; other similar drafting modes or expressions all indicate that they can be the same or different);

$R_{a2}$, $R_{b2}$, $R_{c2}$, $R_{d2}$, $R_{e2}$, $R_{f2}$ and $R_{g2}$ are all $R_{y2}$;

$R_{a3}$, $R_{b3}$, $R_{c3}$, $R_{d3}$, $R_{e3}$, $R_{f3}$ and $R_{g3}$ are all $R_{y3}$;

$R_{y1}$, $R_{y2}$ and $R_{y3}$ are each independently selected from hydrogen, deuterium, tritium, $R_Y$, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$ or $-C(=S)R_k$;

or, $R_{y2}$ is connected to $R_{y1}$ (or $R_{y3}$) to form $R_{ring\ A}$;

$R_Y$ is selected from a C1-C16 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl (including fused aryl, and the like) or a 5-14 membered heteroaryl (including fused heteroaryl);

$R_{ring\ A}$ is a 3-12 membered heterocyclic alkyl or a 5-14 membered heteroaryl;

Ry and $R_{ring\ A}$ are optionally substituted with one or more substituents $P_1$ or unsubstituted;

the substituent $P_1$ is selected from deuterium, tritium, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$ or $R_W$;

$R_W$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl; and $R_W$ is optionally substituted with one or more substituents $P_2$ or unsubstituted;

the substituent $P_2$ is selected from deuterium, tritium, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$ or $R_X$;

$R_X$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl; and $R_X$ is optionally substituted with one or more substituents $P_3$ or unsubstituted;

the substituent $P_3$ is selected from deuterium, tritium, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$ or $R_V$;

$R_V$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl; and $R_V$ is optionally substituted with one or more substituents $P_4$ or unsubstituted;

the substituent $P_4$ is selected from deuterium, tritium, halogen, hydroxyl, mercapto, nitro, cyano, oxo, thio, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, a sulfo-substituted C1-C6 alkyl, a C1-C6 alkoxy, an amino-substituted C1-C6 alkoxy, a carboxyl-substituted C1-C6 alkoxy, a sulfo-substituted C1-C6 alkoxy, a C1-C6 alkyl thioether group, an amino-substituted C1-C6 alkyl thioether group, a carboxyl-substituted C1-C6 alkyl thioether group, a sulfo-substituted C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl;

$R_k$ is selected from hydrogen, deuterium, tritium, halogen, $-NR_iR_j$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_1$, $R_2$, $R_3$, $R_i$ and $R_j$ are each independently selected from hydrogen, deuterium, tritium, hydroxyl, mercapto, nitro, cyano, halogen, $-S(=O)R_z$, $-S(=O)OR_z$, $-S(=O)(=O)R_z$, $-S(=O)(=O)OR_z$, a C1-C6 alkyl, a C1-C6 alkoxy, a C1-C6 alkyl

thioether group, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_z$ is selected from hydrogen, deuterium, tritium, halogen, a C1-C6 alkyl, a deuterated C1-C6 alkyl, a tritiated C1-C6 alkyl, or a halogenated C1-C6 alkyl;

a, b, c, d, e, f and g are each independently selected from integers between 0 and 6 (for example, they may be 0, 1, 2, 3, 4, 5, 6);

$L_1$ is selected from a 3-12 membered cycloalkyl, a 3-12 membered heterocyclic alkyl, a 5-14 membered aryl or a 5-14 membered heteroaryl; wherein the 3-12 membered cycloalkyl, the 3-12 membered heterocyclic alkyl, the 5-14 membered aryl or the 5-14 membered heteroaryl is optionally substituted with one or more substituents $P_1$ or unsubstituted;

$L_2$ is selected from $-L_3-$ or $-NH-(CH_2)_t-L_3-$ (for example, $L_2$ includes, but is not limited to, the group L as defined in claim 4 of international patent application WO2023030509A1);

for example, $L_2$ includes, but is not limited to, the following groups:

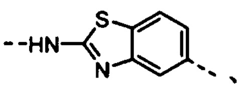, 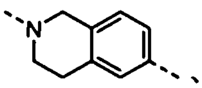, 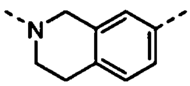, 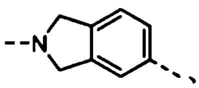, 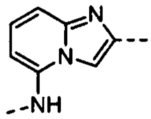,

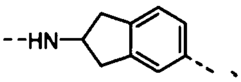, 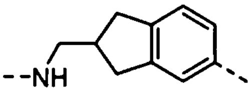, 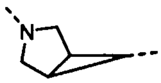, 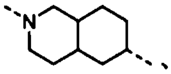, 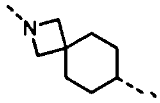,

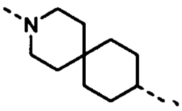, 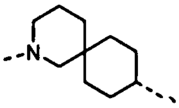, 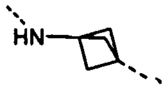, 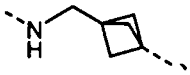, 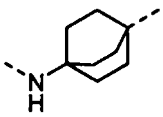, 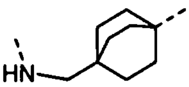,

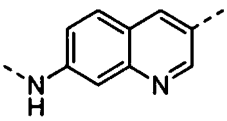, 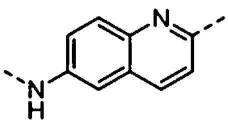, 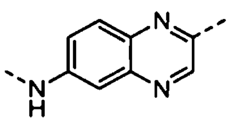, 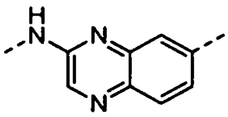,

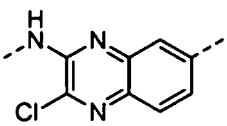, 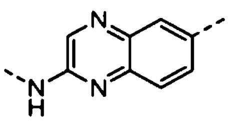, 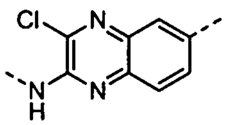, 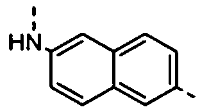,

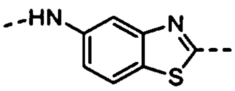, 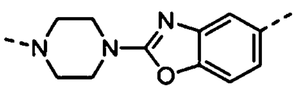, 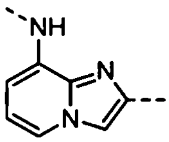, 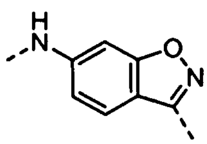,

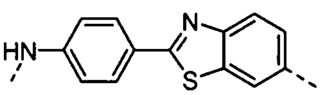, 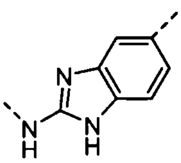, 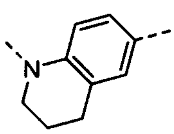, 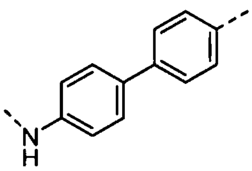,

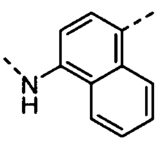, 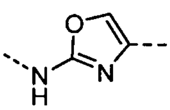, 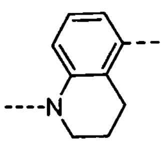, 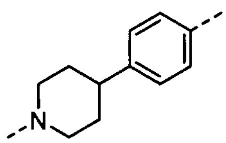, 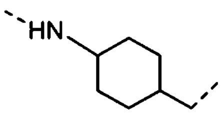,

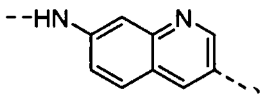 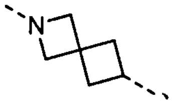 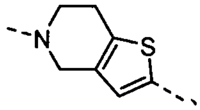 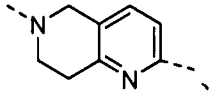 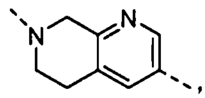 ;

$L_3$ is selected from a 3-12 membered cycloalkyl, a 3-12 membered heterocyclic alkyl, a 5-14 membered aryl or a 5-14 membered heteroaryl; the 3-12 membered cycloalkyl, the 3-12 membered heterocyclic alkyl, the 5-14 membered aryl or the 5-14 membered heteroaryl is optionally substituted with one or more substituents $P_1$ or unsubstituted;

the heteroatom of the 3-12 membered heterocyclic alkyl or the 5-14 membered heteroaryl is selected from one or more of N, O and S, and the 3-12 membered heterocyclic alkyl or the 5-14 membered heteroaryl has 1, 2, or 3 heteroatoms;

m, n and t are each independently selected from integers between 0 and 6 (for example, they may be 0, 1, 2, 3, 4, 5, 6).

In a preferred embodiment, $L_1$ is a 5-14 membered aryl or a 5-14 membered heteroaryl; the 5-14 membered aryl or the 5-14 membered heteroaryl is optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl.

In a preferred embodiment, $L_1$ is selected from phenyl, pyrimidinyl, pyridinyl, pyrrolyl, oxazolyl, imidazolyl, furanyl, thiazolyl, thienyl, thiopyranyl, naphthyl, benzopyrrolyl (including: indolyl, isoindolyl), benzopyridyl (including: quinolinyl, isoquinolinyl), benzoxazolyl, benzimidazolyl, benzofuranyl, benzothiazolyl, benzothienyl, benzothiopyranyl, purinyl, anthryl, or phenanthryl;

preferably, $L_1$ is naphthyl, benzopyridyl, or anthryl.

In a preferred embodiment, $L_2$ is $-L_3-$; $L_3$ is selected from a 3-12 membered heterocyclic alkyl, a 5-14 membered aryl or a 5-14 membered heteroaryl; the 3-12 membered heterocyclic alkyl, the 5-14 membered aryl or the 5-14 membered heteroaryl is optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl.

In a preferred embodiment, $L_3$ is selected from a 3-12 membered heterocyclic alkyl, the 3-12 membered heterocyclic alkyl comprises 1-4 heteroatoms N;

preferably, $L_3$ is $R_{ring\ B}$-$R_{ring\ C}$, $R_{ring\ B}$ and $R_{ring\ C}$ share one carbon atom; $R_{ring\ B}$ is selected from a 3-9 membered cycloalkyl or a 3-9 membered heterocyclic alkyl; $R_{ring\ C}$ is selected from a 3-9 membered heterocyclic alkyl; wherein the heteroatom of the 3-9 membered heterocyclic alkyl is selected from one or more of N, O and S, and the 3-9 membered heterocyclic alkyl has 1, 2, or 3 heteroatoms; $R_{ring\ C}$ comprises 1-2 heteroatoms N;

$R_{ring\ B}$ and $R_{ring\ C}$ are optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

more preferably, $R_{ring\ B}$ is selected from cyclobutyl, cyclopentyl, cyclohexyl, or cycloheptyl; and $R_{ring\ C}$ is selected from pyrrolidinyl, piperidinyl, oxazolidinyl, morpholinyl, thiomorpholinyl, azepanyl, oxazepanyl, or thiazepanyl.

In a preferred embodiment, $L_3$ is selected from:

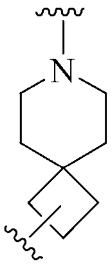 , 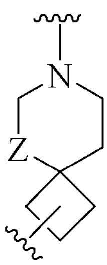 , 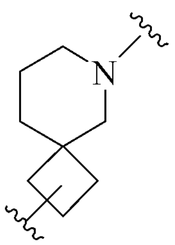 , 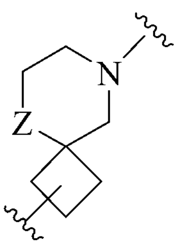 , 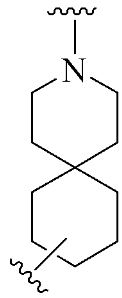 , 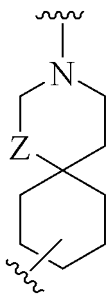 , 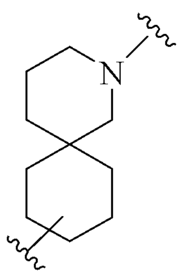 ,

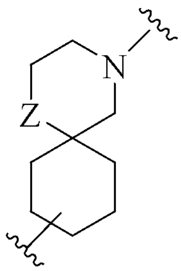;

and Z is O or S.

In a preferred embodiment, $L_3$ is selected from a 5-14 membered heteroaryl, and the 5-14 membered heteroaryl comprises 1-4 heteroatoms N;

preferably, $L_3$ is $R_{ring\ D}$-$R_{ring\ E}$, $R_{ring\ D}$ and $R_{ring\ E}$ share two carbon atoms; $R_{ring\ D}$ is selected from a 5-6 membered aryl or a 5-6 membered heteroaryl; $R_{ring\ E}$ is selected from a 3-9 membered heterocyclic alkyl; wherein the heteroatom of the 5-6 membered heteroaryl or the 3-9 membered heterocyclic alkyl is selected from one or more of N, O and S, and the 3-9 membered heterocyclic alkyl has 1, 2, or 3 heteroatoms; $R_{ring\ E}$ comprises 1-2 heteroatoms N;

$R_{ring\ D}$ and $R_{ring\ E}$ are optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

more preferably, $R_{ring\ D}$ is selected from phenyl, pyrimidinyl, pyridinyl, pyrrolyl, oxazolyl, imidazolyl, furanyl, thiazolyl, thienyl, or thiopyranyl; and $R_{ring\ E}$ is selected from pyrrolidinyl, piperidinyl, oxazolidinyl, morpholinyl, thiomorpholinyl, azepanyl, oxazepanyl, or thiazepanyl.

In a preferred embodiment, $L_3$ is selected from:

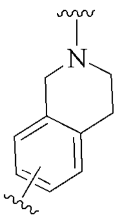, 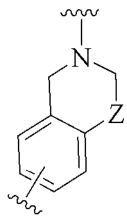, 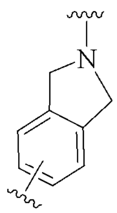, 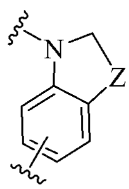, 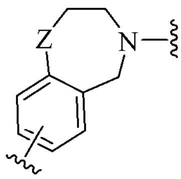, 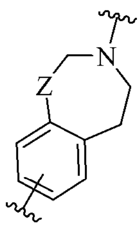, 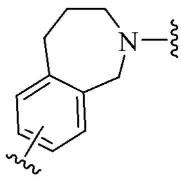,

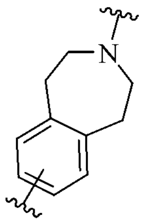;

and Z is O or S;
preferably,
$L_3$ is

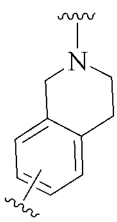, 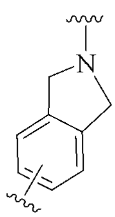 or 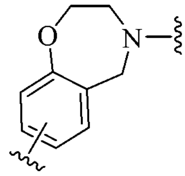.

In a preferred embodiment, the chelating agent is selected from 1,4,7-triazacyclononane-1,4,7-triacetic acid

(NOTA), 1,4,7,10-tetraazacyclododecane-1,4,7,10-tetraacetic acid (DOTA:

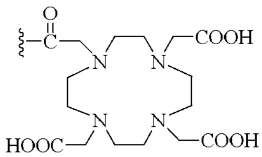

), 2-(4,7,10-tris(carboxymethyl)-1,4,7,10-tetraazacyclododecane-1-yl)-glutaric acid (DOTAGA:

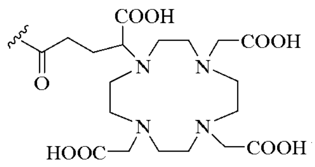

), N,N"-bis[2-hydroxy-5-(carboxyethyl)-benzyl]ethylenediamine-N,N"-diacetic acid (HBED-CC), 2-(4,7-bis(carboxymethyl)-1,4,7-triazacyclononane-1-yl)glutaric acid (NODAGA), 1,4,7-triazacyclononane phosphinic acid (TRAP), 1,4,7-triazacyclononane-1-[methyl(2-carboxyethyl)phosphinic acid]-4,7-bis[methyl(2-hydroxymethyl)phosphinic acid] (NOPO), 3,6,9,15-tetraazabicyclo[9,3,1]pentadecane-1(15), 11, 13-triene-3,6,9-triacetic acid (PCTA), N'-{5-[acetyl(hydroxy)amino]pentyl}-N-[5-({4-[(5-aminopentyl)(hydroxy)amino]-4-oxobutyryl}amino)pentyl]-N-hydroxysuccinamide (DFO), diethylenetriaminepentaacetic acid (DTPA:

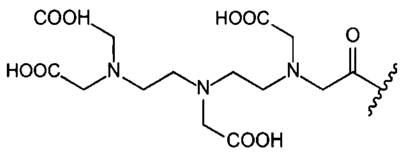

),

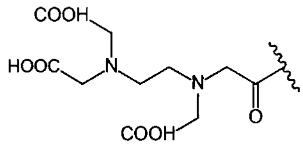

,

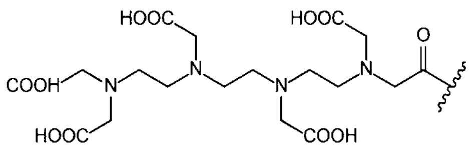

,

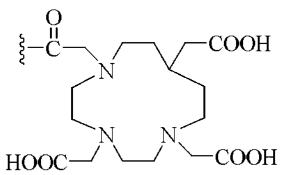

or

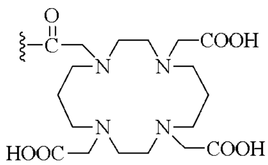

;

preferably, the chelating agent is DOTA or DOTAGA.

In a preferred embodiment, m, n and t are each independently selected from 1 or 2; $R_1$ is selected from hydrogen, deuterium, tritium, a C1-C6 alkyl or a deuterated C1-C6 alkyl; and $R_2$ and $R_3$ are each independently selected from hydroxyl or C1-C6 alkoxy.

In a preferred embodiment, the $R_{y1}$ or $R_{y3}$ is of S or R configuration; and/or, the configuration of the compound of Formula I is:

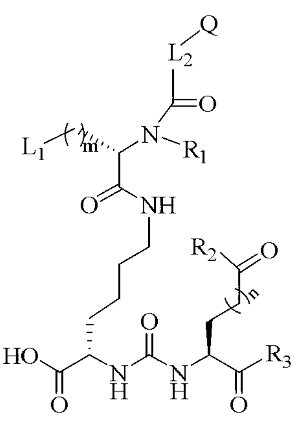

.

In a preferred embodiment,

$X_b$ is a chelating agent;
$R_{y1}$ and $R_{y3}$ are each independently selected from hydrogen, deuterium, tritium, Ry, hydroxyl, mercapto, cyano, amino, carboxyl, sulfo-group; $R_Y$ is selected from a C1-C6 alkyl, a deuterated C1-C6 alkyl, a tritiated C1-C6 alkyl, a halogenated C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl;
$R_{y2}$ is selected from hydrogen, deuterium, tritium, halogen, hydroxyl, mercapto, nitro or cyano; or, $R_{y2}$ is connected to $R_{y1}$ (or $R_{y3}$) to form $R_{ring A}$, the $R_{ring A}$ is a tetrahydropyrrole ring or a piperidine ring; the $R_{ring 1}$ is optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;
preferably, the compound of Formula I is:

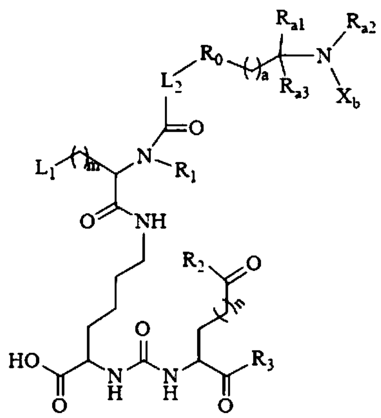

Formula Ia

wherein $X_b$ is a chelating agent; $R_{a1}$ and $R_{a3}$ are each independently selected from hydrogen, deuterium, tritium, $R_Y$; $R_Y$ is selected from a C1-C6 alkyl, a deuterated C1-C6 alkyl or a tritiated C1-C6 alkyl; $R_{a2}$ is selected from hydrogen, deuterium, or tritium; or, $R_{a2}$ is connected to $R_{a1}$ (or $R_{a3}$) to form $R_{ring A}$, the $R_{ring A}$ is a tetrahydropyrrole ring; and a is 0, 1 or 2.

In a preferred embodiment, the compound of Formula Ia is selected from:

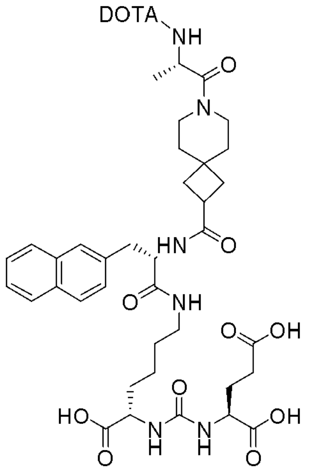

VWT011400 ,

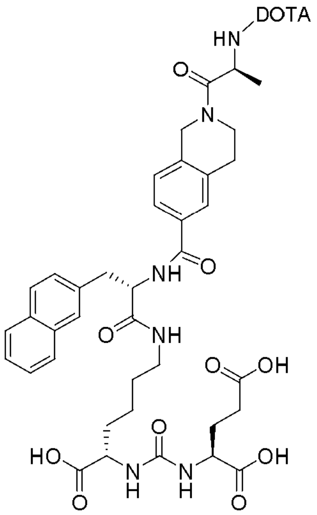

VWT011600 ,

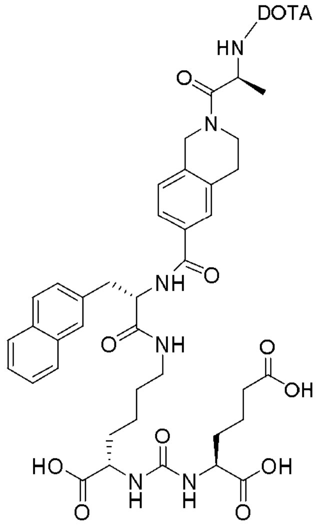

VWT019000 ,

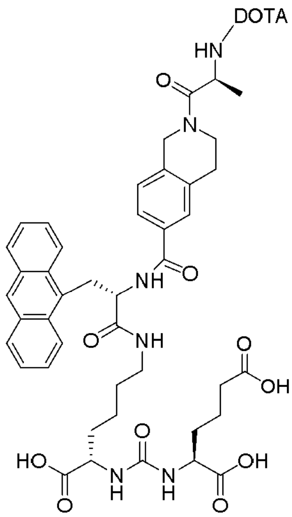

VWT019300 ,

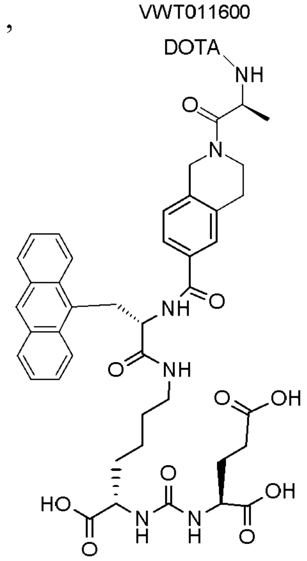

VWT020100 ,

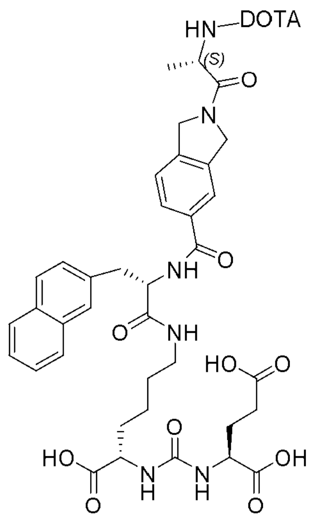

VWT021400 ,

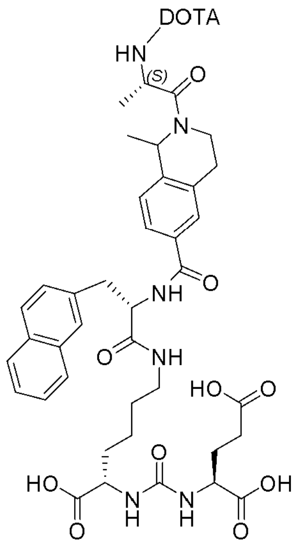

VWT021700 ,

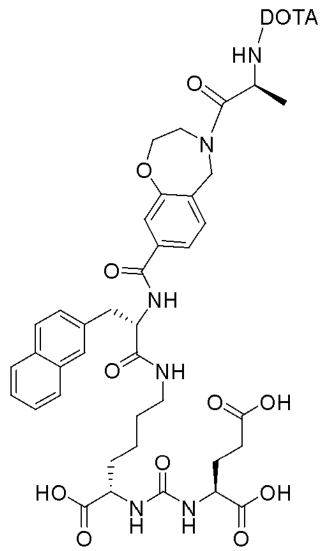

VWT022100 ,

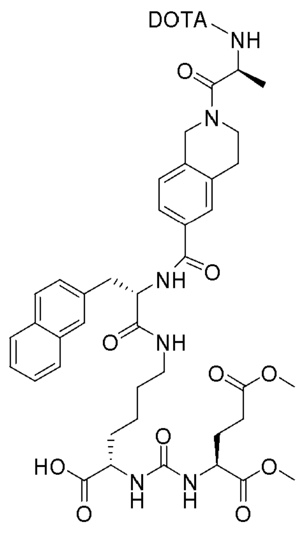

VWT023800 ,

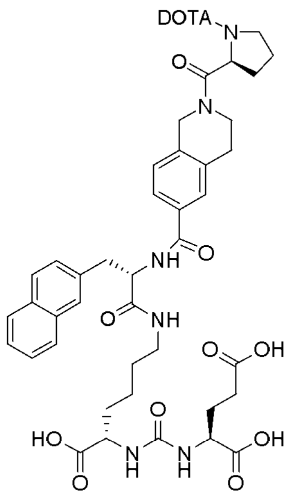
VWT032100 ,
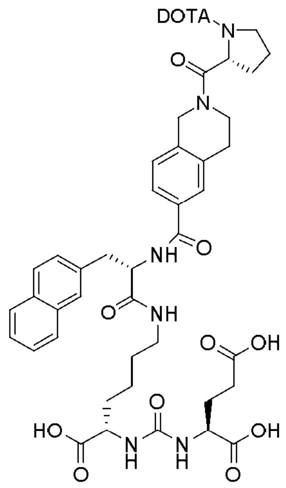
VWT032200 ,
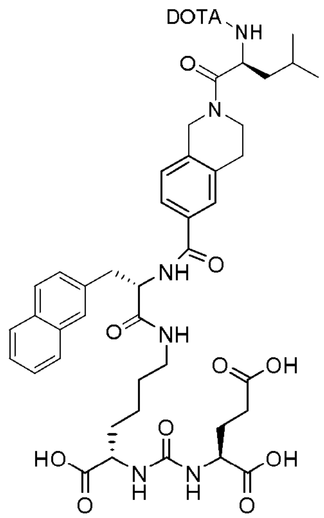
VWT044400 ,

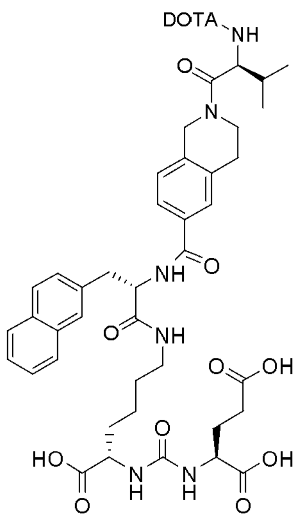
VWT044600 ,
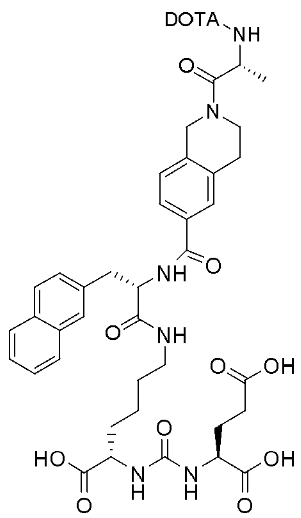
VWT044800 ,
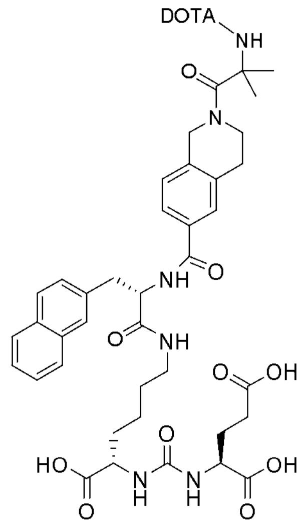
VWT044900 .

In a preferred embodiment,

$X_b$ is a chelating agent;
$R_{y1}$ and $R_{y3}$ are each independently selected from hydrogen, deuterium, tritium, Ry, hydroxyl, mercapto, cyano, amino, carboxyl, or sulfo-group; $R_Y$ is selected from a C1-C6 alkyl, a deuterated C1-C6 alkyl, a tritiated C1-C6 alkyl, a halogenated C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl;
$R_{y2}$ is a C1-C16 alkyl that is optionally substituted with one or more substituents $P_1$ or unsubstituted; and the substituent $P_1$ is selected from deuterium, tritium, amino, carboxyl, sulfo-group, or $R_W$;
$R_W$ is selected from a C1-C6 alkoxy or a C1-C6 alkyl thioether group; and $R_W$ is optionally substituted with one or more substituents $P_2$ or unsubstituted; and the substituent $P_2$ is selected from -C(=O)$R_k$, -C(=O)O$R_k$ or $R_X$;
$R_X$ is selected from a C1-C6 alkyl, a C1-C6 alkoxy or a C1-C6 alkyl thioether group; $R_X$ is optionally substituted with one or more substituents $P_3$ or unsubstituted; and the substituent $P_3$ is selected from -C(=O)$R_k$ or -C(=O)O$R_k$;
$R_k$ is selected from hydrogen, deuterium, tritium, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;
preferably, the compound of Formula I is:

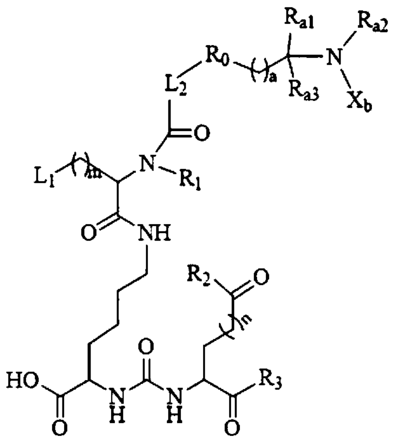

Formula Ib

wherein $X_b$ is a chelating agent; $R_{a1}$ and $R_{a3}$ are each independently selected from hydrogen, deuterium, tritium or $R_Y$; $R_Y$ is selected from a C1-C6 alkyl, a deuterated C1-C6 alkyl or a tritiated C1-C6 alkyl; $R_{a2}$ is a C1-C14 alkyl that is optionally substituted with one or more substituents $P_1$; and the substituent $P_1$ is selected from deuterium, tritium, carboxyl, sulfo-group, or $R_W$;

$R_W$ is selected from a C1-C6 alkoxy; and $R_W$ is optionally substituted with one or more substituents $P_2$ or unsubstituted; and the substituent $P_2$ is selected from -C(=O)OR$_k$ or $R_X$;

$R_X$ is selected from a C1-C6 alkoxy or a C1-C6 alkyl thioether group; $R_X$ is optionally substituted with one or more substituents $P_3$ or unsubstituted; and the substituent $P_3$ is selected from -C(=O)OR$_k$; $R_k$ is selected from hydrogen, deuterium, tritium, a C1-C6 alkyl, a deuterated C1-C6 alkyl, or a tritiated C1-C6 alkyl; and a is 0, 1 or 2.

In a preferred embodiment, the compound of Formula Ib is selected from:

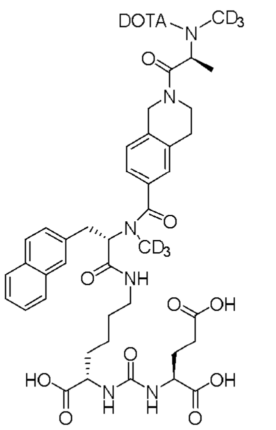

VWT023600 ,

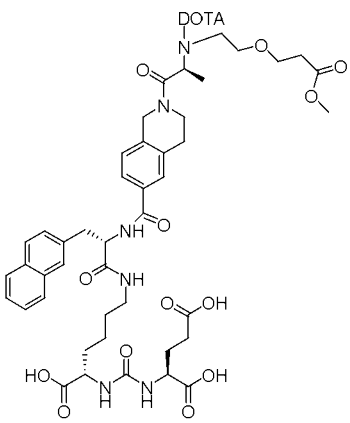

VWT026100 ,

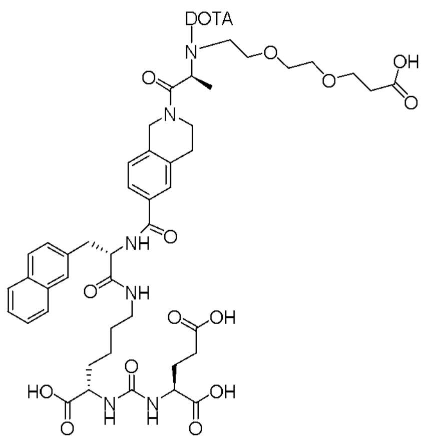

VWT026300 ,

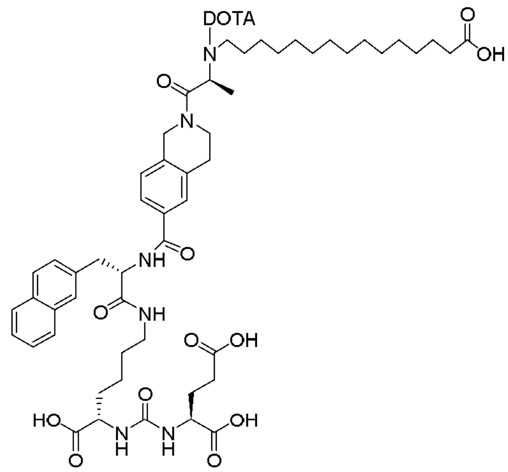

VWT026000

.

In a preferred embodiment,

$X_b$ is a chelating agent;

$R_{y1}$ and $R_{y3}$ are each independently selected from hydrogen, deuterium, tritium, $R_Y$, hydroxyl, mercapto, nitro, cyano, oxo, thio, halogen, amino, carboxyl, or sulfo-group;

$R_Y$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl (including but not limited to: oxadiazolidinyl), a 5-14 membered aryl (including but not limited to: phenyl) or a 5-14 membered heteroaryl (including but not limited to: imidazolyl, oxadiazolyl, benzopyrrolyl);

$R_Y$ is optionally substituted with one or more substituents $P_1$ or unsubstituted; and the substituent $P_1$ is selected from deuterium, tritium, hydroxyl, mercapto, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$ or $R_W$;

$R_W$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl; and $R_W$ is optionally substituted with one or more substituents $P_2$ or unsubstituted; and the substituent $P_2$ is selected from deuterium, tritium, hydroxyl, mercapto, cyano, oxo, thio, halogen, amino, carboxyl, sulfo-group, or $R_X$;

$R_X$ is selected from a C1-C6 alkyl, a C1-C6 alkoxy, or a C1-C6 alkyl thioether group;

$R_k$ is selected from hydrogen, deuterium, tritium, $-NR_iR_j$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_i$ and $R_j$ are each independently selected from hydrogen, deuterium, tritium, hydroxyl, mercapto, cyano, $-S(=O)R_z$, $-S(=O)OR_z$, $-S(=O)(=O)R_z$, $-S(=O)(=O)OR_z$, a C1-C6 alkyl, a C1-C6 alkoxy, a C1-C6 alkyl thioether group, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_z$ is selected from hydrogen, deuterium, tritium, halogen, a C1-C6 alkyl, a deuterated C1-C6 alkyl, a tritiated C1-C6 alkyl, or a halogenated C1-C6 alkyl;

$R_{y2}$ is selected from hydrogen, deuterium, tritium, a C1-C6 alkyl, a deuterated C1-C6 alkyl, or a tritiated C1-C6 alkyl; or, $R_{y2}$ is connected to $R_{y1}$ (or $R_{y3}$) to form $R_{ring\,A}$, and the $R_{ring\,A}$ is a tetrahydropyrrole ring or a piperidine ring; the $R_{ring\,A}$ is optionally substituted with one or more substituents $P_{1a}$ or unsubstituted; and the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

preferably, $X_b$ is a chelating agent; $R_{y1}$ is selected from hydrogen, deuterium, tritium, a C1-C6 alkyl, or a deuterated C1-C6 alkyl; $R_{y3}$ is Ry; $R_Y$ is selected from a C1-C6 alkyl; $R_Y$ is optionally substituted with one or more substituents $P_1$; and $R_{y2}$ ($R_{a2}$) is selected from hydrogen, deuterium, tritium, a C1-C6 alkyl, or a deuterated C1-C6 alkyl; or, $R_{y2}$ is connected to $R_{y1}$ (or $R_{y3}$) to form $R_{ring\,A}$, and the $R_{ring\,A}$ is a tetrahydropyrrole ring; and a is 0, 1 or 2.

In a preferred embodiment, the compound of Formula I is:

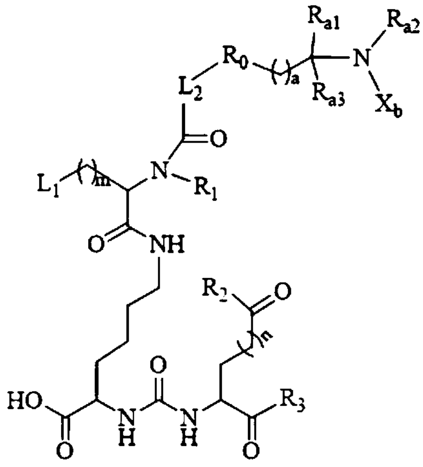

Formula Ic

wherein $X_b$ is a chelating agent;

$R_{a1}$ is selected from hydrogen, deuterium, tritium, or a C1-C6 alkyl;

$R_{a3}$ is Ry; $R_Y$ is selected from a C1-C6 alkyl; $R_Y$ is optionally substituted with one or more substituents $P_1$; the substituent $P_1$ is selected from deuterium, tritium, -S(=O)$R_k$, - S(=O)O$R_k$, -S(=O)(=O)$R_k$, -S(=O)(=O)O$R_k$ or $R_W$;

$R_W$ is selected from oxadiazolyl, phenyl or benzopyrrolyl; and $R_W$ is optionally substituted with one or more substituents $P_2$ or unsubstituted; and the substituent $P_2$ is selected from deuterium, tritium, hydroxyl, mercapto, cyano, oxo, thio, halogen, or $R_X$;

$R_X$ is a C1-C6 alkyl;

$R_k$ is selected from hydrogen, deuterium, tritium, a C1-C6 alkyl, a deuterated C1-C6 alkyl, or a tritiated C1-C6 alkyl;

$R_{a2}$ is selected from hydrogen, deuterium, tritium; or, $R_{a2}$ is connected to $R_{a1}$ (or $R_{a3}$) to form $R_{ring\,A}$, and the $R_{ring\,A}$ is a tetrahydropyrrole ring; and a is 0, 1 or 2;

preferably, the compound of Formula Ic is selected from:

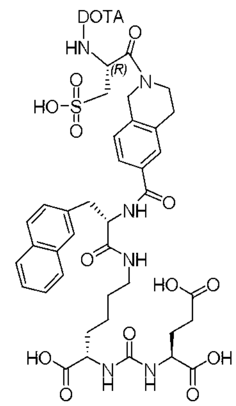

VWT022600 ,

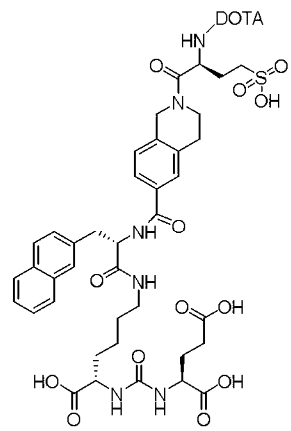

VWT032000 ,

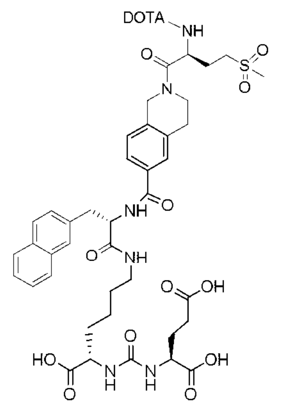

VWT032900 ,

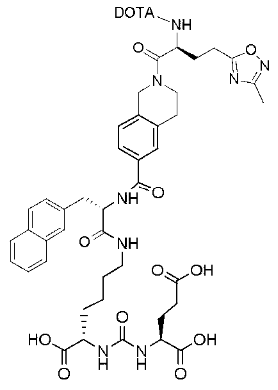
VWT033000 ,
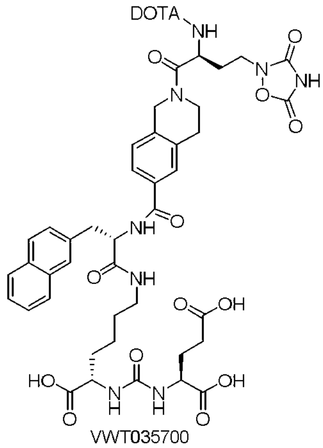
VWT035700 ,
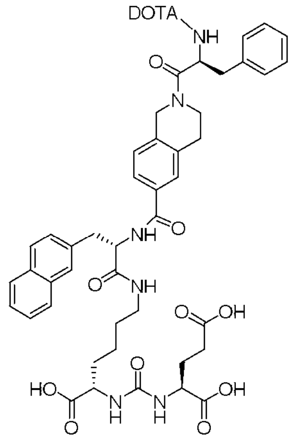
VWT044700 .

In another preferred embodiment, the compound of Formula I is:

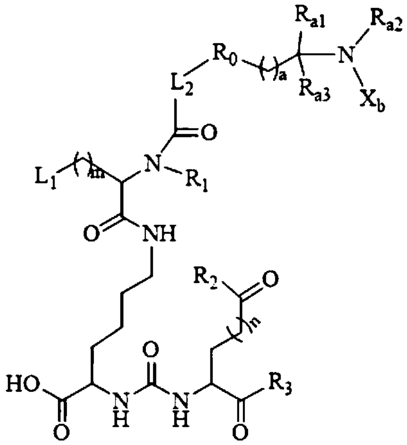

Formula Id

wherein $X_b$ is a chelating agent;

$R_{a1}$ is selected from hydrogen, deuterium, tritium, or a C1-C6 alkyl;

$R_{a3}$ is Ry; $R_Y$ is selected from a C1-C6 alkyl; Ry is optionally substituted with one or more substituents $P_1$; the substituent $P_1$ is selected from -C(=O)$R_k$, or -C(=O)O$R_k$; $R_k$ is selected from hydrogen, deuterium, tritium, -N$R_i$$R_j$, a C1-C6 alkyl, a deuterated C1-C6 alkyl, or a tritiated C1-C6 alkyl; $R_i$ and $R_j$ are each independently selected from hydrogen, deuterium, tritium, hydroxyl, mercapto, cyano, -S(=O)$R_z$, -S(=O)O$R_z$, -S(=O)(=O)$R_z$ or - S(=O)(=O)O$R_z$; $R_z$ is selected from hydrogen, deuterium, tritium, a C1-C6 alkyl, a deuterated C1-C6 alkyl, or a tritiated C1-C6 alkyl; $R_{a2}$ is selected from hydrogen, deuterium, tritium, or a deuterated C1-C6 alkyl; or, $R_{a2}$ is connected to $R_{a1}$ (or $R_{a3}$) to form $R_{ring\ A}$, and the $R_{ring\ A}$ is a tetrahydropyrrole ring; and a is 0, 1 or 2;

preferably, the compound of Formula Id is selected from:

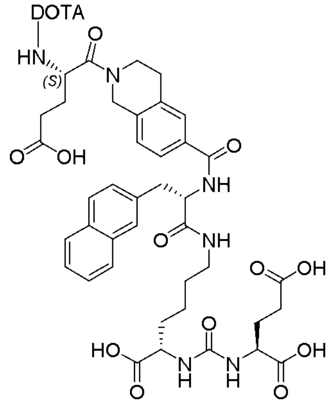

VWT024000 ,

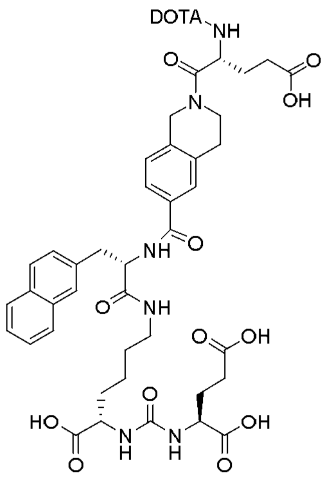

VWT031600 ,

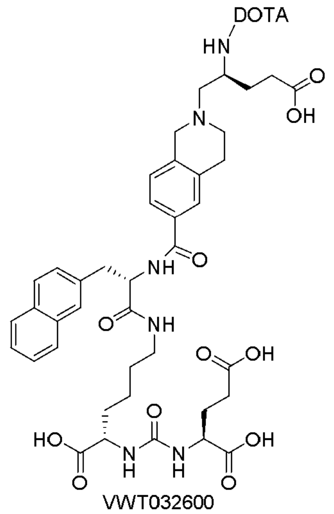

VWT032600 ,

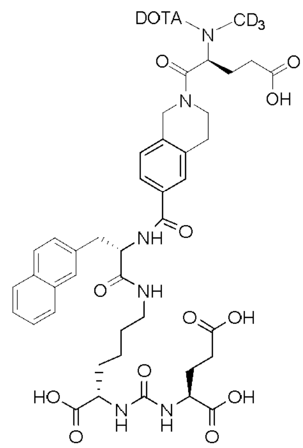

VWT032700 ,

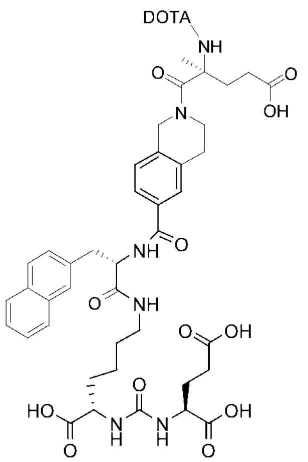

VWT032800 ,

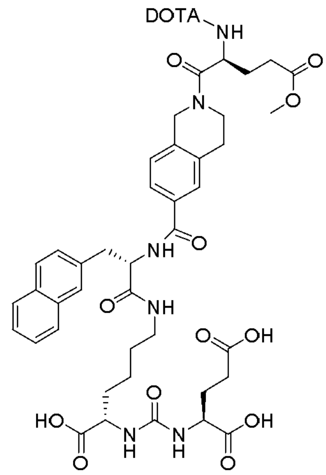

VWT024100 ,

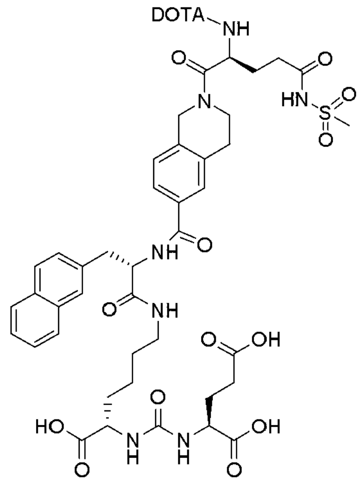

VWT031700 ,

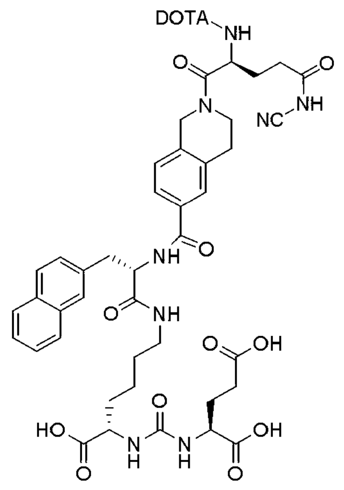

VWT031800 ,

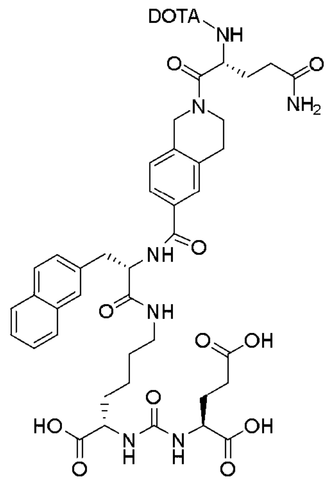

VWT032300 ,

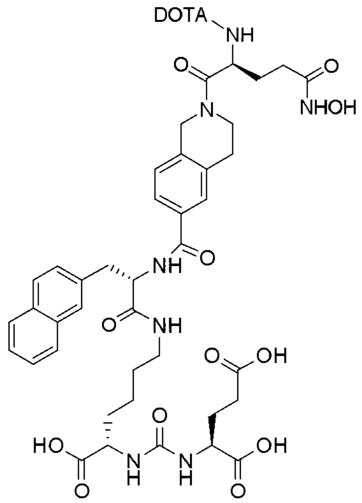

VWT035400

.

In a preferred embodiment, the compound of Formula I is:

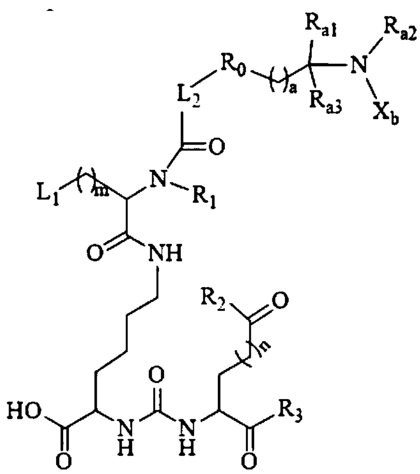

Formula Iz

wherein,

$X_b$ is a chelating agent;
$R_{a1}$ is selected from hydrogen, deuterium, tritium, or a C1-C6 alkyl;
$R_{a3}$ is Ry; wherein Ry is selected from a C1-C6 alkyl; and $R_Y$ is optionally substituted with one or more substituents $P_1$; the substituent $P_1$ is selected from $R_W$; $R_W$ is selected from a 5-14 membered aryl or a 5-14 membered heteroaryl; and $R_W$ is optionally substituted with one or more substituents $P_2$ or unsubstituted; and the substituent $P_2$ is selected from deuterium, tritium, hydroxyl, mercapto, cyano, oxo, thio, halogen, amino, carboxyl, sulfo-group, or $R_X$; wherein $R_X$ is selected from a C1-C6 alkyl, a C1-C6 alkoxy, or a C1-C6 alkyl thioether group;
$R_{a2}$ is selected from hydrogen, deuterium, tritium, or a deuterated C1-C6 alkyl; or, $R_{a2}$ is connected to $R_{a1}$ (or $R_{a3}$) to form $R_{ring\ A}$, and the $R_{ring\ A}$ is a tetrahydropyrrole ring; and a is 0, 1 or 2;
preferably, $R_{a3}$ is Ry; wherein Ry is selected from a C1-C6 alkyl; and $R_Y$ is optionally substituted with one or more substituents $P_1$; the substituent $P_1$ is pyridyl;
more preferably, the compound of Formula Iz is:

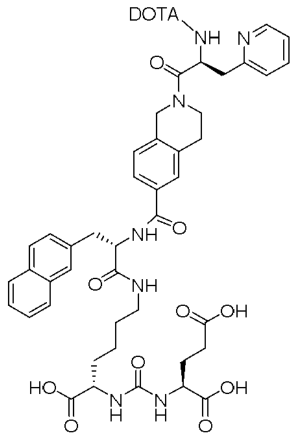

VWT045000

.

In a preferred embodiment,

$X_b$ is

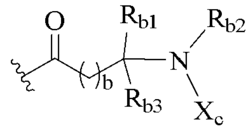

;

$X_c$ is a chelating agent or

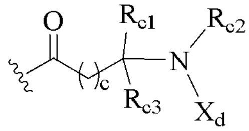

;

$X_a$ is a chelating agent or

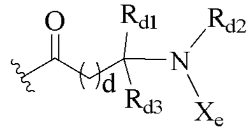

;

$X_e$ is a chelating agent;
$R_{a1}$, $R_{a1}$, $R_{c1}$ and $R_{a1}$ are all $R_{y1}$;
$R_{a2}$, $R_{b2}$, $R_{c2}$ and $R_{d2}$ are all $R_{y2}$;
$R_{a3}$, $R_{b3}$, $R_{c3}$ and $R_{d3}$ are all $R_{y3}$;
$R_{y1}$ and $R_{y3}$ are each independently selected from hydrogen, deuterium, tritium, Ry, hydroxyl, mercapto, nitro, cyano, oxo, thio, halogen, amino, carboxyl, sulfo-group; $R_Y$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl (including but not limited to: oxadiazolidinyl), a 5-14 membered aryl (including but not limited to: phenyl), or a 5-14 membered heteroaryl (including but not limited to: imidazolyl, oxadiazolyl, benzopyrrolyl);
$R_Y$ is optionally substituted with one or more substituents $P_1$ or unsubstituted; the substituent $P_1$ is selected from deuterium, tritium, hydroxyl, mercapto, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $- S(=O)(=O)OR_k$, $-C(=S)R_k$ or $R_W$;
$R_W$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl; and $R_W$ is optionally substituted with one or more substituents $P_2$ or unsubstituted; the substituent $P_2$ is selected from deuterium, tritium, hydroxyl, mercapto, cyano, oxo, thio, halogen, amino, carboxyl, sulfo-group, or $R_X$; and $R_X$ is selected from a C1-C6 alkyl, a C1-C6 alkoxy, a C1-C6 alkyl thioether group;

$R_k$ is selected from hydrogen, deuterium, tritium, $-NR_iR_j$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_i$ and $R_j$ are each independently selected from hydrogen, deuterium, tritium, hydroxyl, mercapto, cyano, $-S(=O)R_z$, $-S(=O)OR_z$, $-S(=O)(=O)R_z$, $-S(=O)(=O)OR_z$, C1-C6 alkyl, C1-C6 alkoxy, C1-C6 alkyl thioether group, deuterated C1-C6 alkyl, tritiated C1-C6 alkyl, halogenated C1-C6 alkyl, amino-substituted C1-C6 alkyl, carboxyl-substituted C1-C6 alkyl or sulfo-substituted C1-C6 alkyl;

$R_z$ is selected from hydrogen, deuterium, tritium, halogen, a C1-C6 alkyl, a deuterated C1-C6 alkyl, a tritiated C1-C6 alkyl, or a halogenated C1-C6 alkyl;

$R_{y2}$ is selected from hydrogen, deuterium, tritium, a C1-C6 alkyl, a deuterated C1-C6 alkyl, or a tritiated C1-C6 alkyl; or, $R_{y2}$ is connected to $R_{y1}$ (or $R_{y3}$) to form $R_{ring\ A}$, the $R_{ring\ A}$ is a tetrahydropyrrole ring or a piperidine ring; the $R_{ring\ A}$ is optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

preferably,

$X_b$ is a chelating agent;

$R_{y1}$ is selected from hydrogen, deuterium, tritium, a C1-C6 alkyl, or a deuterated C1-C6 alkyl;

$R_{y3}$ is hydrogen, deuterium, tritium, carboxyl, sulfo-group or $R_Y$;

$R_Y$ is selected from a C1-C6 alkyl; Ry is optionally substituted with one or more substituents $P_1$ or unsubstituted; and the substituent $P_1$ is selected from deuterium, tritium, hydroxyl, mercapto, cyano, oxo, thio, halogen, $-C(=O)R_k$, $-C(=O)OR_k$, $-S(=O)R_k$, $-S(=O)OR_k$, $-S(=O)(=O)R_k$, $-S(=O)(=O)OR_k$ or $R_W$;

$R_W$ is selected from a C1-C6 alkoxy, a C1-C6 alkyl thioether group, phenyl, benzopyrrolyl, imidazolyl, oxadiazolyl; and $R_W$ is optionally substituted with one or more substituents $P_2$ or unsubstituted; the substituent $P_2$ is selected from deuterium, tritium, hydroxyl, mercapto, cyano, oxo, thio, halogen or $R_X$; and $R_X$ is a C1-C6 alkyl;

$R_k$ is selected from hydrogen, deuterium, tritium, $-NR_iR_j$, a C1-C6 alkyl, a deuterated C1-C6 alkyl, or a tritiated C1-C6 alkyl; $R_i$ and $R_j$ are each independently selected from hydrogen, deuterium, tritium, hydroxyl, mercapto, cyano, $-S(=O)R_z$, $-S(=O)OR_z$, $-S(=O)(=O)R_z$, $-S(=O)(=O)OR_z$; $R_z$ is selected from hydrogen, deuterium, tritium, halogen, a C1-C6 alkyl, a deuterated C1-C6 alkyl, or a tritiated C1-C6 alkyl;

$R_{y2}$ is selected from hydrogen, deuterium, tritium, a C1-C6 alkyl, or a deuterated C1-C6 alkyl; or, $R_{y2}$ is connected to $R_{y1}$ (or $R_{y3}$) to form $R_{ring\ A}$, the $R_{ring\ A}$ is a tetrahydropyrrole ring;

a, b, c and d are each independently selected from 0, 1 or 2.

In a preferred embodiment,

$R_{a1}$, $R_{b1}$, $R_{f1}$ and $R_{d1}$ are each independently selected from hydrogen, deuterium, tritium, or a C1-C6 alkyl;

$R_{a3}$, $R_{b3}$, $R_{c3}$ and $R_{a3}$ are each independently selected from hydrogen, deuterium, tritium, carboxyl, sulfo-group or $R_Y$; $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group or $R_W$; $R_W$ is selected from a C1-C6 alkoxy, a C1-C6 alkyl thioether group, phenyl, benzopyrrolyl or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;

$R_{a2}$, $R_{b2}$, $R_{c2}$ and $R_{d2}$ are each independently selected from hydrogen, deuterium, tritium or a deuterated C1-C6 alkyl; or, $R_{a2}$ is connected to $R_{a1}$ to form $R_{ring\ A}$, $R_{b2}$ is connected to $R_{b1}$ to form $R_{ring\ A}$, $R_{c2}$ is connected to $R_{c1}$ to form $R_{ring\ A}$, $R_{d2}$ is connected to $R_{d1}$ to form $R_{ring\ A}$, the $R_{ring\ A}$ is a tetrahydropyrrole ring; and a, b, c and d are each independently selected from 0, 1 or 2;

preferably, the compound of Formula I includes:

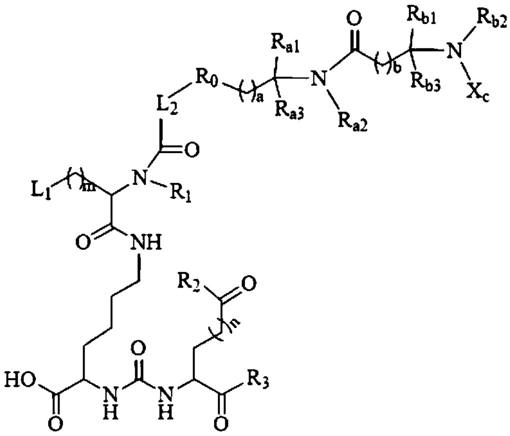

Formula Ie

wherein $X_c$ is a chelating agent;
further preferably, the compound of Formula Ie is selected from:

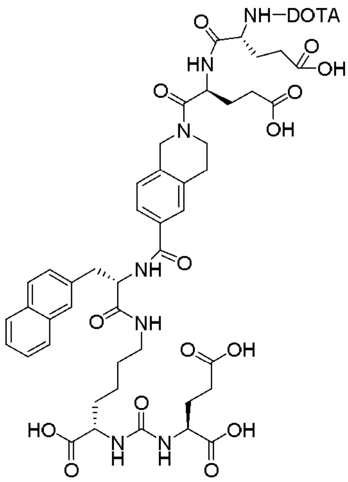

VWT023900 ,

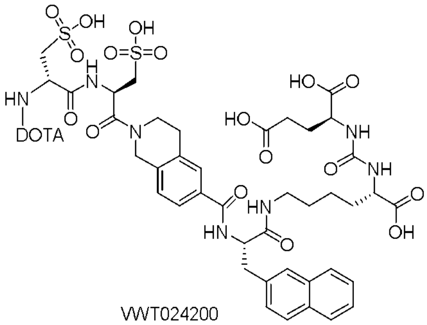

VWT024200 ,

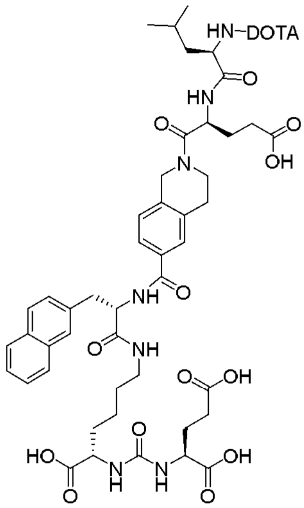

VWT037100 ,

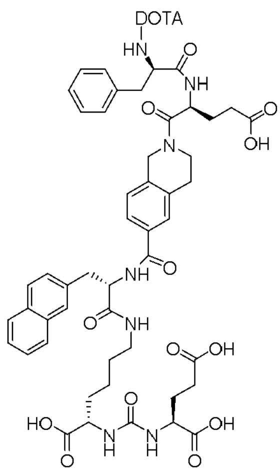

VWT037200 ,

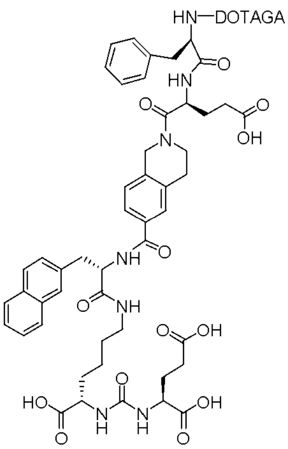

VWT038200 ,

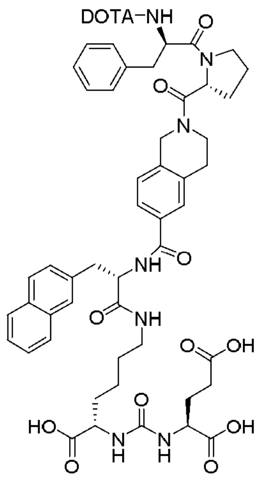

VWT038400 ,

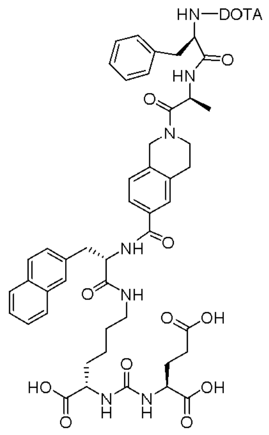

VWT039600 ,

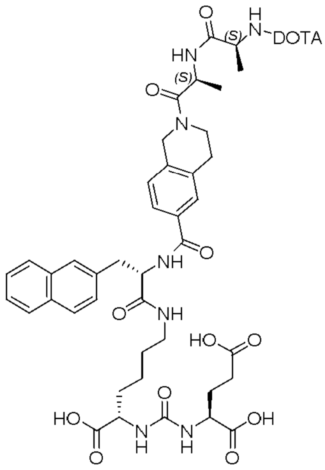

VWT043300 ,

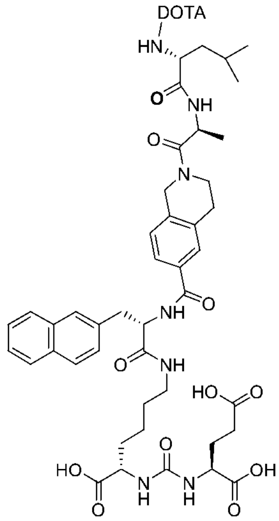

VWT044500 ,

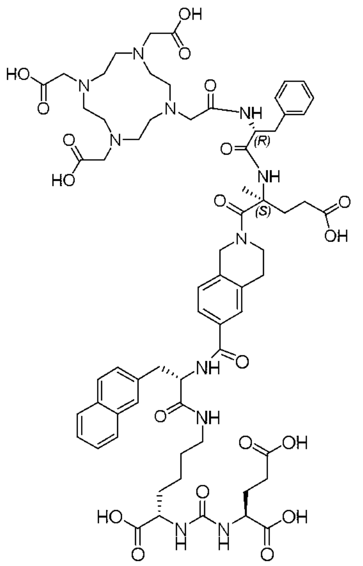

VWT045900 ,

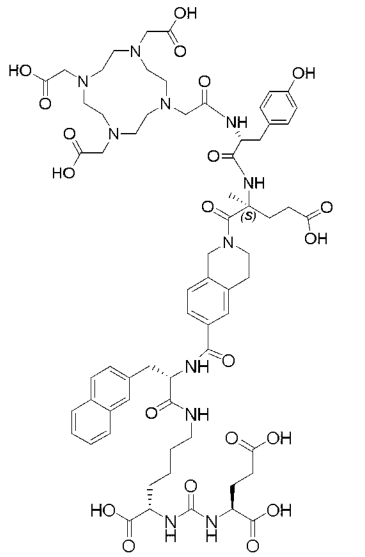

VWT046100 ;

preferably, the compound of Formula I is:

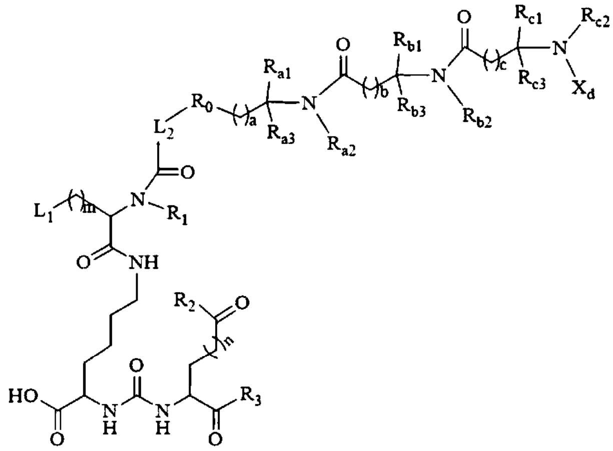

Formula If

wherein $X_d$ is a chelating agent;

further preferably, the compound of Formula If is selected from:

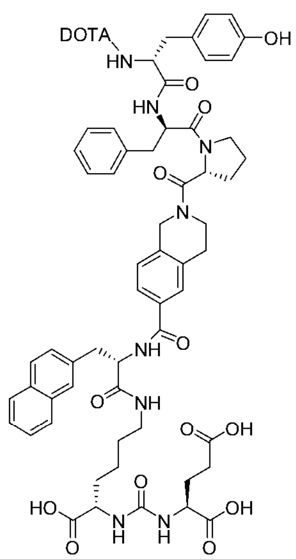

VWT038300 ,

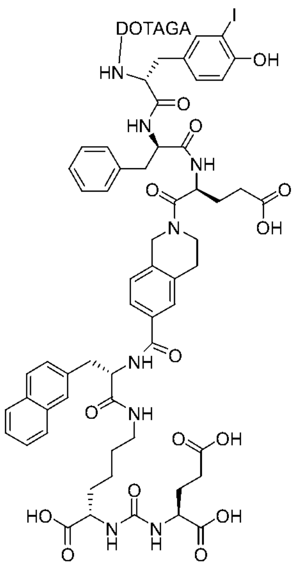

VWT038500 ,

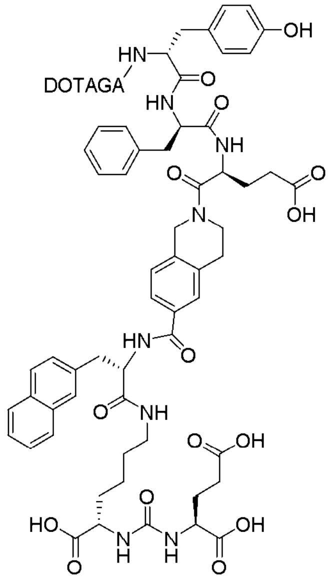

VWT038600 ,

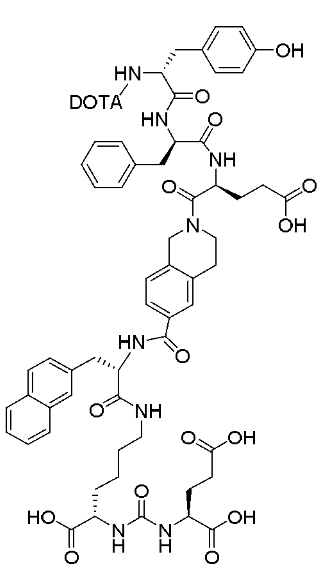
VWT038700 ,

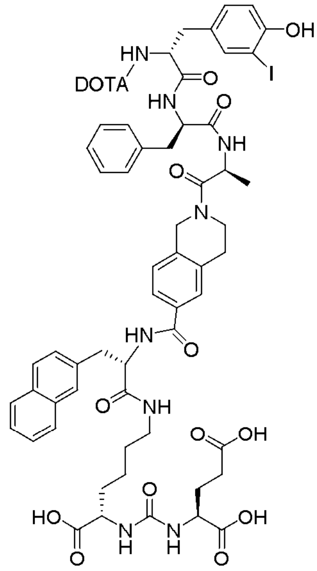
VWT039500 ,

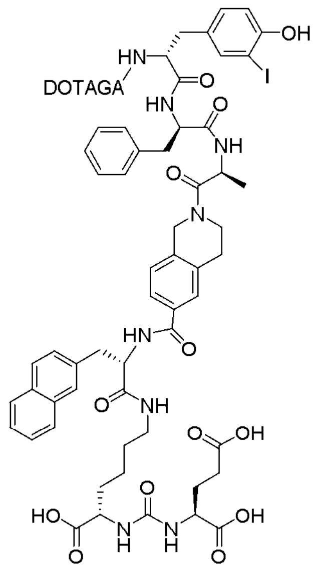
VWT039700 ,

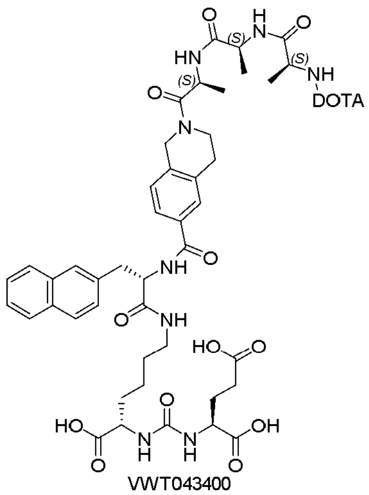
VWT043400 ,

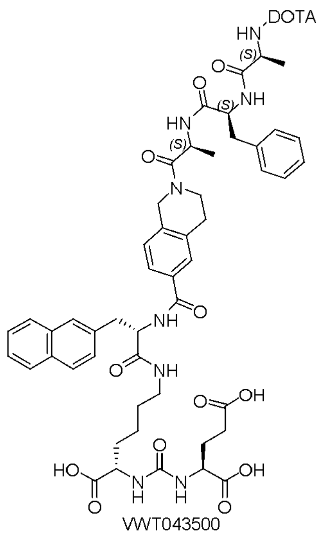
VWT043500 ,

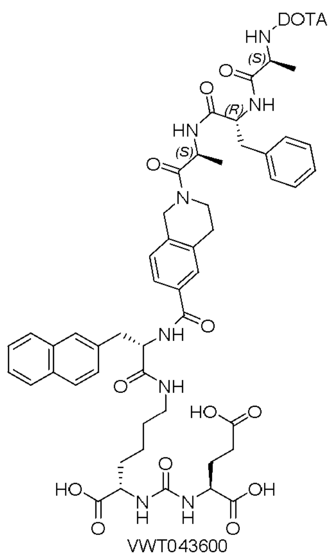
VWT043600 ;

preferably, the compound of Formula I is:

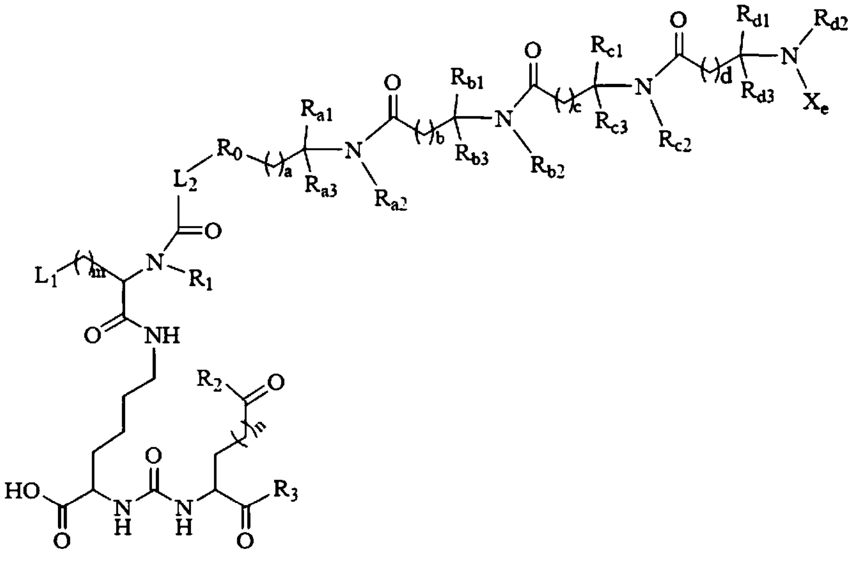

Formula Ig

wherein $X_e$ is a chelating agent;
further preferably, the compound of Formula Ig is:

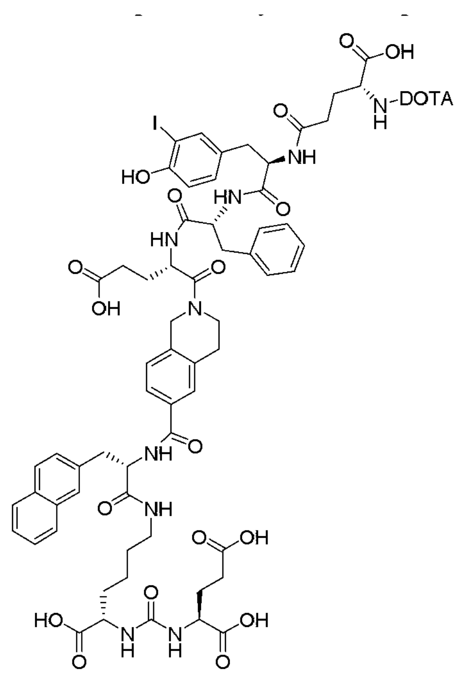

VWT037400

,

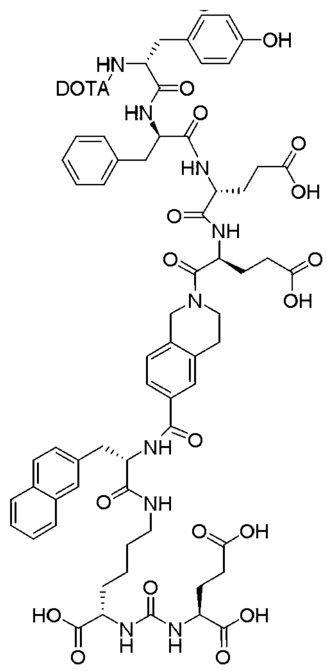

VWT039800

.

In a preferred embodiment,

$R_{a1}$, $R_{b1}$, $R_{c1}$ and $R_{d1}$ are each independently selected from hydrogen, deuterium, tritium or a C1-C6 alkyl;
$R_{a3}$, $R_{b3}$, $R_{c3}$ and $R_{d3}$ are each independently selected from hydrogen, deuterium, tritium, carboxyl, sulfo-group or $R_Y$; wherein $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group, -S(=O)$R_k$, -S(=O)O$R_k$, -S(=O)(=O)$R_k$, -S(=O)(=O)O$R_k$ or $R_W$; wherein $R_k$ is selected from hydrogen, deuterium, tritium, amino, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl or a sulfo-substituted C1-C6 alkyl; $R_W$ is selected from a C1-C6 alkoxy, a C1-C6 alkyl thioether group, phenyl, benzopyrrolyl or imidazolyl; and $R_W$ is optionally substituted with one or more

substituents $P_2$ or unsubstituted; the substituent $P_2$ is selected from deuterium, tritium, hydroxyl, mercapto, halogen or a C1-C6 alkyl;

$R_{a2}$, $R_{b2}$, $R_{c2}$ and $R_{d2}$ are each independently selected from hydrogen, deuterium, tritium or a deuterated C1-C6 alkyl; or, $R_{a2}$ is connected to $R_{a1}$ to form $R_{\text{ring A}}$, $R_{b2}$ is connected to $R_{b1}$ to form $R_{\text{ring A}}$, $R_{c2}$ is connected to $R_{c1}$ to form $R_{\text{ring A}}$, $R_{d2}$ is connected to $R_{d1}$ to form $R_{\text{ring A}}$, the $R_{\text{ring A}}$ is a tetrahydropyrrole ring; and a, b, c and d are each independently selected from 0, 1 or 2;

preferably, $R_{a3}$, $R_{b3}$, $R_{c3}$ and $R_{d3}$ are each independently selected from hydrogen, deuterium, tritium, carboxyl, sulfo-group or $R_Y$; wherein Ry is a C1-C6 alkyl; and $R_Y$ is optionally substituted with one or more substituents $P_1$ or unsubstituted; the substituent $P_1$ is selected from -S(=O)(=O)$R_k$, -S(=O)(=O)O$R_k$ or $R_W$; wherein $R_k$ is selected from hydrogen, deuterium, tritium, or a C1-C6 alkyl; $R_W$ is selected from phenyl, benzopyrrolyl or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;

more preferably, the compound of Formula I is:

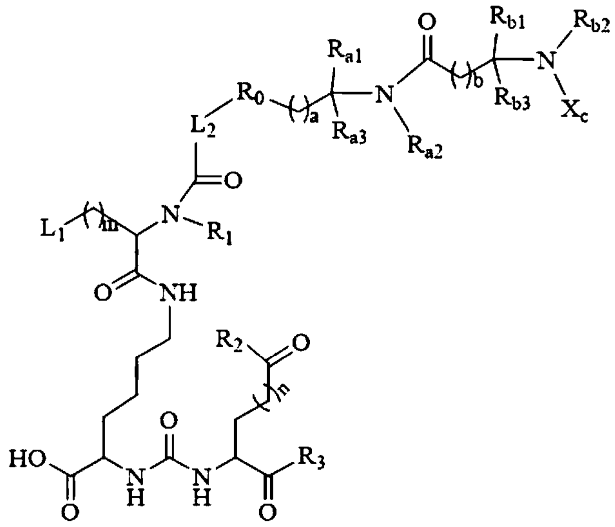

Formula Iy

wherein $X_c$ is a chelating agent;

further preferably, the compound of Formula Iy is selected from:

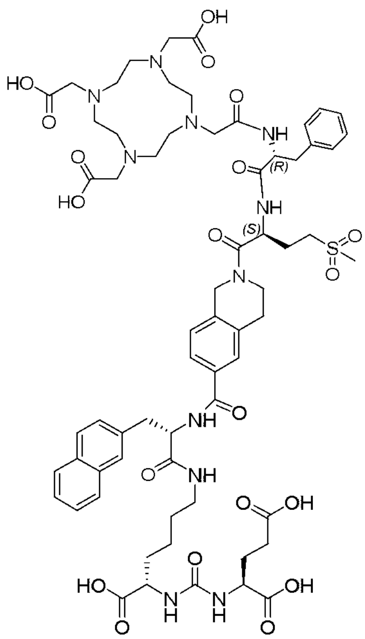

VWT045800 ,

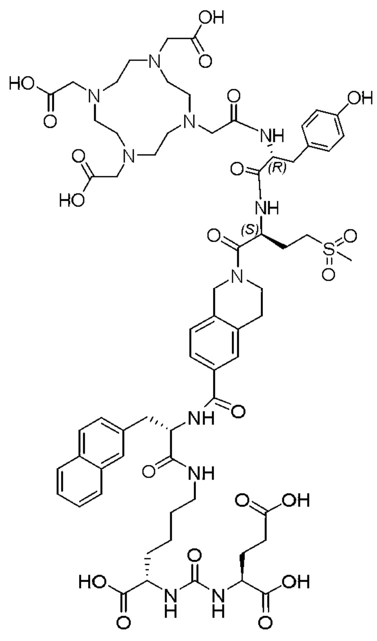

VWT046000 .

In a preferred embodiment,

Q is

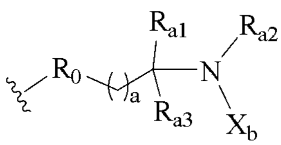

or

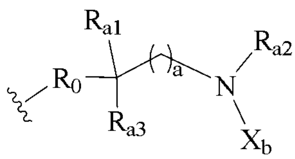

;

$R_0$ is selected from carbonyl or methylene;

$X_b$ is a chelating agent or

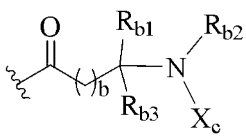

,

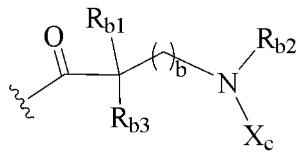

;

$X_c$ is a chelating agent or

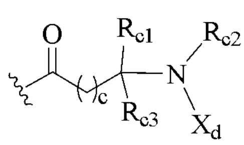

,

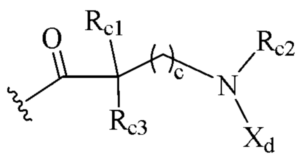

;

$X_d$ is a chelating agent or

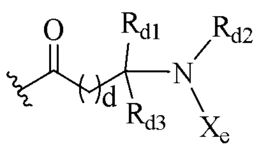

,

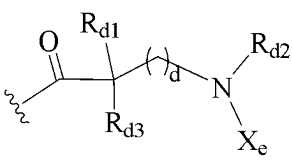

;

$X_e$ is a chelating agent;
wherein $R_{a1}$, $R_{b1}$, $R_{c1}$ and $R_{d1}$, $R_{a2}$, $R_{b2}$, $R_{c2}$ and $R_{d2}$, $R_{a3}$, $R_{b3}$, $R_{c3}$ and $R_{d3}$, a, b, c and d are as defined in claim 20;
preferably, the compound of Formula I is:

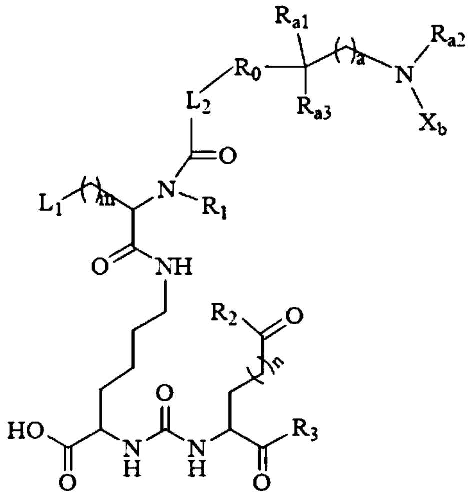

Formula Ix

wherein $X_b$ is

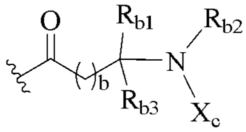

or

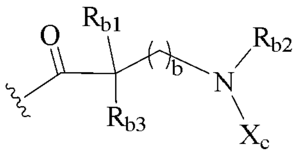

,

$X_c$ is a chelating agent;
and/or,
$R_{a1}$ and $R_{a1}$ are each independently selected from hydrogen, deuterium, tritium, or a C1-C6 alkyl;
$R_{a3}$ and $R_{b3}$ are each independently selected from hydrogen, deuterium, tritium, carboxyl, sulfo-group or $R_Y$; wherein $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group or $R_W$; wherein $R_W$ is selected from a C1-C6 alkoxy, a C1-C6 alkyl thioether group, phenyl, benzopyrrolyl or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;
$R_{a2}$ and $R_{b2}$ are each independently selected from hydrogen, deuterium, tritium or a deuterated C1-C6 alkyl; or, $R_{a2}$ is connected to $R_{a1}$ to form $R_{ring\,A}$, $R_{b2}$ is connected to $R_{b1}$ to form $R_{ring\,\Lambda}$, and the $R_{ring\,\Lambda}$ is a tetrahydropyrrole ring; and a and b are each independently selected from 0, 1 or 2;
more preferably, the compound of Formula Ix is:

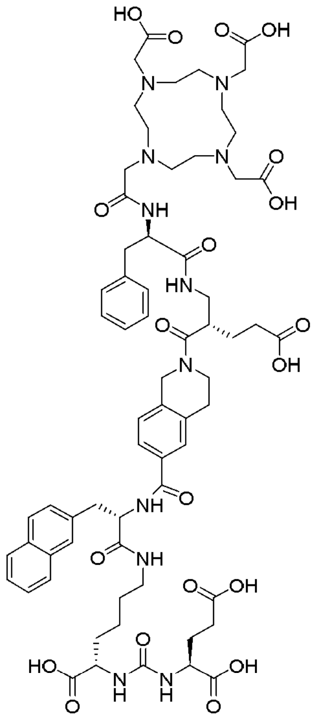

VWT037300

.

In a preferred embodiment, $L_2$ is -NH-$(CH_2)_t$-$L_3$-, wherein t is 0, 1 or 2;

preferably, $L_3$ is selected from a 3-12 membered cycloalkyl, or a 3-12 membered heterocyclic alkyl; and/or,
Q is

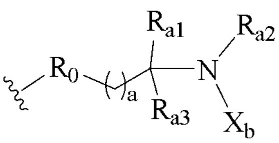

or

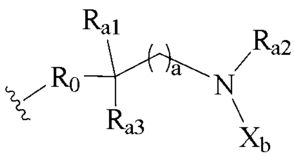

;

and $R_0$ is selected from carbonyl or methylene;
$X_b$ is a chelating agent or

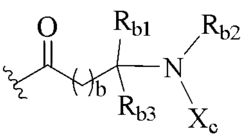

,

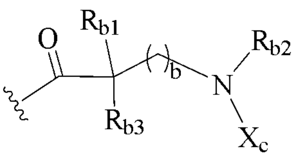

;

$X_c$ is a chelating agent or

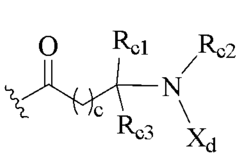

,

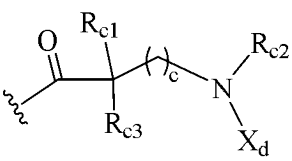

;

$X_d$ is a chelating agent or

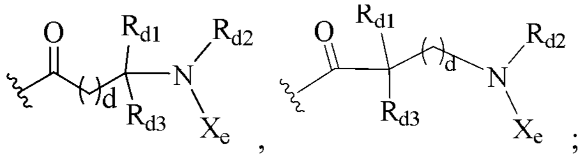

$X_e$ is a chelating agent;

wherein $R_{a1}$, $R_{b1}$, $R_{c1}$ and $R_{a1}$, $R_{a2}$, $R_{b2}$, $R_{c2}$ and $R_{d2}$, $R_{a3}$, $R_{b3}$, $R_{c3}$ and $R_{d3}$, a, b, c and d, are as defined above (for example, in claim 20).

In a preferred embodiment, the compound of Formula I is:

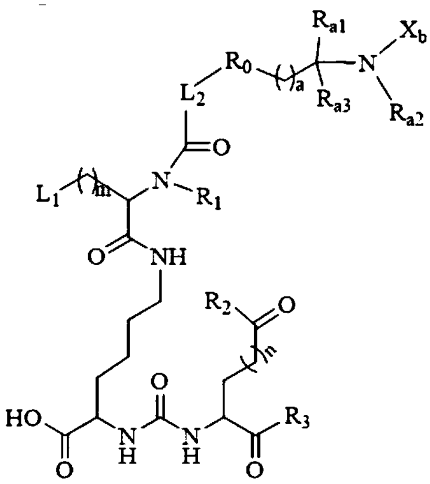

Formula Iw

wherein $X_b$ is a chelating agent or

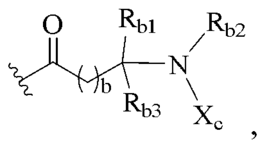

$X_c$ is a chelating agent;

and/or,

$R_{a1}$ and $R_{a1}$ are each independently selected from hydrogen, deuterium, tritium or a C1-C6 alkyl;

$R_{a3}$ and $R_{b3}$ are each independently selected from hydrogen, deuterium, tritium, carboxyl, sulfo-group or $R_Y$; wherein $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group or $R_W$; wherein $R_W$ is selected from a C1-C6 alkoxy, a C1-C6 alkyl thioether group, phenyl, benzopyrrolyl or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;

$R_{a2}$ and $R_{b2}$ are each independently selected from hydrogen, deuterium, tritium or a deuterated C1-C6 alkyl; or, $R_{a2}$ is connected to $R_{a1}$ to form $R_{ring\,A}$, $R_{b2}$ is connected to $R_{a1}$ to form $R_{ring\,A}$, and the $R_{ring\,A}$ is a tetrahydropyrrole ring; and a and b are each independently selected from 0, 1 or 2;

preferably, the compound of Formula Iw is selected from:

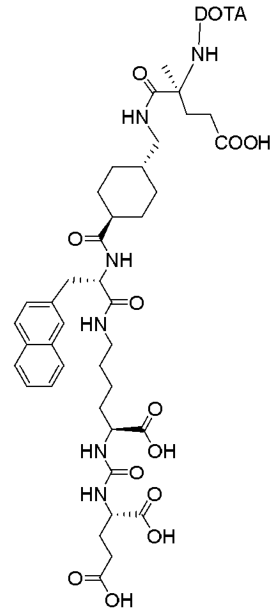

VWT049200 ,

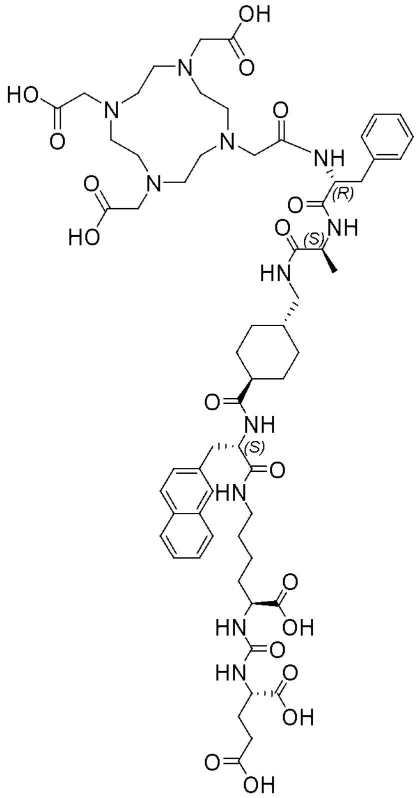

VWT049400 ,

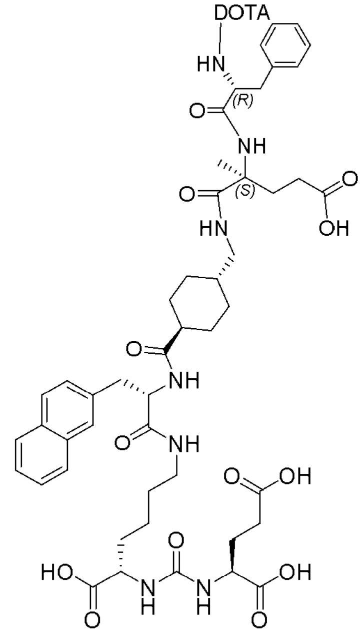

VWT0049900 ,

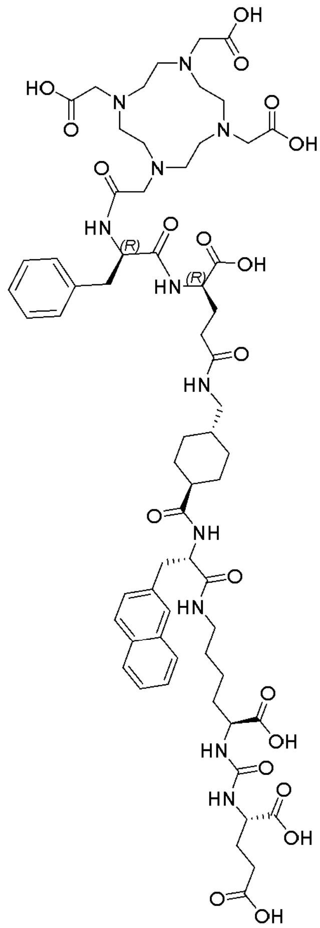

VWT050000 .

In a preferred embodiment, the pharmaceutically acceptable salt is a base addition salt; preferably, the base addition salt is a sodium salt, potassium salt, ammonium salt, or calcium salt.

The disclosure also provides the use of any of the above compound, or pharmaceutically acceptable salt, ester, or solvate thereof in the preparation of a radiolabeled complex.

The disclosure also provides a radiolabeled complex comprising any of the above compound or pharmaceutically acceptable salt, ester or solvate thereof, and a radionuclide.

Preferably, the radionuclide is selected from $^{94}$Tc, $^{99m}$Tc, $^{90}$In, $^{111}$In, $^{67}$Ga, $^{68}$Ga, $^{86}$Y, $^{90}$Y, $^{177}$Lu, $^{151}$Tb, $^{186}$Re, $^{188}$Re, $^{64}$Cu, $^{67}$Cu, $^{55}$Co, $^{57}$Co, $^{43}$Sc, $^{44}$Sc, $^{47}$Sc, $^{225}$Ac, $^{213}$Bi, $^{212}$Bi, $^{212}$Pb, $^{227}$Th, $^{153}$Sm, $^{166}$Ho, $^{152}$Gd, $^{153}$Gd, $^{157}$Gd, $^{166}$Dy, $^{55}$Fe, $^{18}$F, $^{11}$C, $^{89}$Zr, $^{123}$I, $^{124}$I, $^{125}$I, $^{131}$I or $^{211}$At.

More preferably, the radionuclide is $^{68}$Ga or $^{177}$Lu; or, the radionuclide is $^{225}$Ac.

The disclosure also provides a pharmaceutical composition comprising the above radiolabeled complex, and one or more pharmaceutically acceptable excipients, diluents, or carriers.

The disclosure also provides the use of the above radiolabeled complex and/or the pharmaceutical composition in the preparation of a medicament for the diagnosis and/or treatment and/or prevention of tumors; preferably, the tumor is cancer; more preferably, the cancer is prostate cancer.

The disclosure also provides the use of the above radiolabeled complex and/or the above pharmaceutical composition in the preparation of a medicament for imaging in patients; or, the use of the above radiolabeled complex and/or the above pharmaceutical composition in the preparation of a contrast agent.

Unless otherwise stated, the terms used in the description and claims shall have the following meanings.

"Aryl" refers to an aryl group having 5 to 20 carbon atoms, preferably a C5-C14 aryl (i.e., a 5-14 membered aryl), usually a 5- to 8-membered (e.g., 5, 6, 7, 8-membered) monocyclic, a 5- to 12-membered (e.g., 5, 6, 7, 8, 9, 10, 11, 12-membered) bicyclic, or a 10-to 15-membered (e.g., 10, 11, 12, 13, 14, 15-membered) tricyclic system. It can be a bridged ring or a spiro ring. Non-limiting examples include phenyl, naphthyl, or anthryl.

"C1-C6 alkyl" refers to a linear or branched saturated hydrocarbon group having 1 to 6 carbon atoms, preferably an alkyl group having 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and their various branched isomers.

"C1-C6 alkoxy" refers to R-O, wherein R is a C1-C6 alkyl and O is oxygen. Non-limiting examples include methoxy, ethoxy, n-propoxy, isopropoxy, n-butoxy, sec-butoxy, tert-butoxy, n-pentoxy, n-hexoxy, cyclopropoxy, and cyclobutoxy. It is preferably C1-C4 alkoxy.

"Deuterated" refers to the substitution of some or all of the hydrogen atoms in the substituted group by deuterium atoms;

"Halogenated" refers to the substitution of some or all of the hydrogen atoms in the substituted group by a halogen;

"Halogen" refers to fluorine, chlorine, bromine, or iodine;

"Heterocyclic alkyl" refers to a saturated or unsaturated non-aromatic heterocycle containing 2 to 50 carbon atoms (preferably 2 to 15 carbon atoms) and 1 to 4 (e.g., 1, 2, 3, 4) heteroatoms selected from N, O, or S. It can be a 3- to 10-membered (e.g., 3, 4, 5, 6, 7, 8, 9, 10-membered) monocyclic, a 4- to 12-membered (e.g., 4, 5, 6, 7, 8, 9, 10, 11, 12-membered) bicyclic, or a 10- to 15-membered (e.g., 10, 11, 12, 13, 14, 15-membered) tricyclic system, and containing 1 to 4 (e.g., 1, 2, 3, 4) heteroatoms selected from N, O, or S.

"Heterocyclic alkyl" can be a bridged ring or a spiro ring. Non-limiting examples of "heterocyclic alkyl" include epoxide ethyl, epioxide propyl, aziridinyl, oxetanyl, azetidinyl, thietanyl, 1,3-dioxolanyl, 1,4-dioxolanyl, 1,3-dioxanyl, pyrrolidinyl, oxazolidinyl, morpholinyl, thiomorpholinyl, azepanyl, oxepanyl, thiepanyl, oxazepinyl, diazepinyl, thiazepinyl, piperidinyl, and the like.

"Heteroaryl" refers to a 3- to 8-membered (e.g., 3, 4, 5, 6, 7, 8-membered) monocyclic, a 5- to 12-membered (e.g., 5, 6, 7, 8, 9, 10, 11, 12-membered) bicyclic, or a 10- to 15-membered (e.g., 10, 11, 12, 13, 14, 15-membered) tricyclic system, and containing 1 to 6 (e.g., 1, 2, 3, 4, 5, 6) heteroatoms selected from N, O, or S, preferably a 5-14 membered heteroaryl; it can be a bridged ring or a spiro ring. Non-limiting examples include pyridinyl, furanyl, thienyl, pyranyl, pyrrolyl, pyrimidinyl, pyrazinyl, pyridazinyl, imidazolyl, oxazolyl, thiazolyl, thiopyranyl, benzimidazolyl, benzoxazolyl, benzopyridyl (including: indolyl, isoindolyl), benzimidazolyl, benzofuranyl, benzothiazolyl, benzothienyl, benzothiopyranyl, pyrrolopyridinyl, and purinyl.

"Pharmaceutically acceptable salt" includes acid addition salts and/or base addition salts, wherein the "base addition salts" refer to pharmaceutically acceptable salts formed with organic or inorganic bases; the inorganic bases include, but are not limited to, sodium, potassium, ammonium, calcium, magnesium, iron, zinc, copper, manganese, and aluminum salts; salts derived from organic bases, including primary, secondary, and tertiary amines, substituted amines, cyclic amines, and basic ion exchange resins (e.g., isopropylamine, trimethylamine, diethylamine, triethylamine, tripropylamine, ethanolamine, 2-diethylaminoethanol, tromethamine, dicyclohexylamine, lysine, arginine, histidine, caffeine, procaine, hydrabamine, choline, betaine, ethylenediamine, glucosamine, methylglucosamine, theobromine, purine, piperazine, piperidine, N-ethylpiperidine, and polyamine resins).

In some embodiments, compounds of general formulas such as Formula I, Formula Ia, Formula Ib, Formula Ic, Formula Id, Formula Ie, Formula If, Formula Ig, Formula Ix, Formula Iy, Formula Iz, and Formula Iw, as well as respective specific compounds, react with aqueous solutions of sodium hydroxide, ammonia, etc., to form base addition salts, which can improve the stability, solubility, and/or bioavailability of the compounds. For example, 100 mg of VWT032900 is added to 2 ml of an aqueous solution of sodium hydroxide with a concentration of approximately 225 mmol/L, stirred until completely dissolved, and then lyophilized to obtain the sodium salt of VWT032900.

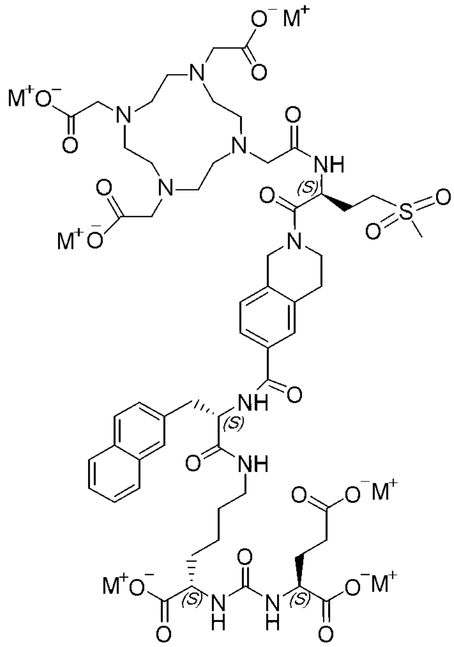

In the formula, M can be sodium (Na), potassium (K), ammonium ($NH_4$), and the like.

"Solvate" refers to a compound or a pharmaceutically acceptable salt thereof, wherein molecules of a suitable solvent are bound in a crystal lattice. The suitable solvent is physiologically permissible at the administered dose. Examples of suitable solvents include ethanol, water, and the like. When water is the solvent, the molecule is called as a "hydrate".

"Ester" refers to the esterification of the free acid group in the compounds of the disclosure. Suitable esters include various alkyl esters, such as C1-C10 alkyl esters of saturated or unsaturated C1-C18 fatty acids.

In the chemical structure of the compound described in the disclosure, the "bond" does not specify the configuration, that is, the bond " /" can be S configuration, R configuration, D configuration, L configuration, Z configuration or E configuration, or simultaneously includes multiple configurations (e.g., two, three, four, and the like).

## BRIEF DESCRIPTION OF THE DRAWINGS

Figure 1 is a visualized image after administration of $^{177}$Lu-VWT011600 in Experimental Example 1.
Figure 2 is a visualized image after administration of $^{177}$Lu-VWT032900 in Experimental Example 1.
Figure 3 shows a trend of tumor size change of the 22RV1 subcutaneous tumor model in Experimental Example 1.

## DETAILED DISCLOSURE

The present invention will now be clearly and completely described with reference to specific examples. Those skilled in the art will understand that the examples described below are only some examples, and not all examples of the present invention, and are only used to illustrate the present invention, and should not be regarded as limiting the protection scope of the present invention.

In the present invention, unless otherwise specified, the procedures shall be performed under conventional conditions or conditions recommended by the manufacturer. Reagents or instruments whose manufacturers are not specified are all commercially available conventional products.

Regarding the definitions of terms used in the present invention, unless otherwise specified, the initial definitions provided herein apply to the term throughout the context; for terms not specifically defined herein, meanings that those skilled in the art can assign to them may be given based on the disclosure and/or context.

### Example 1

Preparation of compound VWT011600

Step 1 (preparation of VWT016100)

(1-1) preparation of VWT016101

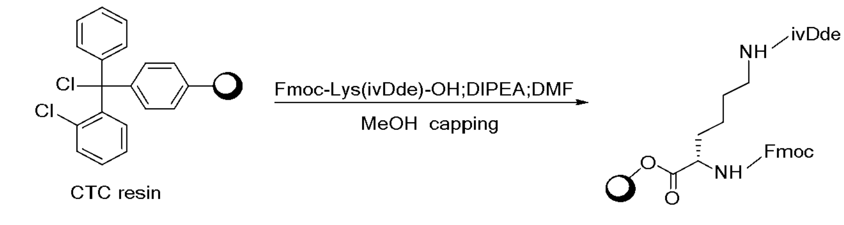


VWT016101

CTC resin (2-chlorotriphenylmethyl chloride bonded to polystyrene, 1.00 g, initial degree of substitution: 1.09 mmol/g) was swollen in DMF (N,N-dimethylformamide: 10 mL) for 30 minutes, and washed twice with DMF (10 mL × 2). The aforementioned CTC resin was treated with a mixture of Fmoc-Lys(ivDde)-OH (940 mg, CAS No.: 204777-78-6; commercially available or synthesized by known methods; Fmoc refers to fluorenylmethyloxycarbonyl) and DIPEA (N,N-diisopropylethylamine: 426 mg) in DMF (4 mL) for 5 hours, and added with methanol (0.5 mL) for another 1 hour, then washed with DMF (10 mL) to obtain VWT016101.

(1-2) preparation of VWT016102

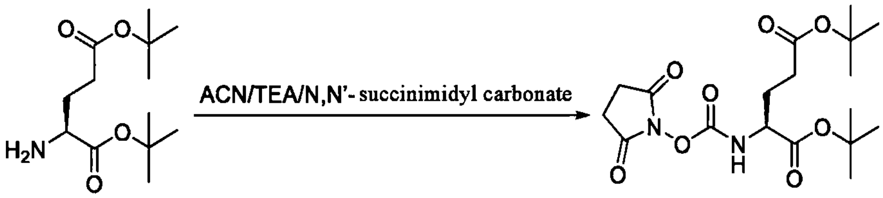


**VWT016102**

H-Glu(OtBu)-OtBu-HCl *(5.00* g, CAS No.: 32677-01-3; commercially available or synthesized by known methods; tBu refers to tert-butyl) was added to 200 mL of anhydrous acetonitrile (ACN) and stirred until dissolved. At room temperature, DSC (N,N'-succinimidyl carbonate: 4.32 g, CAS No.: 74124-79-1) was added with continuous stirring, and triethylamine (2.60 g) was slowly added dropwise. After reacting overnight at room temperature, the mixture was washed with 10% citric acid aqueous solution and saturated brine, separated, and the organic phase was concentrated and distilled to obtain 7.12 g of VWT016102.

(1-3) preparation of VWT016103

VWT016101 resin (0.34 mmol) was swollen in DMF (10 mL) for 30 minutes, washed twice with DMF (10 mL × 2), treated with 20% piperidine/DMF (10 mL, volume ratio: piperidine/DMF = 1/4) for 30 minutes, washed with DMF (10 mL), and then reacted with VWT016102 (3.49 g) in DMF (7 mL) under DIPEA alkaline conditions for 5 hours. The resin was then washed with DMF (10 mL) to obtain compound VWT016103.

(1-4) preparation of VWT016100

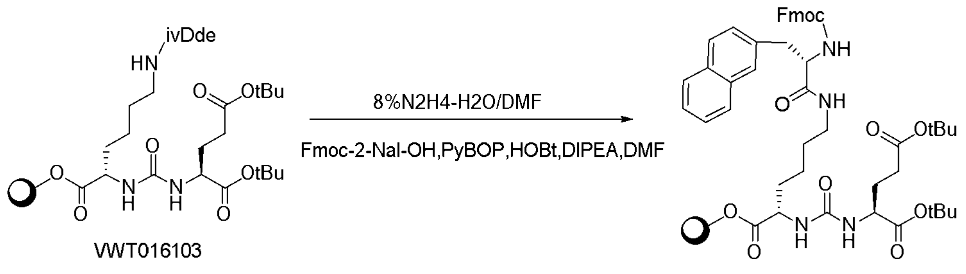


VWT016103

VWT016100

VWT016103 resin (0.34 mmol) was swollen in DMF (10 mL) for 30 min, treated with 8% hydrazine hydrate/DMF (10 mL, volume ratio: hydrazine hydrate-DMF = 8:92) for 30 min to remove the ivDde protecting group, then washed with DMF (10 mL). The reaction was treated with a mixture of Fmoc-2-Nal-OH (765 mg, CAS No.: 112883-43-9; commercially available or synthesized by known methods), PyBOP (benzotriazole-1-yl-oxytripyrrolidinophosphonium hexafluorophosphate: 918 mg, CAS No.: 128625-52-5), HOBT (1-hydroxybenzotriazole: 236 mg, CAS No.: 2592-95-2), and DIPEA (460 mg) in DMF (7 mL) for 4 hours, and the resin was washed with DMF (10 mL) to obtain compound VWT016100.

Step 2 (preparation of VWT011602)

(2-1) Synthesis of Compound 2: (2-(tert-butyl)-6-ethyl-3,4-dihydroisoquinoline-2,6(1H)-dicarboxylate)

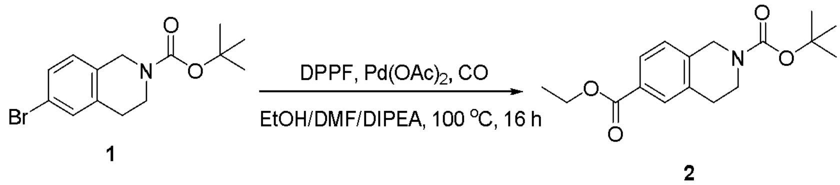


1

2

Compound 1 (tert-Butyl 6-bromo-3,4-dihydroisoquinoline-2(1H)-carboxylate: 1.0 g), DPPF (1,1'-bis(diphenylphosphine)ferrocene: 355 mg), and $Pd(OAc)_2$ (palladium acetate: 71.9 mg) were added to a DMF-EtOH-DIPEA solution (30 mL, volume ratio: DMF-EtOH-DIPEA = 8:2:1). The reaction mixture was reacted at 100°C under a CO (carbon monoxide) atmosphere for 16 hours, and added with ethyl acetate (100 mL), then washed with water and saturated brine, dried over anhydrous sodium sulfate, and concentrated to obtain a residue, which was purified by silica gel column chromatography (volume ratio: petroleum ether/ethyl acetate = 3/1) to obtain compound 2 (600 mg, 2-(tert-butyl)-6-ethyl-3,4-dihydroisoquinoline-2,6(1H)-dicarboxylate (61%) as a yellow solid.

(2-2) Synthesis of Compound 3: (Ethyl 1,2,3,4-tetrahydroisoquinoline-6-carboxylate)

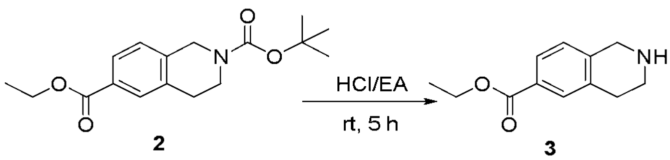


2

3

Compound 2 (600 mg) was added to a 4.0 mol/L hydrochloric acid/ethyl acetate solution (20 mL, CAS: 7647-01-0). The reaction mixture was reacted at room temperature for 5 hours. The solid was collected and washed with ethyl acetate to give compound 3 (400 mg, ethyl 1,2,3,4-tetrahydroisoquinoline-6-carboxylate, 99%) as a white solid.

(2-3) Synthesis of Compound 5: (Ethyl 2-(((benzyloxy)carbonyl)-L-alanyl)-1,2,3,4-tetrahydroisoquinoline-6-carboxylate)

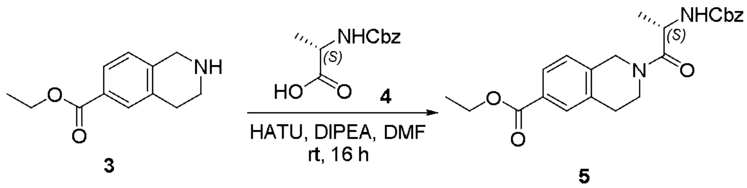


3

5

Compound 4 (benzyloxycarbonyl-L-alanine: 243 mg), HATU (2-(7-azabenzotriazole)-N,N,N',N'-tetramethylurea hexafluorophosphate: 621 mg, CAS No.: 148893-10-1), compound 3 (335 mg), and DIPEA (422 mg) were added to DMF (30 mL), respectively. After reacting at room temperature for 16 hours, the reaction mixture was added with ethyl acetate (100 mL), then washed with water and saturated brine, dried over anhydrous sodium sulfate, and concentrated to obtain a residue, which was purified by silica gel column chromatography (volume ratio: petroleum ether/ethyl acetate = 1/1) to give compound 5 (440 mg, ethyl 2-(((benzyloxy)carbonyl)-L-alanyl)-1,2,3,4-tetrahydroisoquinoline-6- carboxylate, 98%) as a colorless oil.

(2-4) Compound 6: (2-(((benzyloxy)carbonyl)-L-alanyl)-1,2,3,4-tetrahydroisoquinoline-6-carboxylic acid:

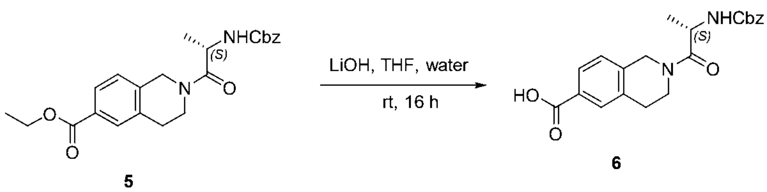


5

6

122.5 mg of lithium hydroxide (dissolved in 10 mL of water) was added to a solution of compound 5 (420 mg) in tetrahydrofuran (10 mL), and the reaction was carried out at room temperature for 16 hours. The reaction mixture was concentrated to remove the tetrahydrofuran, and adjusted to pH 7 with 1N hydrochloric acid, resulting in a large amount of solid. The solid was collected, washed with water, and dried to give compound 6 (360 mg, 2-(((benzyloxy)carbonyl)-L-alanyl)-1,2,3,4-tetrahydroisoquinoline-6-carboxylic acid, 90%) as a white solid.

(2-5) Synthesis of Compound VWT011602

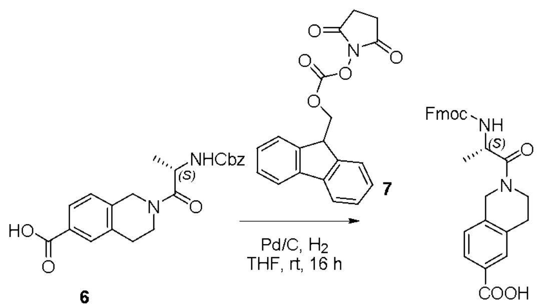


6

VWT011602

Compound 6 (360 mg), compound 7 (N-(9-fluorenylmethoxycarbonyl)succinimide: 476 mg; can be represented as Fmoc-OSu), and Pd/C (40 mg) were added to tetrahydrofuran (10 mL). The reaction mixture was reacted at room temperature under a hydrogen atmosphere for 16 hours. The solid was filtered off, and the filtrate was concentrated under reduced pressure to obtain a residue, which was purified by prep-HPLC (mobile phase: 0.1% formic acid/acetonitrile/-water) to obtain compound VWT011602 (336.8 mg, 2-((((9H-fluoren-9-yl)methoxy)carbonyl)-L-alanyl)-1,2,3,4-tetrahydroisoquinoline-6-carboxylic acid) as a white solid.

Step 3 (preparation of VWT011603)

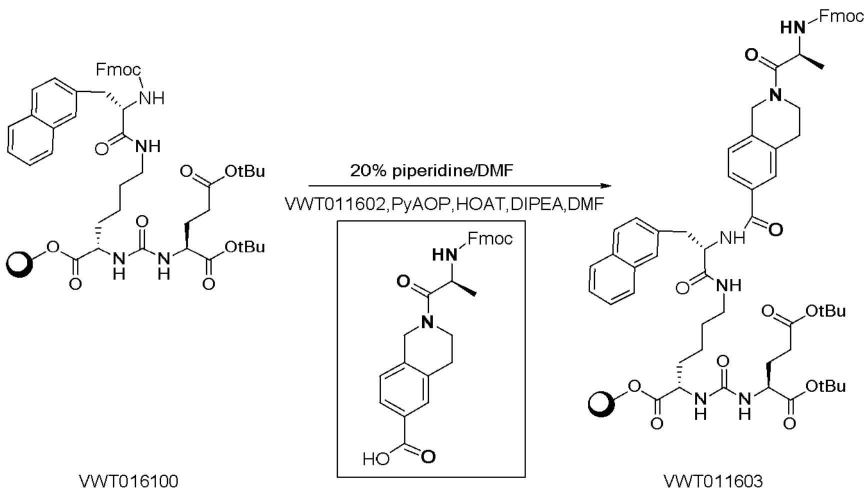


VWT016100 VWT011603

VWT016100 resin (0.34 mmol) was swollen in DMF (3 mL) for 30 minutes, treated with 20% piperidine/DMF (3 mL) for 30 minutes to remove the Fmoc protecting group, then washed with DMF (3 mL). The reaction was treated with a mixture of VWT011602 (191 mg), PyAOP ((7-azabenzotriazole-1-oxo)tripyrrolidinophosphonium hexafluorophosphate: 217 mg, CAS No.: 156311-83-0), HOAT (1-hydroxy-7-azabenzotriazole: 55 mg, CAS No.: 39968-33-7), and DIPEA (112 mg) in DMF (3 mL) for 3 hours, and the resin was then washed with DMF (3 mL) to obtain compound VWT011603.

Step 4 (preparation of VWT011604)

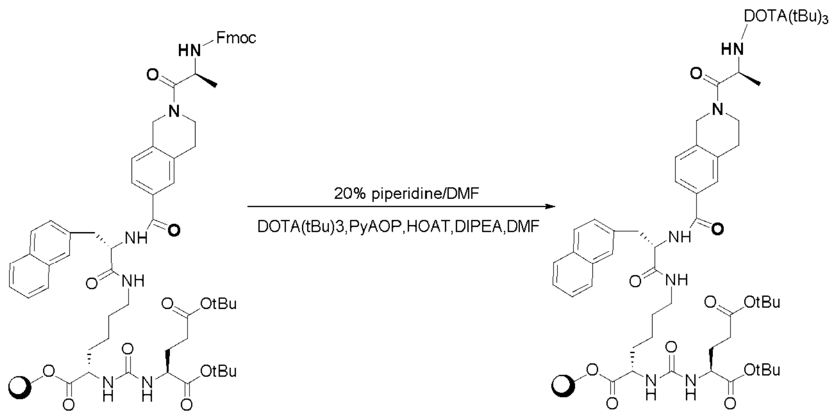


VWT011603 VWT011604

VWT016103 resin (0.34 mmol) was swollen in DMF (3 mL) for 30 minutes, then treated with 20% piperidine/DMF (3 mL) for 30 minutes to remove the Fmoc protecting group, then washed with DMF (3 mL). The reaction was treated with a mixture of DOTA(tBu)$_3$ (390 mg, CAS No.: 137076-54-1), PyAOP (362 mg), HOAT (95 mg), and DIPEA (178 mg) in DMF (3 mL) for 5 hours, and the resin was then washed with DMF (3 mL) to obtain compound VWT011604.

Step 5 (preparation of VWT011600)

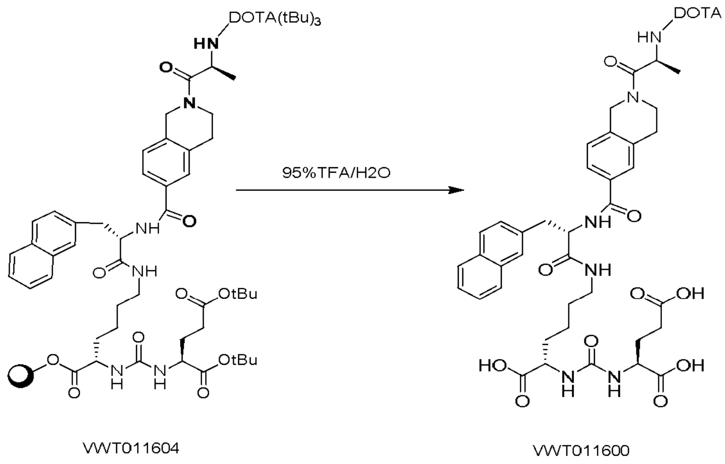


VWT011604 was treated in 95% TFA/5% $H_2O$ Cleavage Cocktail (10 mL; TFA refers to trifluoroacetic acid) for 3 hours, crystallized with the addition of methyl tert-butyl ether (MTBE), and then centrifuged and dried to obtain a white crude solid, VWT011600. This crude solid was then purified by preparative HPLC to obtain the target product VWT011600 with a purity of 97.86%; LCMS: $[M+H]^+$= 1133.29.

Example 2

Preparation of compound VWT011400

Step 1 (preparation of VWT016100)

VWT016100 was prepared using the same method as in Example 1.

Step 2 (Preparation of compound 7-(((9H-fluorene-9-yl)methoxy)carbonyl)-7-azaspiro[3.5]nonane-2-carboxylic acid)

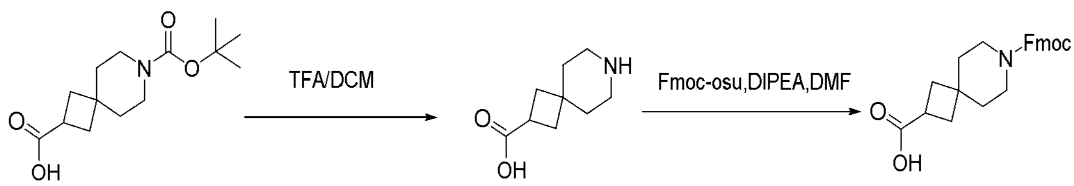


Raw material 7-(tert-butoxycarbonyl)-7-azaspiro[3.5]nonane-2-carboxylic acid (0.5 g, CAS No.: 873924-12-0; commercially available or synthesized by known methods) was added to TFA/DCM (10 mL, volume ratio: TFA/DCM = 2/1; DCM refers to dichloromethane), stirred at room temperature for 4 hours, and concentrated to obtain a residue, which was slurried with methyl tert-butyl ether, crystallized, and centrifuged and dried to obtain intermediate 7-azaspiro[3.5]nonane-2-carboxylic acid (0.49 g) as a white solid.

Then, the intermediate 7-azaspiro[3.5]nonane-2-carboxylic acid (0.49 g) and Fmoc-OSu (0.92 g) were added to dichloromethane (50 mL) and stirred until dissolved. Then, DIPEA (0.85 mL) was slowly added dropwise, and the reaction was carried out at room temperature for 16 hours. The reaction mixture was added with purified water (100 mL) and ethyl acetate (100 mL), stirred thoroughly, and separated. The organic phase was washed with saturated brine, dried over anhydrous sodium sulfate, and concentrated to obtain a residue, which was purified by silica gel column chromatography (volume ratio EA:PE = 11:1; EA refers to ethyl acetate; and PE refers to petroleum ether) to obtain compound 7-(((9H-fluorene-9-yl)methoxy)carbonyl)-7-azaspiro[3.5]nonane-2-carboxylic acid (0.46 g) as a white solid.

Step 3 (preparation of VWT0011402)

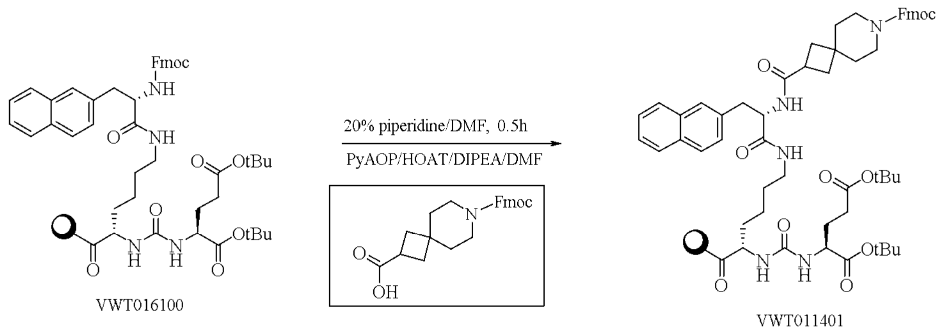


VWT016100 VWT011401

VWT016100 resin (0.35 mmol) was treated with 20% piperidine/DMF (3 mL) for 30 min, then washed with DMF. 7-(((9H-fluorene-9-yl)methoxy)carbonyl)-7-azaspiro[3.5]nonane-2-carboxylic acid was coupled in the order of the sequence in a PyAOP/HOAt/DIPEA condensation system for 7 h. The resin was then washed with DMF to obtain compound VWT011401.

Compound VWT011401 was treated with 20% piperidine/DMF (3 mL) for 30 minutes, followed by washing the resin with DMF. Fmoc-alanine (Fmoc-Ala-OH) was then coupled in a DIC/HOBT condensation system (DIC refers to N,N'-diisopropylcarbodiimide) for 5 hours. The resin was then washed with DMF to obtain compound VWT011402.

Step 4 (Preparation of compound VWT011400)

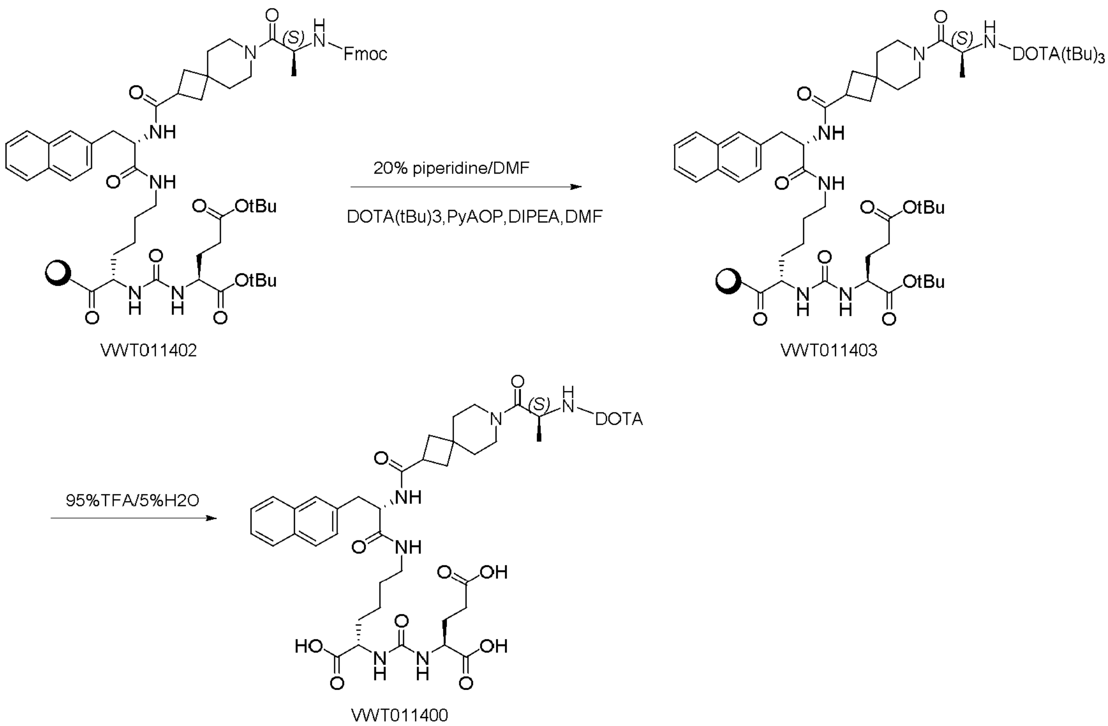


VWT011402 VWT011403 VWT011400

VWT011402 resin (0.35 mmol) was treated with 20% piperidine/DMF (3 mL) for 30 minutes, then washed with DMF. Compound DOTA$(tBu)_3$ was coupled in the PyAOP/DIPEA condensation system for 8 hours. The resin was then washed with DMF to obtain VWT011403.

VWT011403 was treated in 95% TFA/5% $H_2O$ Cleavage Cocktail (10 mL) for 5 hours, crystallized with methyl tert-butyl ether, and then centrifuged and dried to obtain crude VWT011400. This crude product was then purified by preparative HPLC to obtain 63.8 mg of the target product VWT011400 with a purity of 97.35%; LCMS: $[M+H]^+$=1125.80.

**Example 3**

Preparation of compound VWT019300

Step 1 (preparation of VWT019600)

(1-1) Synthesis of VWT016101

VWT016101 was prepared using the same method as in Example 1.

(1-2) Synthesis of VWT019602

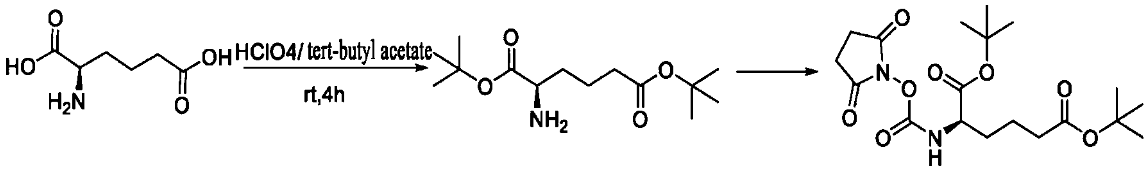


VWT019602

(R)-2-aminoadipic acid (13 g) was added to tert-butyl acetate (150 mL), stirred until dissolved, and then slowly added dropwise with perchloric acid aqueous solution (12.20 g) at a concentration of 70% to 72% under ice-water bath conditions. After the addition was complete, the reaction was carried out at room temperature for 4 hours. The reaction mixture was concentrated, added with ethyl acetate (400 mL) under stirring, and separated, and the organic phase was washed with purified water and saturated brine, then distilled and concentrated to obtain 10.50 g of di-tert-butyl (R)-2-aminoadipate as a colorless oil.

Di-tert-butyl (R)-2-aminoadipate (10.5 g) was added to anhydrous acetonitrile (300 mL), stirred until dissolved, and added with DSC (9.36 g) at room temperature with continuous stirring, then slowly added dropwise with triethylamine (5.6 g). After the addition was complete, the reaction was carried out at room temperature overnight. The mixture was separated, and the organic phase was washed with 10% citric acid aqueous solution and saturated brine, then concentrated and distilled to obtain 17.09 g of compound VWT019602: Di-tert-butyl (R)-2-((((2,5-dioxopyrrolidin-1-yl) oxy)carbonyl)amino)adipate.

(1-3) preparation of VWT019603

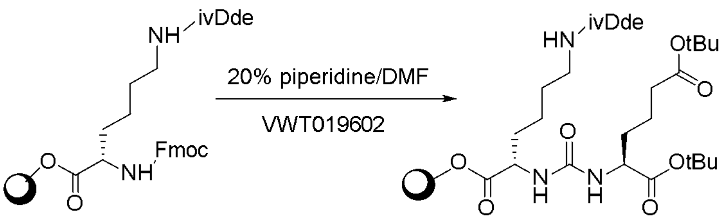


VWT016101 VWT019603

VWT016101 resin (3.24 mmol) was swollen in DMF (30 mL) for 30 minutes and washed with DMF (30 mL), then treated with 20% piperidine/DMF (30 mL) for 30 minutes and washed with DMF (240 mL), then reacted with VWT019602 (13.4 g) in DMF (25 mL) under DIPEA alkaline conditions for 4 hours, and washed with DMF (150 mL) to obtain compound VWT019603.

(1-4) preparation of VWT019600

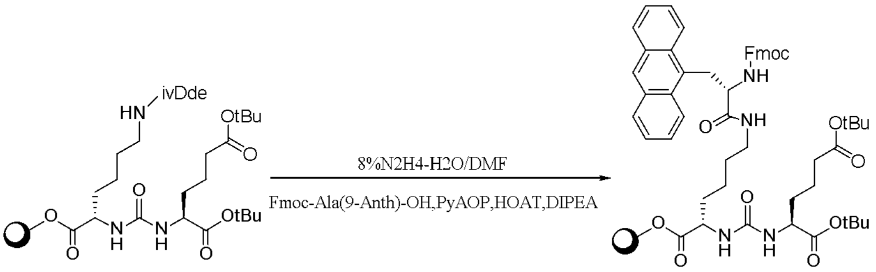


VWT019603 VWT019600

VWT019603 resin (2.64 mmol) was swollen in DMF (30 mL) for 30 min, treated with 8% hydrazine hydrate/DMF (25 mL) for 30 min to remove the ivDde protecting group, then washed with DMF (300 mL). The reaction was then treated with a mixture of Fmoc-Ala(9-Anth)-OH (Fmoc-L-9-anthryalanine, 2.57 g, CAS No.: 268734-27-6; commercially available or synthesized by known methods), PyAOP (2.76 g), HOAT (0.54 g), and DIPEA (1.37 g) in 25 mL DMF for 24 h, and the resin was washed with DMF (150 mL) to obtain compound VWT019600.

Step 2 (preparation of VWT019301)

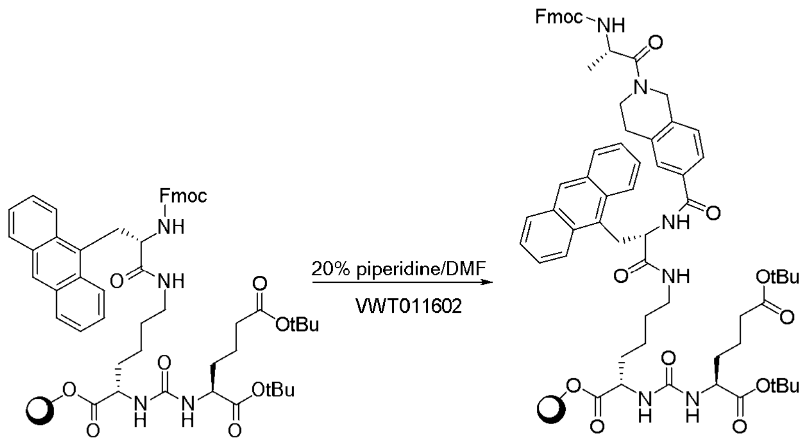


VWT019600 VWT019301

VWT019600 resin (0.25 mmol) was swollen in DMF (5 mL) for 30 minutes, treated with 20% piperidine/DMF (5 mL) for 30 minutes to remove the Fmoc protecting group, and then washed with DMF (24 mL). The reaction was then treated with a mixture of VWT011602 (144 mg), PyAOP (159 mg), HOAT (35 mg), and DIPEA (82 mg) in DMF (4 mL) for 4 hours, and the resin was washed with DMF (18 mL) to obtain compound VWT019301.

Step 3 (preparation of VWT019302)

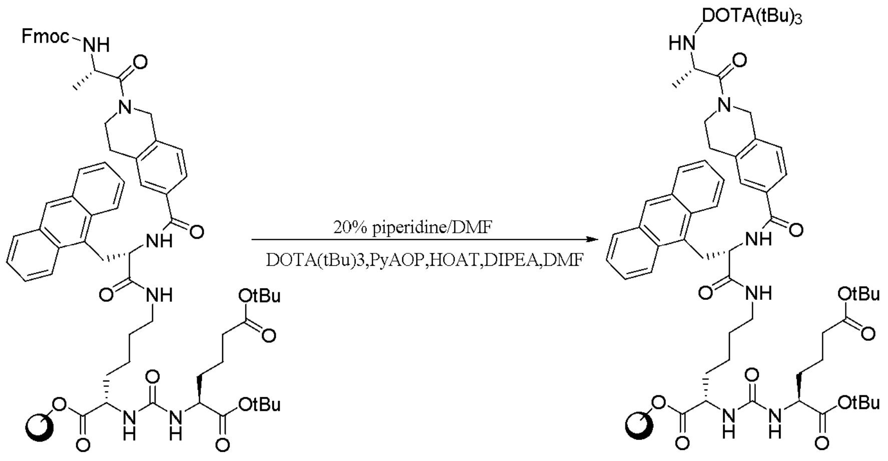


VWT019301 VWT019302

VWT019301 resin (0.25 mmol) was swollen in DMF (3 mL) for 30 minutes, then treated with 20% piperidine/DMF (3 mL) for 30 minutes to remove the Fmoc protecting group, and then washed with DMF (3 mL). The reaction was then treated with a mixture of DOTA(tBu)$_3$ (285 mg), PyAOP (260 mg), HOAT (65 mg), and DIPEA (130 mg) in DMF (4 mL) for 5 hours, and the resin was then washed with DMF (18 mL) to obtain compound VWT019302.

Step 4 (preparation of VWT019300)

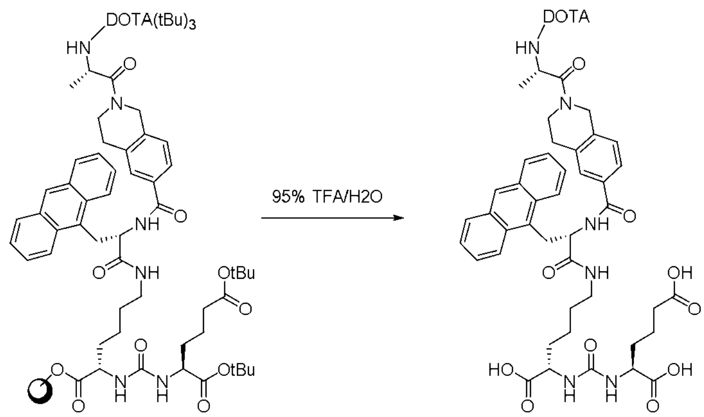


VWT019302 VWT019300

VWT019302 was treated in 95% TFA/5% $H_2O$ Cleavage Cocktail (5 mL) for 4 hours, crystallized with the addition of methyl tert-butyl ether, and centrifuged and dried to obtain 261 mg of crude VWT019300 as a white solid, which was further purified by preparative HPLC to obtain 41.3 mg of the target product VWT019300 with a purity of 92.43%, LCMS: $[M+H]^+$ = 1197.80.

**Example 4**

Preparation of compound VWT019000

Step 1 (Preparation of intermediate VWT019603)

VWT019603 was prepared using the same method as in Example 3.

Step 2 (Preparation of compound VWT011601)

(2-1) Synthesis of Compound 2

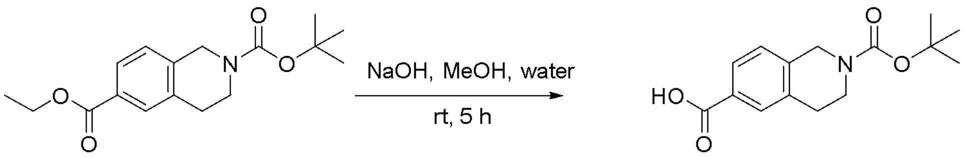


1 2

Sodium hydroxide (3.16 g, 79 mmol, 10.0 eq, dissolved in 20 mL of water) was added to a solution of compound 1 (2.4 g, 7.9 mmol, 1.0 eq, CAS No.: 1443238-78-5) in methanol (20 mL), and the reaction was carried out at room temperature for 5 hours. The reaction mixture was concentrated to remove methanol, and adjusted to pH 7 with 1N hydrochloric acid, resulting in a large amount of solid, which was extracted with ethyl acetate (50 mL × 3), and the organic phases were combined and washed successively with water and saturated brine, dried over anhydrous sodium sulfate, and concentrated to obtain compound 2: 2-(tert-butoxycarbonyl)-1,2,3,4-tetrahydroisoquinoline-6-carboxylic acid (1.77 g, yield 81%) as a white solid.

(2-2) Synthesis of Compound 3

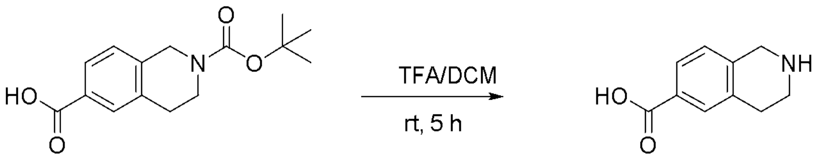


2 3

Compound 2 (1.77 g, 6.4 mmol, 1.0 eq) and trifluoroacetic acid (5 mL) were added to dichloromethane (20 mL) and reacted at room temperature for 5 hours. The reaction mixture was concentrated to give Compound 3: 1,2,3,4-tetrahydroisoquinoline-6-carboxylic acid (1.13 g, 100% yield) as a colorless liquid.

(2-3) Synthesis of Compound VWT011601

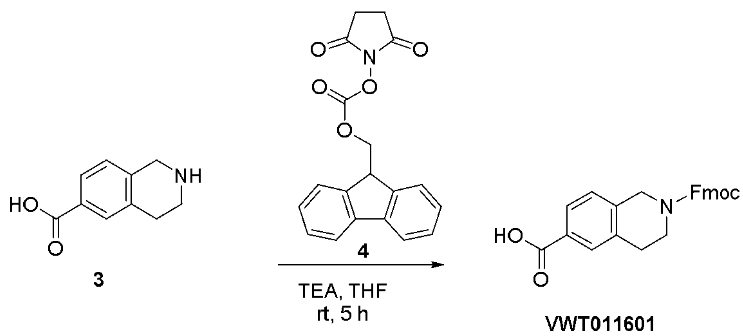


3 4 VWT011601

Compound 3 (1.13 g, 6.4 mmol, 1.0 eq), Compound 4 (3.24 g, 9.6 mmol, 1.5 eq), and triethylamine (6.46 g, 64 mmol, 10 eq) were added to tetrahydrofuran (50 mL) and reacted at room temperature for 5 hours. The reaction mixture was concentrated and purified by silica gel column chromatography (volume ratio: dichloromethane/methanol = 20:1) to give compound VWT011601: 2-(((9H-fluorene-9-yl)methoxy)carbonyl)-1,2,3,4-tetrahydroisoquinoline-6-carboxylic acid (1.79 g, yield 68%) as a white solid.

Step 3 (Preparation of compound VWT019003)

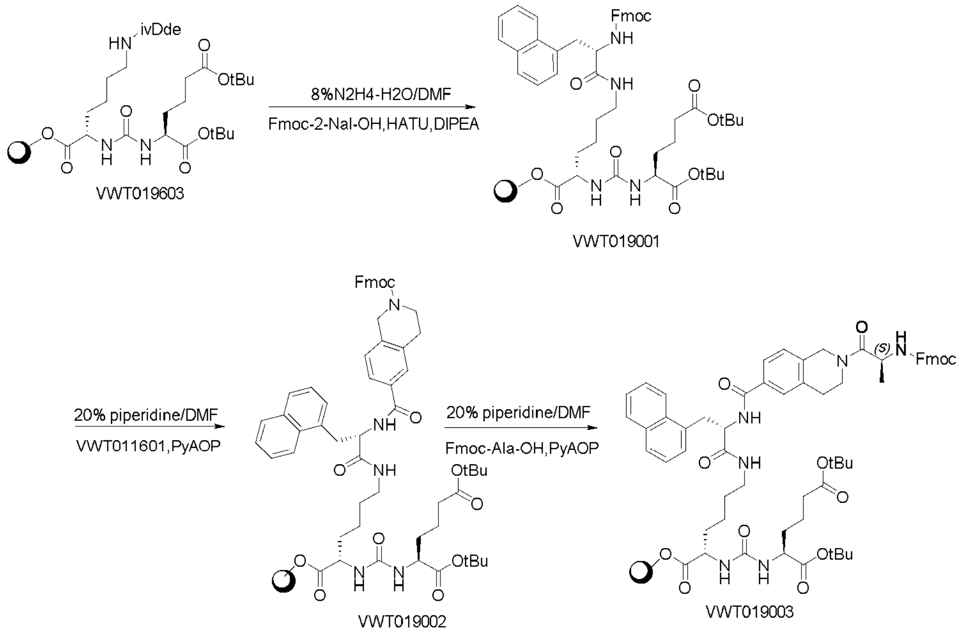


VWT019603 VWT019001 VWT019002 VWT019003

VWT019603 resin (0.6 mmol) was swollen in DMF (10 mL) for 30 minutes, then washed with DMF, and treated with 8% hydrazine hydrate/DMF (5 mL) for 30 minutes to remove the ivDde protecting group. The resin was then washed with DMF again, and coupled with Fmoc-2-Nal-OH, HATU, and DIPEA for 18 h, then washed with DMF to obtain intermediate VWT019001.

VWT019001 resin (0.3 mmol) was treated with 20% piperidine/DMF (5 mL) for 30 minutes to remove the Fmoc protecting group, then washed with DMF again. The resin was then treated with a mixture of VWT011601 (CAS No.: 2138226-21-6), PyAOP, HOAT, and DIPEA in DMF (5 mL) for 5 hours, and washed with DMF to obtain compound VWT019002.

Compound VWT019002 was treated with 20% piperidine/DMF (5 mL) for 30 minutes to remove the Fmoc protecting group. The resin was then washed with DMF, and treated with a mixture of Fmoc-Ala-OH, PyAOP, HOAT, and DIPEA in DMF (3 mL) for 2 hours, then washed with DMF to obtain compound VWT019003.

Step 4 (Preparation of compound VWT019000)

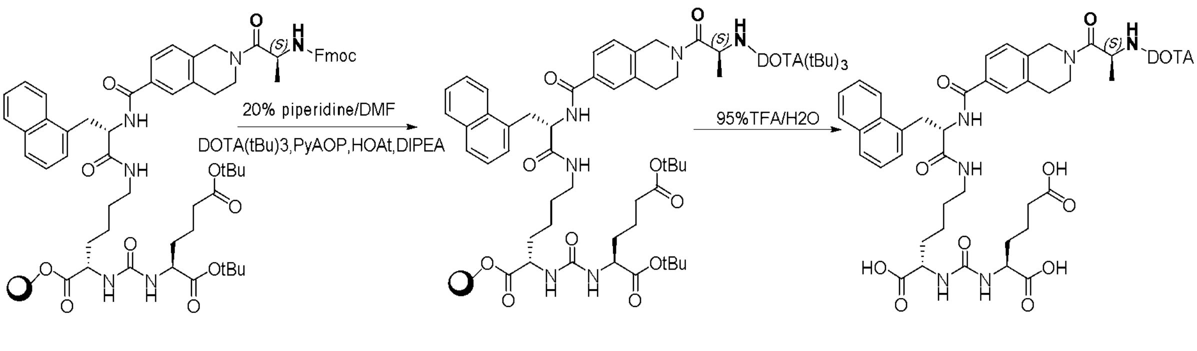


VWT019003 VWT019004 VWT019000

VWT019003 resin (0.3 mmol) was treated with 20% piperidine/DMF (5 mL) for 30 minutes to remove the Fmoc protecting group, then washed with DMF and treated with a mixture of DOTA$(tBu)_3$, PyAOP, HOAT, and DIPEA in DMF (3 mL) for 17 hours, then washed with DMF to obtain resin VWT019004.

VWT019004 was treated with 95% TFA/5% $H_2O$ Cleavage Cocktail (7 mL) for 5 hours, crystallized with methyl tert-butyl ether, and centrifuged and dried to obtain 477 mg of crude VWT019000 as a white solid. This crude product was then purified by preparative HPLC to obtain 45 mg of the target product VWT019000 with a purity of 94.8%; LCMS: $[M+H]^+$ = 1147.70.

**Example 5**

Preparation of compound VWT021400

Step 1 (preparation of VWT016100)

VWT016100 was prepared using the same method as in Example 1.

Step 2 (preparation of VWT021401)

(2-1) Synthesis of Compound 2: (5-carboxyl-isodihydroindole)

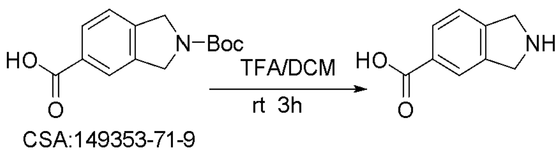


2-(tert-butoxycarbonyl)isoindoline-5-carboxylic acid (250 mg, CAS No.: 149353-71-9) was added to a trifluoroacetic acid/DCM solution (10 mL, volume ratio 2:1). The reaction mixture was reacted at room temperature for 3 hours, distilled and concentrated to remove dichloromethane and most of the trifluoroacetic acid, and then crystallized by methyl tert-butyl ether, and centrifuged and dried to obtain 295 mg of crude 5-carboxyl-isodihydroindole as an off-white solid.

(2-2) Synthesis of Compound VWT021401

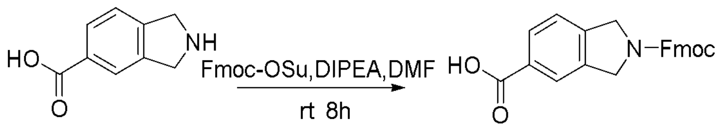


Compound 5-carboxyl-isodihydroindole (295 mg), compound N-(9-fluorenylmethoxycarbonyloxy)succinimide (596 mg, CAS No.: 82911-69-1), and DIPEA (470 mg) were added to DMF (4 mL), and the reaction mixture was reacted at room temperature for 8 hours. The solid was filtered off, and the filtrate was concentrated under reduced pressure to obtain a residue, which was purified by prep-HPLC to obtain compound VWT024101 (150 mg, yield 80%; CAS No.: 149353-71-9) as a white solid.

(2-3) Preparation of compound VWT021402

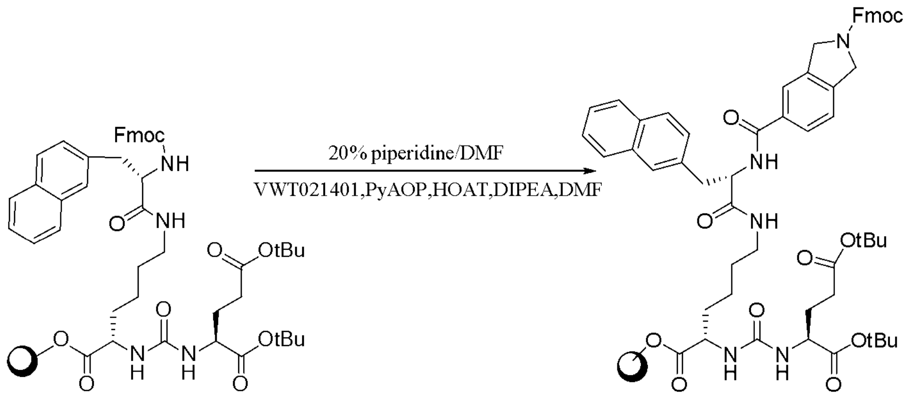


VWT016100 VWT021402

VWT016100 resin (0.2 mmol) was swollen in DMF (5 mL) for 30 minutes, and washed with DMF (5 mL), then treated with 20% piperidine/DMF (5 mL) for 30 minutes to remove the Fmoc protecting group, and washed with DMF (40 mL) again. The reaction was then treated with a mixture of VWT021401 (265 mg), PyAOP (320 mg), HOAT (95 mg), and DIPEA (160 mg) in DMF (6 mL) for 24 hours, and the resin was washed with DMF (30 mL) to obtain compound VWT021402.

(2-4) Preparation of compound VWT021403

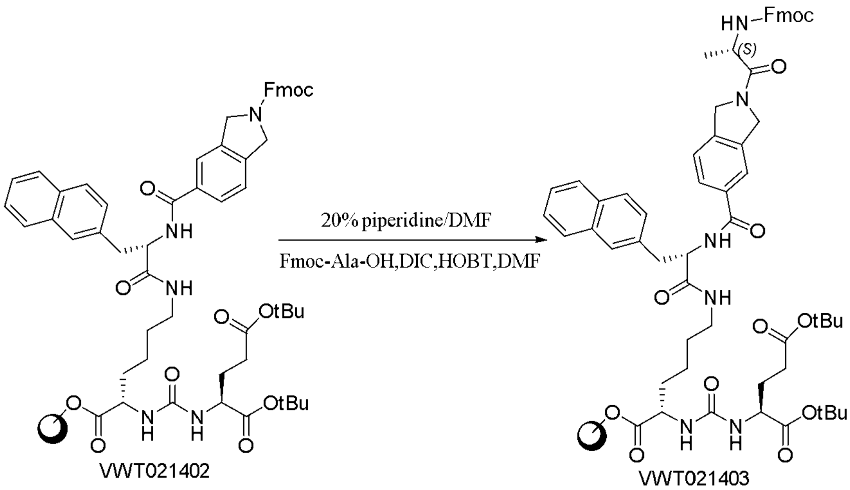


VWT021402 VWT021403

VWT021402 resin (0.2 mmol) was swollen in DMF (5 mL) for 30 minutes, and washed with DMF (5 mL), and then treated with 20% piperidine/DMF (5 mL) for 30 minutes to remove the Fmoc protecting group, and then washed with DMF (40 mL) again. The reaction was treated with a mixture of Fmoc-Ala-OH (310 mg), DIC (130 mg), and HOBT (155 mg) in DMF (3 mL) for 4 hours, and the resin was then washed with DMF (30 mL) to obtain compound VWT021403.

(2-5) Preparation of compound VWT021404

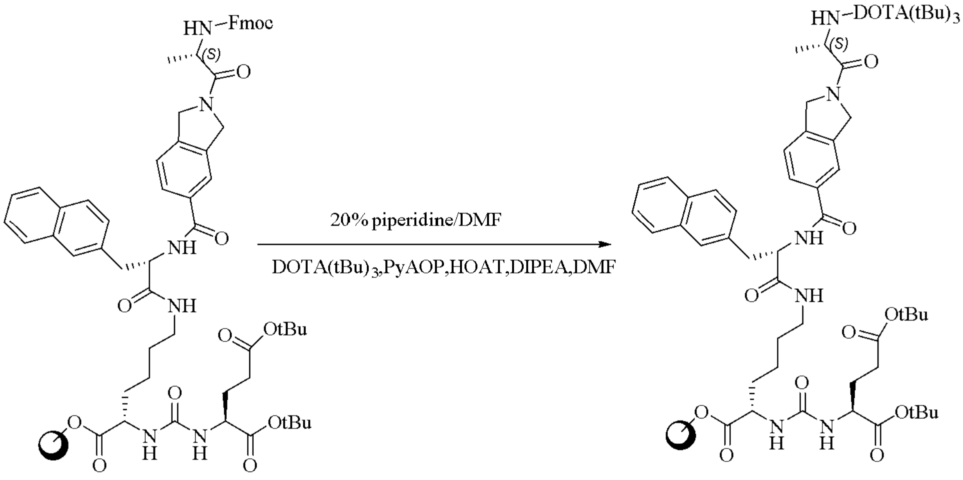


VWT021403 VWT021404

VWT021403 resin (0.2 mmol) was treated with 20% piperidine/DMF (3 mL) for 30 min to remove the Fmoc protecting group, and then washed with DMF (24 mL). The reaction was then treated with a mixture of DOTA(tBu)$_3$ (230 mg), PyAOP (210 mg), HOAT (60 mg), and DIPEA (105 mg) in DMF (3 mL) for 5 h, and the resin was washed with DMF (18 mL) to obtain compound VWT021404.

(2-6) Preparation of compound VWT021400

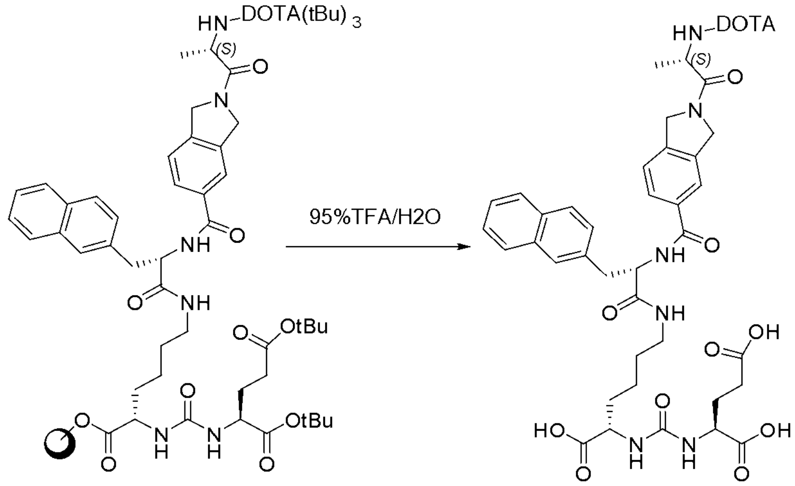


VWT021404 VWT021400

VWT021404 was treated in 95% TFA/5% $H_2O$ Cleavage Cocktail (4.5 mL) for 3 hours, crystallized with methyl tert-butyl ether, and centrifuged and dried to obtain 157 mg of crude VWT021400 as a pale red solid, which was further purified by preparative HPLC to obtain 37 mg of the target product VWT021400 with a purity of 96.81%; LCMS: $[M+H]^+$ = 1119.90.

## Example 6

Preparation of compound VWT021700

Step 1 (Synthesis of VWT016100)

VWT016100 was prepared using the same method as in Example 1.

Step 2 (preparation of VWT021702)

(2-1) Synthesis of Compound 2

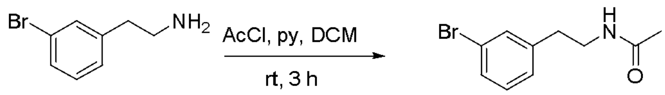


1 2

Acetyl chloride (3.2 g) and pyridine (50 mL) were added sequentially to a solution of compound 3-bromophenylethylamine 1 (8.0 g) in dichloromethane (50 mL) at 0 °C. The reaction mixture was reacted at room temperature for 3 hours, filtered, and the filtrate was concentrated to obtain compound 2 (9.6 g, N-(3-bromophenylethyl)acetamide, 99%) as a brown liquid.

(2-2) Synthesis of Compound 3

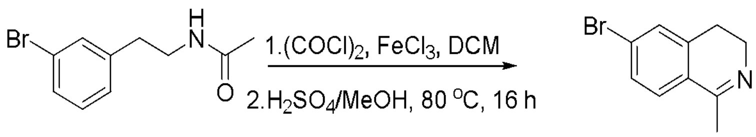


2 3

Oxaloyl chloride (55.4 g) was added to a solution of compound 2 (9.6 g) in dichloromethane (200 mL) at 0 °C. The reaction mixture was reacted at room temperature for 2 hours. The temperature of the reaction mixture was then lowered to -78 °C, and ferric chloride (38.6 g) was added. The reaction mixture was warmed to room temperature and stirred for another 16 hours and filtered, and the filtrate was concentrated. Then, a sulfuric acid/methanol solution (100 mL, v/v ratio 1:10; sulfuric acid concentration 98%) was added, and the reaction mixture was reacted at 80 °C for another 16 hours. The reaction mixture was then concentrated, and added with water (300 mL). The pH was adjusted to 7 with ammonia. The solid was filtered off, and the filtrate was extracted with dichloromethane. The organic phases were combined and concentrated to obtain a residue, which was purified by silica gel column chromatography (v/v ratio: dichloromethane/-methanol = 50/1) to obtain compound 3 (378 mg, 6-bromo-1-methyl-3,4-dihydroisoquinoline, yield 4%) as a brown liquid.

(2-3) Synthesis of Compound 4

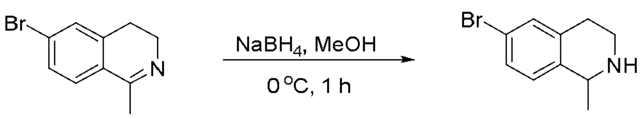


3 4

Sodium borohydride (64 mg) was added to a solution of compound 3 (378 mg) in methanol (10 mL) at 0 °C. The reaction mixture was reacted at 0 °C for 1 hour, and concentrated to obtain a residue, which was purified by silica gel column chromatography (volume ratio: dichloromethane/methanol = 20/1) to obtain compound 4 (328 mg, 6-bromo-1-methyl-1,2,3,4-tetrahydroisoquinoline, 86%) as a colorless liquid.

(2-4) Synthesis of Compound 5

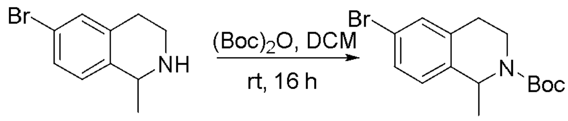


4 5

Di-tert-butyl dicarbonate (475 mg) and triethylamine (293 mg) were added to a solution of compound 4 (328 mg) in dichloromethane (10 mL) at room temperature. The reaction mixture was reacted at room temperature for 16 hours, then concentrated to obtain a residue, which was purified by silica gel column chromatography (volume ratio: petroleum ether/ethyl acetate = 4/1) to obtain compound 5 (380 mg, tert-butyl 6-bromo-1-methyl-3,4-dihydroisoquinoline-2(1H)-carboxylate, 80%) as a colorless liquid.

(2-5) Synthesis of Compound 6

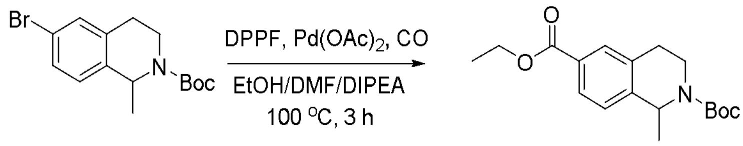


5 6

Compound 5 (280 mg), DPPF (95 mg), and $Pd(OAc)_2$ (19 mg) were added to a DMF-EtOH-DIPEA solution (10 mL, DMF:EtOH:DIPEA = 8:2:1, v/v). The reaction mixture was reacted at 100 °C for 3 hours under a CO atmosphere, then added with ethyl acetate (100 mL), then washed with water and saturated brine, dried over anhydrous sodium sulfate, and concentrated to obtain a residue, which was purified by silica gel column chromatography (v/v: petroleum ether/ethyl acetate = 2/1) to obtain compound 6 (155 mg, ethyl 2-(tert-butyl)-6-ethyl-1-methyl-3,4-dihydroisoquinoline-2,6(1H)-dicarboxylate, 57%) as a colorless liquid.

(2-6) Synthesis of Compound 7

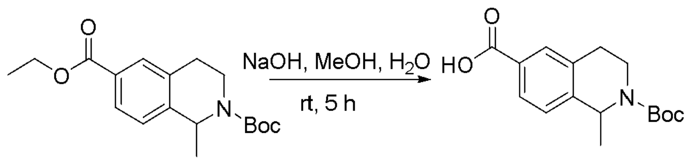


6 7

131 mg of sodium hydroxide (dissolved in 5 mL of water) was added to a solution of compound 6 (210 mg) in methanol (5 mL). The reaction mixture was reacted at room temperature for 5 hours, and adjusted to pH 7 with 1N hydrochloric acid, and then extracted with ethyl acetate. The organic phases were combined, washed with saturated brine, dried over anhydrous sodium sulfate, and concentrated to obtain compound 7 (191 mg, 2-(tert-butoxycarbonyl)-1-methyl-1,2,3,4-tetrahydroisoquinoline-6-carboxylic acid, 100%) as a white solid.

(2-7) Synthesis of Compound 8

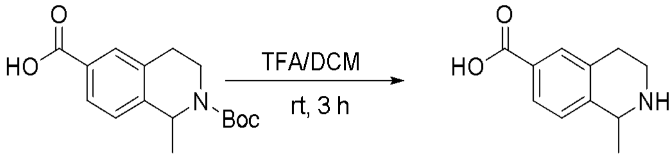


7 8

Compound 7 (191 mg) and trifluoroacetic acid (2 mL) were added to dichloromethane (5 mL). The reaction mixture was reacted at room temperature for 3 hours, and concentrated to obtain compound 8 (124 mg, 1-methyl-1,2,3,4-tetrahydroisoquinoline-6-carboxylic acid, 100%) as a colorless liquid.

(2-8) Synthesis of Compound VWT021702

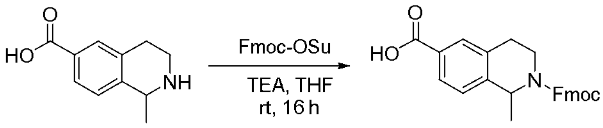


8 VWT021702

Compound 8 (124 mg), N-(9-fluorenylmethoxycarbonyl)succinimide (328 mg), and triethylamine (196 g) were added to tetrahydrofuran (10 mL). The reaction mixture was reacted at room temperature for 16 hours, concentrated and purified by silica gel column chromatography (volume ratio: dichloromethane/methanol = 10/1) to obtain compound VWT021702 (178.2 mg, 2-(((9H-fluoren-9-yl)methoxy)carbonyl)-1-methyl-1,2,3,4-tetrahydroisoquinoline-6-carboxylic acid, 66%) as a white solid.

Step 3 (preparation of VWT021703)

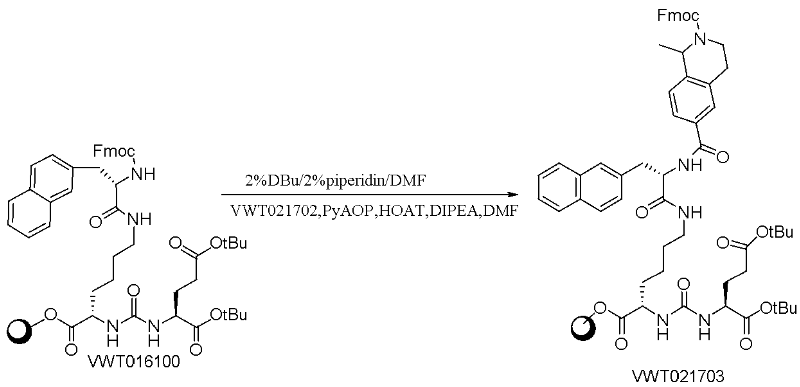


VWT016100 VWT021703

VWT016100 resin (0.15 mmol) was swollen in DMF (5 mL) for 30 minutes, and washed twice with DMF (5 mL), then treated with 2% DBu/2% piperidine/DMF (5 mL; DBu CAS No.: 6674-22-2) for 30 minutes to remove the Fmoc protecting group, and then washed with DMF (40 mL) again. The reaction was then treated with a mixture of VWT021702 (82 mg), PyAOP (115 mg), HOAT (40 mg), and DIPEA (65 mg) in DMF (3 mL) for 5 hours, and the resin was washed with DMF (30 mL) to obtain compound VWT021703.

Step 4 (preparation of VWT021704)

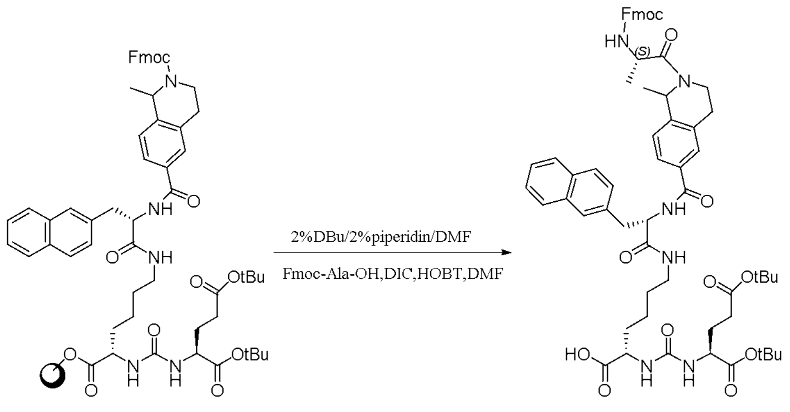


VWT021703 VWT021704

VWT021703 resin (0.15 mmol) was swollen in DMF (5 mL) for 30 minutes and washed with DMF (5 mL), then treated with 2% DBu/2% piperidine/DMF (5 mL) for 30 minutes to remove the Fmoc protecting group, then washed with DMF (40 mL). The reaction was treated with a mixture of Fmoc-Ala-OH (233 mg, CAS No.: 35661-39-3), DIC (95 mg), HOBT (115 mg) in DMF (3 mL) for 4 hours, and the resin was washed with DMF (18 mL) to obtain compound VWT021704.

Step 5 (preparation of VWT021705)

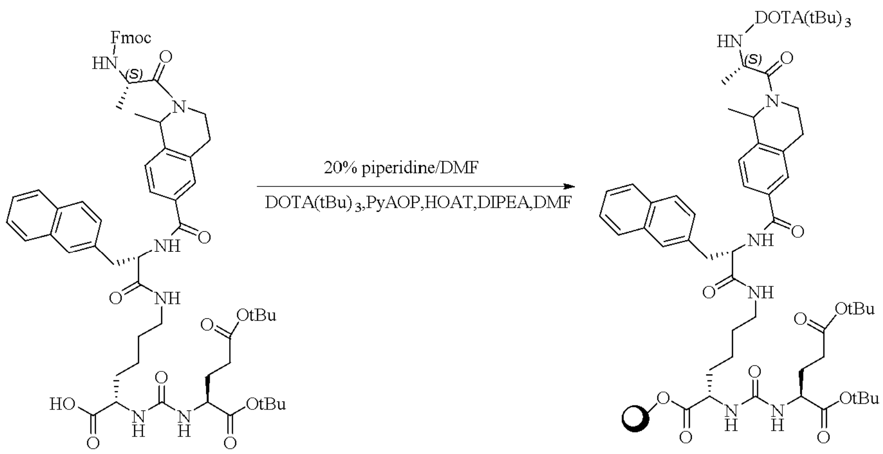


VWT021704 VWT021705

VWT021704 resin (0.15 mmol) was treated with 20% piperidine/DMF (3 mL) for 30 min to remove the Fmoc protecting group, and then washed with DMF (24 mL). The reaction was then treated with a mixture of DOTA(tBu)$_3$ (250 mg), PyAOP (230 mg), HOAT (60 mg), and DIPEA (115 mg) in DMF (3 mL) for 5 h, and the resin was washed with DMF (18 mL) to obtain compound VWT021705.

Step 6 (preparation of VWT021700

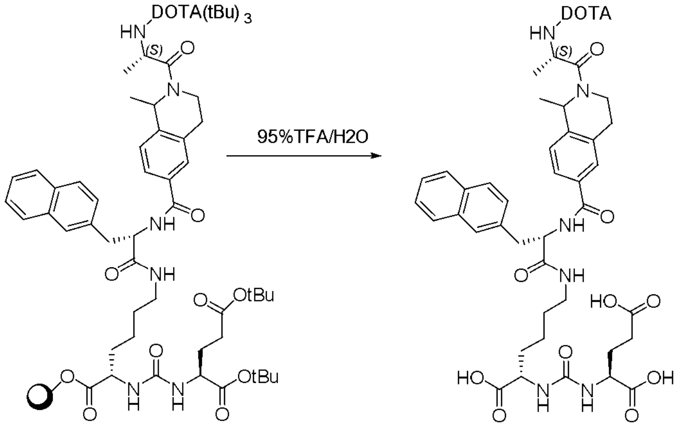


VWT021705 VWT021700

VWT021705 was treated in 95% TFA/5% $H_2O$ Cleavage Cocktail (4 mL) for 5 hours, crystallized with methyl tert-butyl ether, and centrifuged and dried to obtain 177 mg of crude VWT021700 as a white solid, which was further purified by preparative HPLC to obtain 4 mg of the target product VWT021700 with a purity of 97.01%; LCMS: $[M+H]^+$= 1147.60.

Example 7

Preparation of compound VWT022100

Step 1 (preparation of VWT016100)

VWT016100 was prepared using the same method as in Example 1.

Step 2 (preparation of VWT022102)

(2-1) Synthesis of Compound 3

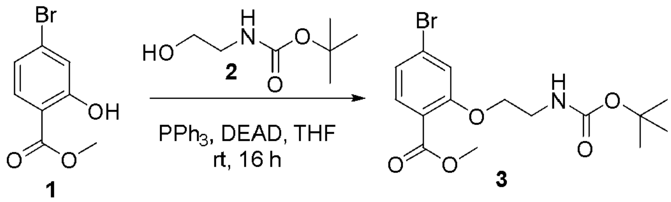


1 3

Compound 1 (5 g, CAS No.: 22717-56-2), compound 2 (3.48 g, N-Boc-ethanolamine, CAS No.: 26690-80-2), and triphenylphosphine (6.23 g) were added to tetrahydrofuran (50 mL). The reaction mixture was added with DEAD (diethyl azodicarboxylate: 4.8 g), and reacted at room temperature for 16 hours, then concentrated to obtain a residue, which was purified by silica gel column chromatography (volume ratio: petroleum ether/ethyl acetate = 10/1) to obtain compound 3 (8.12 g, methyl 4-bromo-2-(2-((tert-butoxycarbonyl)amino)ethoxy)benzoate) as a yellow oily liquid.

(2-2) Synthesis of Compound 4

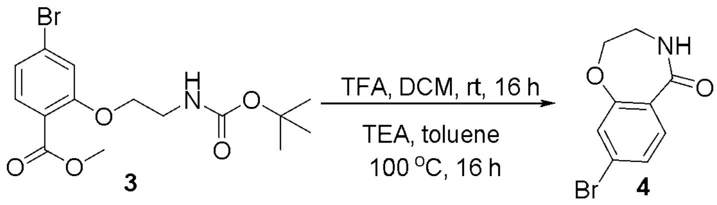


3

4

Compound 3 (8.12 g) was dissolved in dichloromethane (50 mL), and trifluoroacetic acid (9 mL) was added to the reaction mixture. The reaction mixture was reacted at room temperature for 16 hours, and concentrated to obtain a residue. Toluene was added to the residue, and the reaction mixture was concentrated under reduced pressure, then dissolved in toluene again, and added with triethylamine (35 g). The reaction mixture was reacted at 100 °C for 16 hours, cooled to room temperature, and concentrated to obtain a residue, which was purified by silica gel column chromatography (volume ratio: petroleum ether/ethyl acetate = 1/2) to obtain compound 4 (4.2 g, 8-bromo-3,4-dihydrobenzo[F][1,4] oxazepine-5(2H)-one, 79.3%) as a white solid.

(2-3) Synthesis of Compound 5

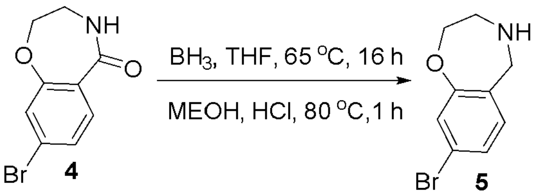


4

5

Compound 4 (4.2 g) was dissolved in tetrahydrofuran (30 mL). Under nitrogen protection, a 1 mol/L borane tetrahydrofuran solution (104 mL) was slowly added to the reaction mixture. The reaction mixture was reacted at 65 °C for 16 hours under nitrogen, then added with methanol (20 mL), followed by 2 mol/L hydrochloric acid (100 mL). The reaction mixture was reacted at 80 °C for 1 hour and concentrated, and water (100 mL) and ethyl acetate (100 mL) were added to the residue. The residue was separated, and the pH of the aqueous phase was adjusted to weakly alkaline with sodium hydroxide. The mixture was extracted three times with ethyl acetate (3 × 100 mL). The organic phase was concentrated to obtain compound 5 (3.56 g, 8-bromo-2,3,4,5-tetrahydrobenzo[f][1,4]oxazepine, 89.1%) as a colorless oily liquid.

(2-4) Synthesis of Compound 6

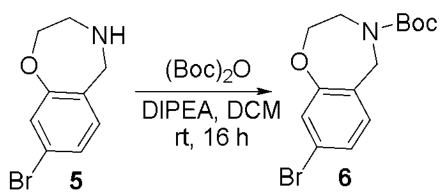


5

6

Compound 5 (3.56 g) and di-tert-butyl dicarbonate (5.1 g) were dissolved in dichloromethane (200 mL). The reaction mixture was added with DIPEA (4.02 g), and reacted at room temperature for 16 hours, concentrated to obtain a residue, which was purified by silica gel column chromatography (volume ratio: petroleum ether/ethyl acetate = 15/1) to obtain compound 6 (5.1 g, tert-butyl 8-bromo-2,3-dihydrobenzo[f][1,4]oxazepine-4(5H)-carboxylate, 98.1%) as a white solid.

(2-5) Synthesis of Compound 7

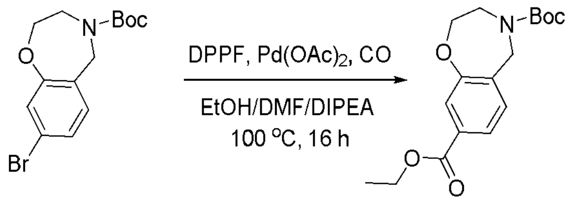


6 7

Compound 6 (5.1 g) was dissolved in an EtOH/DMF/DIPEA solution (200 mL, volume ratio: EtOH/DMF/DIPEA = 2/8/1; EtOH refers to ethanol). The reaction mixture was added with DPPF (1.72 g) and palladium acetate (0.348 g), and reacted at 100°C for 16 hours under carbon monoxide conditions, then added with water (200 mL), and extracted with ethyl acetate (3 × 200 mL). The organic phases were combined, washed with saturated brine (200 mL), dried over anhydrous sodium sulfate, and concentrated to obtain a residue, which was purified by silica gel column chromatography (volume ratio: petroleum ether/ethyl acetate = 10/1) to obtain compound 7 (1.2 g, 4-(tert-butyl)-8-ethyl 2,3-dihydrobenzo[f][1,4] oxazepine-4,8(5H)-dicarboxylate, 23.9%) as a green oily liquid.

(2-6) Synthesis of Compound 8

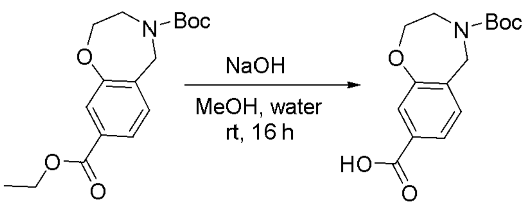


7 8

Compound 7 (1.2 g) was dissolved in methanol (30 mL), and then added with sodium hydroxide (1.48 g) dissolved in water (20 mL). The reaction mixture was reacted at room temperature for 16 hours, concentrated under reduced pressure, and adjusted to acidic pH with 1 N hydrochloric acid, then extracted with ethyl acetate, and the organic phase was concentrated to obtain compound 8 (1 g, 4-(tert-butoxycarbonyl)-2,3,4,5-tetrahydrobenzo[f] [1,4] oxazepine-8-carboxylic acid, 91.9%) as a white solid.

(2-7) Synthesis of Compound 9

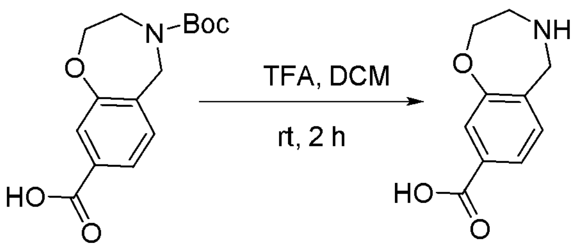


8 9

Compound 8 (1 g) was dissolved in dichloromethane (20 mL), and trifluoroacetic acid (5 mL) was added. The reaction mixture was reacted at room temperature for 2 hours, and then concentrated under reduced pressure to obtain compound 9 (0.6 g, 2,3,4,5-tetrahydrobenzo[f][1,4]oxazepine-8-carboxylic acid, 91.2%) as a white solid.

(2-8) Synthesis of Compound VWT022102

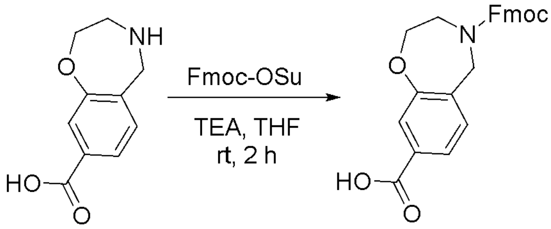


9 VWT022102

Compound 9 (0.6 g) and Fmoc-OSu (1.6 g) were dissolved in tetrahydrofuran (50 mL), and then triethylamine (0.9 g) was added. The reaction mixture was reacted at room temperature for 2 hours, and concentrated under reduced pressure to obtain a residue, which was purified by silica gel column chromatography (volume ratio: dichloromethane/-methanol = 20/1) to obtain a crude product. The crude product was slurried with dichloromethane, filtered, and dried to obtain compound VWT022102 (0.96 g, 4-(9-fluorenylmethoxycarbonyloxy)-2,3,4,5-tetrahydrobenzo[f][1,4]oxazepine-8-carboxylic acid, 74.4%) as a white solid.

Step 3 (preparation of VWT022103)

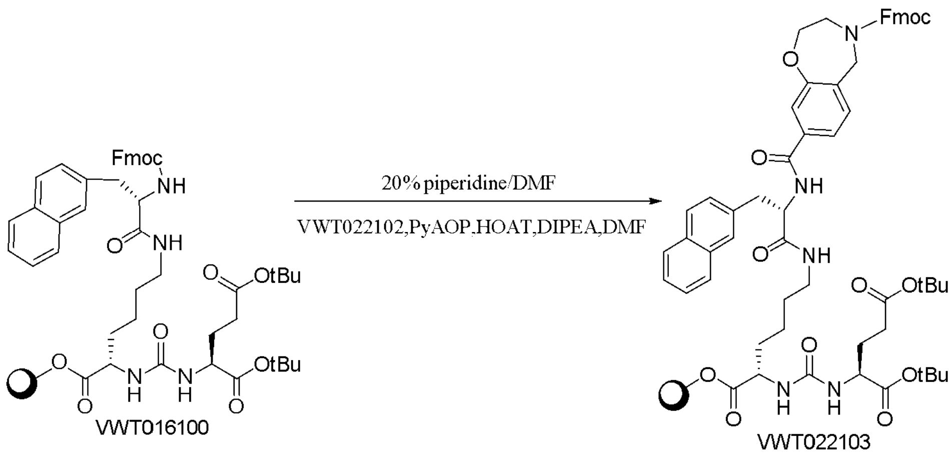


VWT016100 VWT022103

VWT016100 resin (0.12 mmol) was swollen in DMF (5 mL) for 30 minutes, then washed with DMF (5 mL), and then treated with 20% piperidine/DMF (5 mL) for 30 minutes to remove the Fmoc protecting group, then washed with DMF (40 mL). The reaction was treated with a mixture of VWT022102 (75 mg), PyAOP (95 mg), HOAT (25 mg), and DIPEA (50 mg) in DMF (1.5 mL) for 3.5 hours, and the resin was washed with DMF (18 mL) to obtain compound VWT022103.

Step 4 (preparation of VWT022104)

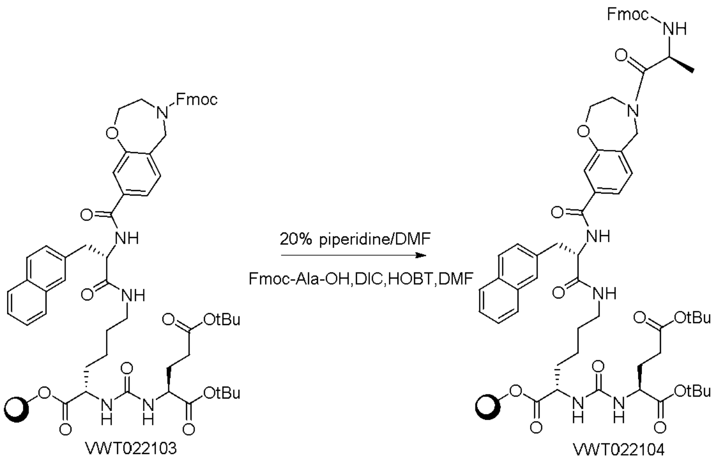


VWT022103 VWT022104

VWT022103 resin (0.12 mmol) was swollen in DMF (5 mL) for 30 minutes, then washed with DMF (5 mL), and then treated with 20% piperidine/DMF (5 mL) for 30 minutes to remove the Fmoc protecting group, then washed with DMF (40 mL). The reaction was treated with a mixture of Fmoc-Ala-OH (190 mg), DIC (80 mg), and HOBT (95 mg) in DMF (1.5 mL) for 3 hours, and the resin was washed with DMF (18 mL) to obtain compound VWT022104.

Step 5 (preparation of VWT022105)

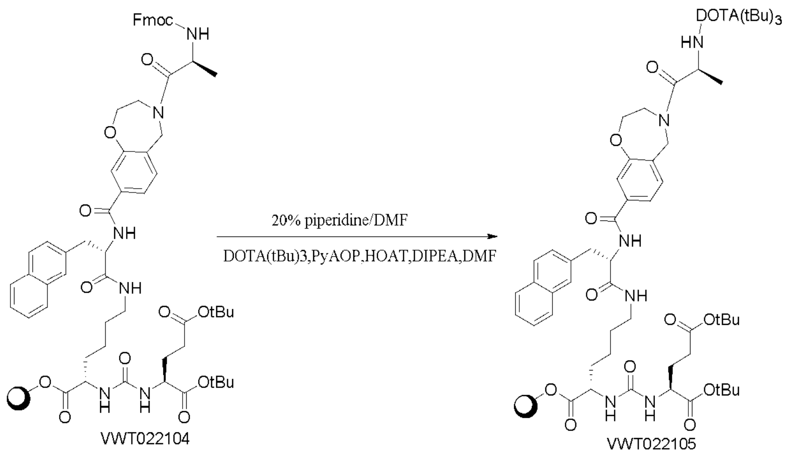


VWT022104 VWT022105

VWT022104 resin (0.12 mmol) was treated with 20% piperidine/DMF (5 mL) for 30 min to remove the Fmoc protecting group, then washed with DMF (40 mL). The reaction was treated with a mixture of DOTA$(tBu)_3$ (210 mg), PyAOP (190 mg), HOAT (50 mg), and DIPEA (95 mg) in DMF (1.5 mL) for 5 h, and the resin was washed with DMF (18 mL) to obtain compound VWT022105.

Step 6 (preparation of VWT022 100)

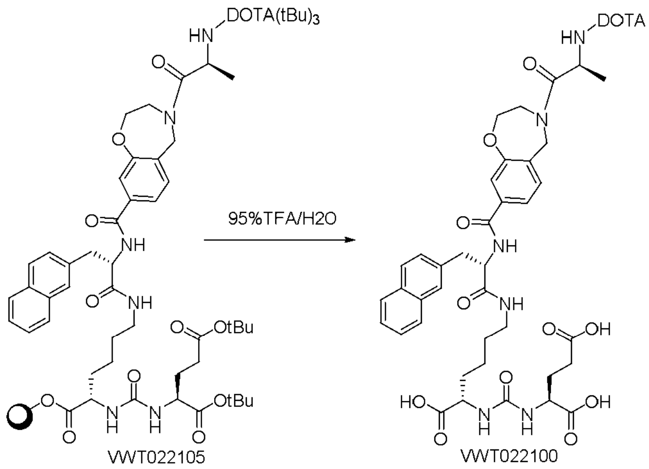


VWT022105 was treated in 95% TFA/5% $H_2O$ Cleavage Cocktail (5.0 mL) for 3 hours, crystallized with methyl tert-butyl ether, and centrifuged and dried to obtain 227 mg of crude VWT022100 as a white solid, which was further purified by preparative HPLC to obtain 53 mg of the target product VWT022100 with a purity of 97.66%; LCMS: $[M+H]^+$ = 1149.70.

**Example 8**

Preparation of compound VWT024000

Step 1 (preparation of VWT016100)

VWT016100 was prepared using the same method as in Example 1.

Step 2 (preparation of VWT011601)

VWT011601 was prepared using the same method as in Example 4.

Step 3 (preparation of VWT024001)

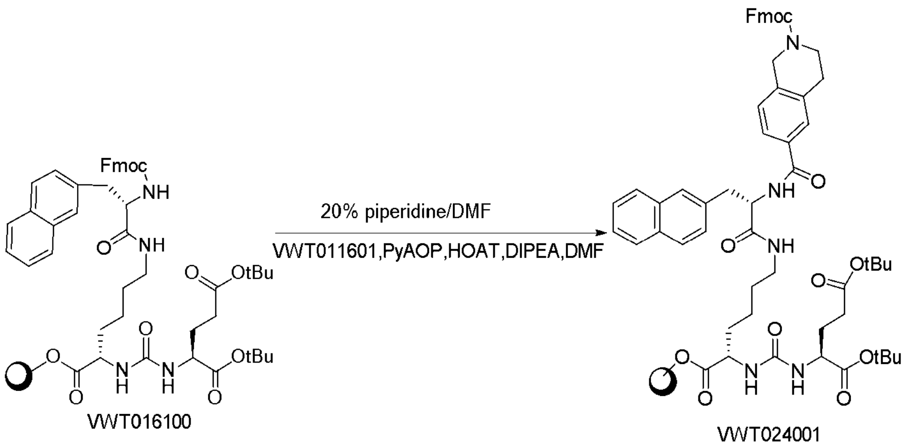


VWT016100 resin (0.2 mmol) was swollen in DMF (5 mL) for 30 minutes, then washed with DMF (5 mL), and then treated with 20% piperidine/DMF (5 mL) for 30 minutes to remove the Fmoc protecting group, and then washed with DMF (40 mL). The reaction was treated with a mixture of VWT011601 (100 mg), PyAOP (125 mg), HOAT (35 mg), and DIPEA (70 mg) in DMF (3 mL) for 3.5 hours, and the resin was washed with DMF (30 mL) to obtain compound VWT024001.

Step 4 (preparation of VWT024002)

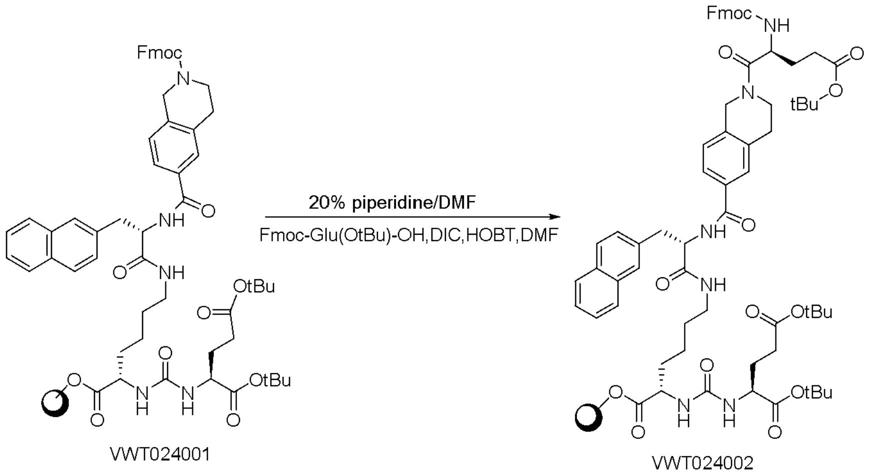


VWT024001 resin (0.2 mmol) was swollen in DMF (5 mL) for 30 minutes, then washed with DMF (5 mL), and then treated with 20% piperidine/DMF (5 mL) for 30 minutes to remove the Fmoc protecting group, and then washed with DMF (40 mL). The reaction was then treated with a mixture of Fmoc-Glu(OtBu)-OH (425 mg, CAS No.: 71989-18-9), DIC (130 mg), and HOBT (155 mg) in DMF (3 mL) for 2 hours, and the resin was washed with DMF (30 mL) to obtain compound VWT024002.

Step 5 (preparation of VWT024003)

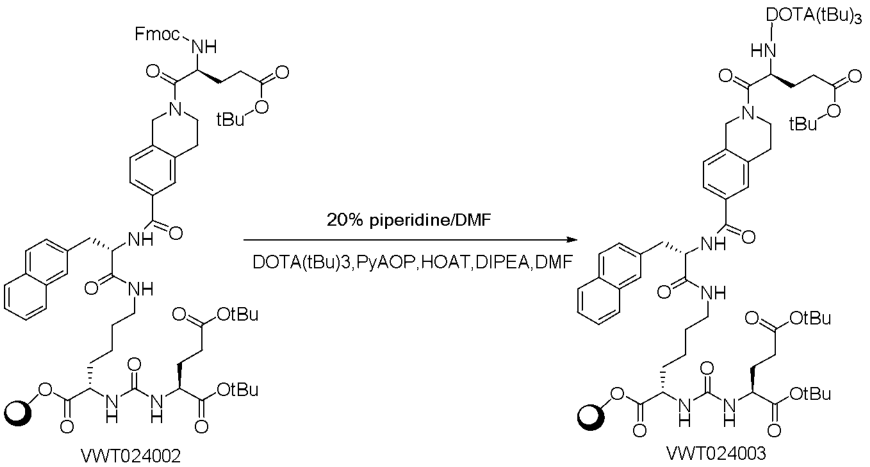


VWT024002 resin (0.2 mmol) was treated with 20% piperidine/DMF (3 mL) for 30 min to remove the Fmoc protecting group, and then washed with DMF (24 mL). The reaction was then treated with a mixture of DOTA(tBu)$_3$ (230 mg), PyAOP (210 mg), HOAT (60 mg), and DIPEA (105 mg) in DMF (3 mL) for 5 h, and the resin was washed with DMF (18 mL) to obtain compound VWT024003.

Step 6 (preparation of VWT024000)

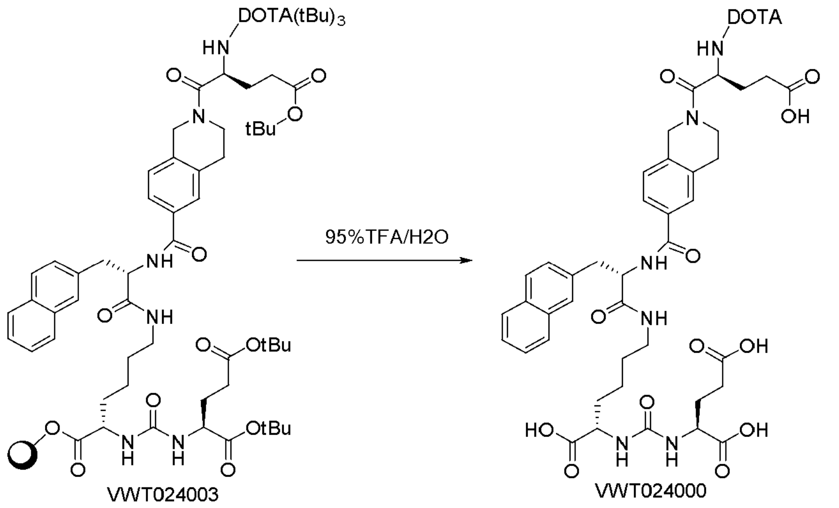


VWT024003 was treated in 95% TFA/5% $H_2O$ Cleavage Cocktail (6.5 mL) for 3 hours, crystallized with methyl tert-butyl ether, and centrifuged and dried to obtain 255 mg of crude VWT024000 as a white solid, which was further purified by preparative HPLC to obtain 110 mg of the target product VWT024000 with a purity of 92.65%; LCMS: $[M+H]^+$ = 1191.90.

Example 9

Preparation of compound VWT024100

Step 1 (preparation of VWT016100)

VWT016100 was prepared using the same method as in Example 1.

Step 2 (preparation of VWT024101)

VWT024101 (i.e., VWT024001) was prepared using the same method as in Example 8.

Step 3 (preparation of VWT024102)

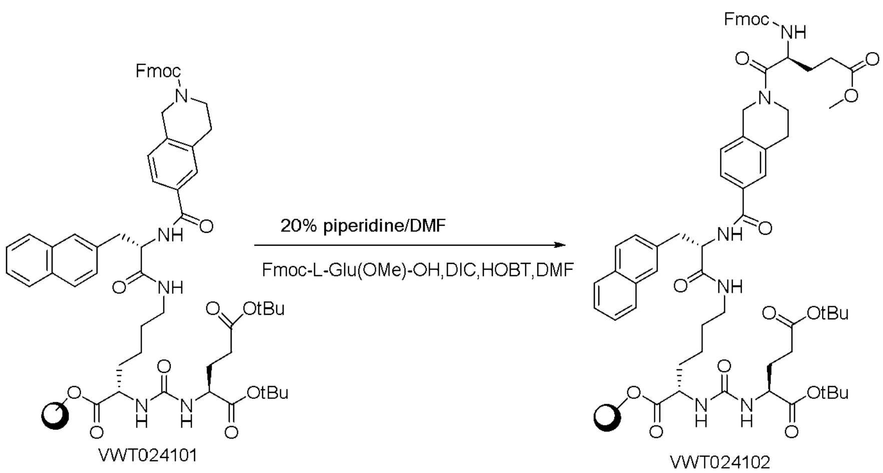


VWT024101 resin (0.2 mmol) was swollen in DMF (5 mL) for 30 minutes, then washed with DMF (5 mL), and then treated with 20% piperidine/DMF (5 mL) for 30 minutes to remove the Fmoc protecting group, and then washed with DMF (40 mL). The reaction was then treated with a mixture of Fmoc-L-Glu(OMe)-OH (380 mg, CAS No.: 145038-50-2), DIC (130 mg), and HOBT (155 mg) in DMF (3 mL) for 2 hours, and the resin was washed with DMF (30 mL) to obtain compound

VWT024102.

Step 4 (preparation of VWT024103)

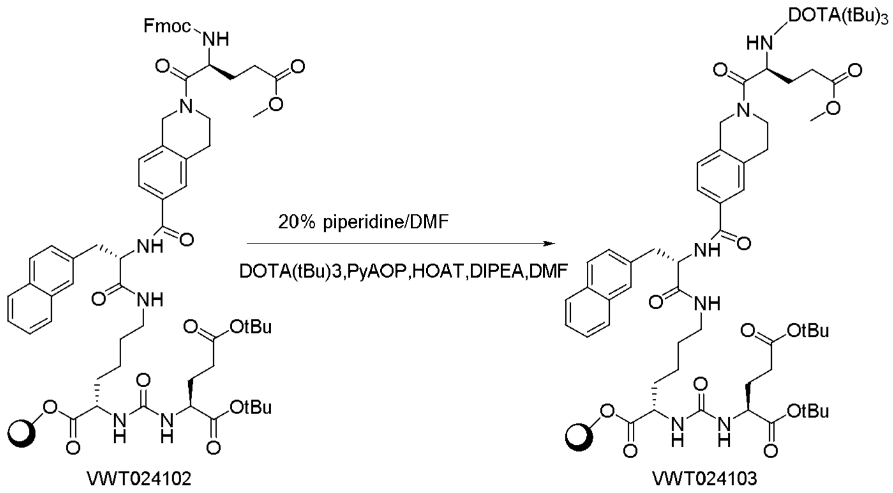


VWT024102 resin (0.2 mmol) was treated with 20% piperidine/DMF (3 mL) for 30 min to remove the Fmoc protecting group, and then washed with DMF (24 mL). The reaction was then treated with a mixture of DOTA(tBu)$_3$ (230 mg), PyAOP (210 mg), HOAT (60 mg), and DIPEA (105 mg) in DMF (3 mL) for 5 h, and the resin was washed with DMF (18 mL) to obtain compound VWT024103.

Step 5 (preparation of VWT024100)

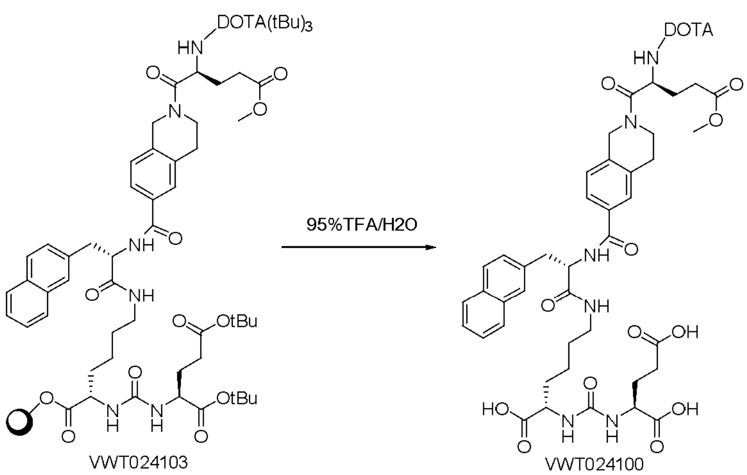


VWT024103 was treated in 95% TFA/5% $H_2O$ Cleavage Cocktail (5.5 mL) for 3 hours, crystallized with methyl tert-butyl ether, and centrifuged and dried to obtain 330 mg of crude VWT024100 as a white solid, which was further purified by preparative HPLC to obtain 90 mg of the target product VWT024100 with a purity of 97.18%; LCMS: [M+H]$^+$ = 1205.90.

**Example 10**

Preparation of compound VWT022600

Step 1 (preparation of VWT024001)

VWT024001 was prepared using the same method as in Example 8.

Step 2 (preparation of VWT022600)

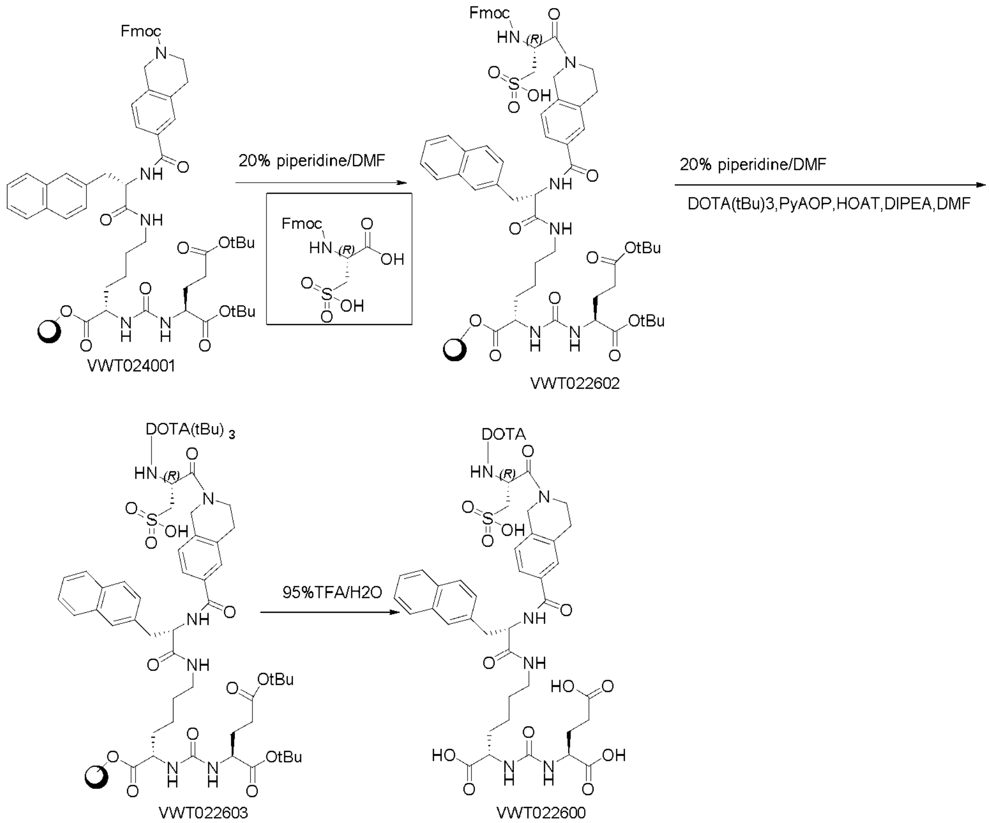


VWT024001 resin (0.22 mmol) was coupled sequentially in the order specified in the sequence with (((9H-fluorene-9-yl)methoxy)carbonyl)(sulfo)-D-alanine (CAS No.: 751470-47-0) under a PyAOP/HOAT/DIPEA condensation system. The resin was then washed with DMF, coupled with compound DOTA$(tBu)_3$ under the PyAOP/HOAT/DIPEA condensation system, and washed with DMF to obtain VWT022603.

VWT022603 was then treated in 95% TFA/5% $H_2O$ Cleavage Cocktail (4 mL) for 4 hours, crystallized with methyl tert-butyl ether, and centrifuged and dried to obtain crude VWT022600, which was then purified by preparative HPLC to obtain 39.5 mg of the target product VWT022600 with a purity of 96.90%; LCMS: $[M+H]^+$ =1213.70.

**Example 11**

Preparation of compound VWT023800

Step 1 (preparation of VWT023803)

(1-1) Synthesis of VWT023801

VWT023801 (i.e., VWT016101) was prepared using the same method as in Example 1.

(1-2) Preparation of derivatives of L-dimethyl glutamate

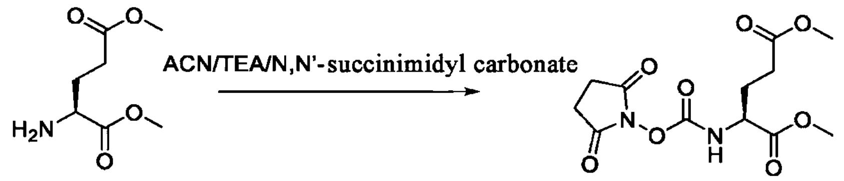


L-dimethyl glutamate hydrochloride (1.06 g) was added to anhydrous acetonitrile (30 mL) and stirred until dissolved. DSC (1.30 g) was added at room temperature with continuous stirring, and triethylamine (1.0 g) was slowly added dropwise. After the addition was complete, the reaction was allowed to proceed overnight at room temperature. The mixture was then separated, and the organic phase was washed with 10% citric acid aqueous solution and saturated brine, then concentrated and distilled to obtain a derivative of L-dimethyl glutamate (1.95 g): dimethyl N-(((2,5-dioxopyrrolidin-1-yl)oxy)carbonyl)-L-glutamate.

(1-3) preparation of VWT023803

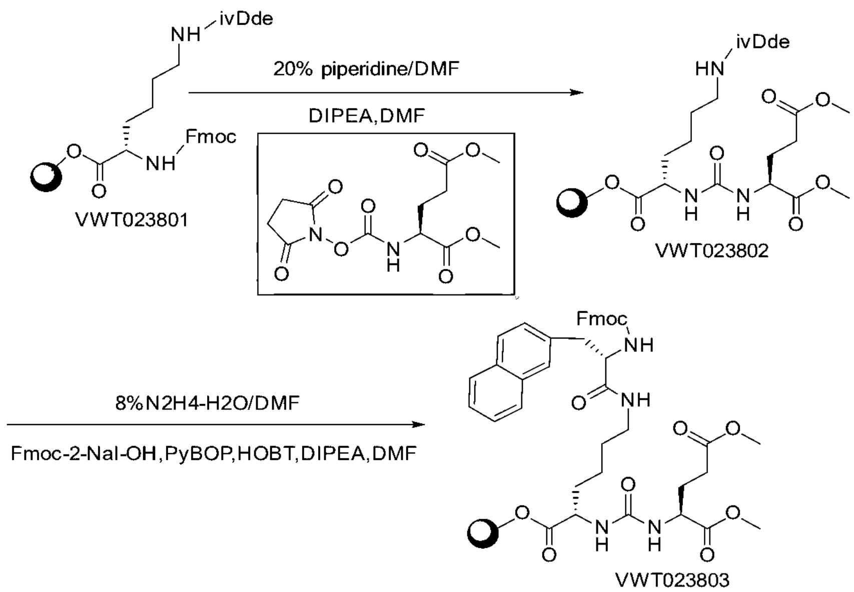


VWT023801 resin (0.5 mmol) was treated with 20% piperidine/DMF (5 mL) for 30 min, then washed with DMF (4 mL); and then coupled with the derivative of L-dimethyl glutamate (dimethyl N-(((2,5-dioxopyrrolidin-1-yl)oxy)carbonyl)-L-glutamate: 1.85 g) sequentially in the order specified in the sequence under the DIPEA/DMF system in DMF (5 mL) for 2 h. The resin was washed with DMF (30 mL), then treated with 8% hydrazine hydrate/DMF solution for 0.5 h to remove the ivDde protecting group. The resin was washed with DMF (60 mL), and then coupled with Fmoc-2-Nal-OH under the PyBOP/HOBT/DIPEA condensation system for 3 h, and then washed with DMF (30 mL) to obtain compound VWT023803.

Step 2 (preparation of VWT011601)

VWT011601 was prepared using the same method as in Example 4.

Step 3 (preparation of VWT023800)

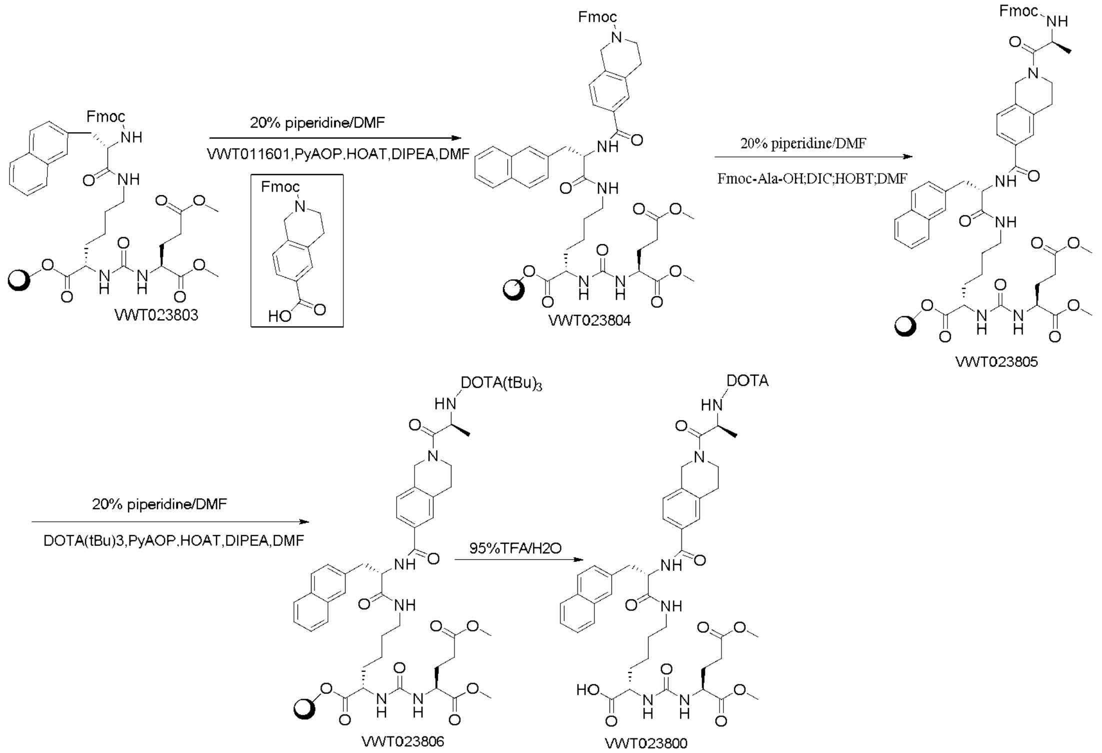


The intermediate VWT023803 resin (0.25 mmol) was treated with 20% piperidine/DMF (3 mL) for 30 minutes to remove the Fmoc protecting group, and washed with DMF (24 mL); then coupled with VWT011601 (CAS No.: 2138226-21-6) sequentially under the PyAOP/HOAT/DIPEA condensation system in the order specified in the sequence. The resin was then washed with DMF (24 mL), treated again with 20% piperidine/DMF (3 mL) for 30 minutes to remove the Fmoc protecting group, and then washed with DMF (24 mL); and then coupled with Fmoc-alanine under the DIC/HOBT condensation system to obtain compound VWT023805. The resin was then washed with 24 mL DMF, treated again with 20% piperidine/DMF (3 mL) for 30 minutes to remove the Fmoc protecting group, and then washed with DMF (24 mL); and then coupled with the compound DOTA(tBu)$_3$ under the PyAOP/HOAT/DIPEA condensation system. The resin was washed with DMF (24 mL) to obtain VWT023806.

VWT023806 was then treated in 95% TFA/5% $H_2O$ Cleavage Cocktail (6.5 mL) for 4 hours, crystallized with methyl tert-butyl ether, and centrifuged and dried to obtain crude VWT023800, which was then purified by preparative HPLC to obtain 41 mg of the target product VWT023800 with a purity of 94.60%; LCMS: $[M+H]^+$ = 1161.70.

**Example 12**

Preparation of compound VWT023900

Step 1 (preparation of VWT016100)

VWT016100 was prepared using the same method as in Example 1.

Step 2 (preparation of VWT023901)

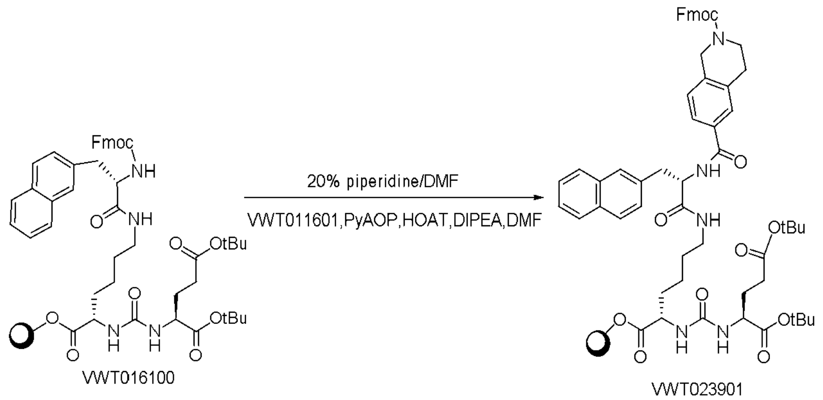


VWT016100

VWT023901

VWT016100 resin (0.2 mmol) was swollen in DMF (5 mL) for 30 minutes, then washed with DMF (5 mL), then treated with 20% piperidine/DMF (5 mL) for 30 minutes to remove the Fmoc protecting group, and then washed with DMF (40 mL). The reaction was then treated with a mixture of VWT011601 (100 mg), PyAOP (125 mg), HOAT (35 mg), and DIPEA (70 mg) in DMF (3 mL) for 5 hours, and the resin was washed with DMF (30 mL) to obtain compound VWT023901.

Step 3 (preparation of VWT023902)

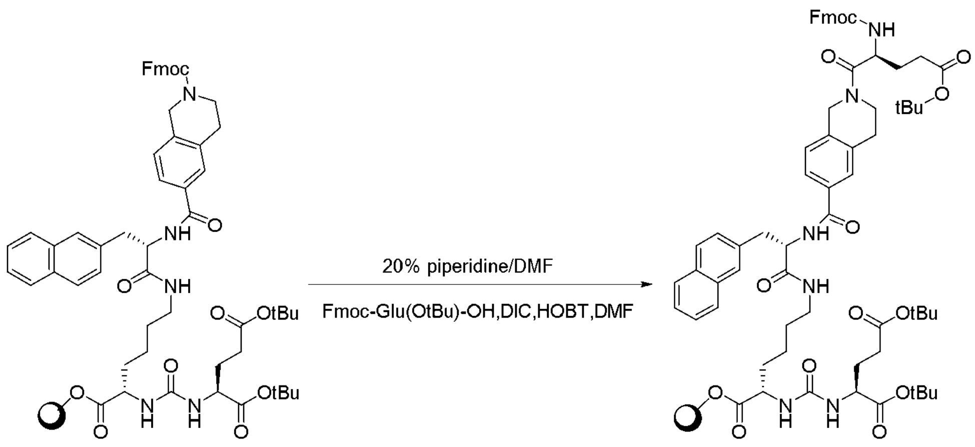


VWT023901

VWT023902

VWT023901 resin (0.2 mmol) was washed with DMF (5 mL), then treated with 20% piperidine/DMF (5 mL) for 30 min to remove the Fmoc protecting group, and then washed with DMF (40 mL). The reaction was treated with a mixture of Fmoc-Glu(OtBu)-OH (425 mg), DIC (130 mg), and HOBT (155 mg) in DMF (3 mL) for 2 h, and the resin was washed with DMF (30 mL) to obtain compound VWT023902.

Step 4 (preparation of VWT023903)

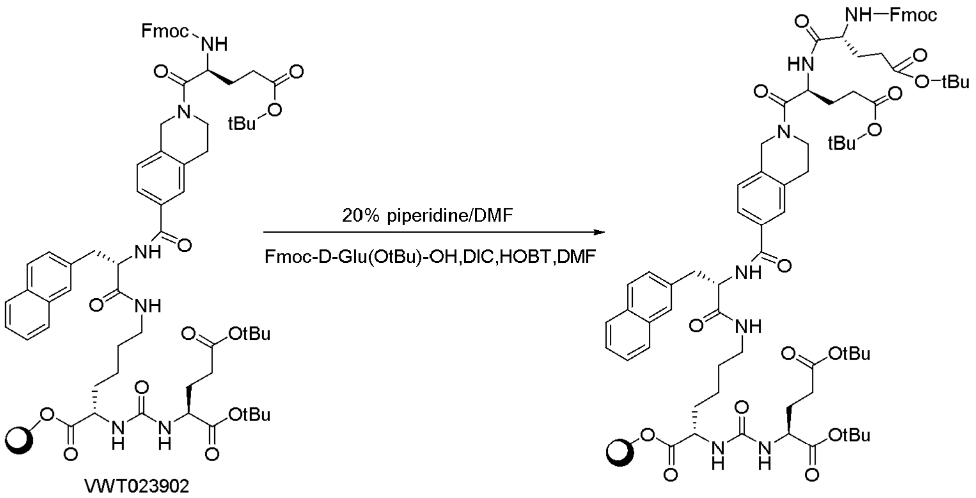


VWT023902 VWT023903

VWT023902 resin (0.2 mmol) was swollen in DMF (5 mL) for 30 minutes, then washed with DMF (5 mL), then treated with 20% piperidine/DMF (5 mL) for 30 minutes to remove the Fmoc protecting group, and then washed with DMF (40 mL). The reaction was then treated with a mixture of Fmoc-D-Glu(OtBu)-OH (425 mg, CAS No.: 104091-08-9), DIC (130 mg), and HOBT (155 mg) in DMF (3 mL) for 3 hours, and the resin was then washed with DMF (30 mL) to obtain compound VWT023903.

Step 5 (preparation of VWT023904)

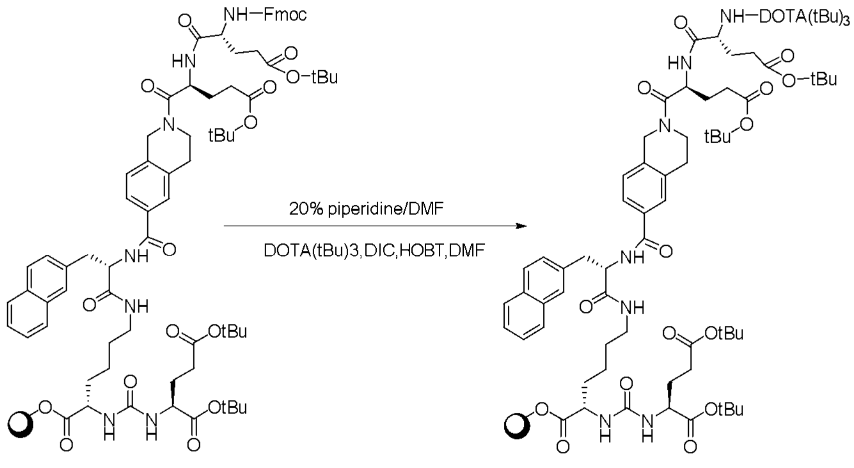


VWT023903 VWT023904

VWT023903 resin (0.2 mmol) was treated with 20% piperidine/DMF (3 mL) for 30 min to remove the Fmoc protecting group, and then washed with DMF (24 mL). The reaction was then treated with a mixture of $DOTA(tBu)_3$ (230 mg), DIC (55 mg), and HOBT (70 mg) in DMF (3 mL) for 5 h, and the resin was washed with DMF (18 mL) to obtain compound VWT023904.

Step 6 (preparation of VWT023900)

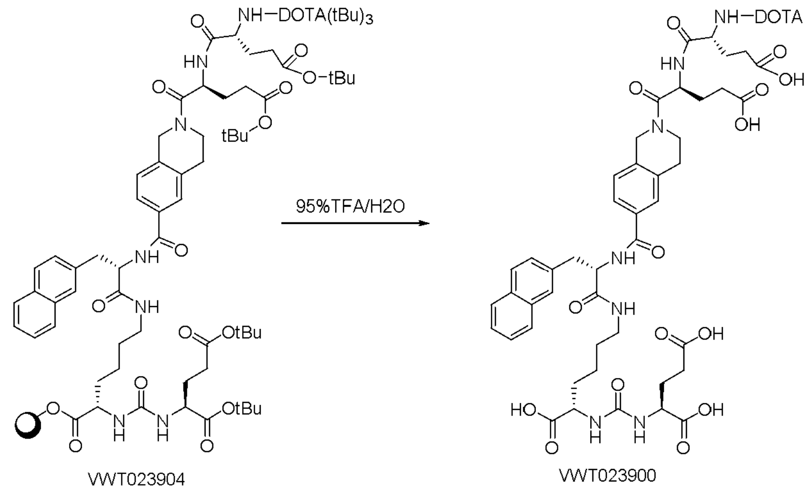


VWT023904 was treated in 95% TFA/5% $H_2O$ Cleavage Cocktail (5.5 mL) for 4 hours, crystallized with methyl tert-butyl ether, and centrifuged and dried to obtain 310 mg of crude VWT023900 as a white solid, which was further purified by preparative HPLC to obtain 105 mg of the target product VWT023900 with a purity of 94.97%; LCMS: $[M+H]^+$ =1320.70.

**Example 13**

Preparation of compound VWT023600

Step 1 (preparation of VWT016103)

VWT016103 was prepared using the same method as in Example 1.

Step 2 (preparation of VWT022502)

(2-1) Synthesis of Compound 2

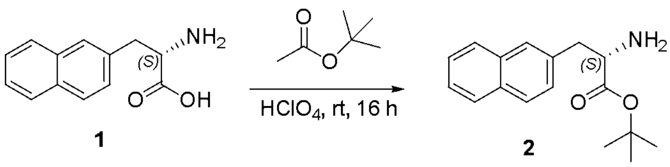


Perchloric acid (1 mL, 70% concentration) was added at room temperature to a solution of compound 1 ((S)-2-amino-3-(naphthalen-2-yl)propionic acid: 2.0 g) in tert-butyl acetate (30 mL). The reaction mixture was reacted at room temperature for 16 hours, added with ethyl acetate (200 mL), and washed with water and saturated brine, dried over anhydrous sodium sulfate, and concentrated to obtain a residue, which was purified by silica gel column chromatography (volume ratio: petroleum ether/ethyl acetate = 1/1) to obtain compound 2 (tert-butyl (S)-2-amino-3-(naphthalen-2-yl) propanoate: 1.55 g) as a colorless liquid.

(2-2) Synthesis of Compound 3

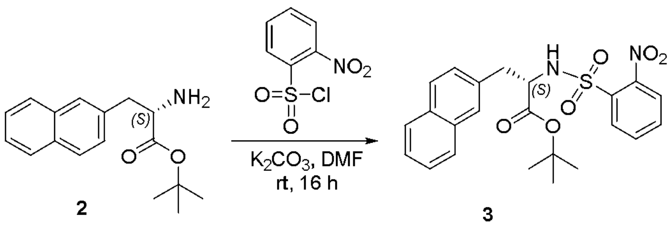


2 3

1.06 g of 2-nitrobenzenesulfonyl chloride and 1.02 g of potassium carbonate were added to a solution of compound 2 (1.0 g) in DMF (20 mL) at room temperature. The reaction mixture was reacted for 16 hours at room temperature, added with ethyl acetate (100 mL), then washed with water and saturated brine, dried over anhydrous sodium sulfate, and concentrated to obtain a residue, which was purified by silica gel column chromatography (volume ratio: petroleum ether/ethyl acetate = 3/1) to obtain compound 3 (tert-butyl (S)-3-(naphthalen-2-yl)-2-((2-nitrophenyl) sulfonamido)propanoate: 1.28 g, 76%) as a white solid

(2-3) Synthesis of Compound 4

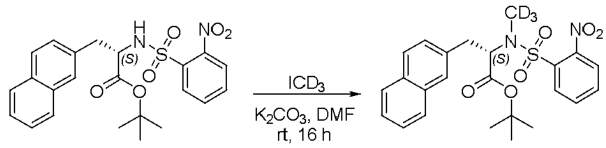


3 4

Deuterated iodomethane (813 mg) and potassium carbonate (775 mg) were added to a solution of compound 3 (1.28 g) in DMF (20 mL) at room temperature. The reaction mixture was reacted at room temperature for 16 hours, added with ethyl acetate (100 mL), and washed with water and saturated brine, dried over anhydrous sodium sulfate, and concentrated to obtain a residue, which was purified by silica gel column chromatography (volume ratio: petroleum ether/ethyl acetate = 4/1) to obtain compound 4 (tert-butyl (S)-2-((N-(methyl-d3)-2-nitrophenyl)sulfonamido)-3-(naphthalen-2-yl)propanoate: 1.2 g) as a yellow liquid.

(2-4) Synthesis of Compound

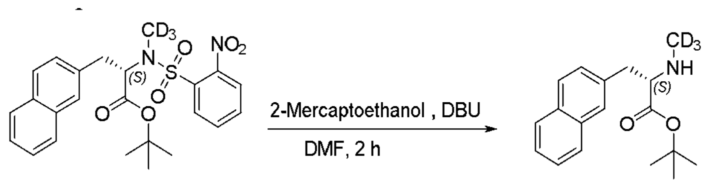


4 5

2-Mercaptoethanol (396 mg) and DBu (772 mg) were added to a solution of compound 4 (1.20 g) in DMF (10 mL) at room temperature, and the reaction mixture was reacted at room temperature for 2 hours, then purified by C18 reversed-phase silica gel column chromatography to obtain compound 5 (tert-butyl (S)-2-((methyl-d3)amino)-3-(naphthalen-2-yl) propanoate: 620 mg) as a yellow liquid.

(2-5) Synthesis of Compound 6

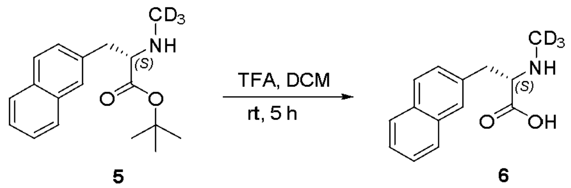


5

6

Trifluoroacetic acid (5 mL) was added to a solution of compound 5 (620 mg) in dichloromethane (5 mL) at room temperature. The reaction mixture was reacted at room temperature for 5 hours, then concentrated to obtain compound 6 ((S)-2-((methyl-d3)amino)-3-(naphthalen-2-yl)propionic acid: 500 mg) as a colorless liquid.

(2-6) Synthesis of Compound VWT022502

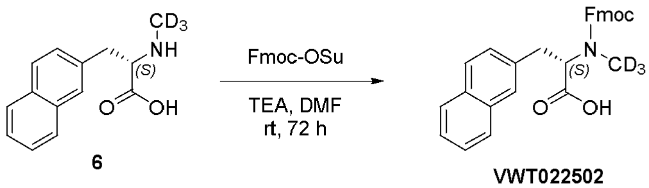


6

VWT022502

Fmoc-OSu (1.09 g) and triethylamine (651 mg) were added to a solution of compound 6 (500 mg) in DMF (300 mL). The reaction mixture was reacted at room temperature for 72 hours, then concentrated, and added with ethyl acetate (200 mL), and then washed with water and saturated brine, dried over anhydrous sodium sulfate, and concentrated to obtain a residue, which was purified by silica gel column chromatography (volume ratio: dichloromethane/methanol = 10/1) to obtain compound VWT022502: (S)-2-((((9H-fluorene-9-yl)methoxy)carbonyl)(methyl-d3)amino)-3-(naphthalen-2-yl)propionic acid (80.3 mg) as a white solid.

Step 3 (preparation of VWT023601)

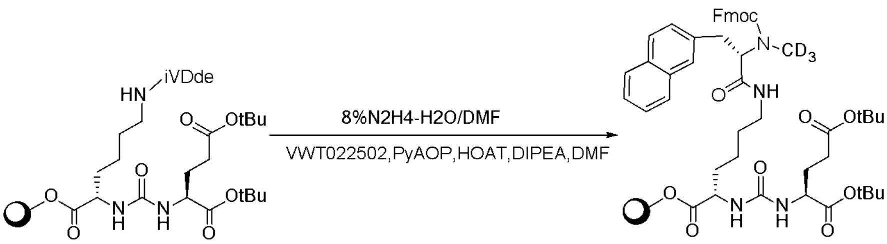


VWT016103

VWT023601

VWT016103 resin (0.06 mmol) was swollen in DMF (5 mL) for 30 minutes, then treated with 8% hydrazine hydrate/DMF (5 mL) for 30 minutes to remove the ivDde protecting group. The resin was then washed with DMF, and then coupled with VWT022502 (40 mg) under a PyAOP/HOAT/DIPEA condensation system (DMF as solvent) for 4 hours, then washed with DMF to obtain compound VWT023601.

Step 4 (preparation of VWT023602)

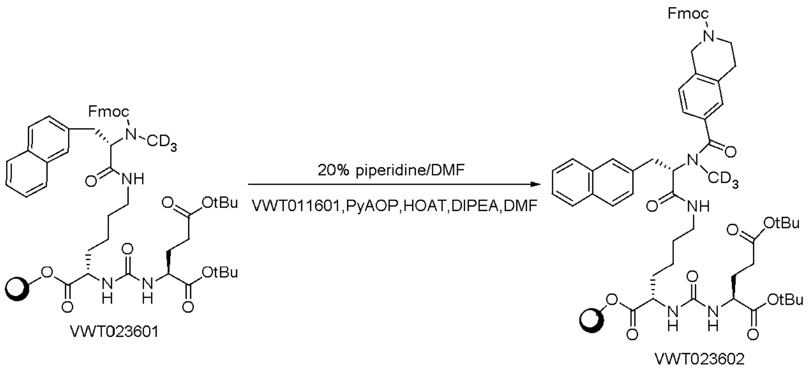


VWT023601 resin (0.06 mmol) was swollen in DMF (5 mL) for 30 minutes, then treated with 20% piperidine/DMF (5 mL) for 30 minutes to remove the Fmoc protecting group. The resin was then washed with DMF, and then coupled with VWT011601 (30 mg) under a PyAOP/HOAT/DIPEA condensation system (DMF as solvent) for 6 hours, and then washed with DMF to obtain compound VWT023602.

Step 5 (preparation of VWT024302)

(5-1) Synthesis of Compound 3

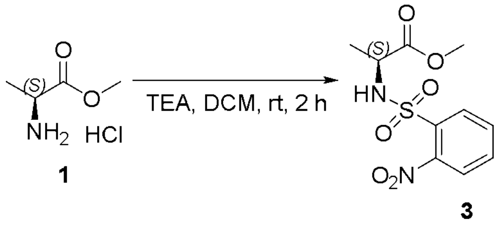


Compound 1 (methyl (2S)-2-aminopropanoate: 1.00 g) and triethylamine (2.94 g) were added to 10 mL of dichloromethane, followed by 2-nitrobenzenesulfonyl chloride (2.15 g). The mixture was stirred at room temperature for 2 hours. After the reaction was complete, the mixture was concentrated under reduced pressure, and the residue was purified by silica gel column chromatography (volume ratio: PE/EA = 5/1) to obtain compound 3 (methyl (2S)-2-[(2-nitrobenzene)sulfonylamino]propanoate: 1.90 g) as a colorless liquid.

(5-2) Synthesis of Compound 4

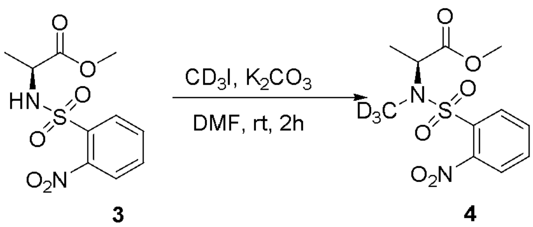


Compound 3 (1.90 g) was dissolved in N,N-dimethylformamide (10 mL), and potassium carbonate (2.74 g) and deuterated iodomethane (1.91 g) were added. The reaction was stirred at room temperature for 2 hours. After the reaction was complete, the reaction mixture was poured into water (100 mL) and extracted with ethyl acetate (100 mL × 3). The organic phases were combined and washed successively with water and saturated brine, then dried over anhydrous sodium sulfate and concentrated. The residue was purified by silica gel column chromatography (volume ratio: PE/EA = 10:1) to obtain compound 4 (Methyl (2S)-2-[N-methyl(2-nitrobenzene)sulfonamido]propanoate: 2.00 g) as a colorless liquid.

(5-3) Synthesis of Compound 6

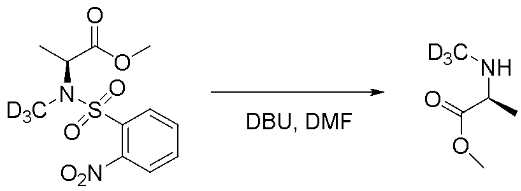


4

6

Compound 4 (2.00 g) was dissolved in N,N-dimethylformamide (20 mL), and 2-thioethanol (2.56 g) and DBU (4.98 g) were added. The reaction was carried out at room temperature for 1 hour. After the reaction was completed, the reaction mixture was concentrated under reduced pressure to obtain compound 6 (Methyl (2S)-2-(methylamino)propanoate: 700 mg). The crude product was a yellow liquid, and was directly added to the next step without purification.

(5-4) Synthesis of Compound 7

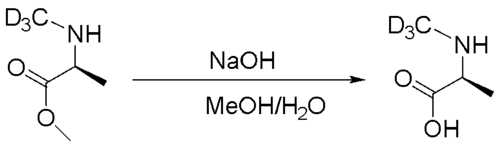


6

7

Compound 6 (700 mg) was dissolved in 10 mL of methanol, and sodium hydroxide (1.16 g dissolved in 2 mL of water) was added. The mixture was reacted at room temperature for 3 hours. After the reaction was complete, the reaction mixture was concentrated under reduced pressure to remove methanol. Dilute hydrochloric acid was added to adjust the pH of the residue to neutral or weakly acidic. The mixture was then concentrated under reduced pressure to obtain compound 7 ((2S)-2-(methylamino)propionic acid: 500 mg). The crude product was a pale yellow solid, and was directly added to the next step without purification.

(5-5) Synthesis of compound VWT024302

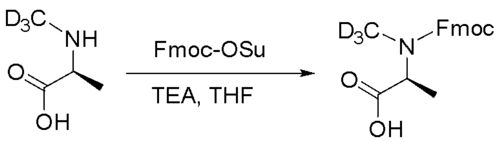


7

**VWT024302**

Compound 7 (500 mg) was added to 10 mL of tetrahydrofuran, followed by Fmoc-OSu (3.18 g) and triethylamine (1.43 g). The reaction mixture was reacted at room temperature for 4 hours. After the reaction was complete, the reaction mixture was poured into water (100 mL) and extracted with ethyl acetate (100 mL × 3). The organic phases were combined and washed successively with water and saturated brine, dried over anhydrous sodium sulfate, and concentrated. The residue was purified by silica gel column chromatography (volume ratio: DCM/MeOH = 10/1) to obtain compound VWT024302 ((2S)-2-({9-[(formyloxy)methyl]-9H-fluorene-4a-yl}(methyl)amino)propionic acid: 252.1 mg) as a yellow liquid.

Step 6 (preparation of VWT023603)

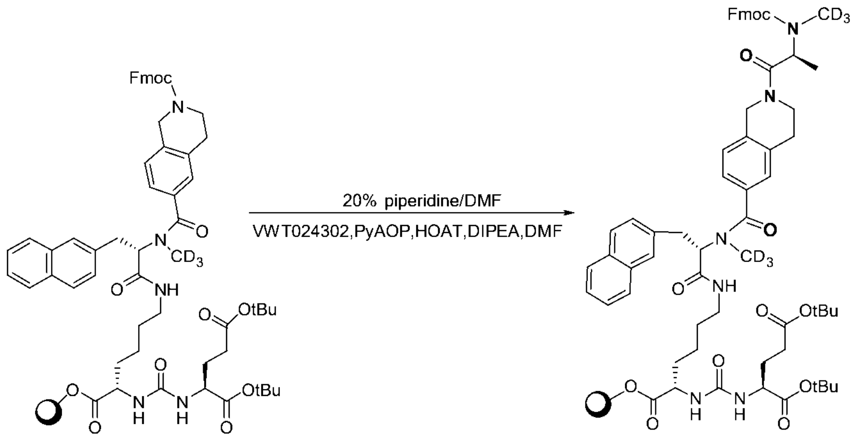


VWT023602 VWT023603

VWT023602 resin (0.06 mmol) was swollen in DMF (5 mL) for 30 minutes, then treated with 20% piperidine/DMF (5 mL) for 30 minutes to remove the Fmoc protecting group. The resin was washed with DMF, and then coupled with VWT024302 (30 mg) under a PyAOP/HOAT/DIPEA condensation system for 6 hours. The resin was then washed with DMF to obtain intermediate VWT023603.

Step 7 (preparation of VWT023600)

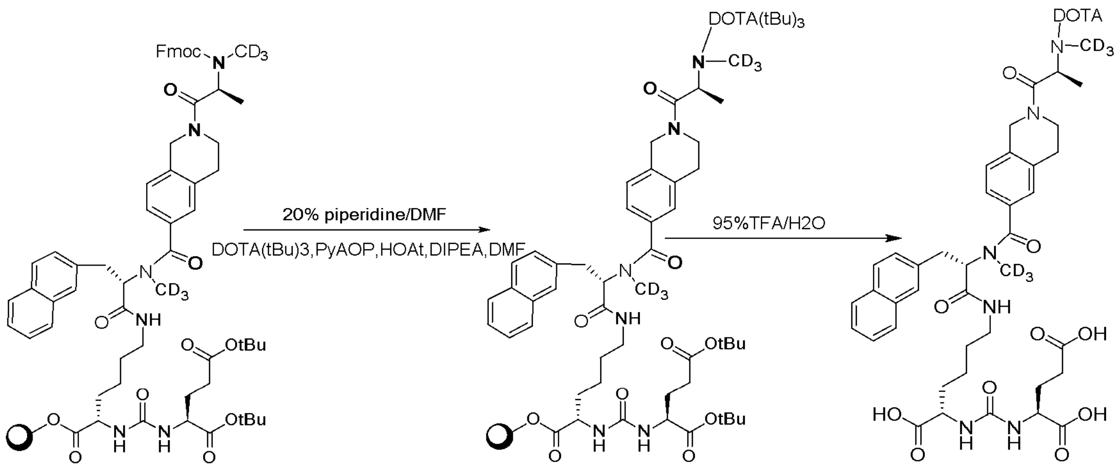


VWT023603 VWT023604 VWT023600

VWT023603 resin (0.06 mmol) was swollen in DMF (5 mL) for 30 minutes, then treated with 20% piperidine/DMF (5 mL) for 30 minutes to remove the Fmoc protecting group. The resin was then washed with DMF, and then coupled with DOTA(tBu)$_3$ (70mg) under a PyAOP/HOAT/DIPEA condensation system (DMF as solvent) for 8 hours. The resin was then washed with DMF to obtain resin VWT023604 (173 mg).

VWT023604 was treated in 95% TFA/5% $H_2O$ Cleavage Cocktail (9.5 mL) for 4 hours, crystallized with methyl tert-butyl ether, and centrifuged and dried to obtain crude VWT023600, which was further purified by preparative HPLC to obtain 17 mg of the target product VWT023600 with a purity of 92.72%; LCMS: $[M+H]^+$ = 1167.80.

**Example 14**

Preparation of compound VWT026300

Step 1 (preparation of VWT024001)

VWT024001 was prepared using the same method as in Example 8.

Step 2 (preparation of VWT026301)

(2-1) Synthesis of Compound 3

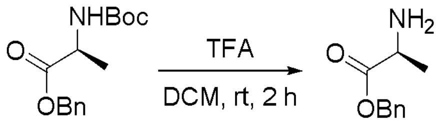


6 3

Trifluoroacetic acid (10 mL) was added to a solution of compound 6 (Benzyl (2S)-2-{[(tert-butoxy)carbonyl] amino}propanoate: 5.0 g, CAS No.: 51814-54-1) in dichloromethane (10 mL). The reaction mixture was reacted at room temperature for 2 hours, and concentrated to obtain compound 3: Benzyl (2S)-2-aminopropanoate as a colorless liquid.

(2-2) Synthesis of Compound 2

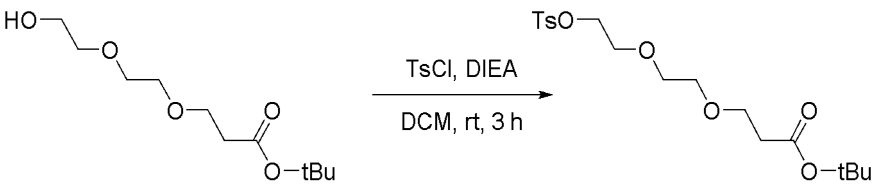


1 2

TsCl (p-toluenesulfonyl chloride: 3.24 g) and triethylamine (3.3 g) were added to a solution of compound 1 (tert-butyl 3-[2-(2-hydroxyethoxy)ethoxy]propanoate: 2.0 g, CAS No.: 133803-81-3) in dichloromethane (20 mL). The reaction mixture was reacted at room temperature for 3 hours. After the reaction was completed, the reaction mixture was concentrated, and the residue was purified by silica gel column chromatography (volume ratio: PE/EA = 10/1) to obtain compound 2: tert-butyl 3-[2-(2-{[(4-methylbenzene)sulfonyl]oxy}ethoxy)ethoxy]propanoate as a pale yellow solid.

(2-3) Synthesis of Compound 4

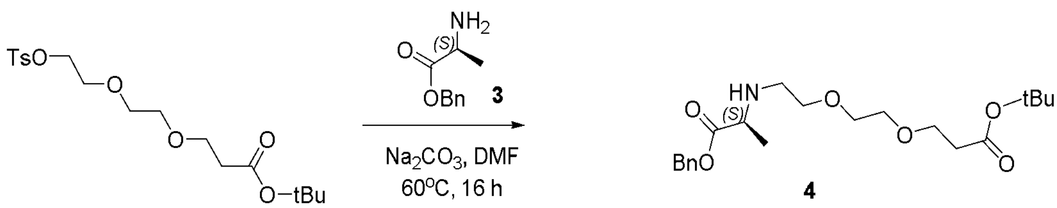


2 4

Compound 3 (1.22 g) and anhydrous sodium carbonate (1.08 g) were added to a solution of compound 2 (1.32 g) in DMF (20 mL). The reaction mixture was reacted at 60 °C for 16 hours. After the reaction was complete, ethyl acetate was added to the reaction mixture. The mixture was then washed with water and saturated brine, dried over anhydrous sodium sulfate, and concentrated to obtain a residue, which was purified by silica gel column chromatography (volume ratio: dichloromethane/methanol = 10/1) to obtain compound 4: benzyl (2S)-2-[(2-{2-[3-(tert-butoxy)-3-oxopropoxy]ethoxy} ethyl)amino]propanoate as a colorless liquid.

(2-4) Synthesis of Compound 5

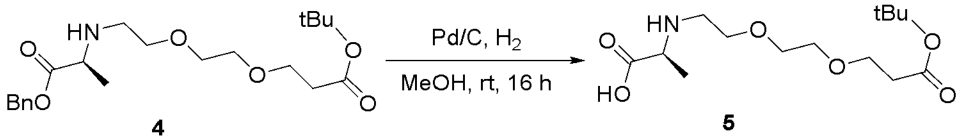


4 5

10% Pd/C (269 mg) was added to a solution of compound 4 (1.0 g) in methanol (10 mL). The reaction mixture was reacted at room temperature under a hydrogen atmosphere for 16 hours. The solid was filtered and evaporated to dryness to obtain compound 5: (2S)-2-[(2-{2-[3-(tert-butoxy)-3-oxopropoxy]ethoxy}ethyl)amino]propionic acid as a colorless liquid.

(2-5) Synthesis of compound VWT026301

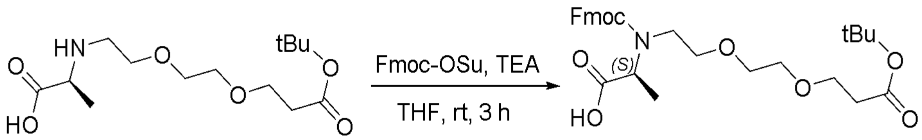


5 **VWT026301**

Fmoc-OSu (1.53 g) and triethylamine (685 mg) were added to a solution of compound 5 (690 mg) in tetrahydrofuran (5 mL). The reaction mixture was reacted at room temperature for 3 hours. After the reaction was complete, the reaction mixture was added with ethyl acetate, then washed with water and saturated brine, dried over anhydrous sodium sulfate, and concentrated to obtain a residue, which was purified by prep-HPLC (mobile phase: 0.1% formic acid aqueous solution-acetonitrile) to obtain compound VWT026301: (2S)-2-[(2-{2-[3-(tert-butoxy)-3-oxopropoxy] ethoxy}ethyl)({9-[(formyloxy)methyl]-9H-fluorene-4a-yl})amino]propionic acid as a brown liquid.

Step 3 (preparation of VWT026302)

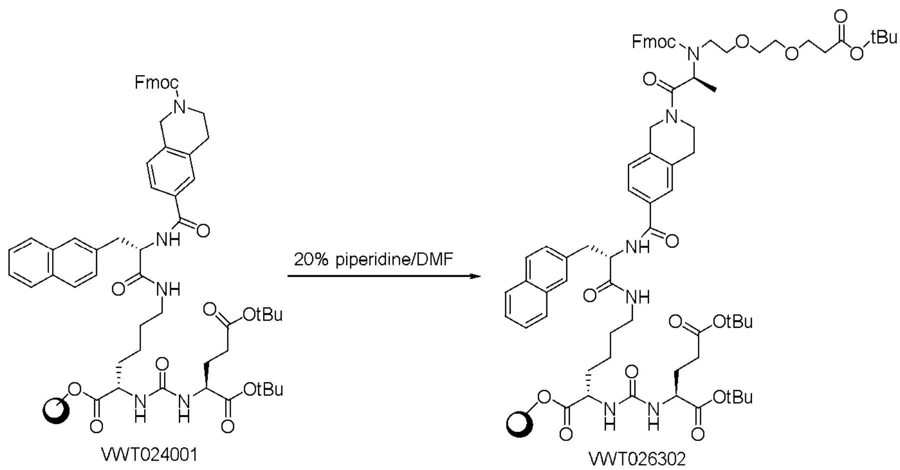


VWT024001 VWT026302

VWT024001 resin was swollen in DMF (2 mL) for 30 minutes, then washed with DMF (2 mL), and then treated with 20% piperidine/DMF (2 mL) for 30 minutes to remove the Fmoc protecting group, and then washed with DMF (16 mL). The reaction was then treated with a mixture of VWT026301 (50 mg), PyAOP (46 mg), HOAT (15 mg), and DIPEA (25 mg) in DMF (1 mL) for 5 hours, and the resin was then washed with DMF (12 mL) to obtain compound VWT026302.

Step 4 (preparation of VWT026300)

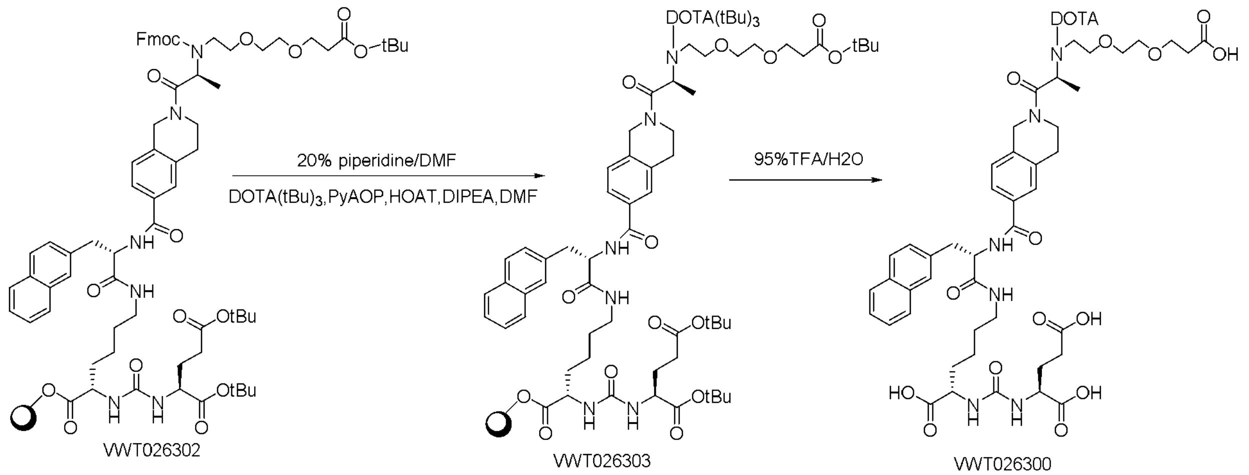

VWT026302 VWT026303 VWT026300

VWT026302 resin was treated with 20% piperidine/DMF (2 mL) for 30 minutes to remove the Fmoc protecting group, and washed with DMF (16 mL), and then coupled with compound DOTA(tBu)$_3$ (25 mg) under a PyAOP/HOAT/DIPEA condensation system, and the resin was washed with DMF (4 mL) to obtain VWT026303.

VWT026303 was then treated in 95% TFA/5% $H_2O$ Cleavage Cocktail (2 mL) for 5 hours, crystallized with methyl tert-butyl ether, and centrifuged and dried to obtain crude VWT026300, which was then purified by preparative HPLC to obtain 14 mg of the target product VWT026300 with a purity of 93.02%; LCMS: [M+H] $^+$ =1293.90.

**Example 15**

Preparation of compound VWT031600

Step 1 (preparation of VWT016100)

VWT016100 was prepared using the same method as in Example 1.

Step 2 (preparation of VWT031601)

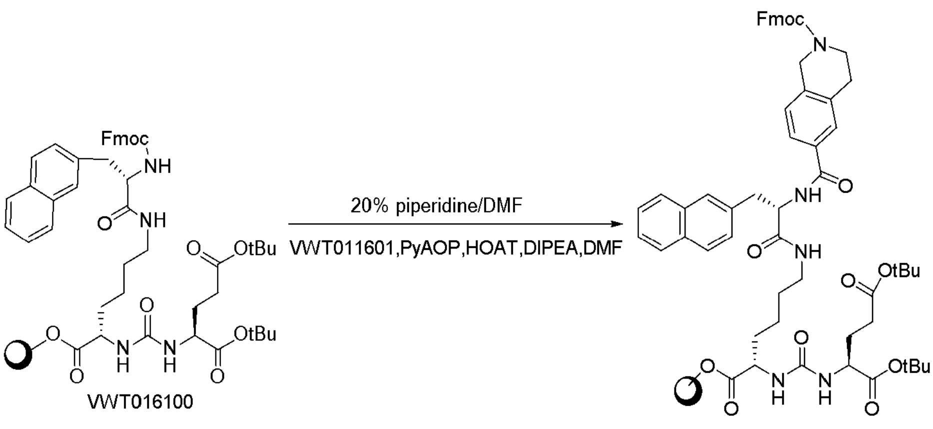

VWT016100 VWT031601

VWT016100 resin (0.1 mmol) was swollen in DMF (3 mL) for 30 minutes, then washed with DMF (3 mL). The swollen resin was then treated with 20% piperidine/DMF (3 mL) for 30 minutes to remove the Fmoc protecting group, and then washed with DMF (24 mL). The reaction was treated with a mixture of VWT011601 (50 mg), PyAOP (65 mg), HOAT (18 mg), and DIPEA (35 mg) in DMF (3 mL) for 3.5 hours, and the resin was washed with DMF (18 mL) to obtain compound VWT031601.

Step 3 (preparation of VWT031602)

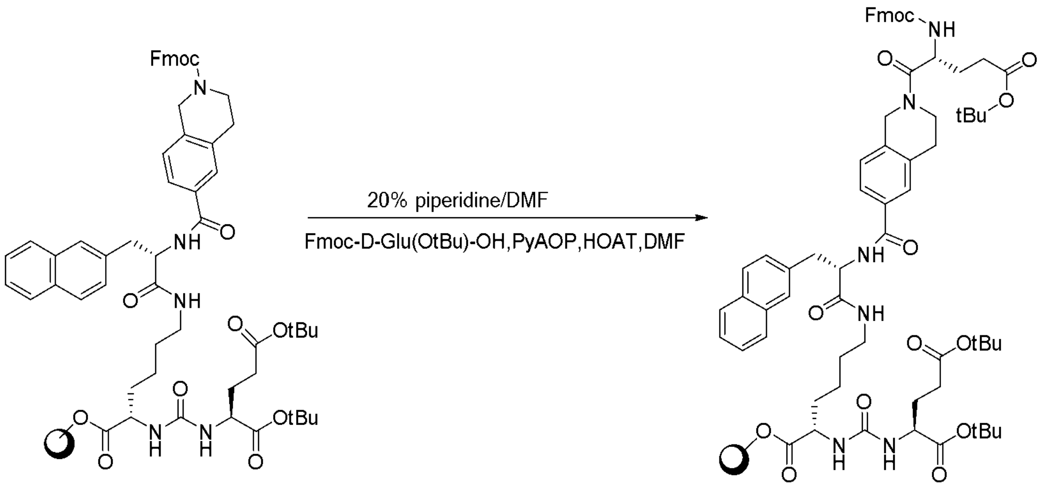


VWT031601 VWT031602

VWT031601 resin (0.1 mmol) was swollen in DMF (5 mL) for 30 minutes, and washed with DMF (5 mL), then treated with 20% piperidine/DMF (5 mL) for 30 minutes to remove the Fmoc protecting group, and then washed with DMF (40 mL). The reaction was then treated with a mixture of Fmoc-D-Glu(OtBu)-OH (130 mg), PyAOP (155 mg), HOAT (45 mg), and DIPEA (85 mg) in DMF (3 mL) for 5 hours, and the resin was washed with DMF (18 mL) to obtain compound VWT031602.

Step 4 (preparation of VWT031603)

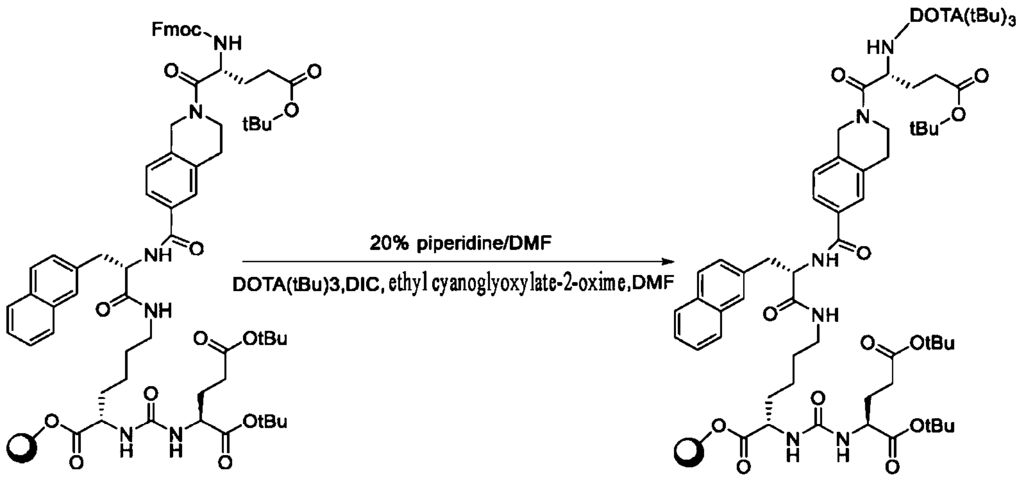


VWT031602 VWT031603

VWT031602 resin (0.1 mmol) was treated with 20% piperidine/DMF (3 mL) for 30 min to remove the Fmoc protecting group, and then washed with DMF (24 mL). The reaction was then treated with a mixture of DOTA(tBu)$_3$ (230 mg), ethyl cyanoglyoxylate-2-oxime (50 mg), and DIC (42 mg) in DMF (3 mL) for 5 h, and the resin was washed with DMF (18 mL) to obtain compound VWT031603.

Step 5 (preparation of VWT031600)

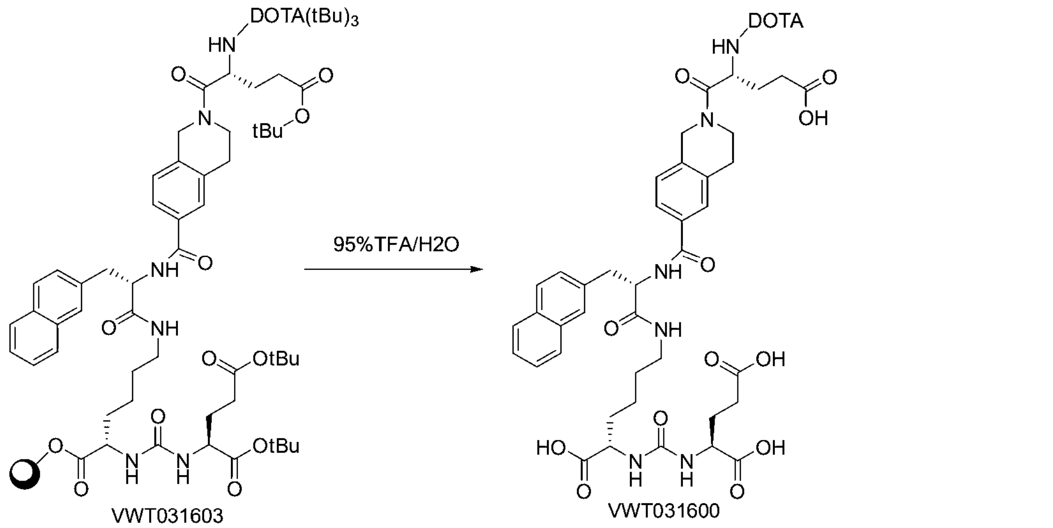


VWT031603 VWT031600

VWT031603 was treated in 95% TFA/5% $H_2O$ Cleavage Cocktail (4.0 mL) for 4 hours, crystallized with methyl tert-butyl ether, and centrifuged and dried to obtain 152 mg of crude VWT031600 as a white solid, which was further purified by preparative HPLC to obtain 47 mg of the target product VWT031600 with a purity of 97.93%; LCMS: $[M+H]^+$ = 1191.80.

**Example 16**

Preparation of compound VWT032000

Step 1 (preparation of VWT024001)

VWT024001 was prepared using the same method as in Example 8.

Step 2 (preparation of VWT032002)

(2-1) Synthesis of Compound 2

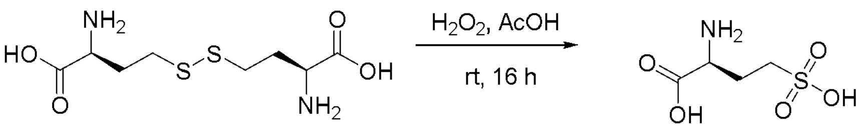


1 2

Compound 1 ((2S,2'S)-4,4'-disulfanediylbis(2-aminobutyric acid): 1 g) was dissolved in acetic acid (30 mL), and hydrogen peroxide (30 mL) was added. The reaction mixture was reacted at room temperature for 16 hours, and lyophilized to obtain compound 2: (S)-2-amino-4-sulfobutyric acid (0.56 g) as a white solid.

(2-2) Synthesis of VWT03200

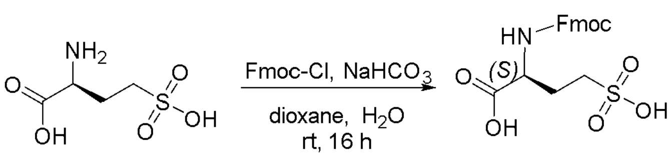


2 **VWT032002**

Compound 2 (0.56 g) was dissolved in dioxane (50 mL). Fmoc-Cl (1.57 g) and sodium bicarbonate (1.3 g dissolved in 50 mL water) were added to the reaction mixture, and the reaction was carried out at room temperature for 16 hours. Ethyl acetate was added to the reaction mixture for separation, and the aqueous phase was retained. The aqueous phase was adjusted to pH 1 with 1N hydrochloric acid, and lyophilized to obtain a residue, which was purified by C18 reversed-phase silica gel column chromatography to obtain compound VWT032002: (S)-2-((((9H-fluorene-9-yl)methoxy) carbonyl)amino)-4-sulfobutyric acid (401.2 mg) as a white solid.

Step 3 (preparation of VWT032003)

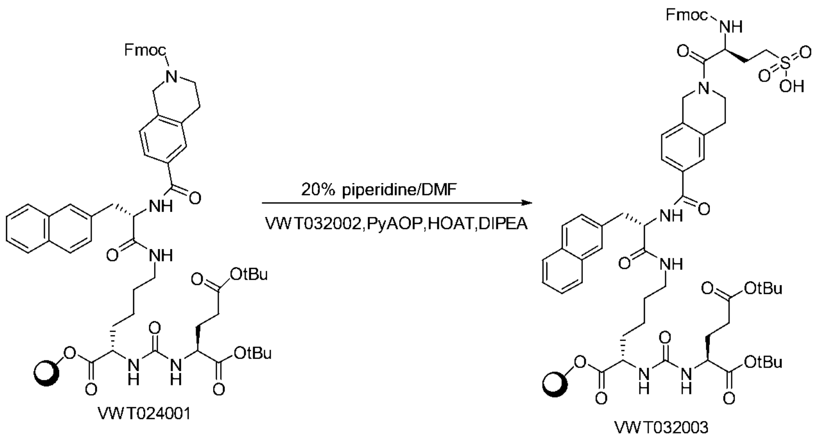


VWT024001 VWT032003

VWT024001 resin was swollen in DMF (2 mL) for 30 minutes, and washed with DMF (2 mL), then treated with 20% piperidine/DMF (2 mL) for 30 minutes to remove the Fmoc protecting group, and then washed with DMF (16 mL). The reaction was then treated with a mixture of VWT032002 (75 mg), PyAOP (94 mg), HOAT (25 mg), and DIPEA (50 mg) in DMF (1 mL) for 5 hours, and the resin was then washed with DMF (12 mL) to obtain compound VWT032003.

Step 4 (preparation of VWT032000)

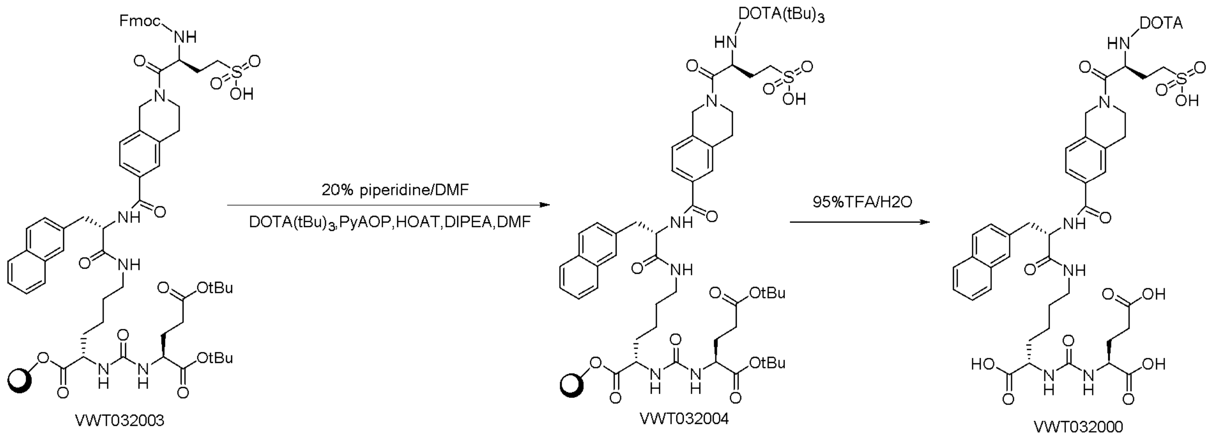


VWT032003 VWT032004 VWT032000

VWT032003 resin was treated with 20% piperidine/DMF (2 mL) for 30 minutes to remove the Fmoc protecting group. The resin was washed with DMF (16 mL), and then coupled with the compound DOTA$(tBu)_3$ (74 mg) under a PyAOP (65 mg)/HOAT (20 mg)/DIPEA (35 mg) condensation system. The resin was washed with DMF (4 mL) to obtain VWT032004.

VWT032004 was then treated in 95% TFA/5% $H_2O$ Cleavage Cocktail (2 mL) for 5 hours, crystallized with methyl tert-butyl ether, and centrifuged and dried to obtain crude VWT032000, which was then purified by preparative HPLC to obtain 11.4 mg of the target product VWT032000; LCMS: $[M+H]^+$ =1228.00.

**Example 17**

Preparation of compound VWT032100

Step 1 (preparation of VWT024001)

VWT024001 was prepared using the same method as in Example 8.

Step 2 (preparation of VWT032 100)

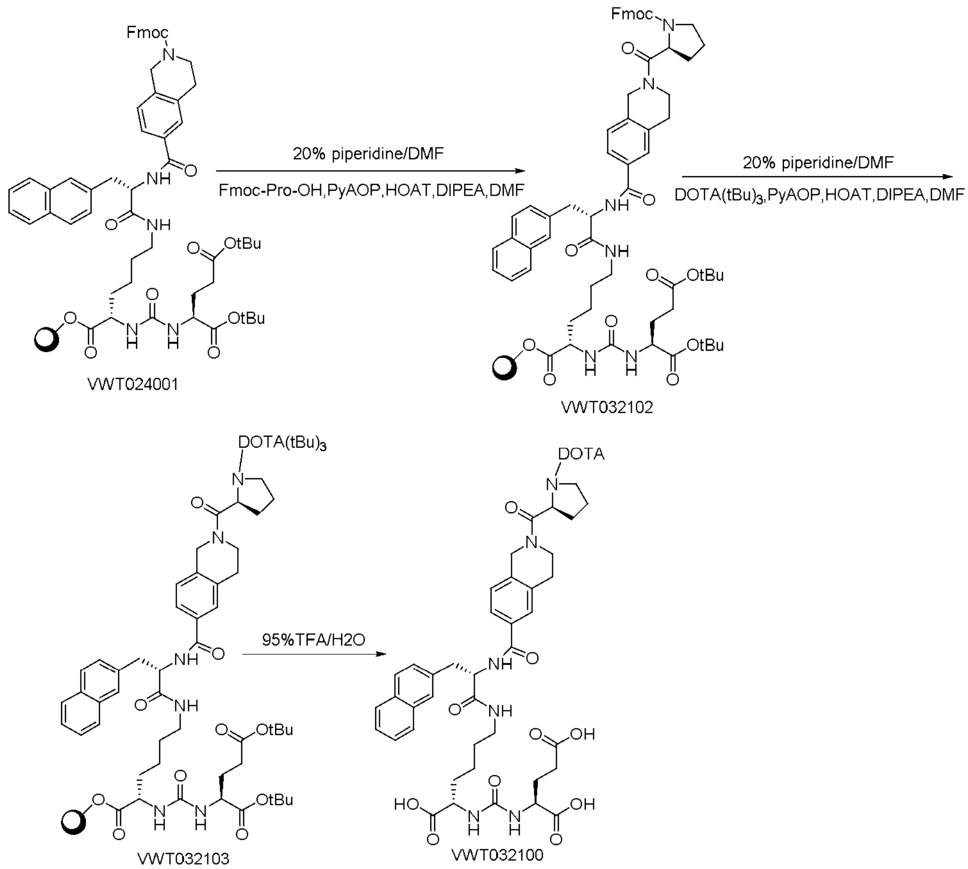

VWT024001
VWT032102
VWT032103
VWT032100

VWT024001 resin (0.1 mmol) was treated with 20% piperidine/DMF (5 mL) for 30 minutes to remove the Fmoc protecting group, and washed with DMF, then coupled with Fmoc-Pro-OH in the order specified in the sequence under a PyAOP/HOAT/DIPEA condensation system, and washed with DMF to obtain an intermediate VWT032102. The intermediate was then treated with 20% piperidine/DMF (5 mL) for 30 minutes, and washed with DMF, then coupled with compound DOTA(tBu)$_3$ under a PyAOP/HOAT/DIPEA condensation system, and the resin was washed with DMF to obtain resin VWT032103.

VWT032103 was treated in 95% TFA/5% $H_2O$ Cleavage Cocktail (5 mL) for 4 hours, crystallized with methyl tert-butyl ether, and centrifuged and dried to obtain crude VWT032100, which was further purified by preparative HPLC to obtain 67 mg of the target product VWT032100 with a purity of 97.53%; LCMS: $[M+H]^+ = 1159.80$.

**Example 18**

Preparation of compound VWT032200

The difference from the preparation steps of VWT032100 in Example 17 is that Fmoc-D-Pro-OH was coupled in Example 18.

**Example 19**

Preparation of compound VWT032600

Step 1 (preparation of VWT016100)

VWT016100 was prepared using the same method as in Example 1.

Step 2 (preparation of VWT032602)

(2-1) Synthesis of Compound 4

Compound 4: ethyl 1,2,3,4-tetrahydroisoquinoline-6-carboxylate (1.34 g) was prepared using the same method as in Example 1.

(2-2) Synthesis of Compound 2

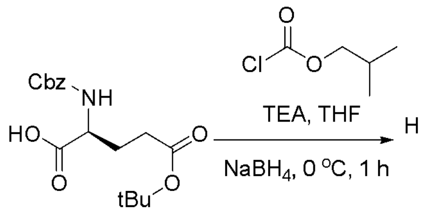


1 2

Isobutyl chloroformate (485.8 mg) was added to a solution of (S)-2-(((benzyloxy)carbonyl)amino)-5-(tert-butoxy)-5-oxopentanoic acid (Cbz-Glu(OtBu)-OH: 1.0 g) and triethylamine (449.9 mg) in tetrahydrofuran (20 mL) at 0 °C. The reaction was carried out at 0 °C for 0.5 h. Sodium borohydride (336.4 mg) was added to the reaction mixture at 0 °C, and the reaction was continued for another 0.5 h. The reaction mixture was quenched with saturated ammonium chloride solution and extracted with ethyl acetate. The combined organic phases were washed with saturated brine, dried over anhydrous sodium sulfate, and concentrated to obtain a residue, which was purified by silica gel column chromatography (volume ratio: petroleum ether/ethyl acetate = 1/1) to obtain compound 2: tert-butyl (S)-4-(((benzyloxy)carbonyl)amino)-5-hydroxyvalerate (529 mg) as a colorless liquid.

(2-3) Synthesis of Compound 3

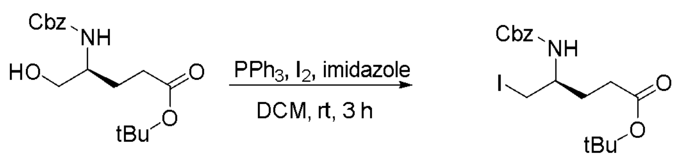


2 3

Triphenylphosphine (1.29 g), iodine (1.25 g), and imidazole (556.8 mg) were added to dichloromethane (50 mL) at room temperature. After reacting for half an hour at room temperature, compound 2 (529 mg) was added to the reaction mixture and the reaction was stirred for another 3 hours. The reaction mixture was concentrated and purified by silica gel column chromatography (volume ratio: petroleum ether/ethyl acetate = 10/1) to obtain compound 3: tert-butyl (S)-4-(((benzyloxy)carbonyl)amino)-5-iodopentanoate (540 mg) as a colorless liquid.

(2-4) Synthesis of Compound 5

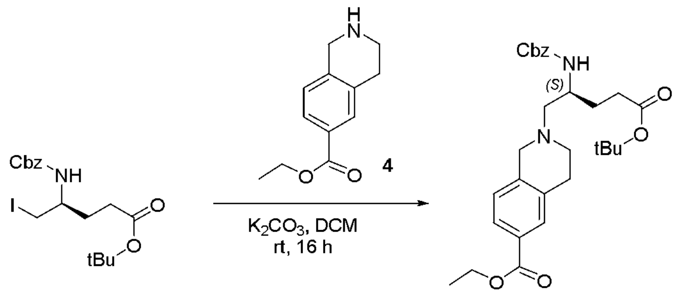


3 5

Ethyl 1,2,3,4-tetrahydroisoquinoline-6-carboxylate (200 mg), compound 3 (506.6 mg), and potassium carbonate (249.3 mg) were added to dichloromethane (10 mL). After reacting at room temperature for 16 hours, the reaction mixture was concentrated and purified by silica gel column chromatography (volume ratio: petroleum ether/ethyl acetate = 1/1) to obtain compound 5: ethyl (S)-2-(2-(((benzyloxy)carbonyl)amino)-5-(tert-butoxy)-5-oxopentyl)-1,2,3,4-tetrahydroisoquinoline-6-carboxylate (190 mg) as a colorless liquid.

(2-5) Synthesis of Compound 6

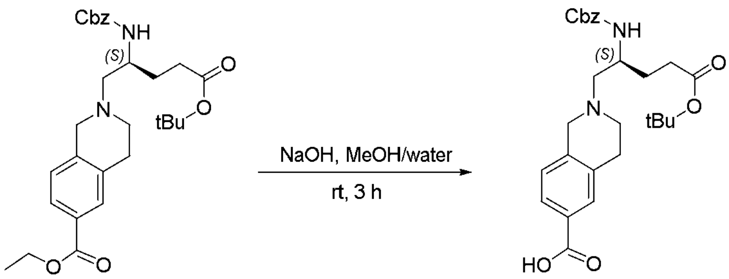


5 6

Sodium hydroxide (74.4 mg dissolved in 5 mL of water) was added to a solution of compound 5 (190 mg) in methanol (5 mL). The reaction was carried out at room temperature for 3 hours. The reaction mixture was adjusted to pH 7 with 1N hydrochloric acid, and extracted with ethyl acetate (50 mL × 3). The combined organic phases were washed with saturated brine, dried over anhydrous sodium sulfate, and concentrated to obtain compound 6: (S)-2-(2-(((benzyloxy) carbonyl)amino)-5-(tert-butoxy)-5-oxopentyl)-1,2,3,4-tetrahydroisoquinoline-6-carboxylic acid (140 mg) as a white solid.

(2-6) Synthesis of Compound 7

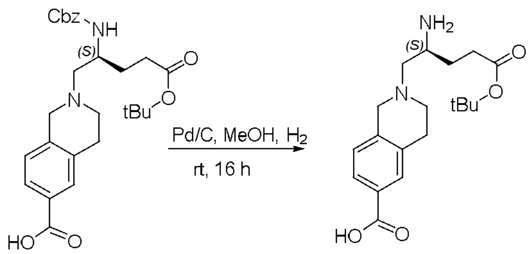


6 7

Pd/C (20 mg) was added to a solution of compound 6 (140 mg) in methanol (10 mL), and the reaction was carried out at room temperature for 16 hours under a hydrogen atmosphere. The reaction mixture was filtered, and the filtrate was concentrated to obtain compound 7: (S)-2-(2-amino-5-(tert-butoxy)-5-oxopentyl)-1,2,3,4-tetrahydroisoquinoline-6-carboxylic acid (100 mg) as a colorless liquid.

(2-7) Synthesis of compound VWT032602

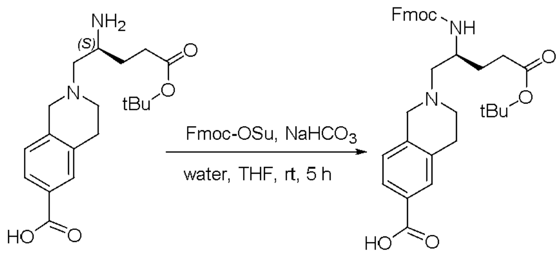


7 VWT032602

Fmoc-OSu (111.4 mg) and sodium bicarbonate (72 mg dissolved in 5 mL of water) were added to a solution of compound 7 (100 mg) in tetrahydrofuran (5 mL), and the reaction was carried out at room temperature for 5 hours. The reaction mixture was concentrated and purified by silica gel column chromatography (volume ratio: dichloromethane/-methanol = 10/1) to obtain compound VWT032602: (S)-2-(2-((((9H-fluorene-9-yl)methoxy)carbonyl)amino)-5-(tert-butoxy)-5-oxopentyl)-1,2,3,4-tetrahydroisoquinoline-6-carboxylic acid (17.2 mg) as a white solid.

Step 3 (preparation of VWT032603)

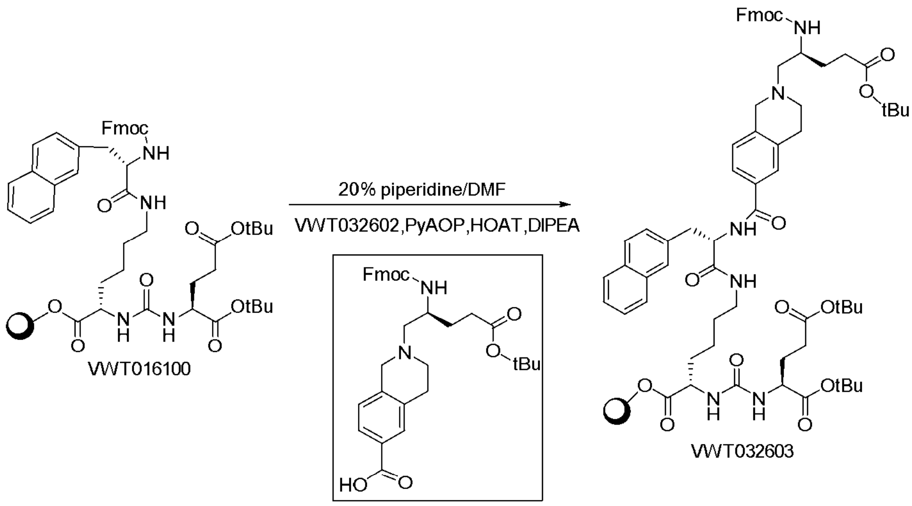


VWT016100 VWT032603

VWT016100 resin was swollen in DMF (1 mL) for 30 minutes, and washed with DMF (2 mL), then treated with 20% piperidine/DMF (1 mL) for 30 minutes to remove the Fmoc protecting group, and then washed with DMF (8 mL). The reaction was then treated with a mixture of VWT032602 (17.2 mg), PyAOP (16 mg), HOAT (5 mg), and DIPEA (10 mg) in DMF (1 mL) for 8 hours, and the resin was then washed with DMF (6 mL) to obtain compound VWT032603.

Step 4 (preparation of VWT032600)

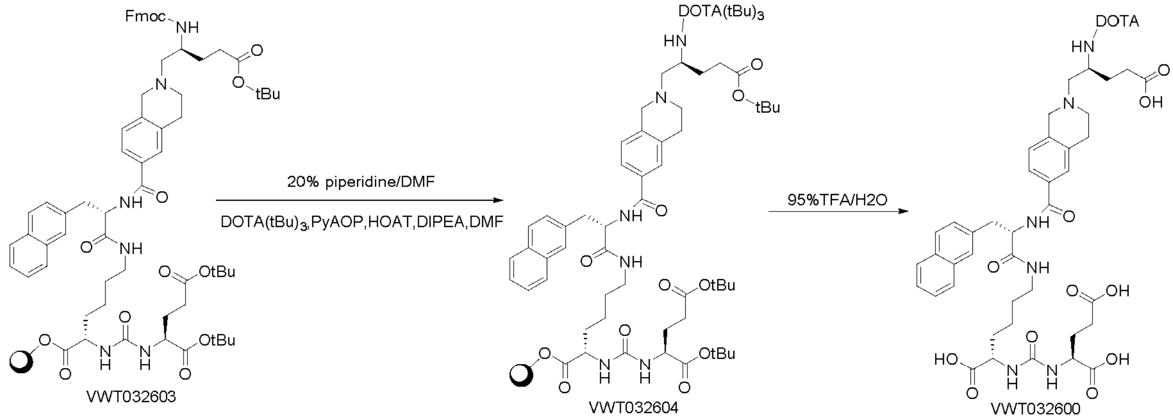


VWT032603 VWT032604 VWT032600

VWT032603 resin was treated with 20% piperidine/DMF (1 mL) for 30 minutes to remove the Fmoc protecting group, and washed with DMF (8 mL), and then coupled with DOTA(tBu)$_3$ (30 mg) under a PyAOP (27 mg)/HOAT (7 mg)/DIPEA (15 mg) condensation system, and washed with DMF (4 mL) to obtain VWT032604.

VWT032604 was then treated in 95% TFA/5% $H_2O$ Cleavage Cocktail (2 mL) for 3 hours, crystallized with methyl tert-butyl ether, and centrifuged and dried to obtain crude VWT032600, which was then purified by preparative HPLC to obtain 36 mg of the target product VWT032600 with a purity of 94.46%; LCMS: $[M+H]^+$ =1178.0.

**Example 20**

Preparation of compound VWT032800

Step 1 (preparation of VWT024001)

VWT024001 was prepared using the same method as in Example 8.

Step 2 (preparation of VWT032802)

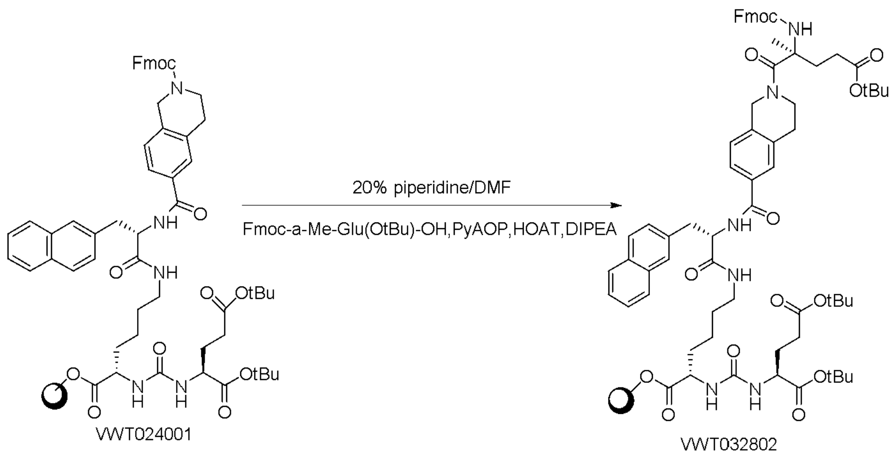


VWT024001 VWT032802

VWT024001 resin was swollen in DMF (2 mL) for 30 minutes, and washed with DMF (2 mL), then treated with 20% piperidine/DMF (2 mL) for 30 minutes to remove the Fmoc protecting group, and then washed with DMF (16 mL). The reaction was treated with a mixture of Fmoc-α-Me-Glu(OtBu)-OH (33 mg, CAS No.: 1072845-48-7), PyAOP (38 mg), HOAT (10 mg), and DIPEA (20 mg) in DMF (1 mL) for 8 hours, and the resin was then washed with DMF (12 mL) to obtain compound VWT032802.

Step 3 (preparation of VWT032803)

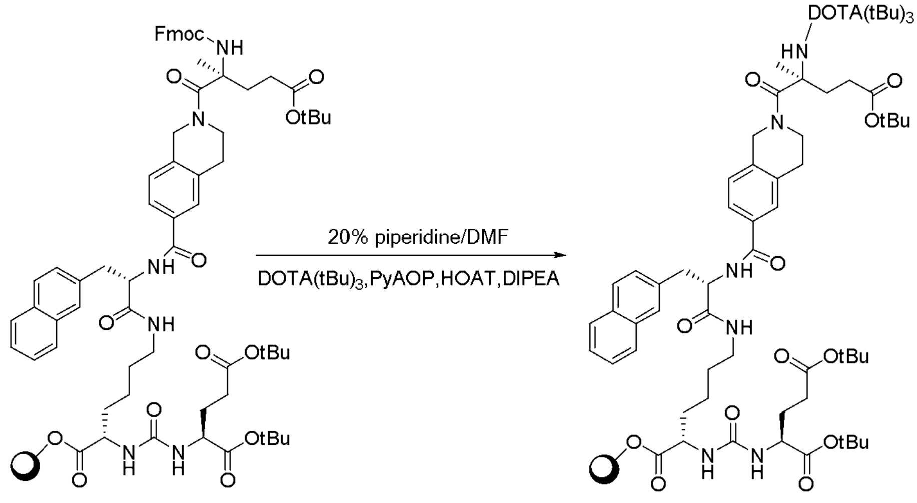


VWT032802 VWT032803

VWT032802 resin was swollen in DMF (2 mL) for 30 minutes, and washed with DMF (2 mL), then treated with 20% piperidine/DMF (2 mL) for 30 minutes to remove the Fmoc protecting group, and then washed with DMF (16 mL). The reaction was then treated with a mixture of DOTA$(tBu)_3$ (105 mg), PyAOP (95 mg), HOAT (26 mg), and DIPEA (50 mg) in DMF (1 mL) for 5 hours, and the resin was then washed with DMF (12 mL) to obtain resin VWT032803.

Step 4 (preparation of VWT032800)

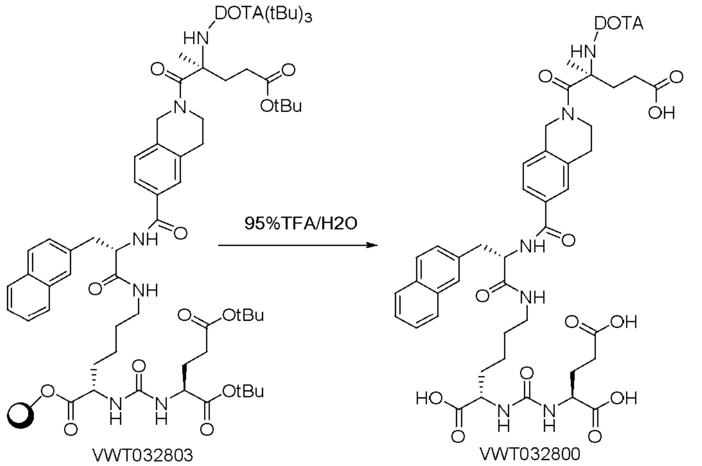


VWT032803 VWT032800

VWT032803 was treated in 2.2 mL of 95% TFA/5% $H_2O$ Cleavage Cocktail for 5 hours, crystallized with methyl tert-butyl ether, and centrifuged and dried to obtain crude VWT032800, which was further purified by preparative HPLC to obtain 3.0 mg of the target product VWT032800 with a purity of 92.13%; LCMS: $[M+H]^+$ =1206.00.

**Example 21**

Preparation of compound VWT032900

Step 1 (preparation of VWT024001)

VWT024001 was prepared using the same method as in Example 8.

Step 2 (preparation of VWT032902)

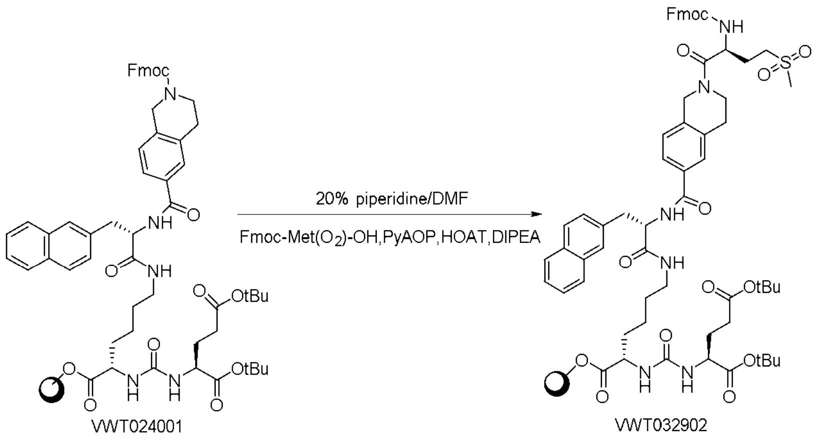


VWT024001 VWT032902

VWT024001 resin was swollen in DMF (2 mL) for 30 minutes, and washed with DMF (2 mL), then treated with 20% piperidine/DMF (2 mL) for 30 minutes to remove the Fmoc protecting group, and then washed with DMF (16 mL). The reaction was treated with a mixture of Fmoc-Met(O2)-OH (51 mg), PyAOP (62 mg), HOAT (18 mg), and DIPEA (40 mg) in DMF (1 mL) for 8 hours, and the resin was then washed with DMF (12 mL) to obtain compound VWT032902.

Step 3 (preparation of VWT032900)

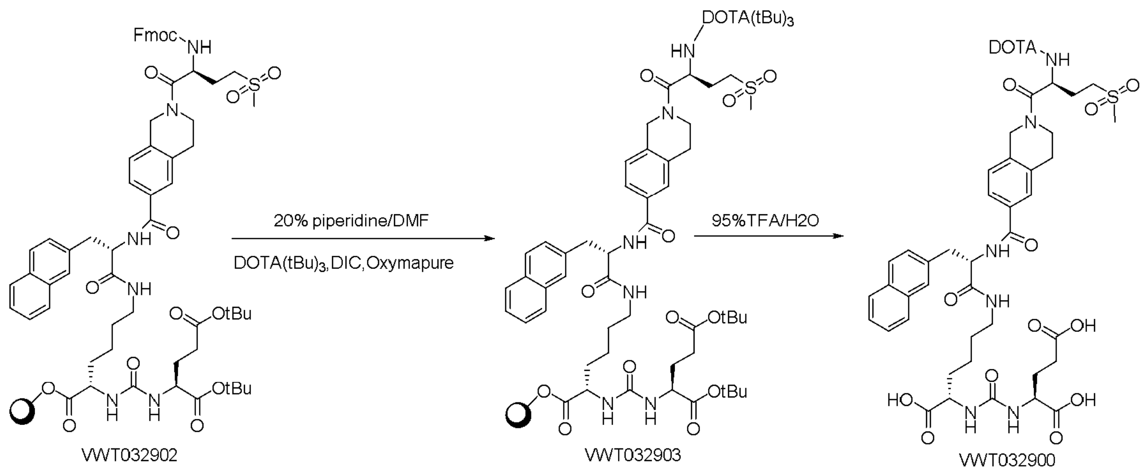


VWT032902 VWT032903 VWT032900

VWT032902 resin was treated with 20% piperidine/DMF (2 mL) for 30 minutes to remove the Fmoc protecting group, and washed with DMF (16 mL), and then coupled with compound DOTA(tBu)$_3$ (170 mg) under a DIC (40 mg)/ethyl cyanoglyoxylate-2-oxime (50 mg) condensation system, and washed with DMF (4 mL) to obtain VWT032903.

VWT032903 was then treated in 95% TFA/5% $H_2O$ Cleavage Cocktail (2 mL) for 3 hours, crystallized with methyl tert-butyl ether, and centrifuged and dried to obtain crude VWT032900, which was then purified by preparative HPLC to obtain 16.1 mg of the target product VWT032900 with a purity of 92.94%; LCMS: $[M+H]^+$ =1225.80.

**Example 22**

Preparation of compound VWT033000

Step 1 (preparation of VWT024001)

VWT024001 was prepared using the same method as in Example 8.

Step 2 (preparation of VWT024902)

(2-1) Synthesis of Compound 3

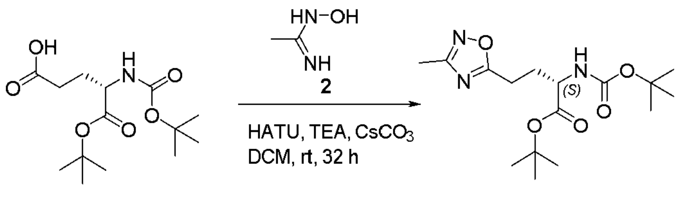


1 3

Compound 1 ((S)-5-(tert-butoxy)-4-((tert-butoxycarbonyl)amino)-5-oxopentanoic acid: 2 g) and HATU (2.76 g) were added to dichloromethane (100 mL), followed by N-hydroxyacetamidine (0.49 g) and triethylamine (2 g). The reaction was carried out at room temperature for 16 hours. The reaction mixture was diluted with sodium bicarbonate solution (100 mL), extracted with dichloromethane (100 mL × 3), and the organic phases were combined, concentrated under reduced pressure to obtain a residue, which was then dissolved in tetrahydrofuran (50 mL). Cesium carbonate (6.44 g) was added to the reaction mixture, and the reaction was carried out at 70 °C for 16 hours. The reaction mixture was concentrated under reduced pressure, diluted with sodium bicarbonate solution (100 mL), extracted with ethyl acetate (100 mL × 3), and the organic phases were combined, concentrated under reduced pressure to obtain a residue, which was purified by silica gel column chromatography (volume ratio: dichloromethane/methanol = 10/1) to obtain compound 3: tert-butyl (S)-2-((tert-butoxycarbonyl)amino)-4-(3-methyl-1,2,4-oxadiazol-5-yl) butyrate (2.1 g) as a colorless liquid.

(2-2) Synthesis of Compound 4

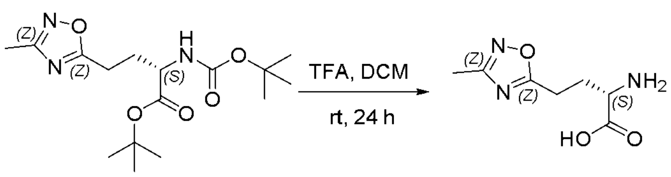


3 4

Compound 3 (2.1 g) was dissolved in dichloromethane (10 mL), and trifluoroacetic acid (10 mL) was added. The mixture was reacted at room temperature for 24 hours, and then concentrated under reduced pressure to obtain compound 4: (S)-2-amino-4-(3-methyl-1,2,4-oxadiazol-5-yl)butyric acid (1.1 g) as a colorless liquid.

(2-3) Synthesis of compound VWT024902

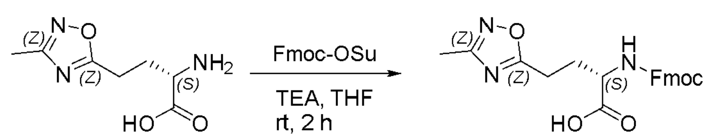


4 **VWT024902**

Compound 4 (1.1 g) and Fmoc-OSu (3 g) were dissolved in tetrahydrofuran, and triethylamine (2 g) was added. The mixture was reacted at room temperature for 2 hours, and concentrated under reduced pressure to obtain a residue, which was purified by C18 reversed-phase silica gel column chromatography to obtain compound VWT024902: (S)-2-((((9H-fluorene-9-yl)methoxy)carbonyl)amino)-4-(3-methyl-1,2,4-oxadiazol-5-yl)butyric acid (427 mg) as a white solid.

Step 3 (preparation of VWT033002)

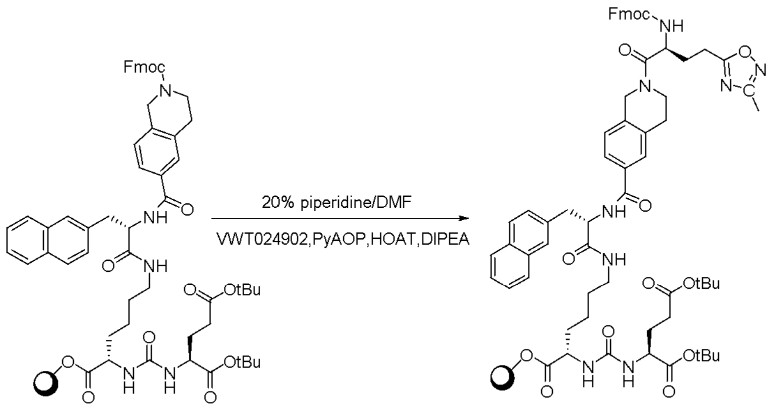


VWT024001 VWT033002

VWT024001 resin was swollen in DMF (2 mL) for 30 minutes, and washed with DMF (2 mL), then treated with 20% piperidine/DMF (2 mL) for 30 minutes to remove the Fmoc protecting group, and then washed with DMF (16 mL). The reaction was treated with a mixture of VWT024902 (50 mg), PyAOP (62 mg), HOAT (17 mg), and DIPEA (37 mg) in DMF (1 mL) for 8 hours, and the resin was then washed with DMF (12 mL) to obtain compound VWT033002.

Step 4 (preparation of VWT033003)

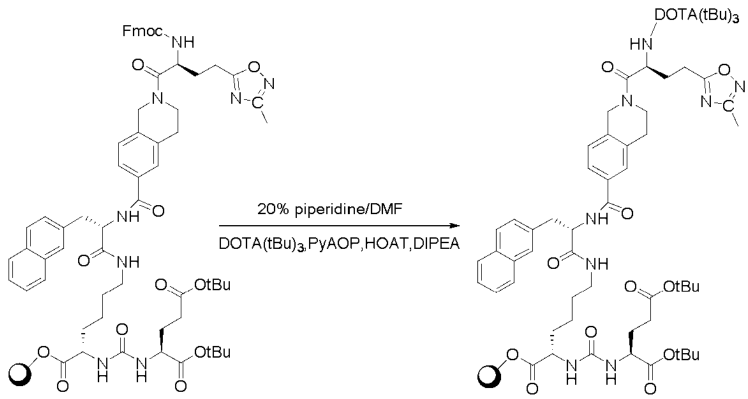


VWT033002 VWT033003

VWT033002 resin was treated with 20% piperidine/DMF (2 mL) for 30 minutes to remove the Fmoc protecting group, then washed with DMF (16 mL). The reaction was treated with a mixture of DOTA(tBu)$_3$ (72 mg), PyAOP (63 mg), HOAT (18 mg), and DIPEA (35 mg) in DMF (1 mL) for 8 hours, and the resin was then washed with DMF (12 mL) to obtain resin VWT033003.

Step 5 (preparation of VWT033000)

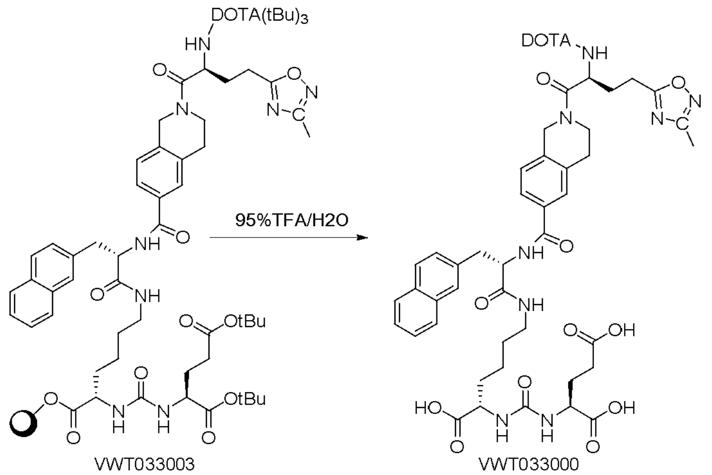

VWT033003 VWT033000

VWT033003 was treated in 95% TFA/5% $H_2O$ Cleavage Cocktail (2 mL) for 5 hours, crystallized with methyl tert-butyl ether, and centrifuged and dried to obtain crude VWT033000, which was further purified by preparative HPLC to obtain 5.7 mg of the target product VWT033000 with a purity of 90.73%; LCMS: $[M+H]^+$ =1230.10.

**Example 23**

Preparation of compound VWT037100

Step 1 (preparation of VWT024001)

VWT024001 was prepared using the same method as in Example 8.

Step 2 (preparation of VWT037102)

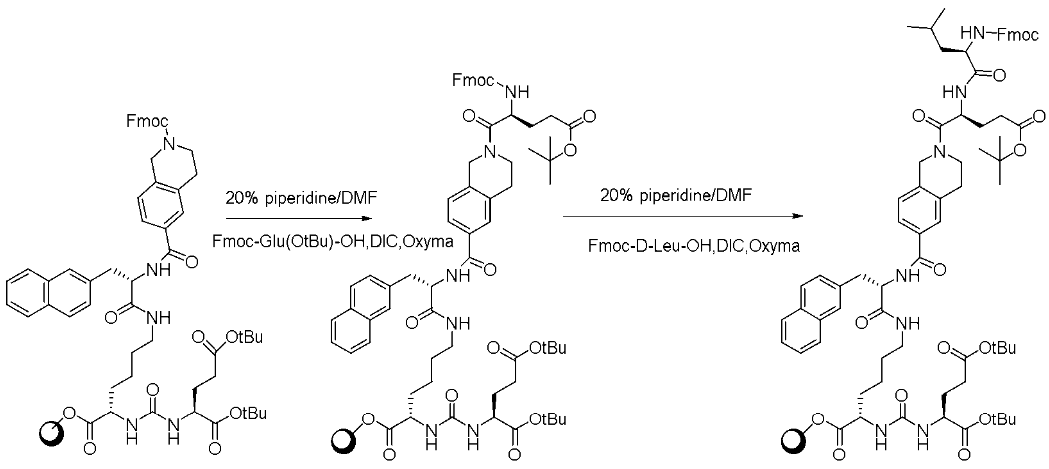

VWT024001 VWT037101 VWT037102

VWT024001 resin was washed twice with DMF (5 mL). Then, it was treated with 20% piperidine/DMF (5 mL) for 30 minutes to remove the Fmoc protecting group, and then washed with DMF (40 mL). The reaction was treated with a mixture of Fmoc-Glu(OtBu)-OH (855 mg), DIC (260 mg), and ethyl cyanoglyoxylate-2-oxime (290 mg) in DMF (3 mL) for 3 hours, and the resin was washed with DMF (30 mL) to obtain compound VWT037101.

VWT037101 resin was swollen in DMF (5 mL) for 30 minutes, and then washed twice with DMF (5 mL), then treated with 20% piperidine/DMF (5 mL) for 30 minutes to remove the Fmoc protecting group, and then washed with DMF (40 mL). The reaction was then treated with a mixture of Fmoc-D-Leu-OH (120 mg), DIC (120 mg), and ethyl cyanoglyoxylate-2-oxime (150 mg) in DMF (3 mL) for 8 hours, and the resin was washed with DMF (30 mL) to obtain VWT037102.

Step 3 (preparation of VWT037100)

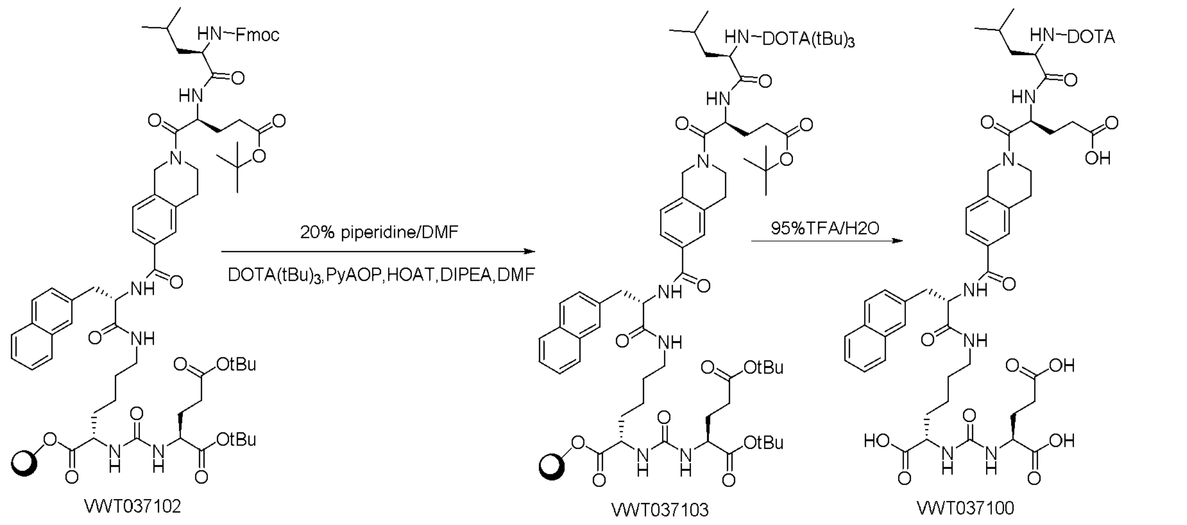


VWT037102 resin was swollen in DMF (2 mL) for 30 minutes, and washed twice with DMF (2 mL), then treated with 20% piperidine/DMF (2 mL) for 30 minutes to remove the Fmoc protecting group, and then washed with DMF (16 mL). The reaction was then treated with a mixture of DOTA(tBu)$_3$ (170 mg), PyAOP (155 mg), HOAT (43 mg), and DIPEA (50 mg) in DMF (1 mL) for 5 hours, and then the resin was washed with DMF (12 mL) to obtain resin VWT037103.

VWT037103 was then treated in 95% TFA/5% $H_2O$ Cleavage Cocktail (3 mL) for 5 hours, crystallized with methyl tert-butyl ether, and centrifuged and dried to obtain 93 mg of crude VWT037100 as a white solid, which was then purified by preparative HPLC to obtain 11.6 mg of the target product VWT037100 with a purity of 96.93%; LCMS: $[M+H]^+$ =1304.60.

## Example 24

Preparation of compound VWT038200

Step 1 (preparation of VWT024001)

VWT024001 was prepared using the same method as in Example 8.

Step 2 (preparation of VWT038202)

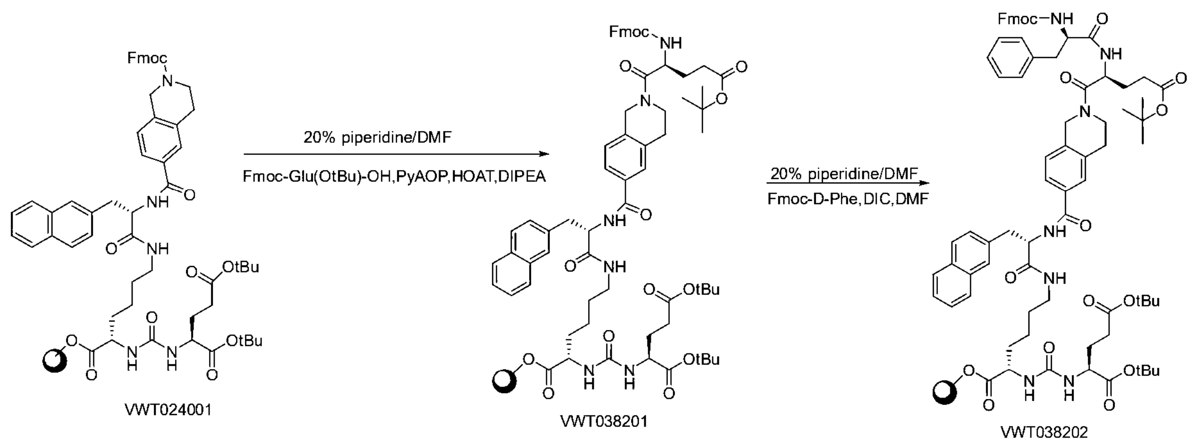


VWT024001 resin was washed twice with DMF (5 mL), then treated with 20% piperidine/DMF (5 mL) for 30

minutes to remove the Fmoc protecting group, and then washed with DMF (40 mL). The reaction was then treated with a mixture of Fmoc-Glu(OtBu)-OH (520 mg), PyAOP (630 mg), HOAT (140 mg), and DIPEA (310 mg) in DMF (5 mL) for 3 hours, and the resin was then washed with DMF (30 mL) to obtain compound VWT038201.

VWT038201 was treated with 20% piperidine/DMF (5 mL) for 30 minutes to remove the Fmoc protecting group, then washed with DMF (40 mL). The reaction was then treated with a mixture of Fmoc-D-phenylalanine (150 mg), DIC (160 mg), and ethyl cyanoglyoxylate-2-oxime (170 mg) in DMF (5 mL) for 2 hours, and the resin was then washed with DMF (30 mL) to obtain VWT038202.

Step 3 (preparation of VWT038200)

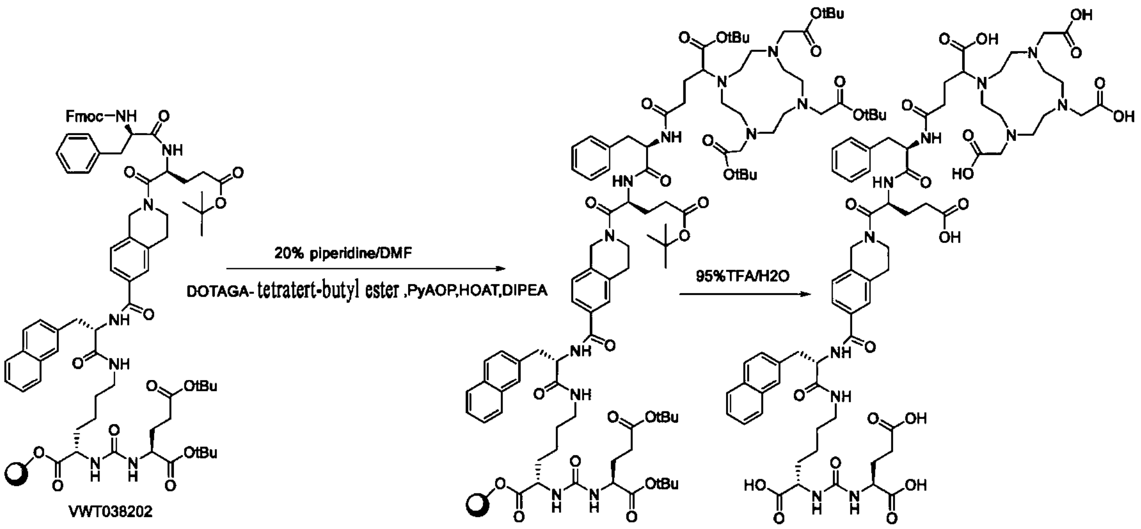


VWT038202 VWT038203 VWT038200

VWT038202 resin was swollen in DMF (2 mL) for 30 minutes, and washed with DMF (2 mL), then treated with 20% piperidine/DMF (3 mL) for 30 minutes to remove the Fmoc protecting group, and then washed with DMF (24 mL). The reaction was treated with a mixture of DOTAGA-tetratert-butyl ester (65 mg), PyAOP (50 mg), HOAT (15 mg), and DIPEA (30 mg) in DMF (1 mL) for 3.5 hours, and the resin was washed with DMF (18 mL) to obtain resin VWT038203.

The VWT038203 resin was then treated in 95% TFA/5% $H_2O$ Cleavage Cocktail (2 mL) for 5 hours, crystallized with methyl tert-butyl ether, and centrifuged and dried to obtain 101 mg of crude VWT038200 as a white solid, which was then purified by preparative HPLC to obtain 11.2 mg of the target product VWT038200; LCMS: $[M+H]^+$ =1410.60.

## Example 25

Preparation of compound VWT038600

Step 1 (preparation of VWT024001)

VWT024001 was prepared using the same method as in Example 8.

Step 2 (preparation of VWT038602)

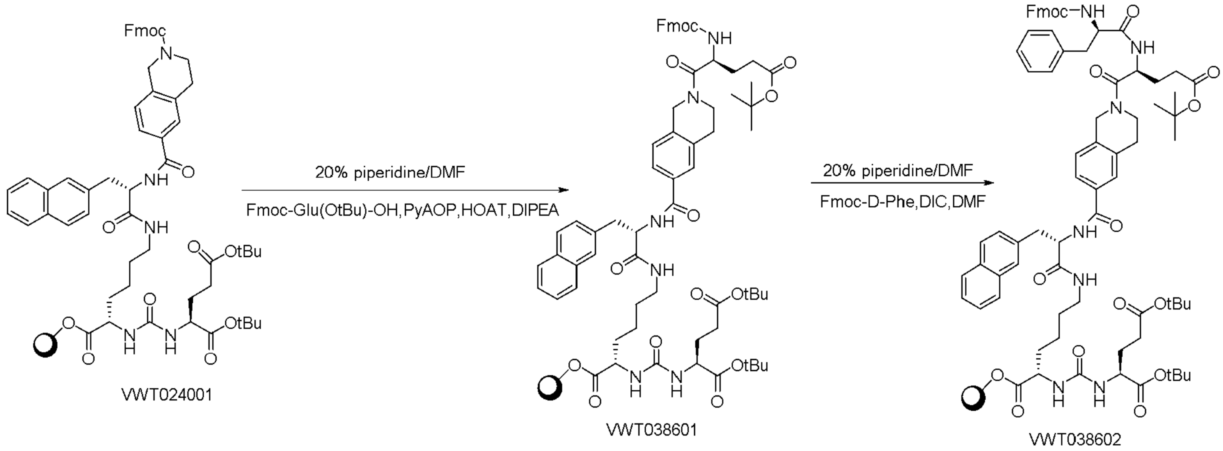


VWT024001 resin was washed twice with DMF (5 mL), then treated with 20% piperidine/DMF (5 mL) for 30 minutes to remove the Fmoc protecting group, and then washed with DMF (40 mL). The reaction was then treated with a mixture of Fmoc-Glu(OtBu)-OH (520 mg), PyAOP (630 mg), HOAT (140 mg), and DIPEA (310 mg) in DMF (5 mL) for 3 hours, and the resin was then washed with DMF (30 mL) to obtain compound VWT038601.

VWT038601 resin was treated in 20% piperidine/DMF (5 mL) for 30 minutes to remove the Fmoc protecting group, then washed with DMF (40 mL). The reaction was then treated with a mixture of Fmoc-D-phenylalanine (150 mg), DIC (160 mg), and ethyl cyanoglyoxylate-2-oxime (170 mg) in DMF (5 mL) for 2 hours, and the resin was then washed with DMF (30 mL) to obtain intermediate VWT038602.

Step 3 (preparation of VWT038600)

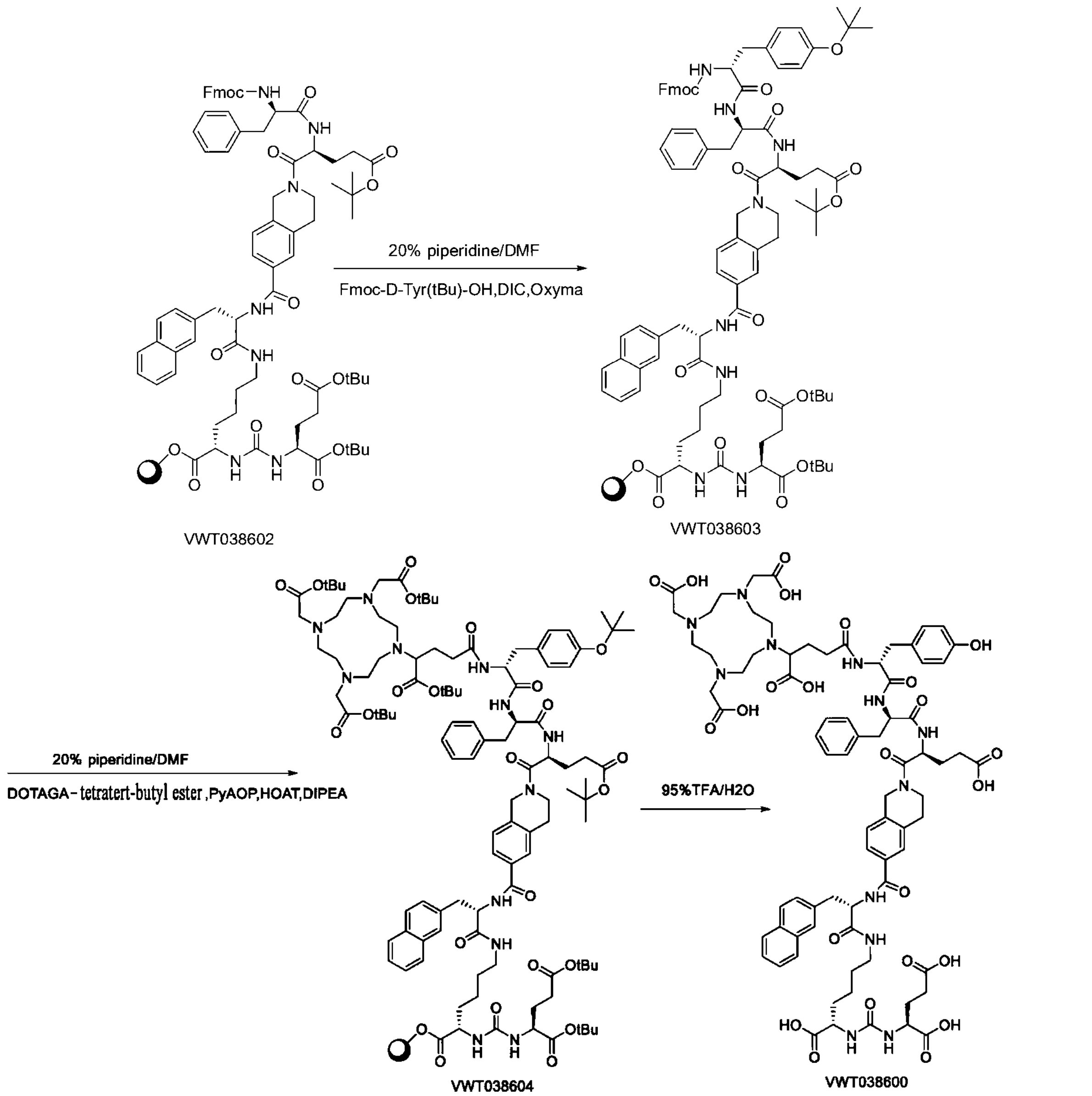


VWT038602 VWT038603 VWT038604 VWT038600

VWT038602 resin was swollen in DMF (5 mL) for 30 minutes, then washed with DMF (2 mL), and treated in 20% piperidine/DMF (3 mL) for 30 minutes to remove the Fmoc protecting group, and then washed with DMF (40 mL). The reaction was treated with a mixture of Fmoc-D-Tyr(tBu)-OH (140 mg), DIC (38 mg), and ethyl cyanoglyoxylate-2-oxime (45 mg) in DMF (3 mL) for 4 hours, and the resin was then washed with DMF (18 mL) to obtain VWT038603.

VWT038603 was treated in 20% piperidine/DMF (3 mL) for 30 minutes to remove the Fmoc protecting group, then washed with DMF (24 mL). The resin was then treated with a mixture of DOTAGA-tetratert-butyl ester (65 mg), PyAOP (50 mg), HOAT (15 mg), and DIPEA (25 mg) in DMF (1 mL) for 5 hours, and the resin was then washed with DMF (18 mL) to obtain resin VWT038604.

VWT038604 was then treated in 2.5 mL of 95% TFA/5% $H_2O$ Cleavage Cocktail for 4 hours, crystallized with methyl tert-butyl ether, and centrifuged and dried to obtain 100 mg of crude VWT038600 as a white solid, which was then purified by preparative HPLC to obtain 24.8 mg of the target product VWT038600 with a purity of 95.25%; LCMS: $[M+H]^+$ =1574.10.

**Example 26**

Preparation of compound VWT038700

Step 1 (preparation of VWT024001)

VWT024001 was prepared using the same method as in Example 8.

Step 2 (preparation of VWT038702)

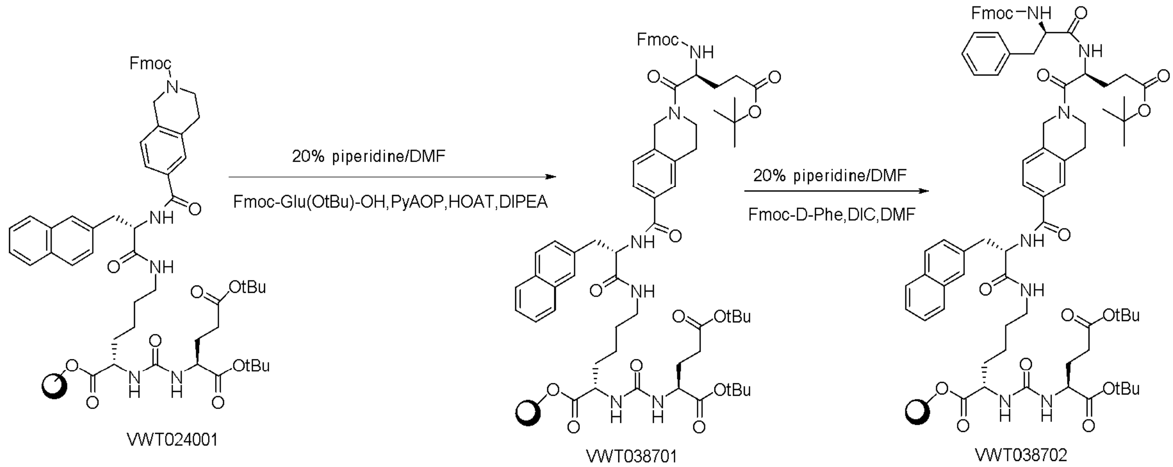


VWT024001 VWT038701 VWT038702

VWT024001 resin was washed twice with DMF (5 mL), then treated with 20% piperidine/DMF (5 mL) for 30 minutes to remove the Fmoc protecting group, and then washed with DMF (40 mL). The reaction was then treated with a mixture of Fmoc-Glu(OtBu)-OH (520 mg), PyAOP (630 mg), HOAT (140 mg), and DIPEA (310 mg) in DMF (5 mL) for 3 hours, and the resin was then washed with DMF (30 mL) to obtain compound VWT038701.

VWT038701 resin was treated in 20% piperidine/DMF (5 mL) for 30 minutes to remove the Fmoc protecting group, then washed with DMF (40 mL). The reaction was then treated with a mixture of Fmoc-D-phenylalanine (150 mg), DIC (160 mg), and ethyl cyanoglyoxylate-2-oxime (170 mg) in DMF (5 mL) for 2 hours, and the resin was then washed with DMF (30 mL) to obtain VWT038702.

Step 3 (preparation of VWT038700)

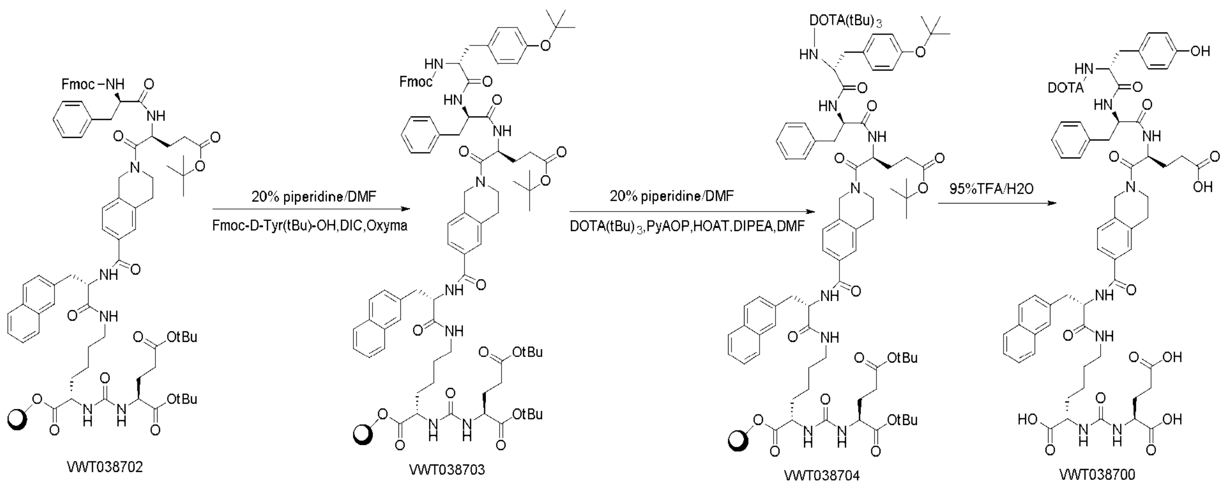


VWT038702 VWT038703 VWT038704 VWT038700

VWT038702 resin was swollen in DMF (5 mL) for 30 minutes, then washed with DMF (2 mL), and treated with 20% piperidine/DMF (3 mL) for 30 minutes to remove the Fmoc protecting group, and then washed with DMF (40 mL). The reaction was treated with a mixture of Fmoc-D-Tyr(tBu)-OH (140 mg), DIC (38 mg), and ethyl cyanoglyoxylate-2-oxime (43 mg) in DMF (3 mL) for 4 hours, and the resin was then washed with DMF (18 mL) to obtain VWT038703.

VWT038703 was treated in 20% piperidine/DMF (3 mL) for 30 minutes to remove the Fmoc protecting group,

then washed with DMF (24 mL). The reaction was then treated with a mixture of DOTA(tBu)$_3$ (105 mg), PyAOP (94 mg), HOAT (25 mg), and DIPEA (60 mg) in DMF (1 mL) for 5 hours, and the resin was then washed with DMF (18 mL) to obtain resin VWT038704.

VWT038704 was then treated in 2.5 mL of 95% TFA/5% $H_2O$ Cleavage Cocktail for 4.5 hours, crystallized with methyl tert-butyl ether, and centrifuged and dried to obtain 138 mg of crude VWT038700 as a white solid, which was then purified by preparative HPLC to obtain 21 mg of the target product VWT038700 with a purity of 95.69%; LCMS: $[M+H]^+$ =1501.70.

**Example 27**

Preparation of compound VWT039500

Step 1 (preparation of VWT024001)

VWT024001 was prepared using the same method as in Example 8.

Step 2 (preparation of VWT039502)

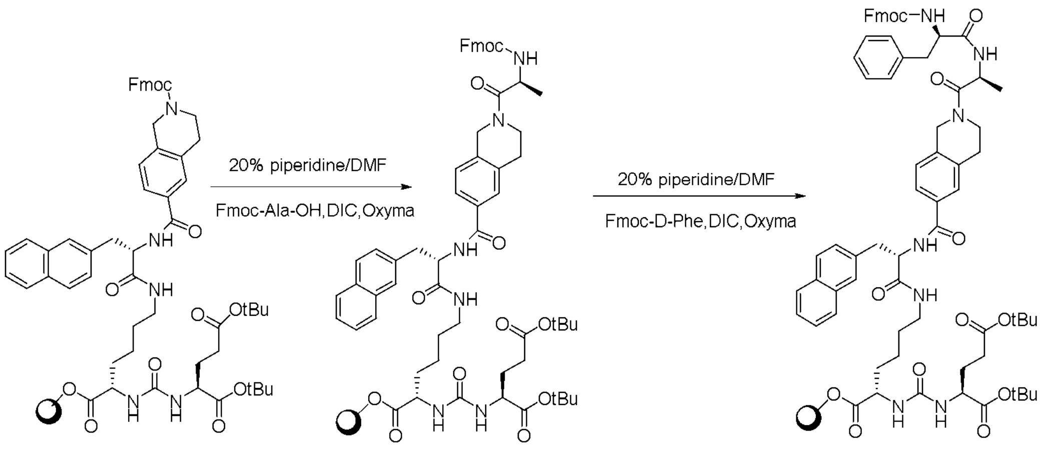


VWT024001 VWT039501 VWT039502

VWT024001 resin was treated with 20% piperidine/DMF (5 mL) for 30 minutes to remove the Fmoc protecting group, then washed with DMF (30 mL), and then coupled with Fmoc-alanine (300 mg) sequentially in the order listed in the sequence under a DIC (140 mg)/ethyl cyanoglyoxylate-2-oxime (150 mg) condensation system. The resin was then washed with DMF (30 mL), treated again with 20% piperidine/DMF (5 mL) for 30 minutes to remove the Fmoc protecting group, washed with DMF (30 mL), and then coupled with Fmoc-D-phenylalanine under a DIC (115 mg)/ethyl cyanoglyoxylate-2-oxime (130 mg) condensation system for 3 hours. The resin was then washed with DMF (30 mL) to obtain the intermediate resin VWT039502.

Step 3 (preparation of VWT039500)

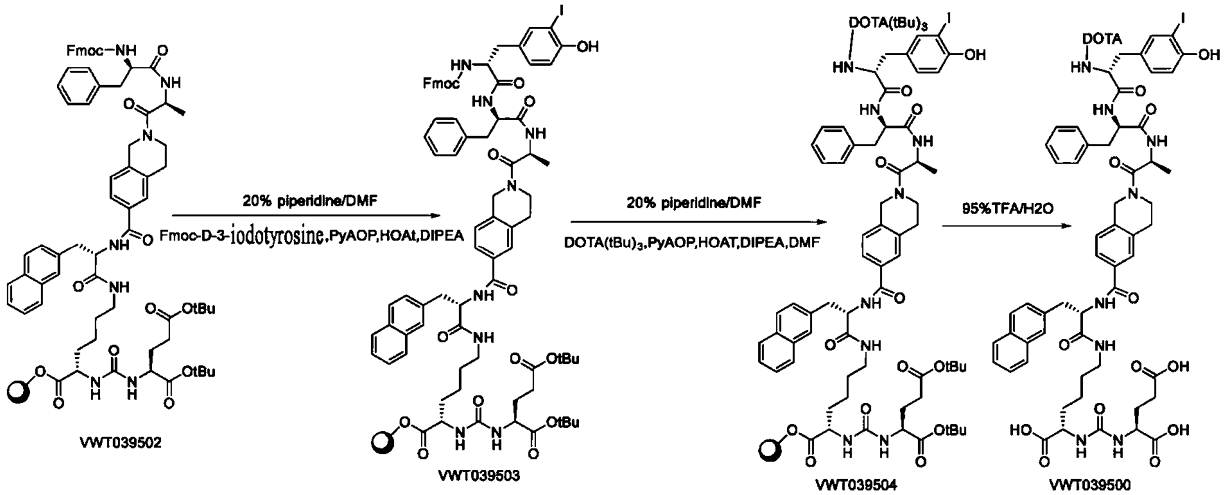


VWT039502 VWT039503 VWT039504 VWT039500

VWT039502 resin was treated in 20% piperidine/DMF (2 mL) for 30 minutes to remove the Fmoc protecting group, and washed with DMF (18 mL), then coupled with Fmoc-D-3-iodotyrosine (65 mg) sequentially in the order listed in the sequence under a PyAOP (65 mg) / HOAT (20 mg) / DIPEA (20 mg) condensation system for 8 hours. The resin was then washed with DMF (12 mL). Subsequently, the resin was treated with 20% piperidine/DMF (2 mL) for 30 minutes to remove the Fmoc protecting group, and washed with DMF (18 mL). The reaction was then treated with a mixture of DOTA(tBu)$_3$ (110 mg), PyAOP (95 mg), HOAT (30 mg), and DIPEA (50 mg) in DMF (1 mL) for 8 hours, and then the resin was washed with DMF (12 mL) to obtain resin VWT039504.

VWT039504 was then treated in 3.5 mL of 95% TFA/5% $H_2O$ Cleavage Cocktail for 4 hours, crystallized with methyl tert-butyl ether, and centrifuged and dried to obtain 160 mg of crude VWT039500 as a white solid, which was then purified by preparative HPLC to obtain 7 mg of the target product VWT039500 with a purity of 96.07%; LCMS: $[M+H]^+$ =1569.60.

## Example 28

Preparation of compound VWT039600

Step 1 (preparation of VWT024001)

VWT024001 was prepared using the same method as in Example 8.

Step 2 (preparation of VWT039602)

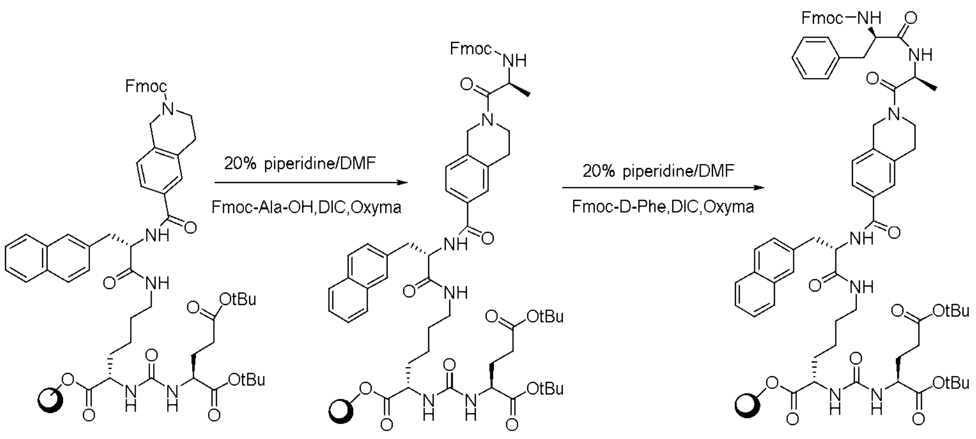


VWT024001 VWT039601 VWT039602

VWT024001 resin was treated with 20% piperidine/DMF (5 mL) for 30 minutes to remove the Fmoc protecting

group, and washed with DMF (30 mL), then coupled with Fmoc-alanine (300 mg) sequentially in the order listed in the sequence under a DIC (140 mg)/ethyl cyanoglyoxylate-2-oxime (150 mg) condensation system. The resin was then washed with DMF (30 mL), and again treated with 20% piperidine/DMF (5 mL) for 30 minutes to remove the Fmoc protecting group, then washed with DMF (30 mL), and then coupled with Fmoc-D-phenylalanine under a DIC (115 mg)/ethyl cyanoglyoxylate-2-oxime (130 mg) condensation system for 3 hours. The resin was then washed with DMF (30 mL) to obtain VWT039602.

Step 3 (preparation of VWT039600)

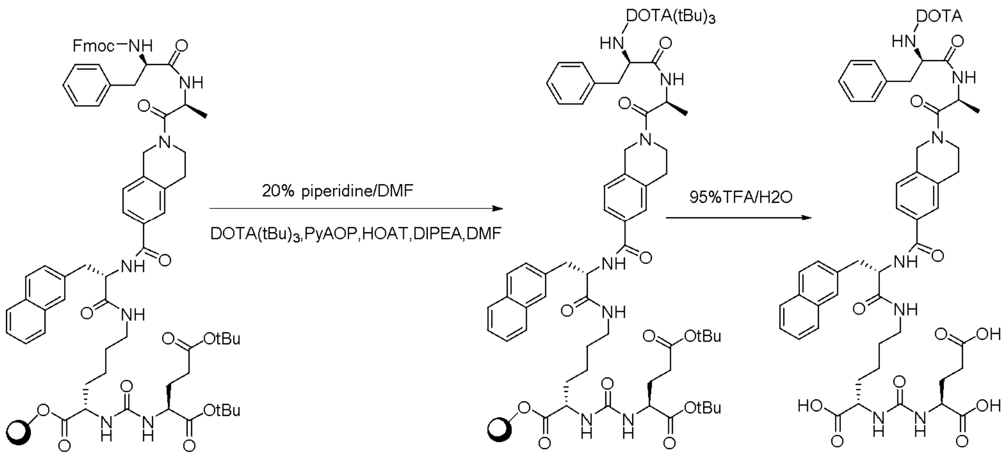


VWT039602 VWT039603 VWT039600

Resin VWT039602 was treated in 20% piperidine/DMF (5 mL) for 30 minutes to remove the Fmoc protecting group, then washed with DMF (40 mL). The reaction was then treated with a mixture of DOTA(tBu)$_3$ (105 mg), PyAOP (95 mg), HOAT (30 mg), and DIPEA (45 mg) in DMF (1 mL) for 8 hours, and the resin was then washed with DMF (30 mL) to obtain resin VWT039603.

VWT039603 was then treated in 3.5 mL of 95% TFA/5% $H_2O$ Cleavage Cocktail for 5 hours, crystallized with methyl tert-butyl ether, and centrifuged and dried to obtain 135 mg of crude VWT039600 as a white solid, which was then purified by preparative HPLC to obtain 6.6 mg of the target product VWT039600 with a purity of 92.13%.

**Example 29**

Preparation of compound VWT039700

Step 1 (preparation of VWT024001)

VWT024001 was prepared using the same method as in Example 8.

Step 2 (preparation of VWT039702)

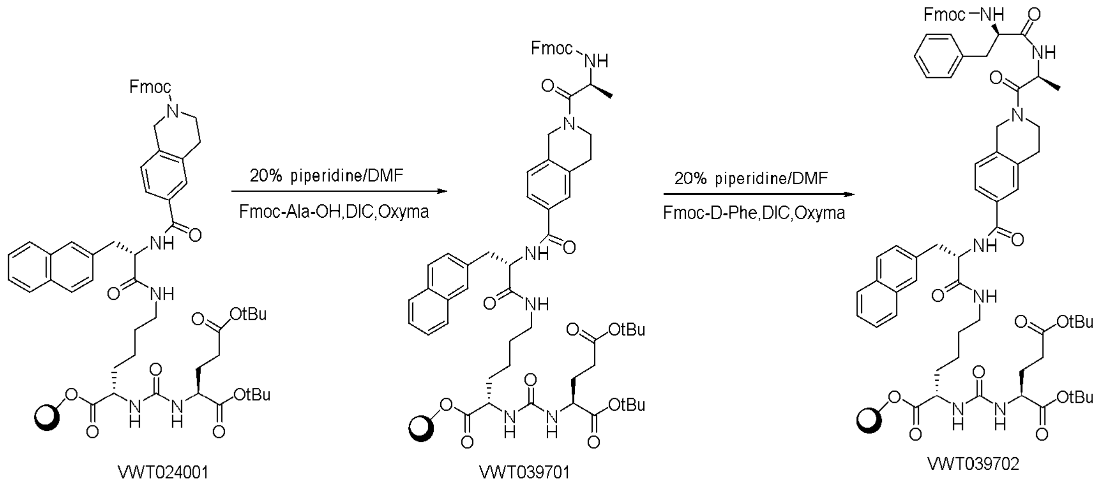


VWT024001 VWT039701 VWT039702

VWT024001 resin was treated with 20% piperidine/DMF (5 mL) for 30 minutes to remove the Fomc protecting group, and washed with DMF (30 mL), then coupled with Fmoc-alanine (300 mg) sequentially in the order listed in the sequence under a DIC (140 mg)/ethyl cyanoglyoxylate-2-oxime (150 mg) condensation system. The resin was then washed with DMF (30 mL), and treated again with 20% piperidine/DMF (5 mL) for 30 minutes to remove the Fomc protecting group, then washed with DMF (30 mL), and then coupled with Fmoc-D-phenylalanine under a DIC (115 mg)/ethyl cyanoglyoxylate-2-oxime (130 mg) condensation system for 3 hours. The resin was then washed with DMF (30 mL) to obtain VWT039702.

Step 3 (preparation of VWT039700)

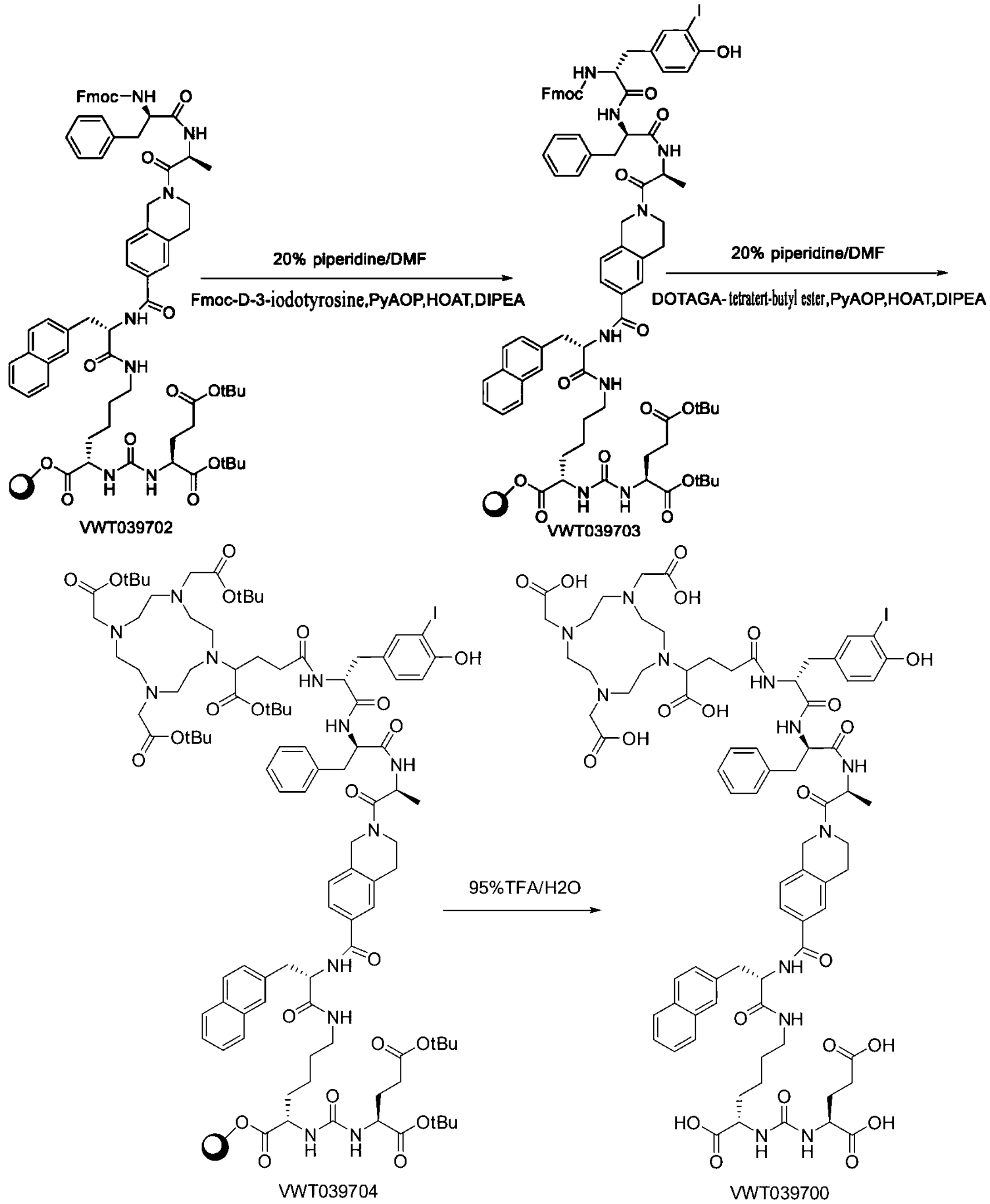


VWT039702 VWT039703 VWT039704 VWT039700

Resin VWT039702 was treated in 20% piperidine/DMF (2 mL) for 30 minutes to remove the Fmoc protecting group, then washed with DMF (18 mL), and then coupled with Fmoc-D-3-iodotyrosine (64 mg) sequentially in the order listed in the sequence under a PyAOP (63 mg)/DIPEA (45 mg) condensation system for 8 hours. The resin was then washed with DMF (12 mL), treated in 20% piperidine/DMF (2 mL) for 30 minutes to remove the Fmoc protecting group, washed with DMF (18 mL), and then treated with a mixture of DOTAGA-tetratert-butyl ester (65 mg), PyAOP (50 mg), HOAT (15 mg), and DIPEA (25 mg) in DMF (1 mL) for 8 hours. The resin was then washed with DMF (12 mL) to obtain resin VWT039704.

VWT039704 was then treated in 3.5 mL of 95% TFA/5% $H_2O$ Cleavage Cocktail for 4 hours, crystallized with methyl tert-butyl ether, and centrifuged and dried to obtain 149 mg of crude VWT039700 as white solid, which was further purified by preparative HPLC to obtain 8.6 mg of the target product VWT039700 with a purity of 94.89%.

**Example 30**

Preparation of compound VWT026000

Step 1 (preparation of VWT024001)

VWT024001 was prepared using the same method as in Example 8.

Step 2 (preparation of VWT026001)

(2-1) Synthesis of Compound 3

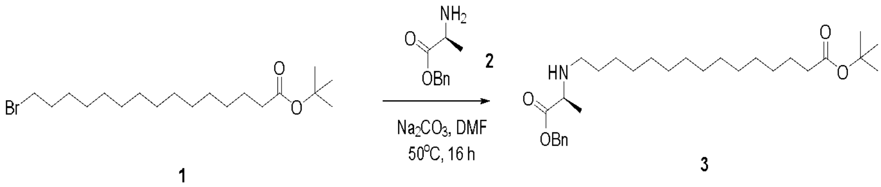


1 3

Compound 1 (tert-butyl 15-bromopentadecanoate: 1 g, 2.6 mmol, 1.0 eq) and compound 2 (benzyl L-alanine: 0.93 g, 5.2 mmol, 2.0 eq) were added to DMF (20 mL), followed by sodium carbonate (0.83 g, 7.8 mmol, 3.0 eq). The reaction mixture was reacted at 50 °C for 16 hours, concentrated under reduced pressure to obtain a residue, which was purified by silica gel column chromatography (volume ratio: petroleum ether/ethyl acetate = 5/1) to obtain compound 3 (tert-butyl (S)-15-((1-(benzyloxy)-1-oxopropane-2-yl)amino)pentadecanoate: 0.65 g, 54%) as a colorless liquid.

(2-2) Synthesis of Compound 4

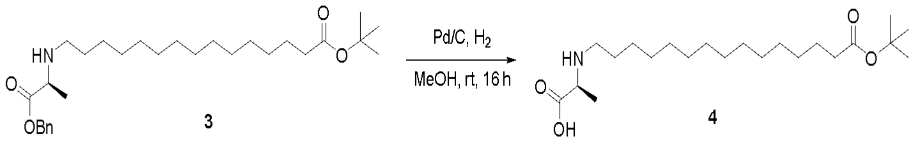


Compound 3 (650 mg, 1.4 mmol, 1.0 eq) was dissolved in methanol (20 mL), and palladium on carbon catalyst (100 mg) was added. The mixture was reacted with hydrogen at room temperature for 16 hours. After filtration, the filtrate was concentrated to obtain compound 4 ((15-(tert-butoxy)-15-oxopentadecyl)-L-alanine: 450 mg, 85%) as a colorless liquid.

(2-3) Synthesis of compound VWT026001

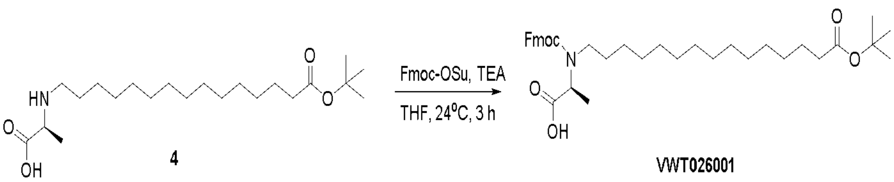


Compound 4 (400 mg, 1.0 mmol, 1.0 eq) and Fmoc-OSu (524 mg, 1.6 mmol, 1.5 eq) were dissolved in tetrahydrofuran, and triethylamine (314 mg, 3.1 mmol, 3.0 eq) was added. The reaction mixture was reacted at 24 °C for 3 h, concentrated under reduced pressure to obtain a residue, which was purified by C18 reversed-phase silica gel column chromatography to obtain compound VWT026001 (N-(((9H-fluorene-9-yl)methoxy)carbonyl)-N-(15-(tert-butoxy)-15-oxopentadecyl)-L-alanine: 146.3 mg, 23%) as a white solid.

Step 3 (Synthesis of VWT026002)

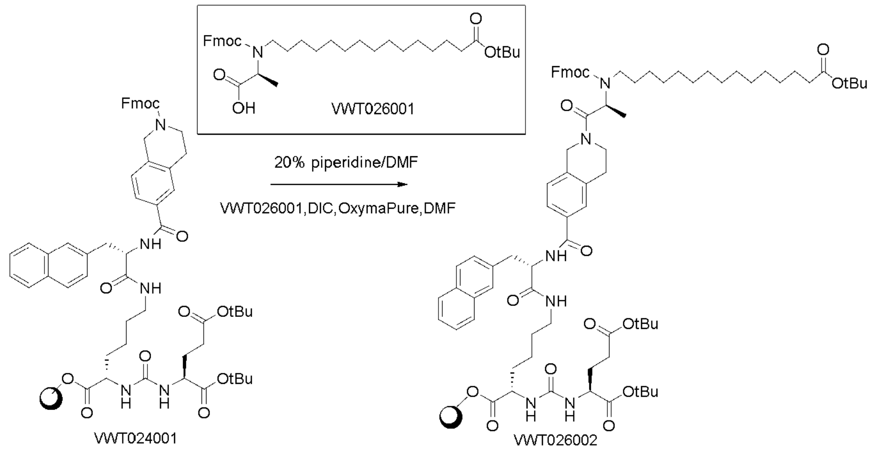


VWT024001 resin was swollen in DMF (2 mL) for 30 minutes, then washed with DMF (2 mL), and then treated with 20% piperidine/DMF (2 mL) for 30 minutes to remove the Fmoc protecting group, then washed with DMF (16 mL). The reaction was treated with a mixture of VWT026001 (97 mg), DIC (22 mg), and ethyl cyanoglyoxylate-2-oxime (25 mg) in DMF (0.5 mL) for 4 hours, and the resin was then washed with DMF (12 mL) to obtain compound VWT026002.

Step 4 (Synthesis of VWT026000)

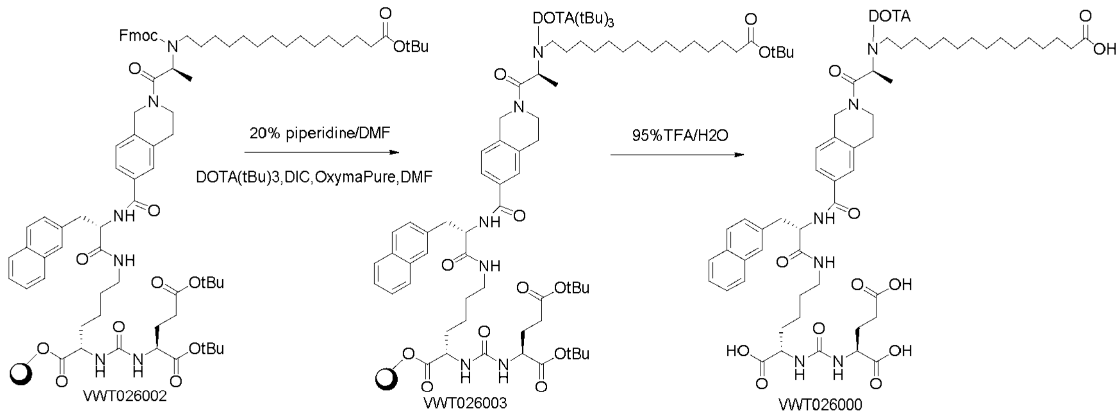


VWT026002 resin was treated with 20% piperidine/DMF (2 mL) for 30 minutes to remove the Fmoc protecting group, then coupled with compound DOTA(tBu)$_3$ (229mg) under a DIC (50 mg)/ethyl cyanoglyoxylate-2-oxime (57 mg) condensation system. After 7 hours of reaction, the resin was washed with DMF (12 mL) to obtain VWT026003. VWT026003 was then treated with 95% TFA/5% $H_2O$ Cleavage Cocktail (3 mL) for 6 hours, crystallized with methyl tert-butyl ether, and centrifuged and dried to obtain crude VWT026000, which was then purified by preparative HPLC to obtain 10 mg of the target product VWT026000 with a purity of 97.39%; LCMS: $[M+H]^+$ =1373.80.

**Example 31**

Preparation of compound VWT035700

Step 1 (preparation of VWT024001)

VWT024001 was prepared using the same method as in Example 8.

Step 2 (preparation of VWT035701)

(2-1) Synthesis of Compound 6

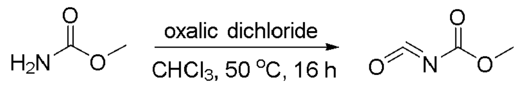


11 6

Compound 11 (methyl carbamate: 3.0 g) and oxaloyl chloride (7.6 g) were added to a chloroform (30 mL) solution at room temperature and reacted at 50 °C for 16 hours. The reaction mixture was filtered and washed with dichloromethane. The filtrate was concentrated to obtain compound 6 (methyl isocyanate: 2.0 g) as a white solid.

(2-2) Synthesis of Compound 2

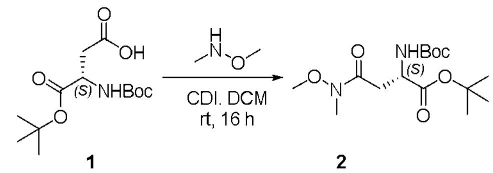


1 2

CDI (N,N'-carbonyldiimidazole: 1.78 g) was added to a solution of compound 1 ((S)-4-(tert-butoxy)-3-((tert-butoxycarbonyl)amino)-4-oxobutyric acid: 2.89 g) in dichloromethane (50 mL), and the reaction was carried out at room temperature for 1 hour. Then, N,O-dimethylhydroxylamine (974 mg) was added, and the reaction was continued at room temperature for 16 hours. Ethyl acetate was added to the reaction solution, and the mixture was washed with 1N hydrochloric acid, saturated sodium bicarbonate solution, and saturated brine, then dried over anhydrous sodium sulfate, and concentrated to obtain compound 2 (tert-butyl N2-(tert-butoxycarbonyl)-N4-methoxy-N4-methyl-L-asparaginic acid: 2.9 g) as a colorless liquid.

(2-3) Synthesis of Compound 3

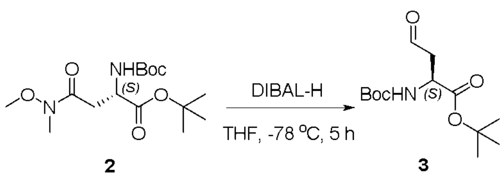


2 3

DIBAL-H (diisobutylaluminum hydride: 11.4 mL) was added to a solution of compound 2 (1.9 g) in tetrahydrofuran (50 mL) at -78 °C, and the reaction was carried out at -78 °C for 5 hours. The reaction mixture was quenched with sodium bisulfate solution and extracted with ethyl acetate. The combined organic phases were washed with 1N hydrochloric acid, saturated sodium bicarbonate solution and saturated brine, dried over anhydrous sodium sulfate, and concentrated to give compound 3 (tert-butyl (S)-2-((tert-butoxycarbonyl)amino)-4-oxobutyrate: 1.5 g), which was a colorless liquid.

(2-4) Synthesis of Compound 4

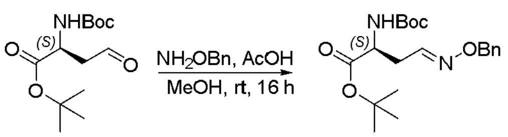


3 4

Compound 3 (1.5 g), o-benzylhydroxylamine (676 mg), and acetic acid (0.1 mL) were added to methanol (20 mL), and the reaction was carried out at room temperature for 16 hours. The reaction mixture was added with ethyl acetate, washed with water and saturated brine, dried over anhydrous sodium sulfate, and concentrated to obtain a residue, which was purified by silica gel column chromatography (volume ratio: petroleum ether/ethyl acetate = 10/1) to obtain compound 4 (tert-butyl (S,E)-4-((benzyloxy)imino)-2-((tert-butoxycarbonyl)amino)butyrate: 1.5 g) as a colorless liquid.

(2-5) Synthesis of Compound 5

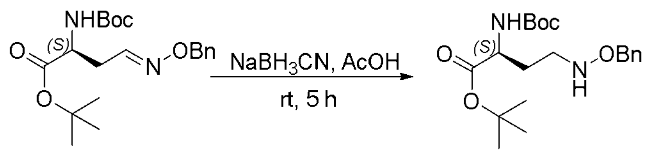


4 5

Sodium cyanoborohydride (498 mg) was added to a solution of compound 4 (1.5 g) in acetic acid (20 mL), and the reaction was allowed to carry out at room temperature for 5 hours. The reaction mixture was added with water, and adjusted to neutral pH with sodium hydroxide, then extracted with ethyl acetate. The combined organic phases were washed with water and saturated brine, dried over anhydrous sodium sulfate, and concentrated to obtain a residue, which was purified by silica gel column chromatography (volume ratio: petroleum ether/ethyl acetate = 3/1) to obtain compound 5 (tert-butyl (S)-4-((benzyloxy)amino)-2-((tert-butoxycarbonyl)amino)butyrate: 1.35 g) as a colorless liquid.

(2-6) Synthesis of Compound 7

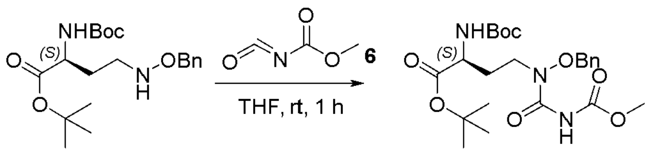


5 7

Compound 6 (531 mg) was added to a solution of compound 5 (1.0 g) in tetrahydrofuran (20 mL), and the reaction was carried out at room temperature for 1 hour. The reaction mixture was concentrated and purified by silica gel column chromatography (volume ratio: petroleum ether/ethyl acetate = 1/1) to obtain compound 7 (tert-butyl (S)-4-(1-(benzyloxy)-3-(methoxycarbonyl)ureido)-2-((tert-butoxycarbonyl)amino)butyrate: 900 mg) as a colorless liquid.

(2-7) Synthesis of Compound 8

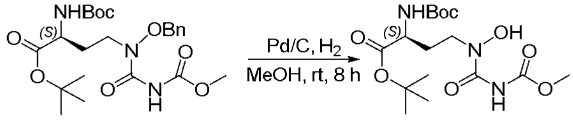


7 8

Pd/C (100 mg) was added to a solution of compound 7 (900 mg) in methanol (20 mL), and the reaction was carried out at room temperature with hydrogen for 8 hours. After filtering the solid, the filtrate was concentrated to obtain compound 8 (tert-butyl (S)-2-((tert-butoxycarbonyl)amino)-4-(1-hydroxy-3-(methoxycarbonyl)ureido)butyrate: 688 mg) as a white solid.

(2-8) Synthesis of Compound 9

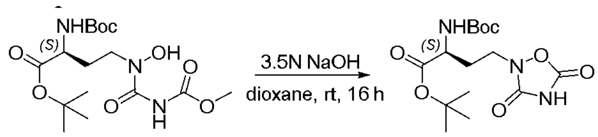


**8** **9**

A 3.5 mol/L sodium hydroxide aqueous solution (1 mL) was added to a solution of compound 8 (680 mg) in dioxane (10 mL), and the reaction was carried out at room temperature for 16 hours. The reaction mixture was concentrated under reduced pressure to remove dioxane. The residue was adjusted to pH 5 with 1N hydrochloric acid, and extracted with ethyl acetate. The combined organic phases were washed with saturated brine, dried over anhydrous sodium sulfate, and concentrated to obtain compound 9 (tert-butyl (S)-2-((tert-butoxycarbonyl)amino)-4-(3,5-dioxo-1,2,4-oxadiazolidine-2-yl)butyrate: 588 mg) as a colorless liquid.

(2-9) Synthesis of Compound 10

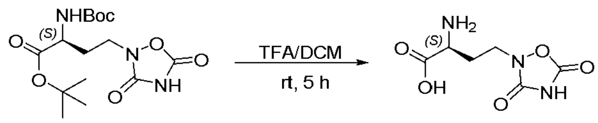


**9** **10**

Trifluoroacetic acid (1 mL) was added to a solution of compound 9 (588 mg) in dichloromethane (5 mL), and the reaction was carried out at room temperature for 5 hours. The reaction mixture was concentrated to obtain compound 10 ((S)-2-amino-4-(3,5-dioxo-1,2,4-oxadiazolidine-2-yl)butyric acid: 332 mg) as a colorless liquid.

(2-10) Synthesis of compound VWT035701

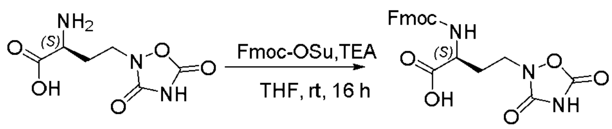


**10** **VWT035701**

Fmoc-OSu (826.9 mg) and triethylamine (826.8 mg) were added to a solution of compound 10 (332 mg) in tetrahydrofuran (10 mL), and the reaction was carried out at room temperature for 16 hours. The reaction mixture was concentrated and purified by C18 reverse-phase column to obtain compound VWT035701 ((S)-2-((((9H-fluorene-9-yl) methoxy)carbonyl)amino)-4-(3,5-dioxo-1,2,4-oxadiazolidine-2-yl)butyric acid: 263.8 mg) as a white solid.

Step 3 (preparation of VWT035702)

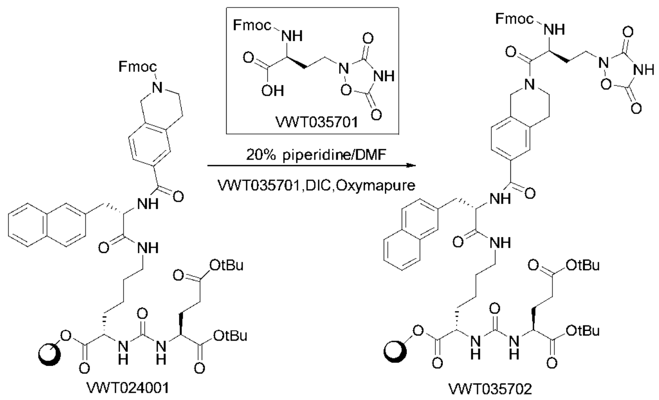


VWT024001 resin was swollen in DMF (2 mL) for 30 minutes, washed with DMF (2 mL), then treated with 20% piperidine/DMF (2 mL) for 30 minutes to remove the Fmoc protecting group. The resin was then washed with DMF (16 mL), and reacted with VWT035701 (52 mg), DIC (16 mg), and ethyl cyanoglyoxylate-2-oxime (20 mg) in DMF (3 mL) for 8 hours. The resin was then washed with DMF (12 mL) to obtain compound VWT035702.

Step 4 (Preparation of compound VWT035700)

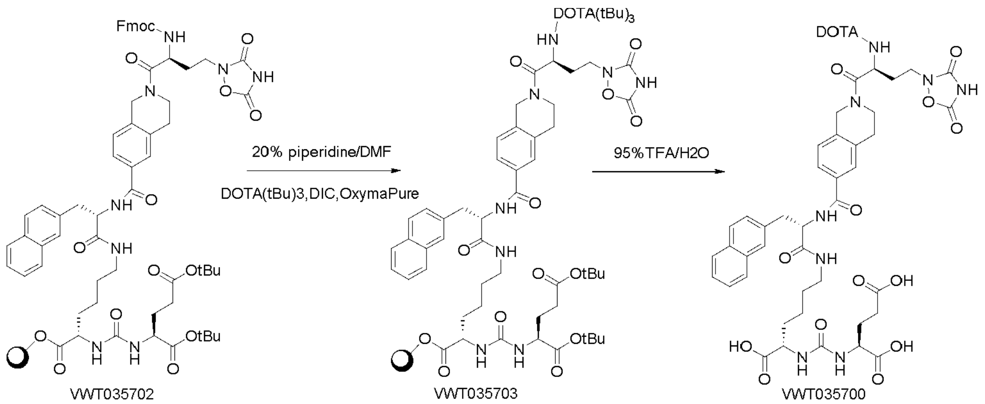


VWT035702 resin was treated with 20% piperidine/DMF (2 mL) for 30 minutes to remove the Fmoc protecting group, then coupled with DOTA(tBu)$_3$ (229mg) under a condensation system of DIC (27 mg) and ethyl cyanoglyoxylate-2-oxime (35 mg). After reacting for 3 hours, the resin was washed with DMF (12 mL) to obtain resin VWT035703. VWT035703 was then treated with 95% TFA/5% $H_2O$ Cleavage Cocktail (2 mL) for 6 hours, crystallized by methyl tert-butyl ether, centrifuged and dried to obtain crude VWT035700, which was then purified by preparative HPLC to obtain 16 mg of the target product VWT035700 with a purity of 93.87%; LCMS: $[M+H]^+$ =1247.60.

**Example 32**

Preparation of compound VWT037200

Step 1 (preparation of VWT024001)

VWT024001 was prepared using the same method as in Example 8.

Step 2 (preparation of VWT037202)

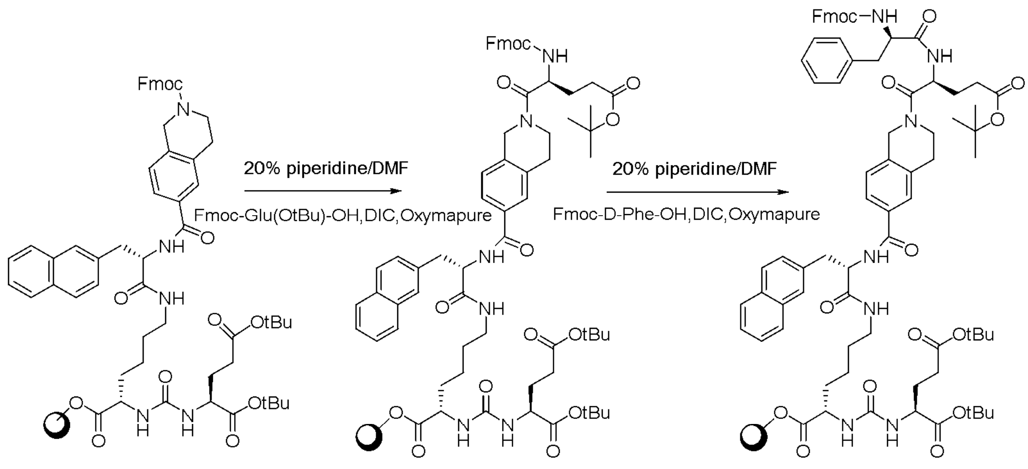


VWT024001 VWT037201 VWT037202

VWT024001 resin was washed with DMF (5 mL), then treated with 20% piperidine/DMF (5 mL) for 30 minutes to remove the Fmoc protecting group. The resin was then washed with DMF (40 mL), and then reacted with Fmoc-Glu(OtBu)-OH (855 mg), DIC (260 mg), and ethyl cyanoglyoxylate-2-oxime (290 mg) in DMF (3 mL) for 3 hours. The resin was then washed with DMF (30 mL) to obtain compound VWT037201.

VWT037201 resin was swollen in DMF (5 mL) for 30 minutes, washed with DMF (5 mL), then treated with 20% piperidine/DMF (5 mL) for 30 minutes to remove the Fmoc protecting group. The resin was then washed with DMF (40 mL), and then reacted with Fmoc-D-Phe-OH (CAS No.: 86123-10-6; 120 mg), DIC (125 mg), and ethyl cyanoglyoxylate-2-oxime (150 mg) in DMF (3 mL) for 6 hours. The resin was then washed with DMF (30 mL) to obtain VWT037202.

Step 3 (Preparation of compound VWT037200)

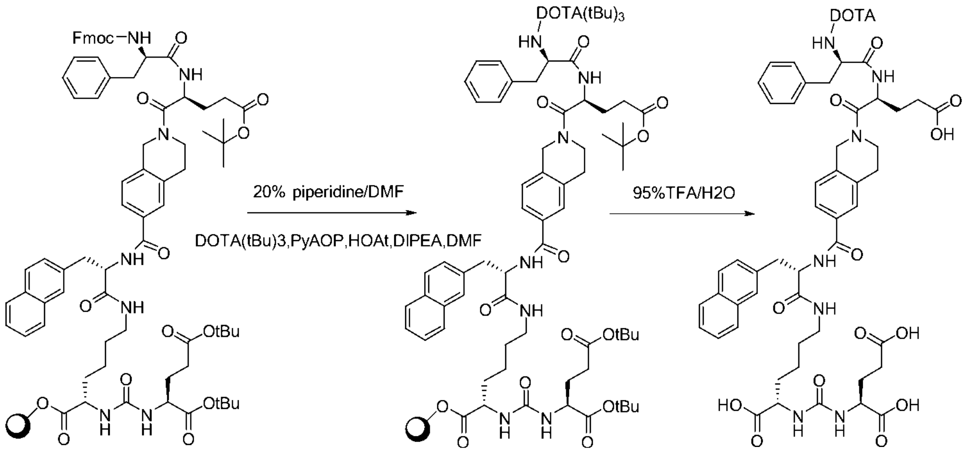


VWT037202 VWT037203 VWT037200

VWT037202 resin was swollen in DMF (2 mL) for 30 minutes, washed with DMF (2 mL), then treated with 20% piperidine/DMF (2 mL) for 30 minutes to remove the Fmoc protecting group, and washed with DMF (16 mL), and then reacted with DOTA(tBu)$_3$ (170mg), PyAOP (158 mg), HOAT (43 mg), and DIPEA (77 mg) in DMF (3 mL) for 6 hours. The resin was then washed with DMF (12 mL) to obtain resin VWT037203. VWT037203 was then treated in 95% TFA/5% $H_2O$ Cleavage Cocktail (3 mL) for 5 hours, crystallized with methyl tert-butyl ether, and centrifuged and dried to obtain 96 mg of crude VWT037200, which was further purified by preparative HPLC to obtain 30 mg of the target product VWT037200 with a purity of 96.37%; LCMS: $[M+H]^+$ =1338.70.

## Example 33

Preparation of compound VWT037400

Step 1 (preparation of VWT024001)

VWT024001 was prepared using the same method as in Example 8.

Step 2 (preparation of VWT037402)

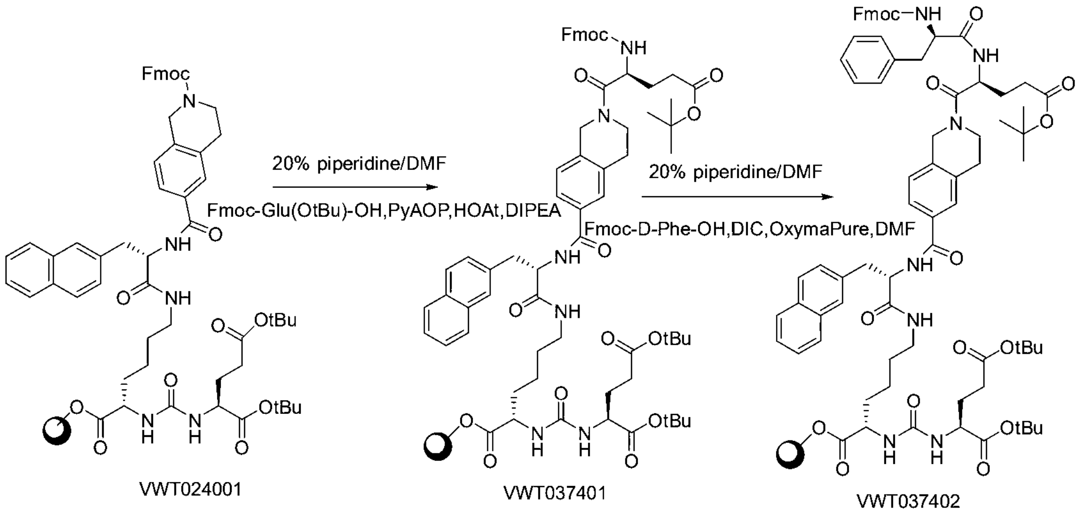


VWT024001 VWT037401 VWT037402

VWT024001 resin was washed with DMF (5 mL), then treated with 20% piperidine/DMF (5 mL) for 30 minutes to remove the Fmoc protecting group, and then washed with DMF (40 mL). The reaction was treated with a mixture of Fmoc-Glu(OtBu)-OH (259 mg), PyAOP (310 mg), HOAT (70 mg), and DIPEA (150 mg) in DMF (1 mL) for 3 hours, and the resin was then washed with DMF (12 mL) to obtain compound VWT037401.

VWT037401 resin was treated with 20% piperidine/DMF (5 mL) for 30 minutes to remove the Fmoc protecting group, then washed with DMF (40 mL). The reaction was then treated with a mixture of Fmoc-D-Phe-OH (36 mg), DIC (38 mg), and ethyl cyanoglyoxylate-2-oxime (45 mg) in DMF (1.5 mL) for 2 hours, and the resin was then washed with DMF (12 mL) to obtain VWT037402.

Step 3 (preparation of VWT037404)

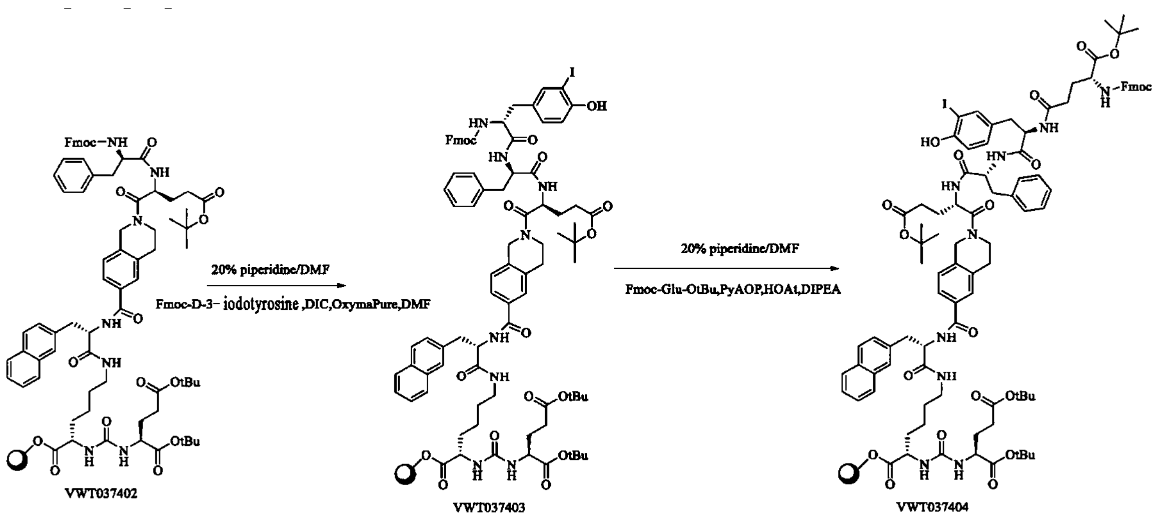


VWT037402 VWT037403 VWT037404

VWT037402 resin was swollen in DMF (5 mL) for 30 minutes, then washed with DMF (10 mL), and then treated with 20% piperidine/DMF (5 mL) for 30 minutes to remove the Fmoc protecting group, and then washed with DMF (40 mL).

The reaction was treated with a mixture of Fmoc-D-3-iodotyrosine (70 mg), DIC (18 mg), and ethyl cyanoglyoxylate-2-oxime (20 mg) in DMF (3 mL) for 3 hours, and the resin was then washed with DMF (18 mL) to obtain VWT037403.

VWT037403 resin was treated with 20% piperidine/DMF (3 mL) for 30 min to remove the Fmoc protecting group, then washed with DMF (24 mL). The reaction was then treated with a mixture of N-(9-fluorenylmethoxycarbonyl)-D-glutamic acid-1-tert-butyl ester (Fmoc-Glu-OtBu, 256 mg), PyAOP (315 mg), HOAT (70 mg), and DIPEA (160 mg) in DMF (3 mL) for 4 h. The resin was then washed with DMF (18 mL) to obtain VWT037404.

Step 4 (preparation of DOTA(tBu)$_3$-NHS)

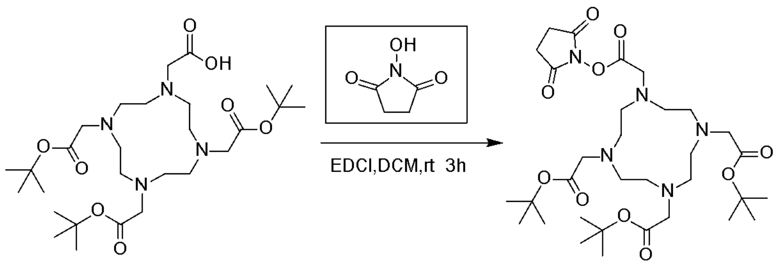


5.00 g of DOTA(tBu)$_3$ was added to 500 mL of dichloromethane (DCM) and stirred until dissolved. 5.00 g of HOSU and 8.35 g of EDCI were added at room temperature and the mixture was stirred continuously for 3 hours at room temperature. After the reaction was complete, the mixture was washed three times with saturated brine, separated, and the organic phases were combined. The mixture was then concentrated and distilled to obtain 7.14 g of DOTA(tBu)$_3$-NHS.

Step 5 (preparation of VWT037400)

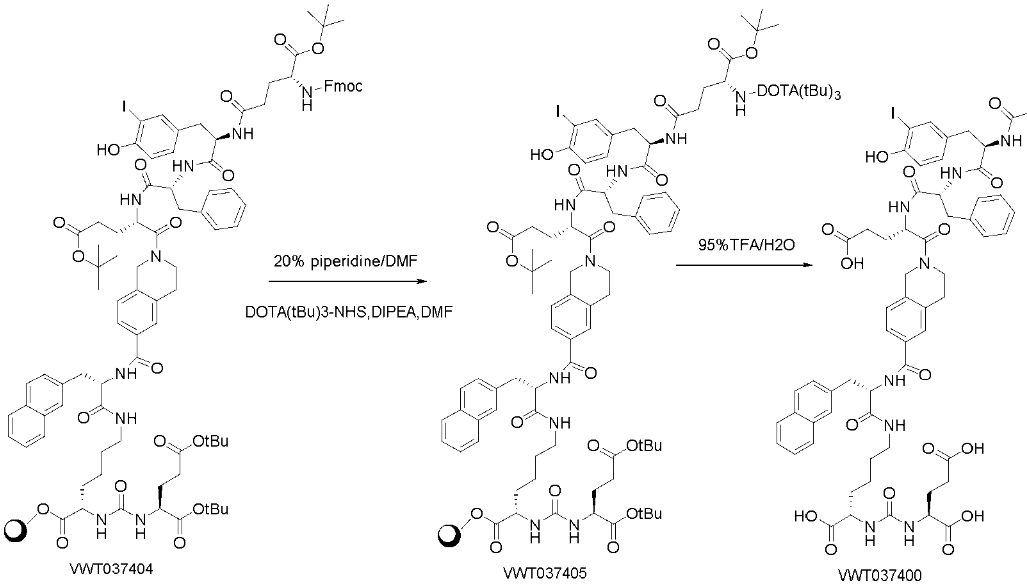


VWT037404 resin was treated with 20% piperidine/DMF (3 mL) for 30 minutes to remove the Fmoc protecting group, then washed with DMF (24 mL). The reaction was treated with a mixture of DOTA(tBu)$_3$-NHS (80mg) and DIPEA (35 mg) in DMF (1 mL) for 5 hours. The resin was then washed with DMF (12 mL) to obtain resin VWT037405. VWT037405 was then treated with 95% TFA/5% $H_2O$ Cleavage Cocktail (3 mL) for 4.5 hours, crystallized with methyl tert-butyl ether, and centrifuged and dried to obtain 122 mg of crude VWT037400, which was then purified by preparative HPLC to obtain 14.5 mg of the target product VWT037400 with a purity of 95.45%; LCMS: [M+H] $^+$ =1757.50.

**Example 34**

Preparation of compound VWT038300

Step 1 (preparation of VWT024001)

VWT024001 was prepared using the same method as in Example 8.

Step 2 (preparation of VWT038302)

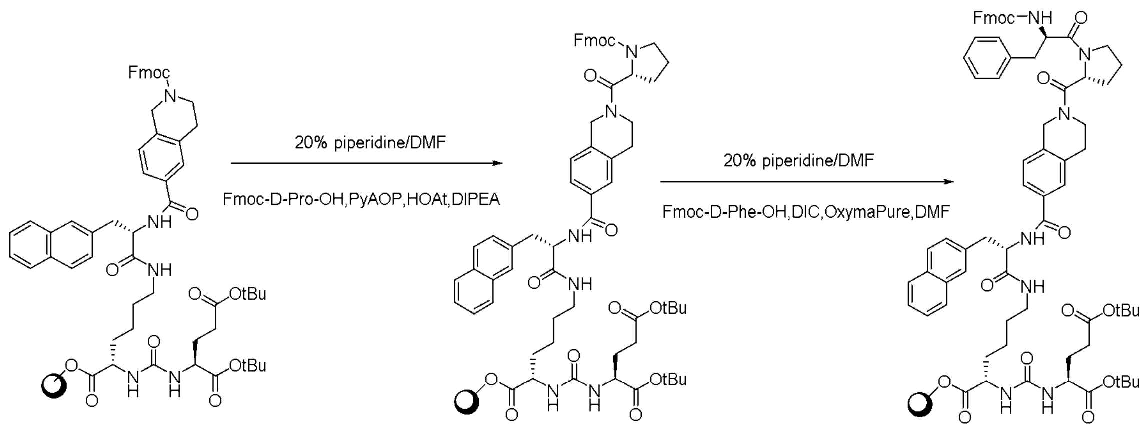


VWT024001 VWT038301 VWT038302

VWT024001 resin was washed with DMF (5 mL), then treated with 20% piperidine/DMF (5 mL) for 30 minutes to remove the Fmoc protecting group, and then washed with DMF (40 mL). The reaction was treated with a mixture of Fmoc-D-Pro-OH (140 mg), PyAOP (230 mg), HOAT (65 mg), and DIPEA (115 mg) in DMF (1 mL) for 3 hours, and the resin was then washed with DMF (12 mL) to obtain compound VWT038301.

VWT038301 was treated with 20% piperidine/DMF (5 mL) for 30 minutes to remove the Fmoc protecting group, then washed with DMF (40 mL). The reaction was then treated with a mixture of Fmoc-D-Phe-OH (36 mg), DIC (38 mg), and ethyl cyanoglyoxylate-2-oxime (45 mg) in DMF (5 mL) for 3 hours, and the resin was then washed with DMF (10 mL) to obtain VWT038302.

Step 3 (preparation of VWT038300

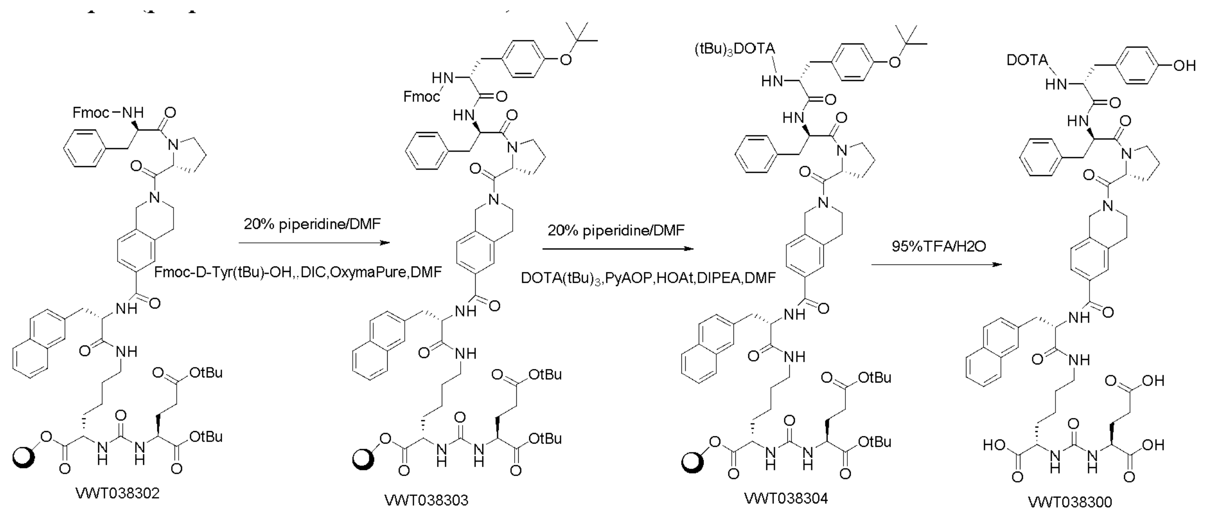


VWT038302 VWT038303 VWT038304 VWT038300

VWT038302 resin was swollen in DMF (5 mL) for 30 minutes, then washed with DMF (10 mL), and then treated with 20% piperidine/DMF (5 mL) for 30 minutes to remove the Fmoc protecting group, and then washed with DMF (40 mL).

The reaction was treated with a mixture of Fmoc-D-Tyr(tBu)-OH (136 mg), DIC (40 mg), and ethyl cyanoglyoxylate-2-oxime (50 mg) in DMF (3 mL) for 3 hours, and the resin was then washed with DMF (18 mL) to obtain VWT038303.

VWT038303 was treated in 20% piperidine/DMF (5 mL) for 30 minutes to remove the Fmoc protecting group, then washed with DMF (40 mL). The reaction was treated with a mixture of $DOTA(tBu)_3$ (110mg), PyAOP (94 mg), HOAT (25 mg), and DIPEA (60 mg) in DMF (1 mL) for 4 hours, and the resin was then washed with DMF (12 mL) to obtain resin VWT038304.

VWT038304 was then treated in 95% TFA/5% $H_2O$ Cleavage Cocktail (2 mL) for 5 hours, crystallized with methyl tert-butyl ether, and centrifuged and dried to obtain 124 mg of crude VWT038300, which was then purified by preparative HPLC to obtain 5.43 mg of the target product VWT038300 with a purity of 99.46%; LCMS: $[M+H]^+$ =1469.70.

**Example 35**

Preparation of compound VWT038400

Step 1 (preparation of VWT038402)

VWT038402 is the same as VWT038302, and VWT038402 was prepared using the same method as in Example 34.

Step 2 (preparation of VWT038400)

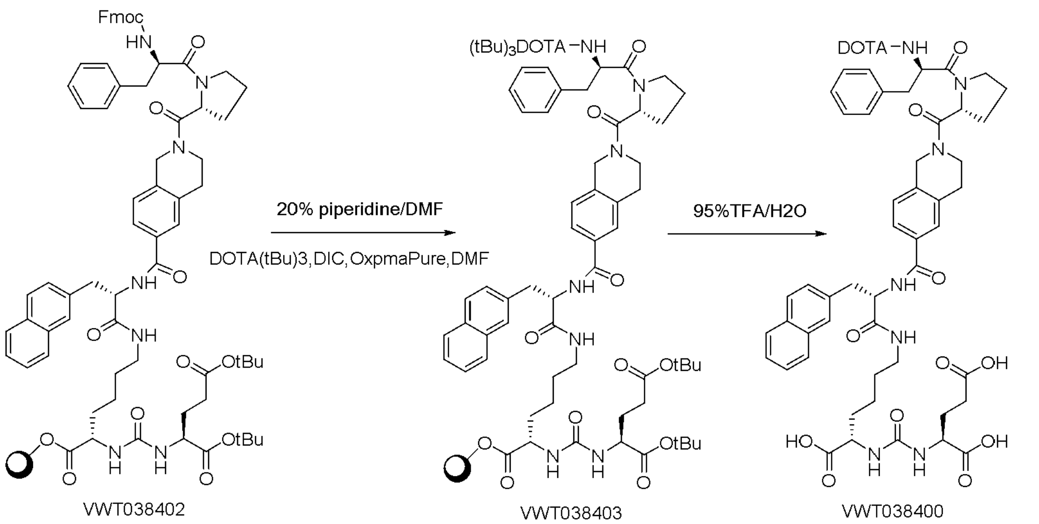


VWT038402 resin was swollen in DMF (5 mL) for 30 minutes, then washed with DMF (10 mL), and then treated with 20% piperidine/DMF (5 mL) for 30 minutes to remove the Fmoc protecting group, then washed with DMF (40 mL). The reaction was treated with a mixture of $DOTA(tBu)_3$ (105mg), DIC (26 mg), and ethyl cyanoglyoxylate-2-oxime (30 mg) in DMF (3 mL) for 3 hours, and the resin was then washed with DMF (18 mL) to obtain resin VWT038403.

VWT038403 was then treated in 2.5 mL of 95% TFA/5% $H_2O$ Cleavage Cocktail for 6 hours, crystallized with methyl tert-butyl ether, and centrifuged and dried to obtain 70 mg of crude VWT038400, which was then purified by preparative HPLC to obtain 0.5 mg of the target product VWT038400 with a purity of 95.23%; LCMS: $[M+H]^+$=1306.60.

**Example 36**

Preparation of compound VWT038500

Step 1 (preparation of VWT038502)

VWT038502 is the same as VWT037202.

Step 2 (preparation of VWT038500)

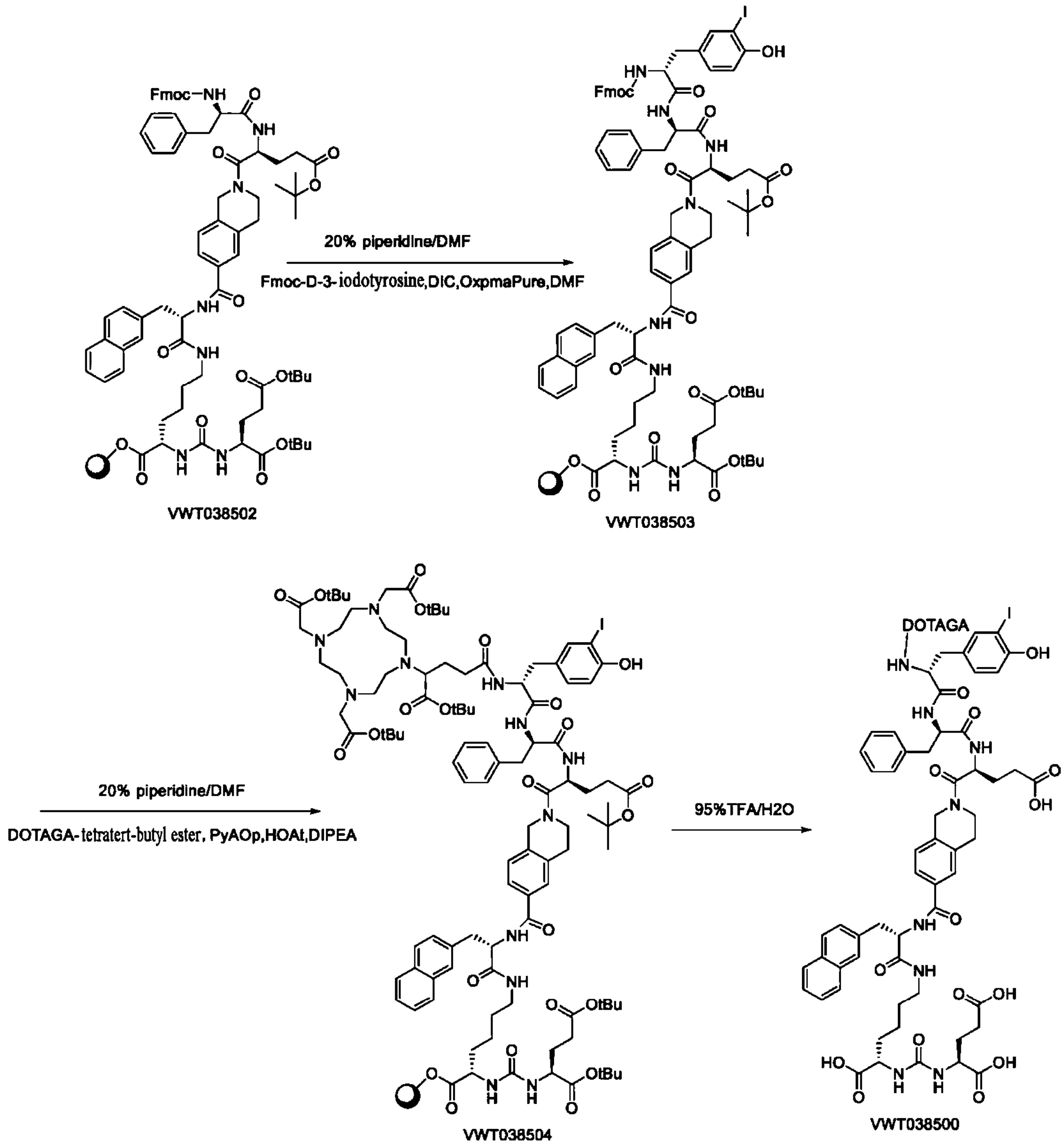


VWT038502

VWT038503

VWT038504

VWT038500

VWT038502 resin was swollen in DMF (5 mL) for 30 minutes, washed with DMF (2 mL), and then treated with 20% piperidine/DMF (3 mL) for 30 minutes to remove the Fmoc protecting group. The resin was then washed with DMF (40 mL), and then reacted with Fmoc-D-3-iodotyrosine (65 mg), DIC (16 mg), and ethyl cyanoglyoxylate-2-oxime (20 mg) in DMF (3 mL) for 4 hours. The resin was then washed with DMF (18 mL) to obtain VWT038503.

VWT038503 was treated with 20% piperidine/DMF (3 mL) for 30 minutes to remove the Fmoc protecting group, then washed with DMF (24 mL), and then reacted with DOTAGA-tetratert-butyl ester (65 mg), PyAOP (50 mg), HOAT (15 mg), and DIPEA (25 mg) in DMF (1 mL) for 5 hours. The resin was then washed with DMF (18 mL) to obtain resin VWT038504.

VWT038504 was then treated in 2.5 mL of 95% TFA/5% $H_2O$ Cleavage Cocktail for 4 hours, crystallized with methyl tert-butyl ether, and centrifuged and dried to obtain 109 mg of crude VWT038500, which was then purified by preparative HPLC to obtain 14.5 mg of the target product VWT038500 with a purity of 94.91%; LCMS: $[M+H]^+$=1699.50.

Example 37

Preparation of compound VWT039800

Step 1 (preparation of VWT039803)

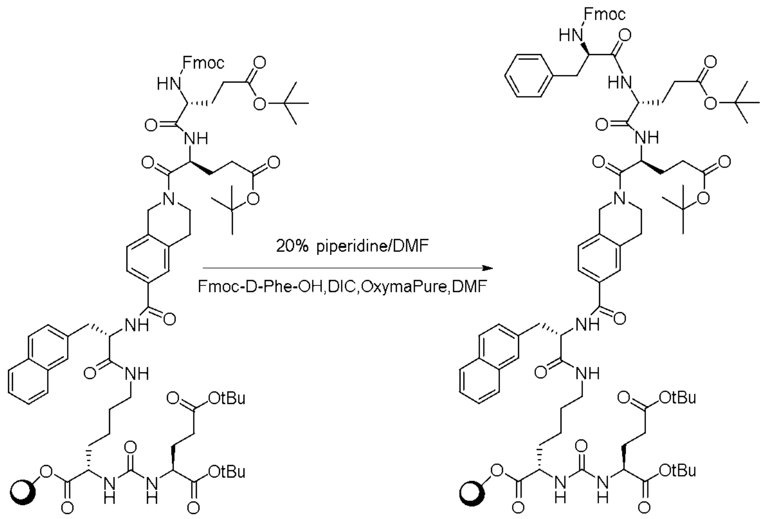


VWT039802 VWT039803

VWT039802 resin was treated with 20% piperidine/DMF (5 mL) for 30 minutes to remove the Fmoc protecting group, then washed with DMF (40 mL), and then reacted with Fmoc-D-Phe-OH (72 mg), DIC (75 mg), and ethyl cyanoglyoxylate-2-oxime (88 mg) in DMF (1 mL) for 2 hours. The resin was then washed with DMF (12 mL) to obtain VWT039803.

Step 2 (preparation of VWT039800)

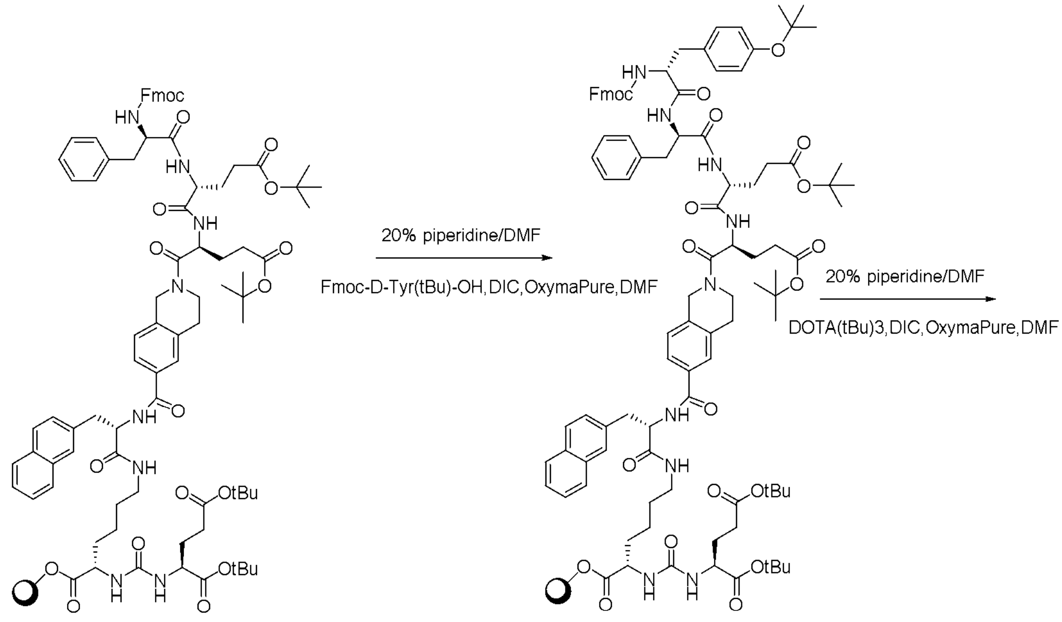


VWT039803 VWT039804

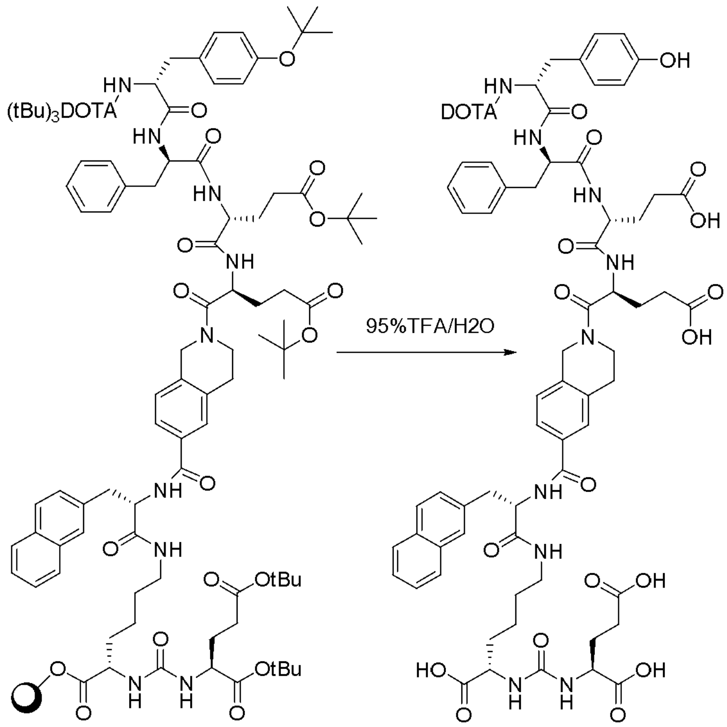


VWT039805 VWT039800

VWT039803 resin was swollen in DMF (5 mL) for 30 minutes, then washed with DMF (2 mL), and then treated with 20% piperidine/DMF (3 mL) for 30 minutes to remove the Fmoc protecting group. The resin was then washed with DMF (40 mL), and reacted with Fmoc-D-Tyr(tBu)-OH (140 mg), DIC (40 mg), and ethyl cyanoglyoxylate-2-oxime (45 mg) in DMF (3 mL) for 4 hours. The resin was then washed with DMF (18 mL) to obtain VWT039804.

VWT039804 was treated in 20% piperidine/DMF (3 mL) for 30 minutes to remove the Fmoc protecting group, then washed with DMF (24 mL), followed by reaction with DOTA(tBu)$_3$ (105mg), DIC (25 mg), and ethyl cyanoglyoxylate-2-oxime (30 mg) in DMF (3 mL) for 6 hours. The resin was then washed with DMF (18 mL) to obtain resin VWT039805. VWT039805 was then treated in 95% TFA/5% $H_2O$ Cleavage Cocktail (2 mL) for 6 hours, crystallized with methyl tert-butyl ether, and centrifuged and dried to obtain 107 mg of crude VWT039800, which was then purified by preparative HPLC to obtain 49.2 mg of the target product VWT039800 with a purity of 95.97%; LCMS: $[M+H]^+$ =1631.70.

**Example 38**

Preparation of compound VWT043300

Step 1 (preparation of VWT043304)

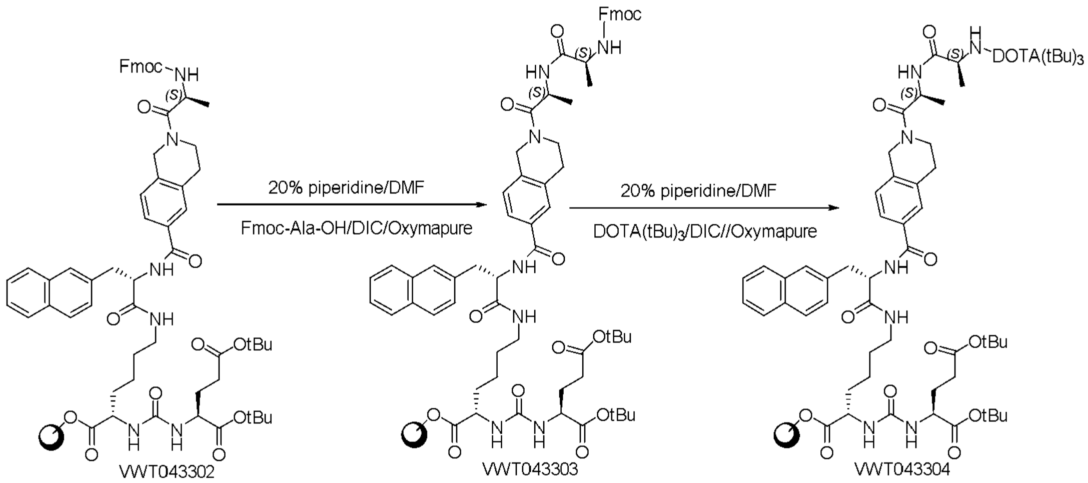


VWT043302 was washed with DMF (18 mL), then treated with 20% piperidine/DMF (5 mL) for 30 minutes to remove the Fmoc protecting group. The resin was then washed with DMF (30 mL), and coupled with a second Fmoc-alanine (310 mg) under a DIC (125 mg)/ethyl cyanoglyoxylate-2-oxime (145 mg) condensation system. The resin was then washed with DMF (18 mL), treated with 20% piperidine/DMF (5 mL) for 30 minutes to remove the Fmoc protecting group, washed with DMF (30 mL), and then coupled with DOTA$(tBu)_3$ (290mg) under a DIC (64 mg)/ethyl cyanoglyoxylate-2-oxime (145 mg) condensation system for 5 hours. The resin was then washed with DMF (30 mL) to obtain the fully protected resin VWT043304.

Step 2 (preparation of VWT043300)

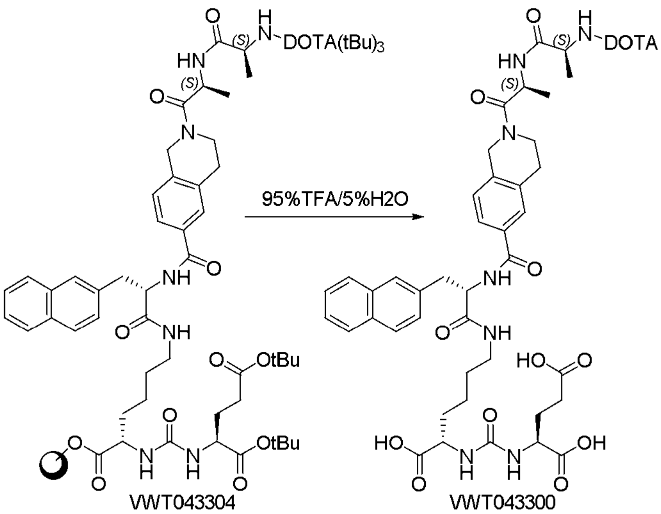


VWT043304 was treated in 95% TFA/5% $H_2O$ Cleavage Cocktail (3 mL) for 4 hours, crystallized with methyl tert-butyl ether, and centrifuged and dried to obtain 117 mg of crude VWT043300, which was further purified by preparative HPLC to obtain 23.7 mg of the target product VWT043300 with a purity of 97.14%; LCMS: $[M+H]^+$ =1204.50.

Example 39

Preparation of compound VWT043400

Step 1 (preparation of VWT043404)

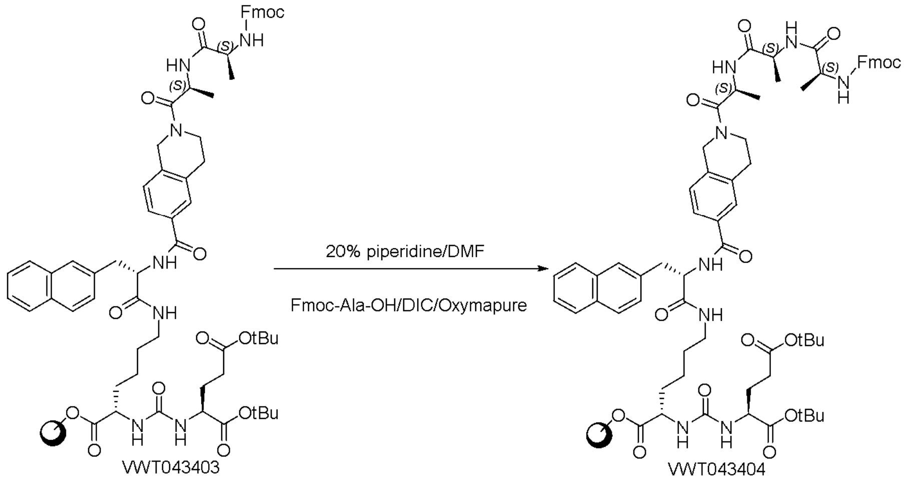


VWT043403 VWT043404

VWT043403 resin was washed with DMF (18 mL), then treated with 20% piperidine/DMF (5 mL) for 30 minutes to remove the Fmoc protecting group. The resin was then washed with DMF (30 mL), and coupled with a third Fmoc-alanine (310 mg) under a DIC (125 mg)/ethyl cyanoglyoxylate-2-oxime (145 mg) condensation system for 3 hours. The resin was then washed with DMF (30 mL) to obtain VWT043404.

Step 2 (preparation of VWT043400)

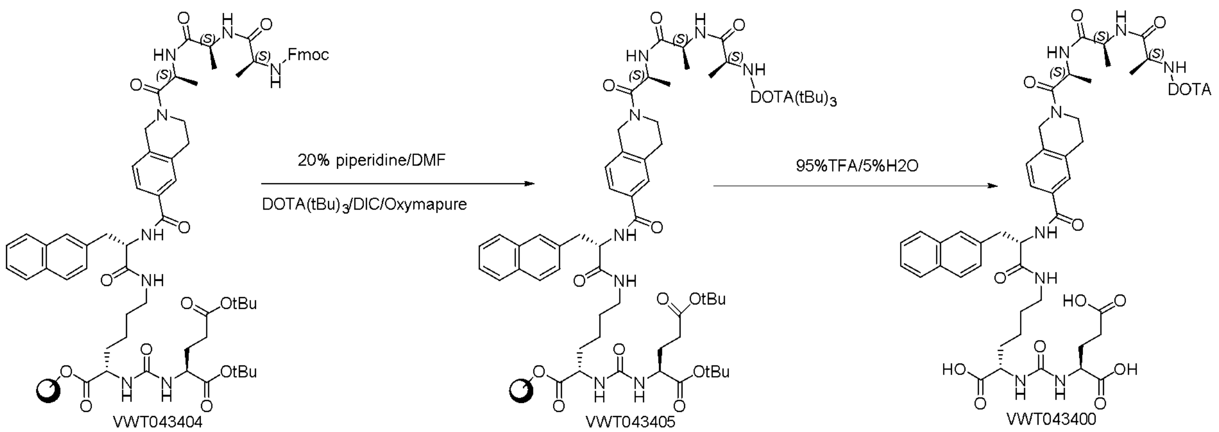


VWT043404 VWT043405 VWT043400

VWT043404 resin was washed with DMF (18 mL), then treated with 20% piperidine/DMF (5 mL) for 30 minutes to remove the Fmoc protecting group. The resin was then washed with 30 mL DMF, and coupled with DOTA(tBu)$_3$ (290mg) under a DIC (64 mg)/ethyl cyanoglyoxylate-2-oxime (145 mg) condensation system for 5 hours. The resin was then washed with DMF (30 mL) to obtain the fully protected resin VWT043405. VWT043405 was treated in 95% TFA/5% $H_2O$ Cleavage Cocktail (2.5 mL) for 4 hours, crystallized by methyl tert-butyl ether, centrifuged and dried to obtain 121 mg of crude VWT043400, which was then purified by preparative HPLC to obtain 13 mg of the target product VWT043400 with a purity of 95.83%; LCMS: $[M+H]^+$ =1275.60.

## Example 40

Preparation of compound VWT043500

Step 1 (preparation of VWT043504)

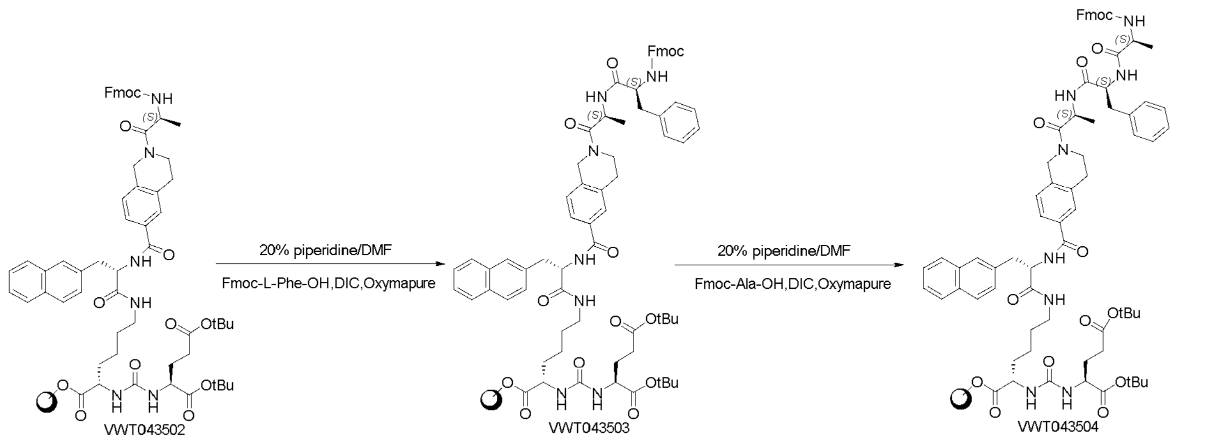


VWT043502 resin was washed with DMF (18 mL), then treated with 20% piperidine/DMF (5 mL) for 30 minutes to remove the Fmoc protecting group. The resin was then washed with DMF (30 mL), and coupled with Fmoc-L-Phe-OH (388 mg) under a DIC (125 mg)/ethyl cyanoglyoxylate-2-oxime (145 mg) condensation system to obtain VWT043503.

VWT043503 resin was washed with DMF (18 mL), then treated with 20% piperidine/DMF (5 mL) for 30 minutes to remove the Fmoc protecting group. The resin was then washed with DMF (30 mL), and coupled with Fmoc-alanine (315 mg) under a DIC (125 mg)/ethyl cyanoglyoxylate-2-oxime (145 mg) condensation system for 3 hours. The resin was then washed with DMF (30 mL) to obtain VWT043504.

Step 2 (preparation of VWT043500)

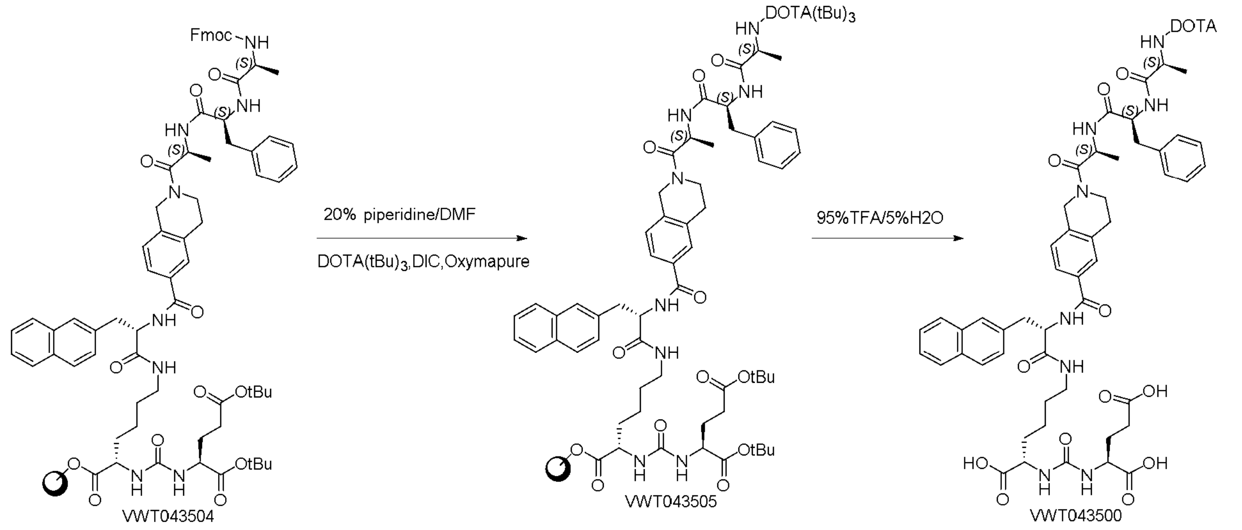


VWT043504 resin was washed with DMF (18 mL), then treated with 20% piperidine/DMF (5 mL) for 30 minutes to remove the Fmoc protecting group. The resin was then washed with DMF (30 mL), and coupled with DOTA$(tBu)_3$ (290mg) under a DIC (64 mg) /ethyl cyanoglyoxylate-2-oxime (145 mg) condensation system for 5 hours. The resin was then washed with DMF (30 mL) to obtain a fully protected resin VWT043505. VWT043505 was treated with 95% TFA/5% $H_2O$ Cleavage Cocktail (3 mL) for 3 hours, crystallized by methyl tert-butyl ether, centrifuged and dried to obtain 127 mg of crude VWT043500, which was then purified by preparative HPLC to obtain 19.3 mg of the target product VWT043500 with a purity of 98.41%; LCMS: $[M+H]^+$ =1351.60.

**Example 41**

Preparation of compound VWT043600

It should be noted that compound VWT043600 only differs from compound VWT043500 in terms of the configuration of phenylalanine.

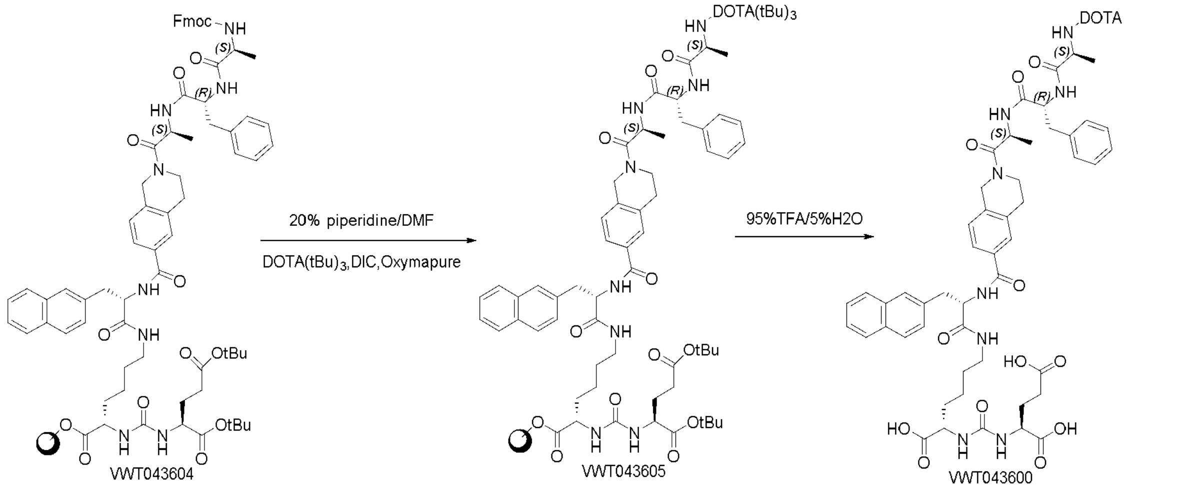


According to a similar method to that used for VWT043500 (the difference being that the coupling raw material was Fmoc-D-Phe-OH), 21.2 mg of the target product VWT043600 with a purity of 94.33% was prepared; LCMS: $[M+H]^+$ =1351.70.

Example 42

Preparation of compound VWT044400

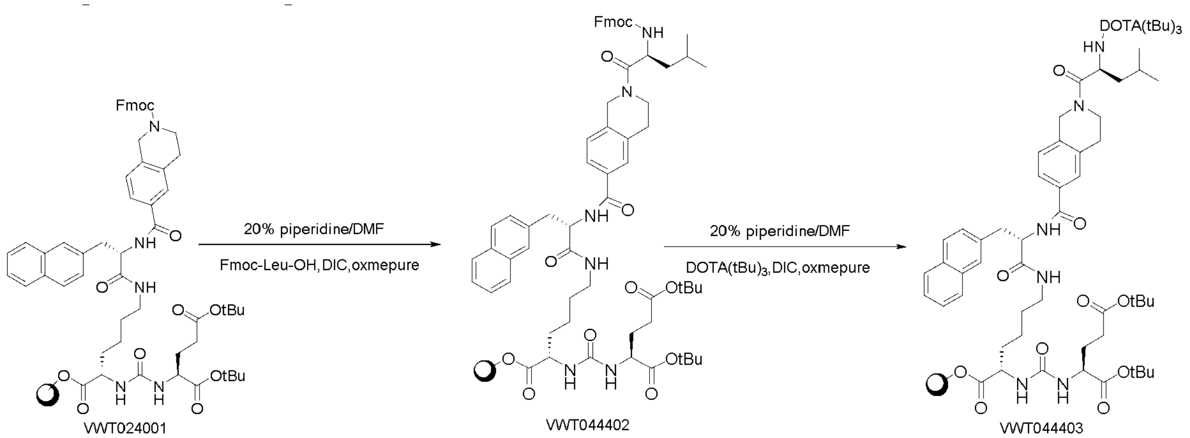

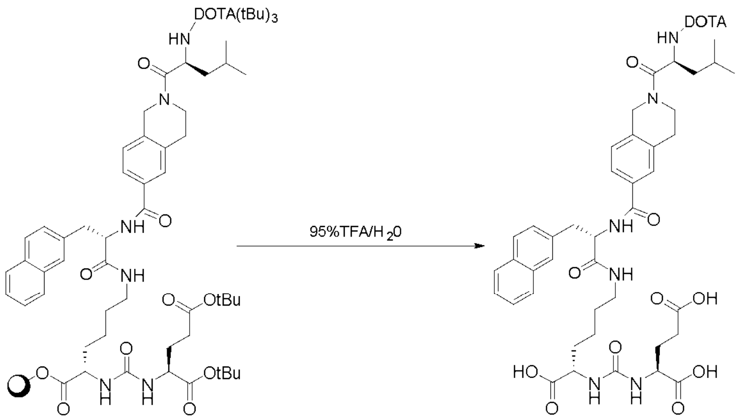


VWT044403 VWT044400

According to a similar method as in Example 1 (the difference being that the coupling raw material was Fmoc-Leu-OH), 11.7 mg of the target product VWT044400 with a purity of 95.77% was prepared; LCMS: [M+H] $^+$ =1175.30.

Examples 43-45

Preparation of compounds VWT044600, VWT044800 and VWT044900

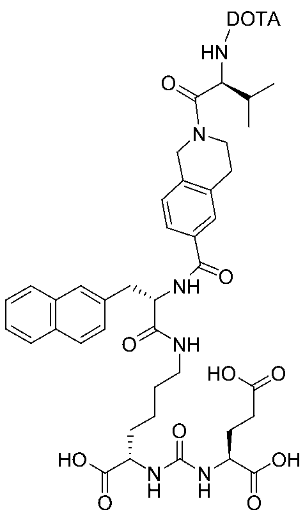

VWT044600 ,

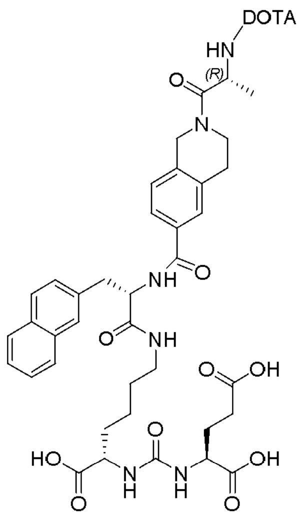

VWT044800 ,

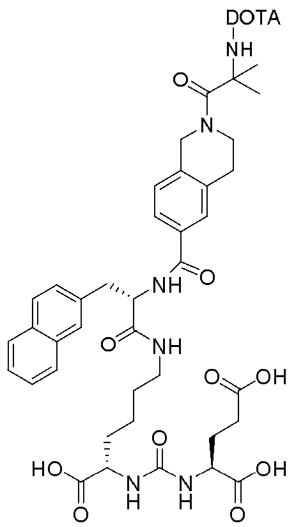

VWT044900

According to a similar method as in Example 1 (the difference being that the coupling raw material was Fmoc-L-Val-OH), 19 mg of the target product VWT044600 with a purity of 94.99% was prepared; LCMS: [M+H] $^+$ =1161.30.
According to a similar method as in Example 1 (the difference being that the coupling raw material was Fmoc-D-Ala-OH), 12.11 mg of the target product VWT044800 with a purity of 94.84% was prepared; LCMS: [M+H] $^+$ =1133.30.
According to a similar method as in Example 1 (the difference being that the coupling raw material was Fmoc-Aib-OH), 1.5 mg of the target product VWT044900 with a purity of 96.42% was prepared; LCMS: [M+H] $^+$ =1147.60.

Examples 46-47

Preparation of compounds VWT044700 and VWT045000

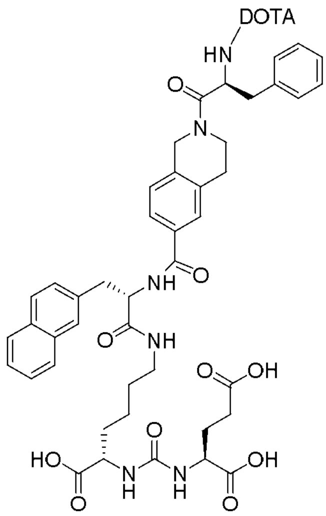

VWT044700 ,

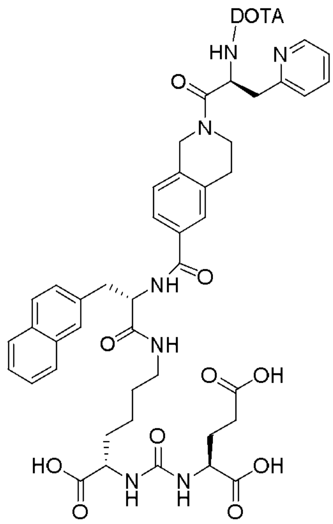

VWT045000

According to a similar method as in Example 1 (the difference being that the coupling raw material was Fmoc-L-Phe-OH), 9 mg of the target product VWT044700 was prepared; LCMS: $[M+H]^+$ =1209.40.

According to a similar method as in Example 1 (the difference being that the coupling raw material was Fmoc-2-Pal-OH), 1.2 mg of the target product VWT045000 was prepared; LCMS: $[M+H]^+$ =1210.60.

**Example 48**

Preparation of compound VWT035400

(1) Preparation of compound VWT035401

a. Synthesis of Compound 3

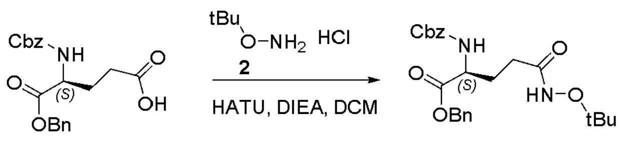


1 3

Compound 1 ((S)-5-(benzyloxy)-4-(((benzyloxy)carbonyl)amino)-5-oxopentanoic acid, 1 g) and HATU (1.23 g) were added to dichloromethane (20 mL), followed by Compound 2 (tert-butoxyhydroxylamine hydrochloride, 0.68 g) and DIPEA (1.04 g). The reaction mixture was reacted at room temperature for 16 hours, concentrated under reduced pressure to obtain a residue, which was purified by silica gel column chromatography (petroleum ether/ethyl acetate = 1/1) to obtain compound 3 (1.1 g) as a colorless liquid.

b. Synthesis of Compound 4

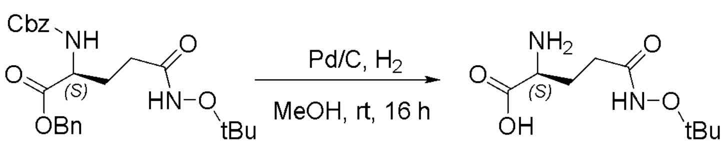


3 4

Compound 3 (1.1 g) was dissolved in methanol (50 mL), and palladium on carbon catalyst (100 mg) was added. The reaction was carried out at room temperature under hydrogen atmosphere for 16 hours. The reaction mixture was filtered, and the filtrate was concentrated to obtain compound 4 (0.45 g) as a colorless liquid.

c. Synthesis of compound VWT035401

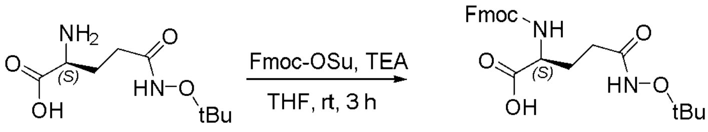


4

VWT035401

Compound 4 (400 mg) and Fmoc-OSu (926.5 mg) were dissolved in tetrahydrofuran, and triethylamine (555.3 mg) was added. The reaction mixture was reacted at room temperature for 3 hours, concentrated under reduced pressure to obtain a residue, which was purified by C18 reversed-phase silica gel column chromatography to obtain compound VWT035401 (177 mg) as a white solid.

(2) Preparation of compound VWT035400

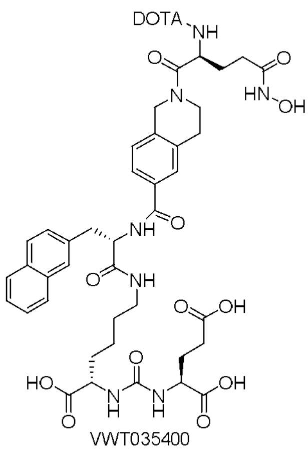

VWT035400

According to a similar method as in Example 1 (the difference being that the coupling raw material was VWT035401), 2.2 mg of the target product VWT035400 was prepared; LCMS: [M+H] $^+$ =1206.40.

Examples 49-51

Preparation of compounds VWT044500, VWT045900 and VWT046100

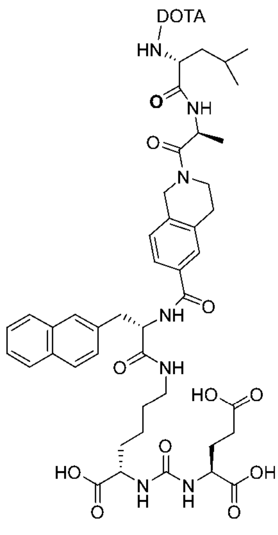

VWT044500 ,

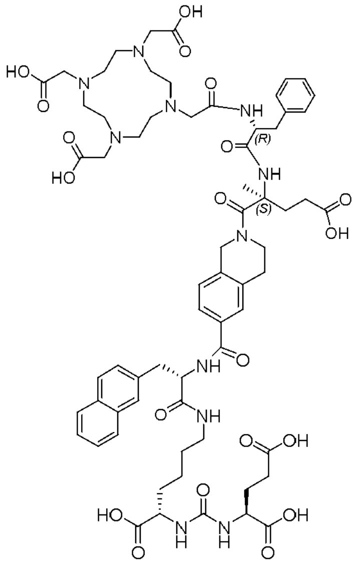

VWT045900 ,

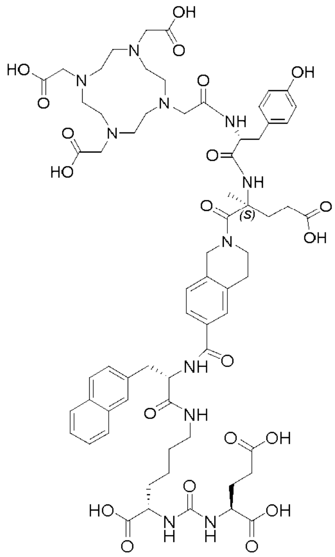

VWT046100

According to similar methods as in Example 12, Example 23 or Example 28 (the difference being that Fmoc-alanine and Fmoc-D-Leu-OH were coupled sequentially), 11.8 mg of the target product VWT044500 with a purity of 95.35% was prepared; LCMS: $[M+H]^+$ =1246.40.

According to similar methods as in Example 12, Example 23 or Example 28 (the difference being that Fmoc-α-Me-Glu(OtBu)-OH and Fmoc-D-Phe-OH were coupled sequentially), 9.7 mg of the target product VWT045900 was prepared; LCMS: $[M+H]^+$ =1352.80.

According to similar methods as in Example 12, Example 23 or Example 28 (the difference being that Fmoc-α-Me-Glu(OtBu)-OH and Fmoc-D-Tyr(tBu)-OH were coupled sequentially), 2.1 mg of the target product VWT046100 with a purity of 94.48% was prepared; LCMS: $[M+H]^+$ =1368.80.

**Examples 52-53**

Preparation of compounds VWT045800 and VWT046000

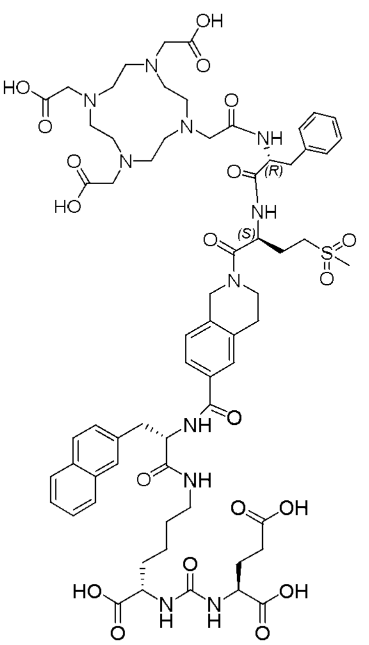

VWT045800 ,

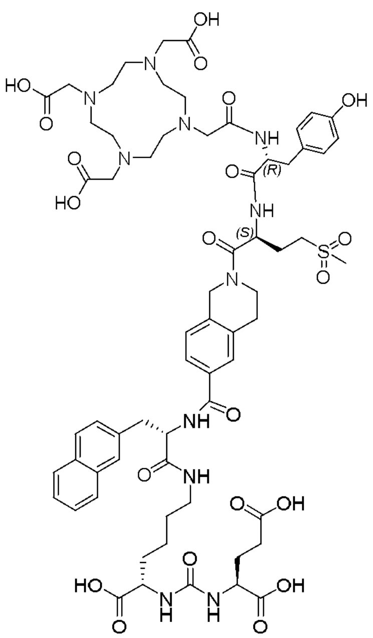

VWT046000

According to similar methods as in Example 12, Example 23 or Example 28 (the difference being that Fmoc-Met($O_2$)-OH and Fmoc-D-Phe-OH were coupled sequentially), 17 mg of the target product VWT045800 with a purity of 94.99% was prepared; LCMS: $[M+H]^+$ =1372.70.

According to similar methods as in Example 12, Example 23 or Example 28 (the difference being that Fmoc-Met($O_2$)-OH and Fmoc-D-Tyr(tBu)-OH were coupled sequentially), 31 mg of the target product VWT046000 was prepared; LCMS: $[M+H]^+$ =1388.70.

**Example 54**

Preparation of compound VWT037300

(1) Preparation of compound VWT037301-A

a. Synthesis of Compound 3

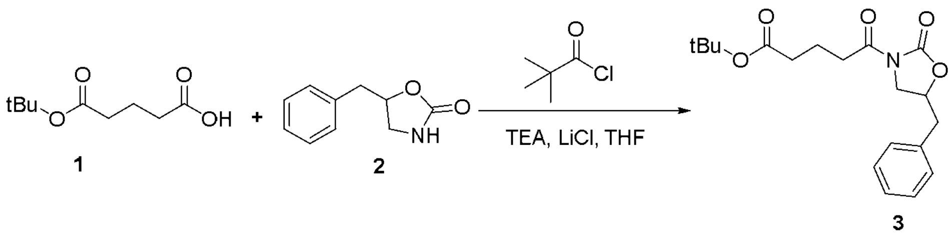


Under nitrogen protection, triethylamine (4.1 g), pivaloyl chloride (2.9 g), and compound 1 (4.6 g) were stirred in tetrahydrofuran (200 mL) at -30 °C for 2 hours. Then, lithium chloride (1.1 g) and compound 2 (4.1 g) were added, and the mixture was warmed to room temperature and stirred for another 16 hours. After the reaction was complete, the reaction mixture was added with ethyl acetate, washed with saturated ammonium chloride aqueous solution, sodium hydroxide aqueous solution (1N), and saturated brine, then dried over anhydrous sodium sulfate, and concentrated to obtain a residue, which was purified by silica gel column chromatography (petroleum ether/ethyl acetate = 2/1) to obtain compound 3 (7.3 g, 86%) as a colorless liquid.

b. Synthesis of Compound 5

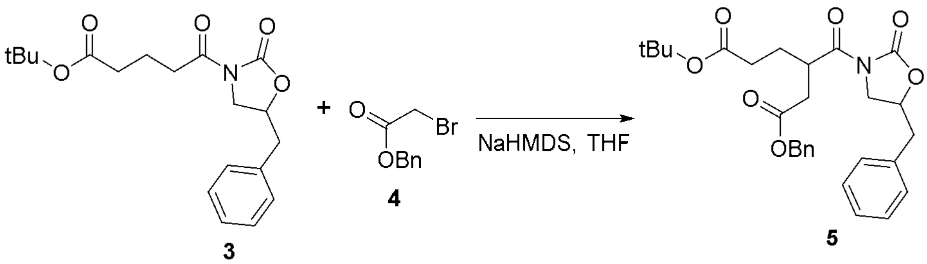


Under nitrogen protection, NaHMDS (7.9 mL) and compound 3 (5.0 g) were stirred in tetrahydrofuran (200 mL) at -78 °C for 1 hour. Then, compound 4 (benzyl 2-bromoacetate, 4.9 g) was added, and the mixture was warmed to room temperature and stirred for another 16 hours. After the reaction was complete, the reaction mixture was added with ethyl acetate, washed with saturated ammonium chloride aqueous solution, 5% sodium bisulfite aqueous solution, and saturated brine, then dried over anhydrous sodium sulfate, and concentrated to obtain a residue, which was purified by silica gel column chromatography (petroleum ether/ethyl acetate = 5/1) to give compound 5 (6.6 g, 93%) as a colorless liquid.

c. Synthesis of Compound 6

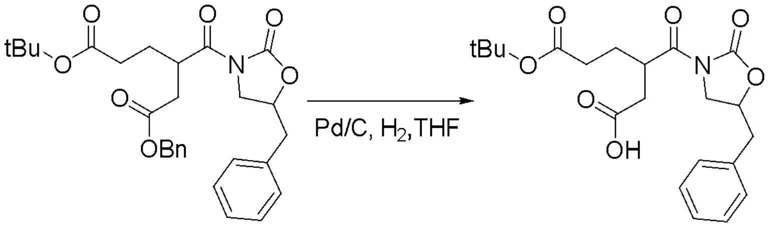


5 6

Compound 5 (6.6 g) and Pd/C (500 mg) were reacted in tetrahydrofuran (200 mL) under continuous hydrogen purging at room temperature for 16 hours. The mixture was filtered, and the filtrate was concentrated under reduced pressure to obtain compound 6 (5.1 g, 94%) as a colorless liquid.

d. Synthesis of Compound 7

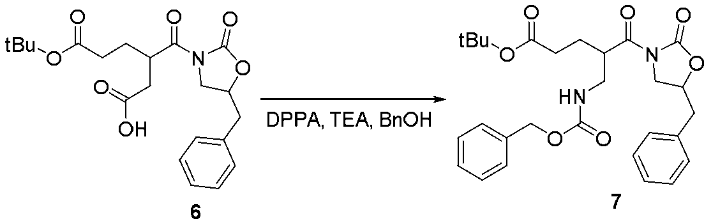


6 7

Under nitrogen protection, compound 6 (5.1 g), DPPA (CAS No.: 26386-88-9, 4.2 g), and triethylamine (1.5 g) were stirred in toluene (100 mL) at 110 °C for 1.5 h. Then, benzyl alcohol (2.7 g) was added, and the reaction was continued at 110 °C for 16 h. After the reaction was complete, the reaction mixture was added with ethyl acetate, washed with saturated sodium bicarbonate aqueous solution and saturated brine, then dried over anhydrous sodium sulfate, and concentrated to obtain a residue, which was purified by silica gel column chromatography (petroleum ether/ethyl acetate = 2/1) to obtain compound 7 (4.08 g, 64%) as a colorless liquid.

e. Synthesis of Compound 8

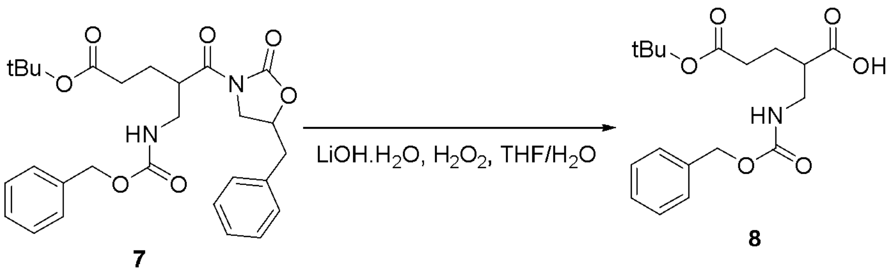


8

7

Lithium hydroxide monohydrate (536 mg), hydrogen peroxide (3.6 g), and compound 7 (4.08 g) were reacted in tetrahydrofuran/water (80 mL/20 mL) at 0 °C for 2 h. The reaction mixture was added with ethyl acetate, and adjusted to pH 4 with 10% citric acid, extracted with ethyl acetate, washed with saturated brine, dried over anhydrous sodium sulfate, and concentrated to obtain a residue, which was purified by silica gel column chromatography (dichloromethane/methanol = 20/1) to obtain compound 8 (2.5 g, 89%) as a colorless liquid.

f. Synthesis of Compound 9

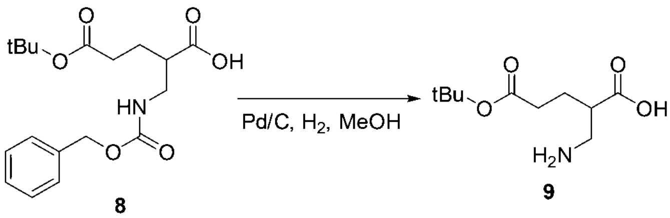


8

9

Compound 8 (2.5 g) and Pd/C (200 mg) were reacted in methanol (100 mL) under continuous hydrogen purging at room temperature for 3 hours. The mixture was filtered, and the filtrate was concentrated under reduced pressure to obtain compound 9 (1.4 g, 91%) as a white solid.

g. Synthesis of compound VWT037301-A

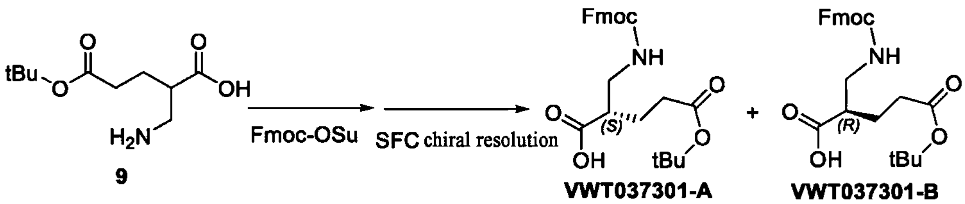


9

VWT037301-A

+

VWT037301-B

Compound 9 (1.4 g) and Fmoc-OSu (2.6 g dissolved in 50 mL acetone) were reacted in an aqueous sodium bicarbonate solution (86 mL) at room temperature for 16 hours. The reaction mixture was concentrated under reduced pressure to remove most of the acetone. The residue was diluted with water and extracted with diethyl ether. The aqueous phase was retained, added with ethyl acetate, and adjusted to pH 4 with 10% citric acid, then extracted with ethyl acetate, washed with saturated brine, dried over anhydrous sodium sulfate, and concentrated to obtain a residue, which was purified by silica gel column chromatography (dichloromethane/methanol = 20/1) and then chirally resolved to obtain compound VWT037301-A (1.74 g, 61%; as a white solid) and its isomer VWT037301-B (47.3 mg, 2%; as a white solid).

(2) Preparation of compound VWT037300

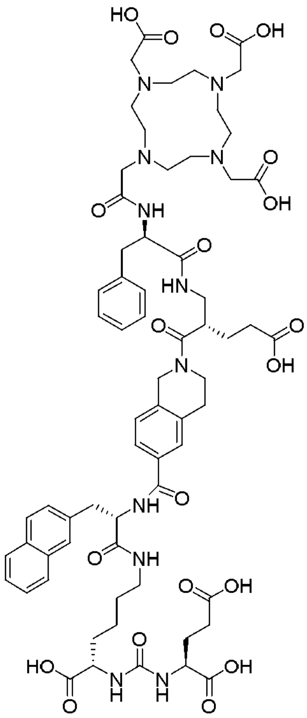

VWT037300

According to similar methods as in Example 12, Example 23 or Example 28 (the difference being that VWT037301-A and Fmoc-D-Phe-OH were coupled sequentially), 22.7 mg of the target product VWT037300 was prepared; LCMS: $[M+H]^+$ =1352.50.

**Example 55**

Preparation of compound VWT049900

(1) Preparation of compound VWT049901

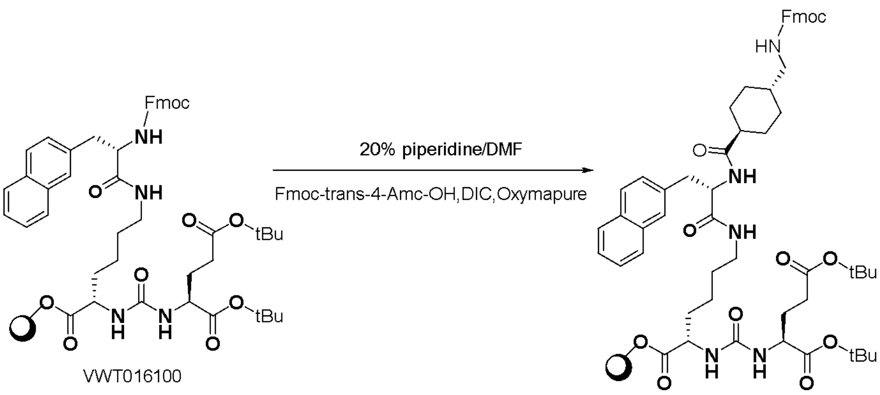


VWT016100

VWT049901

VWT016100 was swollen in DMF (1 ml) for 30 minutes, washed twice with DMF (1 ml), then treated with 20% piperidine/DMF (1 ml) for 30 minutes to remove the Fmoc protecting group, and then washed with DMF (8 ml). The reaction was treated with a mixture of Fmoc-trans-4-Amc-OH (116 mg), DIC (39 mg), and Oxymapure (ethyl cyanoglyoxylate-2-oxime, CAS No.: 3849-21-6, 47 mg) in DMF (0.6 ml) for 3 hours, and the resin was then washed with DMF (6 ml) to obtain compound VWT049901.

(2) Preparation of compound VWT0049902

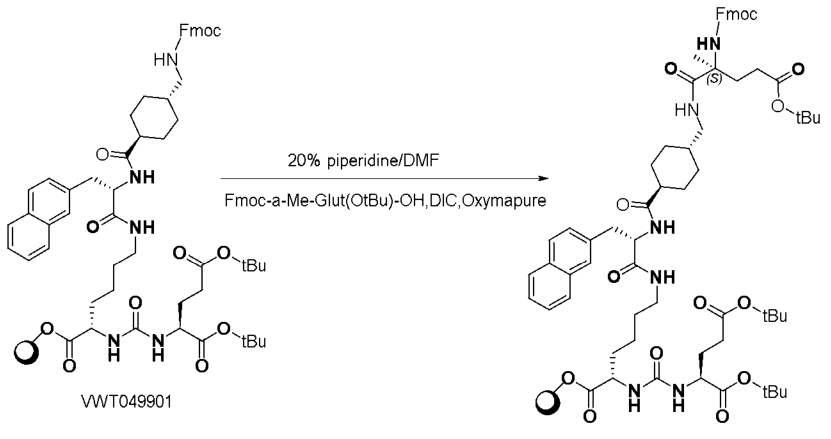


VWT049901 VWT049902

VWT0049901 was swollen in DMF (1 ml) for 30 minutes, washed twice with DMF (1 ml), then treated with 20% piperidine/DMF (1 ml) for 30 minutes to remove the Fmoc protecting group, and then washed with DMF (8 ml). The reaction was treated with a mixture of Fmoc-α-Me-Glu(OtBu)-OH (68 mg), DIC (29 mg), and Oxymapure (40 mg) in DMF (0.6 ml) for 4 hours, and the resin was then washed with DMF (6 ml) to obtain compound VWT049902.

(3) Preparation of compound VWT049903

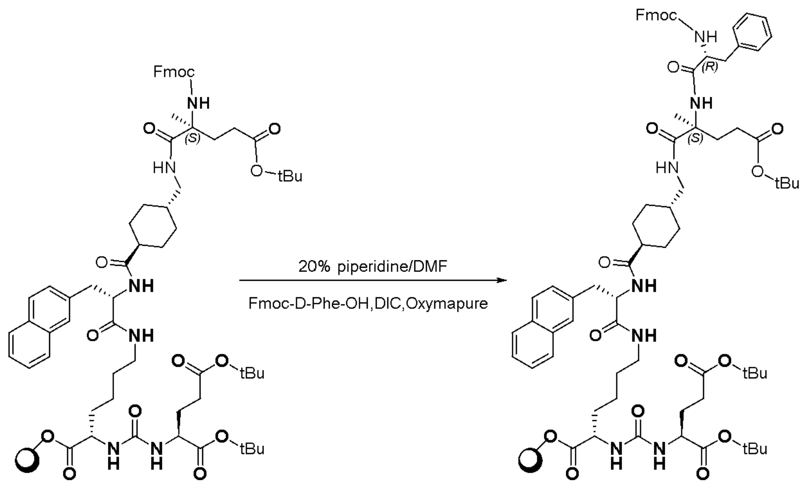


VWT049902 VWT049903

VWT0049902 was swollen in DMF (1 ml) for 30 minutes, washed twice with DMF (1 ml), then treated with 20% piperidine/DMF (1 ml) for 30 minutes to remove the Fmoc protecting group, and then washed with DMF (8 ml). The reaction was treated with a mixture of Fmoc-D-Phe-OH (196 mg), DIC (67 mg), and Oxymapure (73 mg) in DMF (0.6 ml) for 18 hours, and the resin was then washed with DMF (6 ml) to obtain compound VWT049903.

(4) Preparation of compound VWT049904

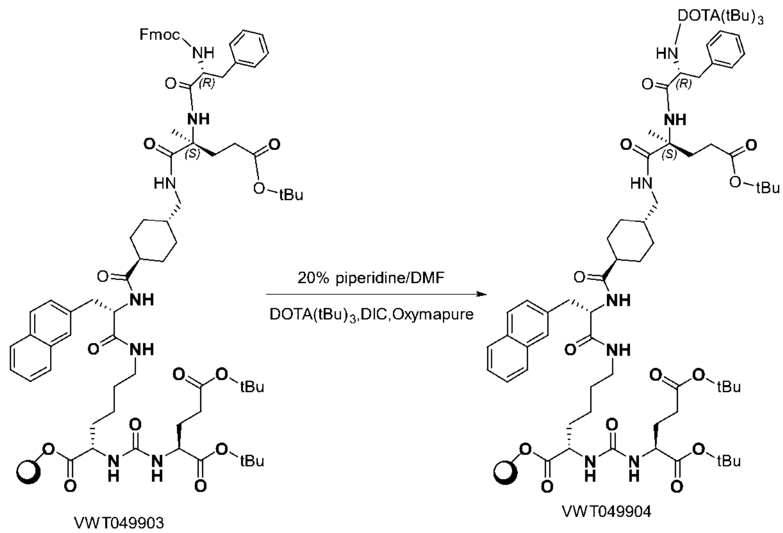


VWT049903 was swollen in DMF (1 ml) for 30 minutes, washed twice with DMF (1 ml), then treated with 20% piperidine/DMF (1 ml) for 30 minutes to remove the Fmoc protecting group, and then washed with DMF (8 ml). The reaction was treated with a mixture of DOTA(tBu)$_3$ (174mg), DIC (39 mg), and Oxymapure (44 mg) in DMF (0.6 ml) for 6 hours, and the resin was then washed with DMF (6 ml) to obtain compound VWT049904.

(5) Preparation of compound VWT049900

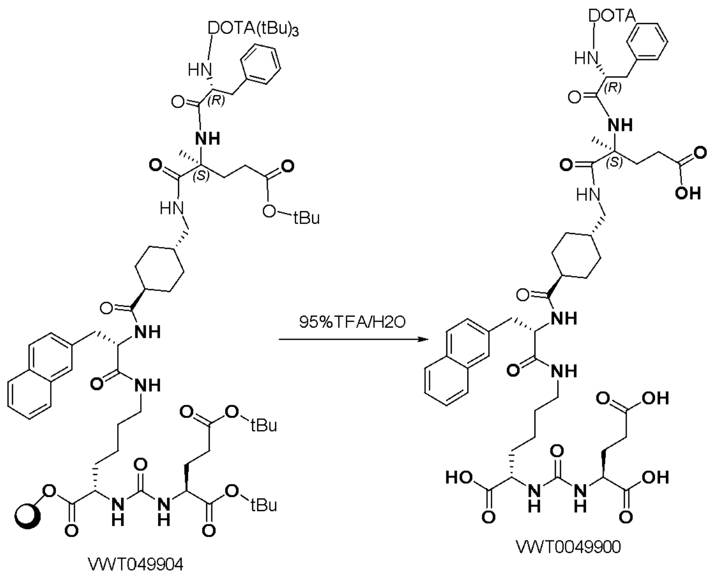


VWT049904 was treated in 95% TFA/5% $H_2O$ Cleavage Cocktail (2 ml) for 3 hours, crystallized with methyl tert-butyl ether, and centrifuged and dried to obtain 88 mg of crude VWT049900 solid, which was further purified by preparative HPLC to obtain 4.6 mg of the target product VWT049900 with a purity of 94.45%; LCMS: [M+H] $^+$ = 1332.80.

**Example 56**

Preparation of compound VWT049200

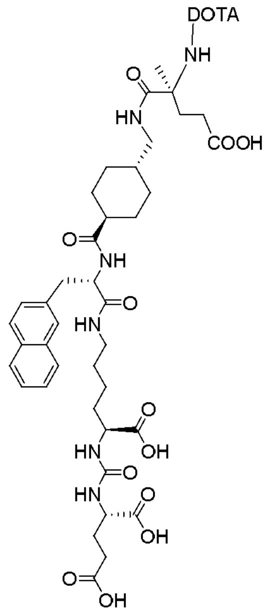

VWT049200

According to similar method as in Example 55 (the difference being that VWT0049901 was coupled with Fmoc-α-Me-Glu(OtBu)-OH and then directly coupled with DOTA(tBu)$_3$), 5.9 mg of the target product VWT049200 was prepared; LCMS: $[M+H]^+$ =1185.70.

Examples 57-58

Preparation of compounds VWT049400 and VWT050000

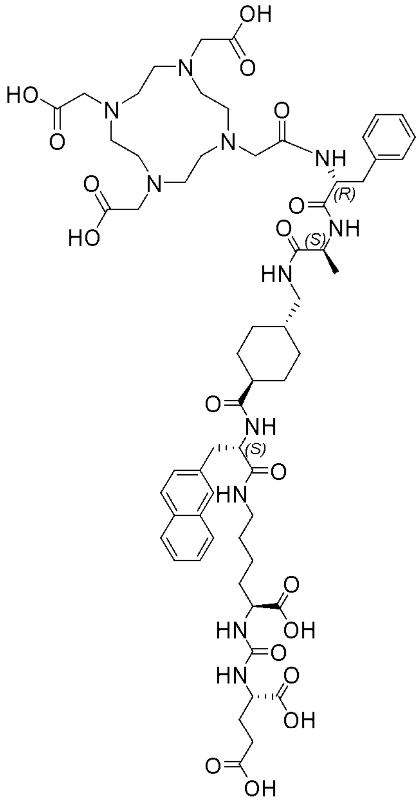

VWT049400

,

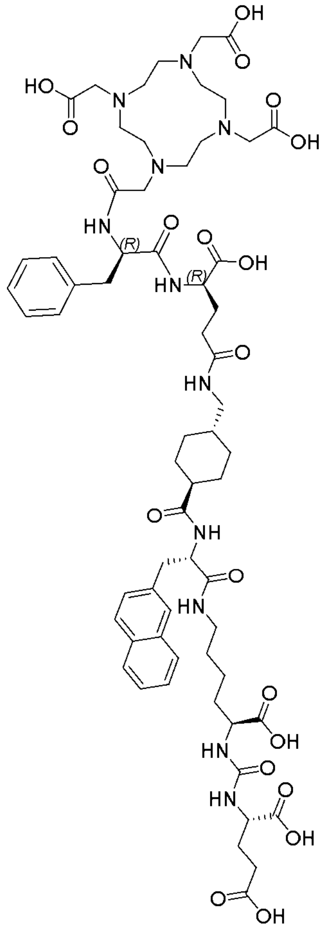

VWT050000

According to similar method as in Example 55 (the difference being that VWT0049901 was coupled sequentially with Fmoc-L-Ala-OH and Fmoc-D-Phe-OH), 11.2 mg of the target product VWT049400 with a purity of 94.75% was prepared; LCMS: $[M+H]^+$ =1260.70.

According to similar method as in Example 55 (the difference being that VWT0049901 was coupled sequentially with Fmoc-D-Glu-OtBu (CAS No.: 109745-15-5) and Fmoc-D-Phe-OH), 9.5 mg of the target product VWT050000 with a purity of 94.98% was prepared; LCMS: $[M+H]^+$ =1318.80.

### Experimental Example 1: Study on Biological Distribution

1. Experimental materials and methods

1.1 Experimental instruments and reagents

Radioactivity meter (CRC-55tR, Capintec, USA); Thermostatic mixer (TS100, Thermo); Electronic balance (ME104E, Mettler Toledo Instruments (Shanghai) Co., Ltd.); Electronic balance (BT457A10, Shenzhen Botu Electronic Technology Co., Ltd.); Small animal SPECT-CT imaging system (U-SPECT+/CT, MI Labs); Sodium acetate (250 g/bottle, SIGMA); $^{177}LuCl_3$ (100 mCi, China Tongfu Co., Ltd.); Physiological saline (250 mL/bag, Sichuan Kelun Pharmaceutical Co., Ltd.).

1.2 Tumor mouse model

The mouse used were LnCap mouse, obtained from Shanghai Southern Model Biotechnology Co., Ltd., 6-8 weeks old, female. The mouse strain was M-NSG nude mouse. 0.1 mL of $10^7$ LnCaP cells (PBS: Matrigel = 1:1) was subcutaneously injected into the right dorsal side of each mouse. The mice were suitable for the study on biological distribution when the average tumor volume reached 200 to 400 $mm^3$. Specific feeding conditions for these mice are shown in Table 1.

Table 1. Environmental conditions of the laboratory animal housing

| | |
|---|---|
| Cage: | Polysulfone IVC cages for mouse under constant temperature and humidity, with an independent air supply system and an air exchange rate of 10-20 times per hour |
| Housing density | Maximum 5 mice per cage |
| Temperature | 20-26°C |
| Humidity | 40-70% |
| Lighting | A standard 12-hour light/dark cycle, light from 7:00 AM to 7:00 PM, darkness from 7:00 PM to 7:00 AM the following day |
| Bedding | Autoclaved wood shavings bedding, changed weekly |
| Food Supply | Ad libitum access to a standard pellet feed, which was continuously supplied and sterilized by Cobalt-60 irradiation |
| Water Supply | Ad libitum access to autoclaved tap water via continuous-supply water bottles |
| Animal Labeling | Ear tagging and ear notching for laboratory animals |

2. Experiment of study on biological distribution

2.1. Preparation of injection solution to be tested

The properties of the compounds to be tested are shown in Table 2.

Table 2. Compounds to be tested

| No. | Compound | Shape and physicochemical properties | Molecular weight | Storage conditions |
|---|---|---|---|---|
| 1 | VWT019000 | Solid | 1146.7 | -20°C |
| 2 | VWT019300 | Solid | 1196.8 | -20°C |
| 3 | VWT011600 | Solid | 1133.2 | -20°C |
| 4 | VWT022600 | Solid | 1212.7 | -20°C |
| 5 | VWT024000 | Solid | 1190.9 | -20°C |
| 6 | VWT024100 | Solid | 1204.9 | -20°C |
| 7 | VWT021400 | Solid | 1118.9 | -20°C |
| 8 | VWT023800 | Solid | 1160.7 | -20°C |
| 9 | VWT023900 | Solid | 1319.7 | -20°C |
| 10 | VWT022100 | Solid | 1148.8 | -20°C |
| 11 | VWT001500 | Solid | 1041.9 | -20°C |
| 12 | VWT032800 | Solid | / | -20°C |
| 13 | VWT032900 | Solid | / | -20°C |
| 14 | VWT039600 | Solid | / | -20°C |

wherein VWT001500 is a known compound (i.e., PSMA-617) with the following structural formula:

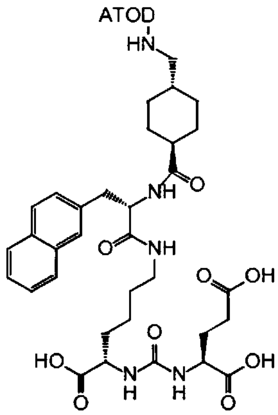

A stock solution with a concentration of 1 μmol/mL (1 nM) was prepared using physiological saline and the compounds to be tested (i.e., the compounds prepared in the Examples), with an initial volume of 200 μL. 100 μL of the stock solution was diluted in 900 μL of acetic acid solution (pH=4.5, 0.4 mol/L). 10 μL or 20 μL of the diluted solution was added to $^{177}LuCl_3$ solution, followed by 40 μL of acetic acid solution (pH=4.5, 0.4 mol/L). The solution was heated at 95°C for 30 min. After the reaction was complete, the final solution was cooled to room temperature to obtain various $^{177}$Lu-compounds to be tested (i.e., the radiolabeled complexes described in the description). Compound $^{177}$Lu-VWT001500 was used as a control.

Similarly, various $^{225}$Ac-compounds to be tested can be obtained respectively by replacing the $^{177}LuCl_3$ solution with a $^{225}AcCl_3$ solution and following the method described above.

For example,

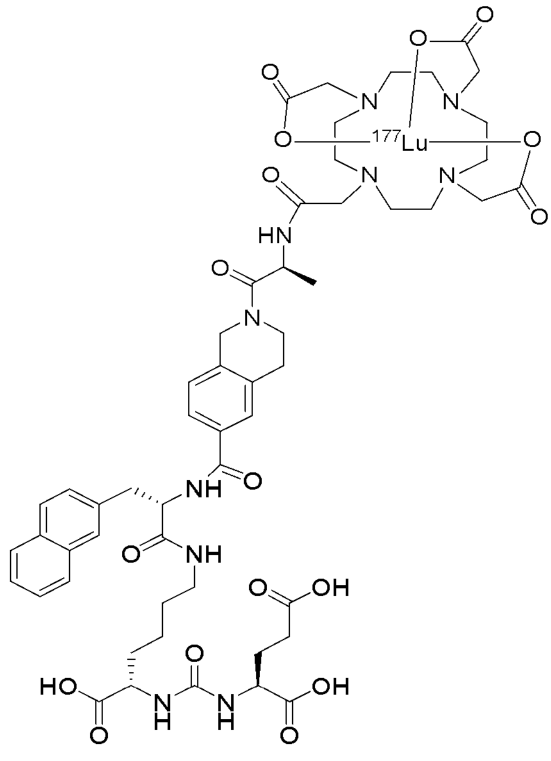

$^{177}$Lu-VWT011600 ,

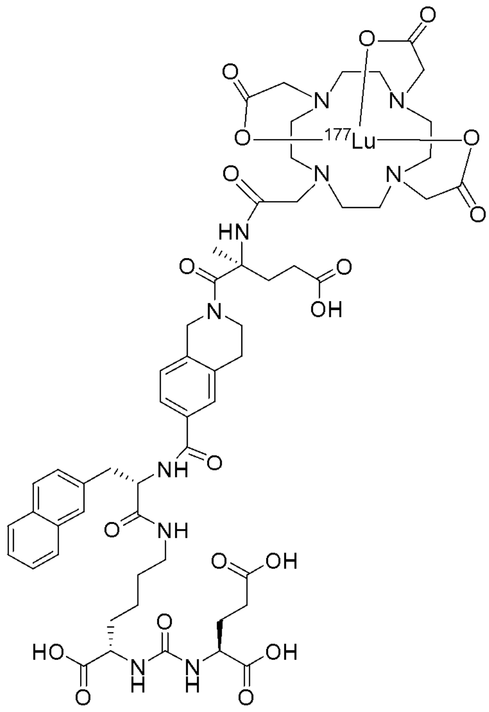

$^{177}$Lu-VWT032800 ,

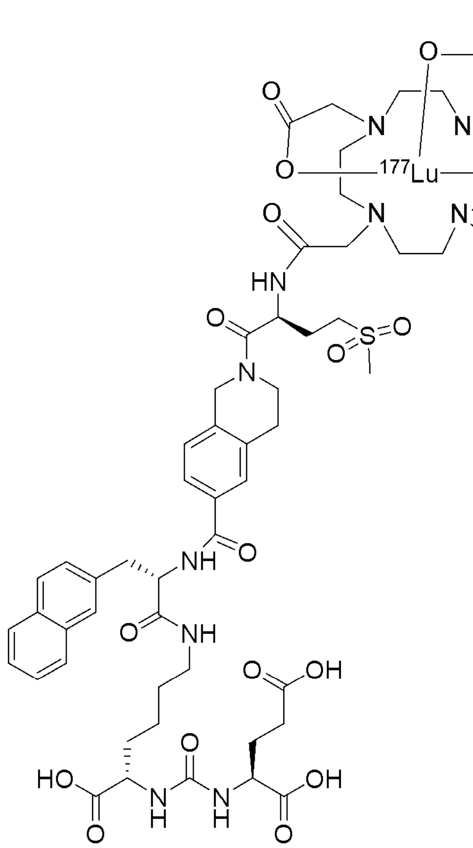

$^{177}$Lu-VWT032900 ,

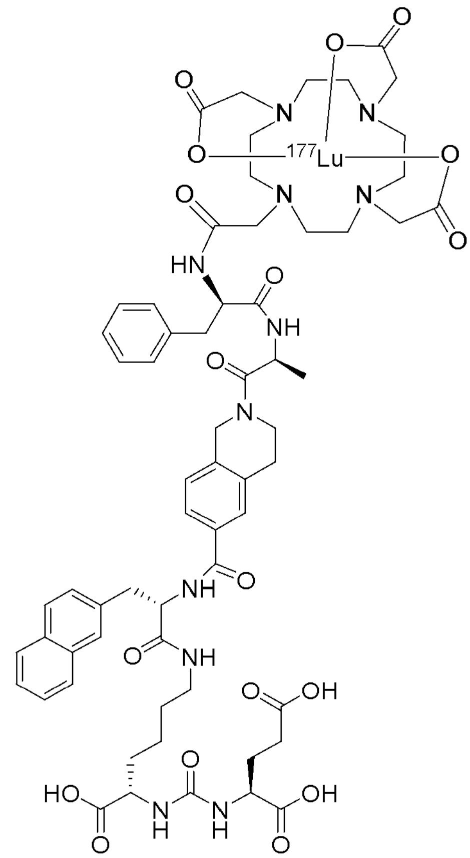

$^{177}$Lu-VWT039600 ,

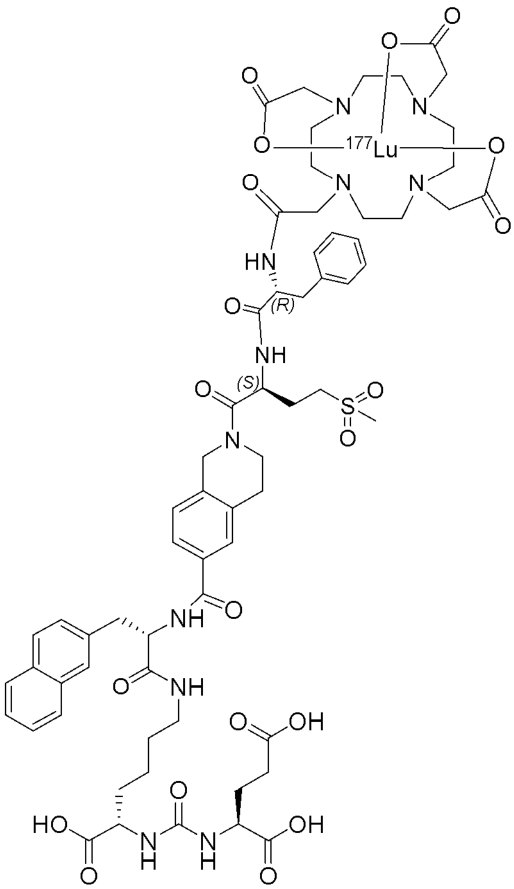

$^{177}$Lu-VWT045800
,

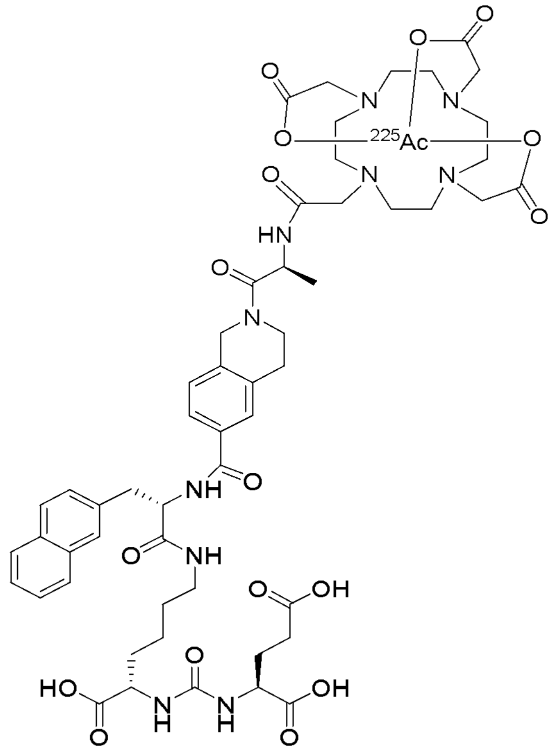

$^{225}$Ac-VWT011600
,

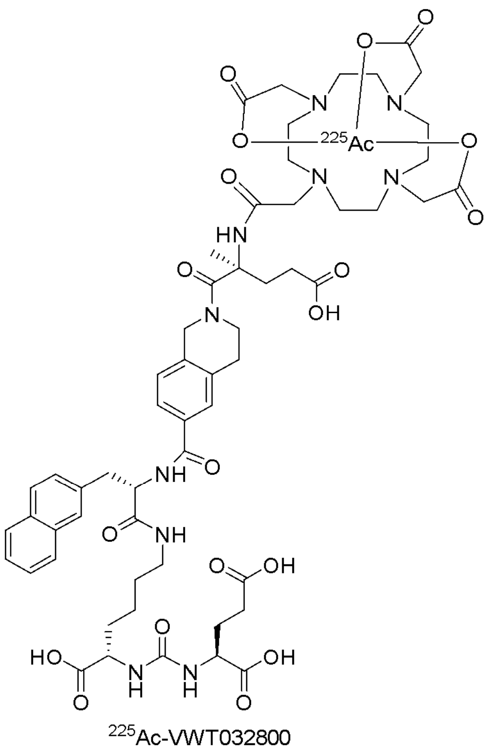

$^{225}$Ac-VWT032800
,

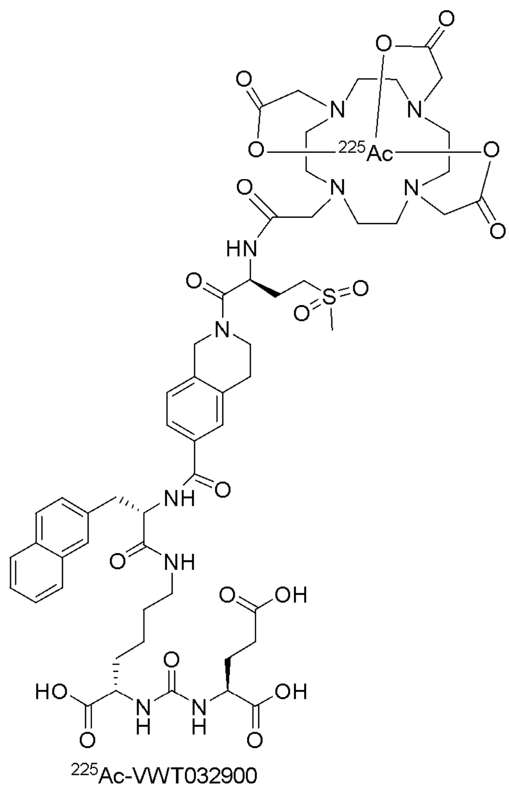

$^{225}$Ac-VWT032900
,

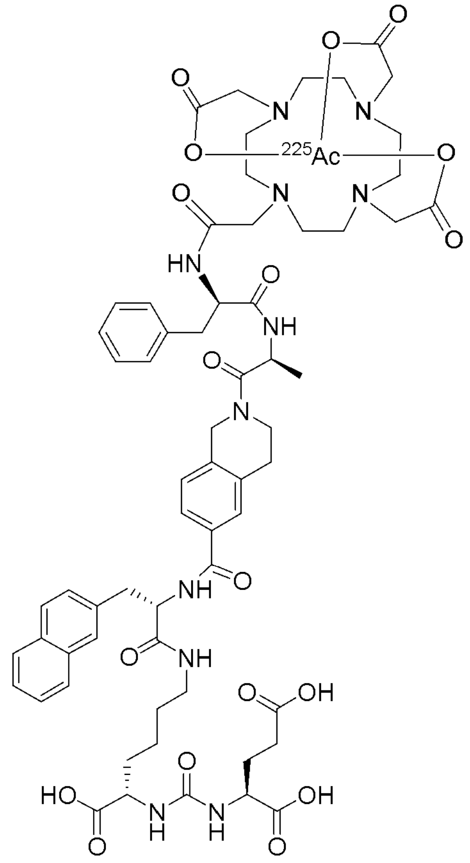

$^{225}$Ac-VWT039600 ,

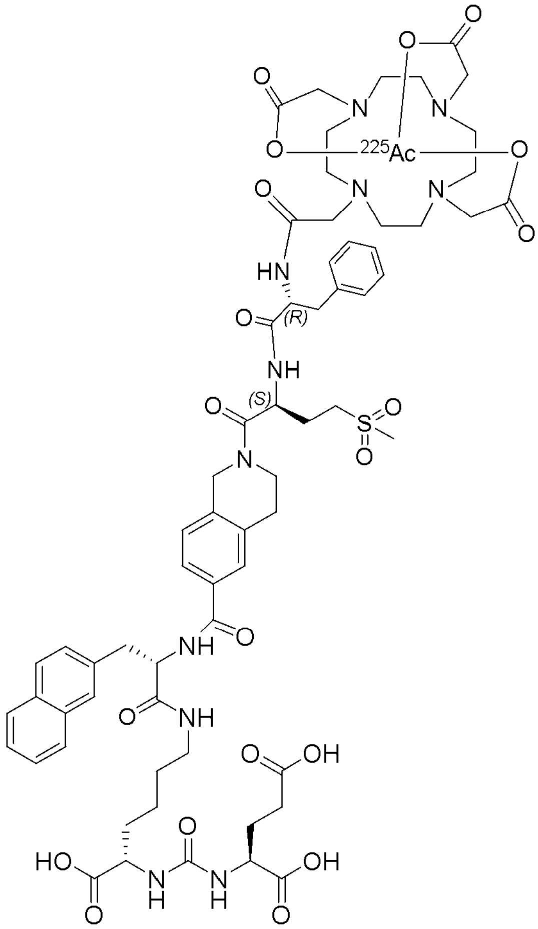

$^{225}$Ac-VWT045800 ,

and the like.

After preparation, the purity of the radioactive compound was determined using chromatography paper (Xinhua No. 1 paper). The developing agent used in the determination was 1% EDTA.

2.2 Administration and testing

The drug solutions prepared above were injected into LnCap tumor-bearing mice via tail vein injection (consistent with the intended clinical administration) with a single dose at an administration concentration (dosage) of 0.2 mL, 200 μCi/nmol; and biological distribution studies were conducted.

SPECT/CT scans were performed using a small animal SPECT-CT imaging system (U-SPECT+/CT, MI Labs). Data acquisition was performed by static SPECT for 10 minutes, followed by a low-resolution whole-body CT scan.

Mice were euthanized by cervical dislocation at 4 h and 24 h after administration. Animal weight, injection dosage, injection time, and residual dosage were recorded, along with the time of measurement of the injection dosage and the residual dosage. Tissue samples were collected from the heart, liver, spleen, lungs, kidneys, and tumors. The net weight of the tissues was first weighed, followed by radioactivity counting to determine the distribution of the radiolabeled isotope across different tissues and organs in mice. Furthermore, the test sample was diluted 100-fold, and 0.1 mL was placed in a counting tube as the standard 1% ID (one-hundredth of the administered dosage). The radioactivity counts of the 1% ID standard and the collected biological samples were measured using a gamma counter to determine the biodistribution of the drug in the mice.

3. Results Analysis

3.1 Labeling efficiency

The labeling efficiency of each compound to be tested was greater than 95%, indicating that the compounds did not require purification.

3.2 Biological distribution

Tissue distribution of each drug was observed using SPECT/CT, and visualized and quantified. The data on the distribution in biological tissues were expressed as the percentage of radioactivity count per gram of tissue or organ relative to the total administered dosage (radioactivity count) (%ID/g).

Visualized images after administration of compounds $^{177}$Lu-VWT011600 and $^{177}$Lu-VWT032900 are shown in Figures 1 and 2, respectively. The results of quantitative analysis are shown in Table 3; wherein T/K refers to the ratio of tumor uptake to renal uptake.

Table 3. Biodistribution of drugs in prostate tumor-bearing mice

| **Experiment 1** | | | | | | | |
|---|---|---|---|---|---|---|---|
| **ID%/g (4h)** | **Heart** | **Liver** | **Spleen** | **Lung** | **Kidney** | **Tumor** | **T/K** |
| $^{177}$Lu-VWT019000 | 0.06 | 0.10 | 0.10 | 0.08 | 1.27 | 4.78 | 3.76 |
| $^{177}$Lu-VWT019300 | 0.14 | 0.19 | 0.14 | 0.26 | 3.28 | 1.03 | 0.32 |
| $^{177}$Lu-VWT011600 | 0.15 | 0.17 | 0.28 | 0.06 | 1.34 | 6.68 | 4.98 |
| $^{177}$Lu-VWT022600 | 0.12 | 0.11 | 0.19 | 0.17 | 1.86 | / | / |
| $^{177}$Lu-VWT024000 | 0.04 | 0.03 | 0.65 | 0.04 | 1.35 | 16.88 | 12.51 |
| $^{177}$Lu- VWT024100 | 0.04 | 0.07 | 0.35 | 0.16 | 1.02 | 5.83 | 5.69 |
| $^{177}$Lu-VWT021400 | 0.04 | 0.05 | 0.17 | 0.05 | 0.62 | 5.40 | 8.75 |
| $^{177}$Lu-VWT023800 | 0.05 | 0.13 | 0.05 | 0.24 | 0.48 | 0.26 | 0.55 |
| $^{177}$Lu-VWT023900 | 0.09 | 0.04 | 1.30 | 0.05 | 2.37 | 18.09 | 7.62 |
| $^{177}$Lu-VWT022100 | 0.11 | 0.08 | 0.59 | 0.15 | 1.23 | 10.40 | 8.44 |
| **ID%/g (24h)** | **Heart** | **Liver** | **Spleen** | **Lung** | **Kidney** | **Tumor** | **T/K** |
| $^{177}$Lu-VWT019000 | 0.02 | 0.06 | 0.05 | 0.03 | 0.62 | 2.94 | 4.71 |
| $^{177}$Lu-VWT019300 | 0.06 | 0.10 | 0.09 | 0.05 | 0.64 | 0.65 | 1.02 |
| $^{177}$Lu-VWT011600 | 0.01 | 0.02 | 0.08 | 0.01 | 0.23 | 6.98 | 30.64 |
| $^{177}$Lu-VWT022600 | 0.04 | 0.05 | 0.05 | 0.04 | 0.38 | 8.88 | 23.07 |
| $^{177}$Lu-VWT024000 | 0.01 | 0.02 | 0.05 | 0.01 | 0.11 | 13.56 | 124.39 |
| $^{177}$Lu-VWT024100 | 0.01 | 0.03 | 0.06 | 0.01 | 0.17 | 6.54 | 37.90 |
| $^{177}$Lu-VWT021400 | 0.02 | 0.04 | 0.08 | 0.02 | 0.58 | 11.68 | 19.97 |
| $^{177}$Lu-VWT023800 | 0.02 | 0.03 | 0.04 | 0.02 | 0.07 | 0.07 | 1.09 |
| $^{177}$Lu-VWT023900 | 0.03 | 0.04 | 0.14 | 0.02 | 0.32 | 12.32 | 39.09 |
| $^{177}$Lu-VWT0221 00 | 0.02 | 0.01 | 0.05 | 0.02 | 0.45 | 6.47 | 14.23 |
| $^{77}$Lu-VWT001500 | 0.00 | 0.02 | 0.04 | 0.00 | 0.15 | 5.98 | 38.63 |
| **Experiment 2** | | | | | | | |
| **ID%/g (4h)** | **Heart** | **Liver** | **Spleen** | **Lung** | **Kidney** | **Tumor** | **T/K** |
| $^{177}$Lu-VWT032800 | 1.12 | 1.28 | 7.65 | 1.04 | 2.00 | 17.17 | 8.59 |
| $^{177}$Lu-VWT032900 | 0.79 | 1.93 | 1.66 | 0.22 | 4.06 | 21.12 | 5.20 |
| $^{77}$Lu-VWT001500 | 4.45 | 1.09 | 2.77 | 1.82 | 3.91 | 14.10 | 3.61 |
| **ID%/g (8h)** | **Heart** | **Liver** | **Spleen** | **Lung** | **Kidney** | **Tumor** | **T/K** |
| $^{177}$Lu-VWT032800 | 1.56 | 0.96 | 3.21 | 1.17 | 2.13 | 17.86 | 8.40 |
| $^{177}$Lu-VWT032900 | 2.08 | 0.78 | 0.39 | 0.76 | 2.16 | 33.56 | 15.51 |
| $^{77}$Lu-VWT001500 | 0.71 | 2.14 | 5.17 | 1.28 | 5.32 | 14.04 | 2.64 |

(continued)

| ID%/g (24h) | Heart | Liver | Spleen | Lung | Kidney | Tumor | T/K |
|---|---|---|---|---|---|---|---|
| $^{177}$Lu-VWT032800 | 1.14 | 1.20 | 2.16 | 0.75 | 2.44 | 13.98 | 5.73 |
| $^{177}$Lu-VWT032900 | 1.08 | 1.15 | 1.18 | 0.21 | 1.16 | 18.85 | 16.27 |
| $^{177}$Lu-VWT039600 | 0.86 | 1.86 | 1.03 | 1.48 | 5.25 | 41.66 | 7.93 |
| $^{77}$Lu-VWT001500 | 1.15 | 2.02 | 1.15 | 2.82 | 2.84 | 11.57 | 4.08 |
| **ID%/g (48h)** | **Heart** | **Liver** | **Spleen** | **Lung** | **Kidney** | **Tumor** | **T/K** |
| $^{177}$Lu-VWT032800 | 2.27 | 2.63 | 2.45 | 0.32 | 2.87 | 12.80 | 4.46 |
| $^{177}$Lu-VWT032900 | 0.63 | 0.27 | 0.83 | 0.70 | 2.12 | 17.07 | 8.03 |
| $^{77}$Lu-VWT001500 | 1.35 | 3.01 | 3.64 | 0.83 | 0.52 | 8.44 | 16.31 |
| **ID%/g (72h)** | **Heart** | **Liver** | **Spleen** | **Lung** | **Kidney** | **Tumor** | **T/K** |
| $^{177}$Lu-VWT032800 | 1.13 | 1.13 | 0.92 | 0.80 | 1.91 | 10.92 | 5.72 |
| $^{177}$Lu-VWT032900 | 0.48 | 1.37 | 1.93 | 0.47 | 1.61 | 14.33 | 8.89 |
| $^{177}$Lu-VWT039600 | 1.26 | 0.59 | 0.69 | 0.88 | 1.15 | 34.14 | 29.81 |
| $^{77}$Lu-VWT001500 | 4.14 | 1.60 | 5.29 | 2.97 | 1.33 | 8.46 | 6.36 |

The results showed that, compared with the existing VWT001500 (the known compound PSMA-617, a marketed drug), the PSMA compound provided in the application has a higher tumor uptake and/or a lower or substantially equivalent renal uptake; wherein the higher tumor uptake means a better targeting effect and/or a better therapeutic effect (a primary evaluation indicator), the lower renal uptake means a lower nephrotoxicity of the drug (a secondary evaluation indicator); and the T/K (a ratio of tumor uptake to renal uptake) is another evaluation indicator for drug screening and/or clinical drug selection.

4. 22RV1 (human prostate cancer cell) subcutaneous tumor model

22RV1 subcutaneous tumor model mice (B-NDG, Biocytogen Jiangsu Gene Biotechnology Co., Ltd.) were used. Mice were randomly assigned to 5 experimental groups (n=5 per group) according to tumor volume. Body weight and tumor size were measured. On the day of grouping, control group (no drug), group 1 ($^{177}$Lu-VWT001500, 0.3 mCi/mouse), group 2 ($^{177}$Lu-VWT032800, 0.3 mCi/mouse), group 3 ($^{177}$Lu-VWT032800, 0.9 mCi/mouse), and group 4 ($^{177}$Lu-VWT032900, 0.3 mCi/mouse) were administrated. Health and appearance were observed daily thereafter, and tumor size was measured before the sampling time point. Tumor volume results are shown in Figure 3.

Experimental results showed that $^{177}$Lu-VWT032800 and $^{177}$Lu-VWT032900 have significantly enhanced efficacy in the mouse 22RV1 (human prostate cancer cell) subcutaneous tumor model; and the efficacy was significantly dose-dependent.

5. Uptake rate and internalization rate

See Tables 4 and 5 for details.

Table 4. Conditions and results of LnCaP cell uptake

| | | VWT011600 | VWT032800 | VWT032900 | VWT001500 |
|---|---|---|---|---|---|
| Labeled nuclides | | $^{177}$Lu | | | |

(continued)

| | | VWT011600 | VWT032800 | VWT032900 | VWT001500 |
|---|---|---|---|---|---|
| Labeled conditions | Stock (mmol) | 5 | | | |
| | Solvent | $^{177}LuCl_3$ solution: physiological saline: sodium acetate solution =5.5: 5: 45; Animal administration: physiological saline | | | |
| | Radiation dose (mci) | 5.08 | 5.21 | 5.35 | 5.37 |
| | Reaction volume (μL) | 55.5 | | | |
| | Reaction time (min) | 20 | | | |
| | Reaction temperature (°C) | 90 | | | |
| Quality control | TLC | | | | |
| | Developing agent | 0.1 M sodium citrate solution (pH = 5.0) | | | |
| | Chromatography paper | iTLC-SG-Glass chromatography plate | | | |
| Radiochemical purity and stability results | | 94.80% (0 h) | 93.30% (0 h) | 95.29% (0 h) | 94.21% (0 h) |
| Cellular uptake conditions | Incubation temperature (°C) | 37 | | | |
| | Incubation time (min) | 45 | | | |
| Cellular uptake results | Total T | 374950 | 370820 | 352702 | 413099 |
| | uptake rate% | 1.95% | 2.49% | 1.89% | 1.01% |
| | internalization rate% | 9.93% | 10.24% | 13.42% | 9.57% |

wherein

Uptake rate% = (Membrane binding+internalization) /total T*100%; and internalization rate% = internalization/ (Membrane binding+internalization) *100%.

Table 5. Detailed results of LnCaP cell uptake

| LnCaP (1E5 cells/well) + $^{177}$Lu-VWT011600 (30 nM) | | | | | | |
|---|---|---|---|---|---|---|
| Time point (min) | Membrane binding | Internalization | uptake rate% | | internalization rate% | |
| | | | Result | Mean | Result | Mean |
| 45 | 7059 | 869 | 2.11 | 1.95 | 10.96 | 9.93 |
| | 6139 | 715 | 1.83 | | 10.43 | |
| | 6505 | 597 | 1.89 | | 8.41 | |
| LnCaP (1E5 cells/well) + $^{177}$Lu-VWT032800 (30 nM) | | | | | | |
| Time point (min) | Membrane binding | Internalization | uptake rate% | | internalization rate% | |
| | | | Result | Mean | Result | Mean |
| 45 | 8265 | 941 | 2.48 | 2.49 | 10.22 | 10.24 |
| | 8421 | 900 | 2.51 | | 9.65 | |
| | 8156 | 993 | 2.47 | | 10.85 | |

(continued)

| LnCaP (1E5 cells/well) + $^{177}$Lu-VWT032900 (30 nM) | | | | | | |
|---|---|---|---|---|---|---|
| Time point (min) | Membrane binding | Internalization | uptake rate% | | internalization rate% | |
| | | | Result | Mean | Result | Mean |
| 45 | 5683 | 769 | 1.83 | 1.89 | 11.92 | 13.42 |
| | 5440 | 953 | 1.81 | | 14.91 | |
| | 6202 | 964 | 2.03 | | 13.45 | |
| LnCaP (1E5 cells/well) + $^{177}$Lu-VWT001500 (30 nM) | | | | | | |
| Time point (min) | Membrane binding | Internalization | uptake rate% | | internalization rate% | |
| | | | Result | Mean | Result | Mean |
| 45 | 3711 | 411 | 1.00 | 1.01 | 9.98 | 9.57 |
| | 3691 | 455 | 1.00 | | 10.98 | |
| | 3912 | 329 | 1.03 | | 7.75 | |

In terms of uptake rate and/or internalization rate, the PSMA-targeting compounds/complexes of the present disclosure, such as $^{177}$Lu-VWT011600 and $^{177}$Lu-VWT032800, are more effective than the known $^{177}$Lu-VWT001500.

The PSMA compounds and radiolabeled complex thereof provided in the disclosure can improve drug targeting efficacy and/or reduce the risk of kidney damage, with a long duration of action, which is beneficial to better meet the medication needs of patients, thereby having broad prospects for clinical application and higher application values, especially in targeted therapy of prostate cancer, imaging technology, and the like.

Of course, the present disclousre can have many other embodiments. Without departing from the spirit and essence of the present invention, those skilled in the art can make various corresponding changes and/or modifications according to the present disclousre, and these corresponding changes and/or modifications should all fall within the protection scope of the appended claims.

## Claims

1. A compound of Formula I or pharmaceutically acceptable salt, ester or solvate thereof,

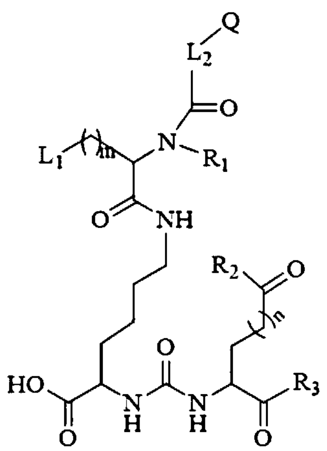

Formula I

wherein

Q is

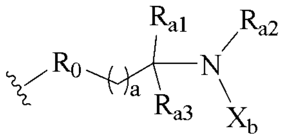;

$R_0$ is selected from carbonyl or methylene;
$X_b$ is a chelating agent or

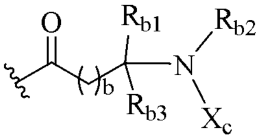;

$X_c$ is a chelating agent or

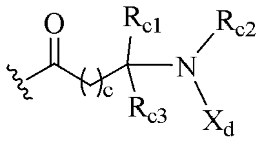;

$X_d$ is a chelating agent or

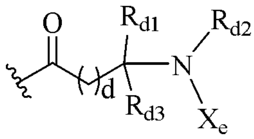;

$X_e$ is a chelating agent or

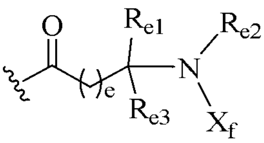;

$X_f$ is a chelating agent or

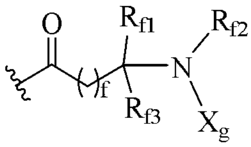;

$X_g$ is a chelating agent or

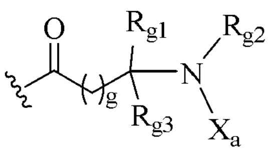;

$X_a$ is a chelating agent;
$R_{a1}$, $R_{b1}$, $R_{c1}$, $R_{d1}$, $R_{e1}$, $R_{f1}$ and $R_{g1}$ are all $R_{y1}$;
$R_{a2}$, $R_{b2}$, $R_{c2}$, $R_{d2}$, $R_{e2}$, $R_{f2}$ and $R_{g2}$ are all $R_{y2}$;

$R_{a3}$, $R_{b3}$, $R_{c3}$, $R_{d3}$, $R_{e3}$, $R_{f3}$ and $R_{g3}$ are all $R_{y3}$;

$R_{y1}$, $R_{y2}$ and $R_{y3}$ are each independently selected from hydrogen, deuterium, tritium, $R_Y$, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$ or $-C(=S)R_k$;

or, $R_{y2}$ is connected to $R_{y1}$ (or $R_{y3}$) to form $R_{ring\ A}$;

$R_Y$ is selected from a C1-C16 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl (including a fused aryl, and the like), or a 5-14 membered heteroaryl (including a fused heteroaryl); and $R_{ring\ A}$ is a 3-12 membered heterocyclic alkyl or a 5-14 membered heteroaryl;

$R_Y$ and $R_{ring\ A}$ are optionally substituted with one or more substituents $P_1$ or unsubstituted;

the substituent $P_1$ selected from deuterium, tritium, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$ or $R_W$; wherein $R_W$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl, or a 5-14 membered heteroaryl; and $R_W$ is optionally substituted with one or more substituents $P_2$ or unsubstituted;

the substituent $P_2$ selected from deuterium, tritium, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$ or $R_X$; wherein $R_X$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl, or a 5-14 membered heteroaryl; and $R_X$ is optionally substituted with one or more substituents $P_3$ or unsubstituted;

the substituent $P_3$ selected from deuterium, tritium, hydroxyl, mercapto, nitro, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$ or $R_V$; wherein $R_V$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl, or a 5-14 membered heteroaryl; and $R_V$ is optionally substituted with one or more substituents $P_4$ or unsubstituted;

the substituent $P_4$ is selected from deuterium, tritium, halogen, hydroxyl, mercapto, nitro, cyano, oxo, thio, $-NR_iR_j$, $-C(=O)R_k$, $-C(=O)OR_k$, $-S(=O)R_k$, $-S(=O)OR_k$, $-S(=O)(=O)R_k$, $-S(=O)(=O)OR_k$, $-C(=S)R_k$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, a sulfo-substituted C1-C6 alkyl, a C1-C6 alkoxy, an amino-substituted C1-C6 alkoxy, a carboxyl-substituted C1-C6 alkoxy, a sulfo-substituted C1-C6 alkoxy, a C1-C6 alkyl thioether group, an amino-substituted C1-C6 alkyl thioether group, a carboxyl-substituted C1-C6 alkyl thioether group, a sulfo-substituted C1-C6 alkyl thioether group, a C2-C6 alkenyl, a C2-C6 alkynyl, a 3-12 membered cycloalkyl, a 3-12 membered heterocyclic alkyl, a 5-14 membered aryl, or a 5-14 membered heteroaryl;

$R_k$ is selected from hydrogen, deuterium, tritium, halogen, $-NR_iR_j$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_1$, $R_2$, $R_3$, $R_i$ and $R_j$ are each independently selected from hydrogen, deuterium, tritium, hydroxyl, mercapto, nitro, cyano, halogen, $-S(=O)R_z$, $-S(=O)OR_z$, $-S(=O)(=O)R_z$, $-S(=O)(=O)OR_z$, a C1-C6 alkyl, a C1-C6 alkoxy, a C1-C6 alkyl thioether group, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_z$ is selected from hydrogen, deuterium, tritium, halogen, a C1-C6 alkyl, a deuterated C1-C6 alkyl, a tritiated C1-C6 alkyl, or a halogenated C1-C6 alkyl;

a, b, c, d, e, f and g are each independently selected from integers between 0 and 6;

$L_1$ is selected from a 3-12 membered cycloalkyl, a 3-12 membered heterocyclic alkyl, a 5-14 membered aryl, or a 5-14 membered heteroaryl; wherein the 3-12 membered cycloalkyl, the 3-12 membered heterocyclic alkyl, the 5-14 membered aryl, or the 5-14 membered heteroaryl is optionally substituted with one or more substituents $P_1$ or unsubstituted;

$L_2$ is selected from $-L_3-$ or $-NH-(CH_2)_t-L_3-$; $L_3$ is selected from a 3-12 membered cycloalkyl, a 3-12 membered heterocyclic alkyl, a 5-14 membered aryl, or a 5-14 membered heteroaryl; wherein the 3-12 membered cycloalkyl, the 3-12 membered heterocyclic alkyl, the 5-14 membered aryl, or the 5-14 membered heteroaryl is optionally substituted with one or more substituents $P_1$ or unsubstituted;

the heteroatom of the 3-12 membered heterocyclic alkyl or the 5-14 membered heteroaryl is selected from one or more of N, O and S, and the 3-12 membered heterocyclic alkyl or the 5-14 membered heteroaryl has 1, 2, or 3 heteroatoms;

m, n and t are each independently selected from integers between 0 and 6.

**2.** The compound or pharmaceutically acceptable salt, ester or solvate thereof according to claim 1, wherein $L_1$ is a 5-14 membered aryl or a 5-14 membered heteroaryl; wherein the 5-14 membered aryl or the 5-14 membered heteroaryl is

optionally substituted with one or more substituents $P_{1a}$ or unsubstituted; and the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl.

**3.** The compound or pharmaceutically acceptable salt, ester or solvate thereof according to claim 2, wherein $L_1$ is selected from phenyl, pyrimidinyl, pyridinyl, pyrrolyl, oxazolyl, imidazolyl, furanyl, thiazolyl, thienyl, thiopyranyl, naphthyl, benzopyrrolyl (including: indolyl, isoindolyl), benzopyridyl (including: quinolinyl, isoquinolinyl), benzoxazolyl, benzimidazolyl, benzofuranyl, benzothiazolyl, benzothienyl, benzothiopyranyl, purinyl, anthryl, or phenanthryl; preferably, Li is naphthyl, benzopyridyl, or anthryl.

**4.** The compound or pharmaceutically acceptable salt, ester or solvate thereof according to claim 1, wherein $L_2$ is $-L_3-$; and $L_3$ is selected from a 3-12 membered heterocyclic alkyl, a 5-14 membered aryl, or a 5-14 membered heteroaryl; wherein the 3-12 membered heterocyclic alkyl, the 5-14 membered aryl or the 5-14 membered heteroaryl is optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl.

**5.** The compound or pharmaceutically acceptable salt, ester or solvate thereof according to claim 4, wherein $L_3$ is selected from a 3-12 membered heterocyclic alkyl, wherein the 3-12 membered heterocyclic alkyl comprises 1-4 heteroatoms N;

preferably, $L_3$ is $R_{ring B}$-$R_{ring C}$, $R_{ring B}$ and $R_{ring C}$ share one carbon atom; $R_{ring B}$ is selected from a 3-9 membered cycloalkyl or a 3-9 membered heterocyclic alkyl; $R_{ring C}$ is selected from a 3-9 membered heterocyclic alkyl; wherein the heteroatom of the 3-9 membered heterocyclic alkyl is selected from one or more of N, O and S, and the 3-9 membered heterocyclic alkyl has 1, 2, or 3 heteroatoms; preferably, $R_{ring C}$, comprises 1-2 heteroatoms N; $R_{ring B}$ and/or $R_{ring C}$ are optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;
more preferably, $R_{ring B}$ is selected from cyclobutyl, cyclopentyl, cyclohexyl, or cycloheptyl; and $R_{ring C}$ is selected from pyrrolidinyl, piperidinyl, oxazolidinyl, morpholinyl, thiomorpholinyl, azepanyl, oxazepanyl, or thiazepanyl.

**6.** The compound or pharmaceutically acceptable salt, ester or solvate thereof according to claim 5, wherein $L_3$ is selected from:

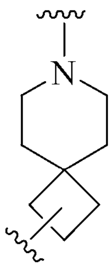, 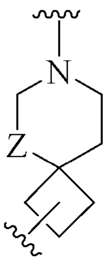, 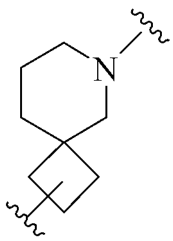, 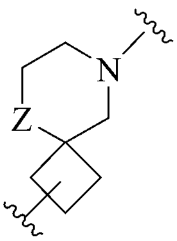, 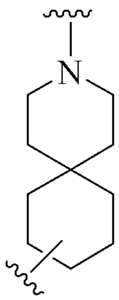, 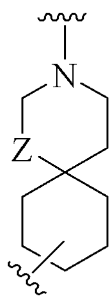, 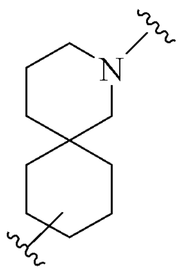,

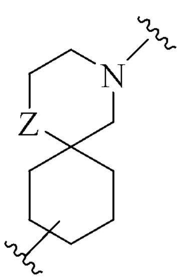;

and Z is O or S.

7. The compound or pharmaceutically acceptable salt, ester or solvate thereof according to claim 4, wherein $L_3$ is selected from a 5-14 membered heteroaryl, and the 5-14 membered heteroaryl comprises 1-4 heteroatoms N;

preferably, $L_3$ is $R_{ring\ D}$-$R_{ring\ E}$, $R_{ring\ D}$ and $R_{ring\ E}$ share two carbon atoms; $R_{ring\ D}$ is selected from a 5-6 membered aryl or a 5-6 membered heteroaryl; $R_{ring\ E}$ is selected from a 3-9 membered heterocyclic alkyl; wherein the heteroatom of the 5-6 membered heteroaryl or the 3-9 membered heterocyclic alkyl is selected from one or more of N, O and S, and the 3-9 membered heterocyclic alkyl has 1, 2, or 3 heteroatoms; and $R_{ring\ E}$ comprises 1-2 heteroatoms N;

$R_{ring\ D}$ and/or $R_{ring\ E}$ are optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

more preferably, $R_{ring\ D}$ is selected from phenyl, pyrimidinyl, pyridinyl, pyrrolyl, oxazolyl, imidazolyl, furanyl, thiazolyl, thienyl, or thiopyranyl; and $R_{ring\ E}$ is selected from pyrrolidinyl, piperidinyl, oxazolidinyl, morpholinyl, thiomorpholinyl, azepanyl, azepanyl, oxazepanyl, or thiazepanyl.

8. The compound or pharmaceutically acceptable salt, ester or solvate thereof according to claim 7, wherein $L_3$ is selected from:

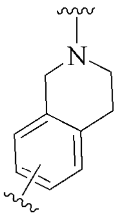 , 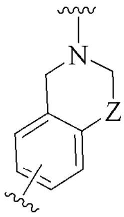 , 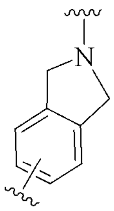 , 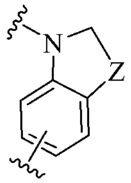 , 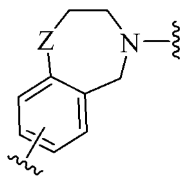 , 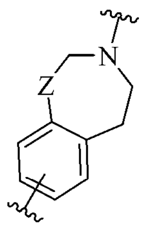 , 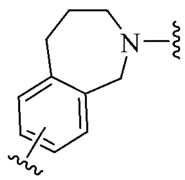 ,

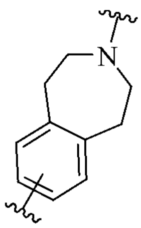 ;

and Z is O or S;
preferably,
$L_3$ is

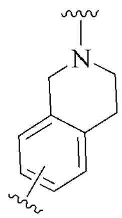 , 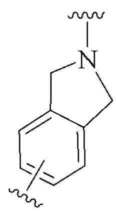 or 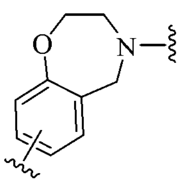 .

9. The compound or pharmaceutically acceptable salt, ester or solvate thereof according to claim 1, wherein the chelating agent is selected from 1,4,7-triazacyclononane-1,4,7-triacetic acid (NOTA), 1,4,7,10-tetraazacyclododecane-1,4,7,10-tetraacetic acid (DOTA:

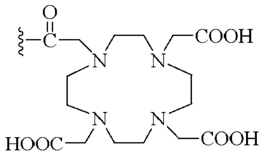

), 2-(4,7,10-tris(carboxymethyl)-1,4,7,10-tetraazacyclododecane-1-yl)-glutaric acid (DOTAGA:

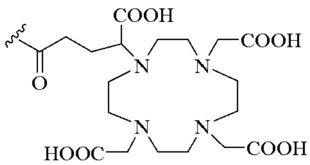

), N,N"-bis[2-hydroxy-5-(carboxyethyl)-benzyl]ethylenediamine-N,N"-diacetic acid (HBED-CC), 2-(4,7-bis(carboxymethyl)-1,4,7-triazacyclononane-1-yl)glutaric acid (NODAGA), 1,4,7-triazacyclononane phosphinic acid (TRAP), 1,4,7-triazacyclononane-1-[methyl(2-carboxyethyl)phosphinic acid]-4,7-bis[methyl(2-hydroxymethyl)phosphinic acid](NOPO), 3,6,9,15-tetraazabicyclo[9,3,1]pentadecane-1(15), 11, 13-triene-3,6,9-triacetic acid (PCTA), N'-{5-[acetyl(hydroxy)amino]pentyl}-N-[5-({4-[(5-aminopentyl)(hydroxy)amino]-4-oxobutyryl}amino)pentyl]-N-hydroxysuccinamide (DFO), diethylenetriaminepentaacetic acid (DTPA:

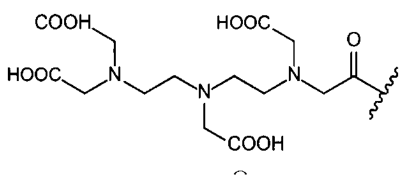 ), 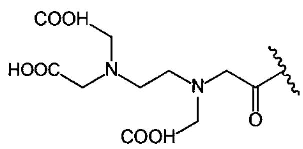 ,

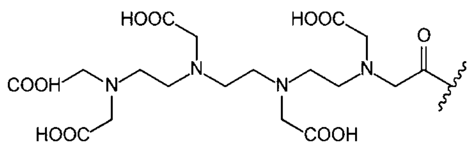 , 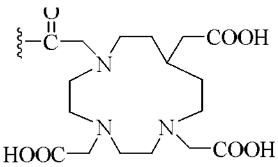 or 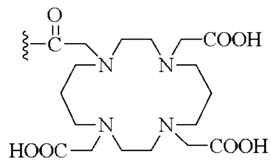 ;

preferably, the chelating agent is DOTA or DOTAGA.

**10.** The compound or pharmaceutically acceptable salt, ester or solvate thereof according to claim 1, wherein m, n and t are each independently selected from 1 or 2; $R_1$ is selected from hydrogen, deuterium, tritium, a C1-C6 alkyl or a deuterated C1-C6 alkyl; and $R_2$ and $R_3$ are each independently selected from hydroxyl or C1-C6 alkoxy.

**11.** The compound or pharmaceutically acceptable salt, ester or solvate thereof according to claim 1, wherein the $R_{y1}$ or $R_{y3}$ is of S or R configuration; and/or, the configuration of the compound of Formula I is:

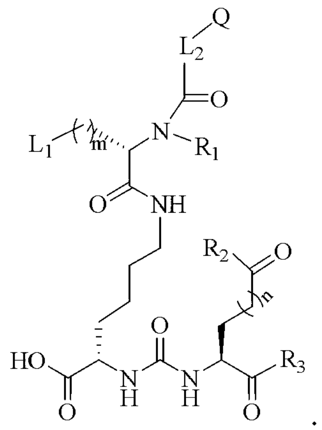

12. The compound or pharmaceutically acceptable salt, ester or solvate thereof according to any one of claims 1 to 11, wherein $X_b$ is a chelating agent;

$R_{y1}$ and $R_{y3}$ are each independently selected from hydrogen, deuterium, tritium, $R_Y$, hydroxyl, mercapto, cyano, amino, carboxyl, sulfo-group; wherein $R_Y$ is selected from a C1-C6 alkyl, a deuterated C1-C6 alkyl, a tritiated C1-C6 alkyl, a halogenated C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl, or a 5-14 membered heteroaryl;

$R_{y2}$ is selected from hydrogen, deuterium, tritium, halogen, hydroxyl, mercapto, nitro or cyano; or, $R_{y2}$ is connected to $R_{y1}$ (or $R_{y3}$) to form $R_{ring\ A}$, the $R_{ring\ A}$ is a tetrahydropyrrole ring or a piperidine ring; the $R_{ring\ A}$ is optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

preferably, the compound of Formula I is:

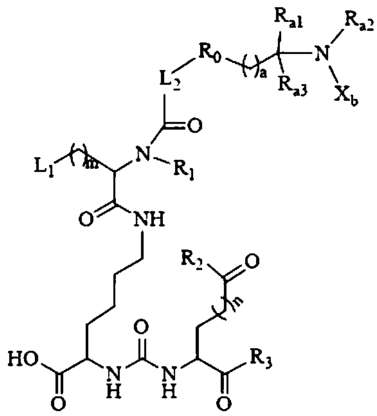

Formula Ia

wherein $X_b$ is a chelating agent; $R_{a1}$ and $R_{a3}$ are each independently selected from hydrogen, deuterium, tritium, $R_Y$; $R_Y$ is selected from a C1-C6 alkyl, a deuterated C1-C6 alkyl or a tritiated C1-C6 alkyl; and $R_{a2}$ is selected from hydrogen, deuterium, or tritium; or, $R_{a2}$ is connected to $R_{a1}$ (or $R_{a3}$) to form $R_{ringA}$, the $R_{ringA}$ is a tetrahydropyrrole ring; and a is 0, 1 or 2.

13. The compound or pharmaceutically acceptable salt, ester or solvate thereof according to claim 12, wherein the compound of Formula Ia is selected from:

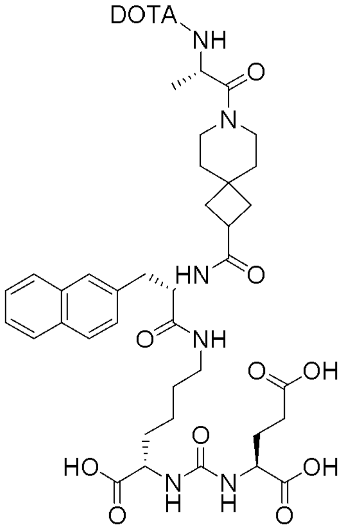

VWT011400 ,

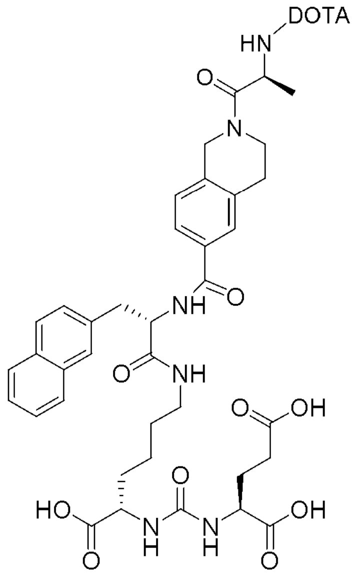

VWT011600 ,

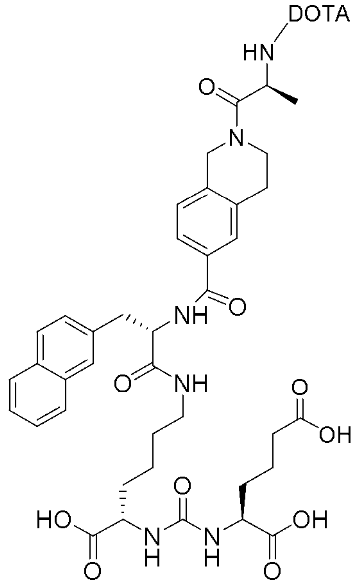

VWT019000 ,

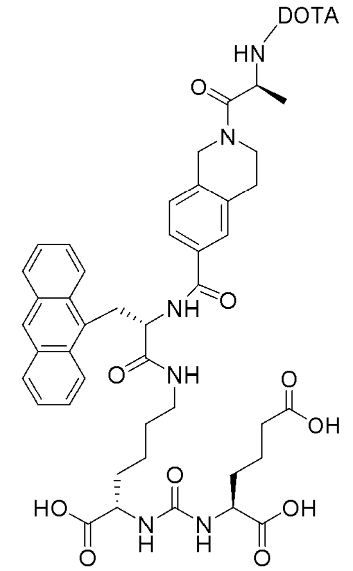

VWT019300 ,

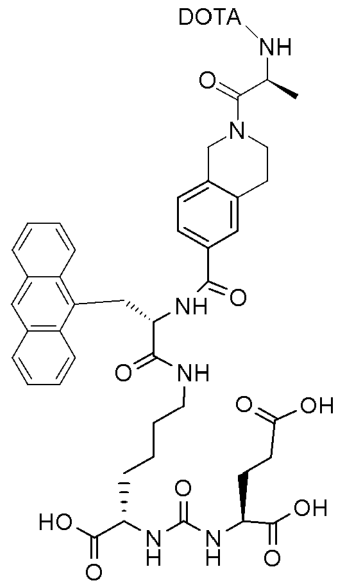

VWT020100 ,

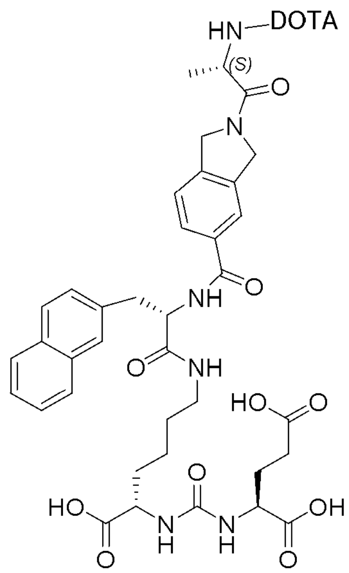

VWT021400 ,

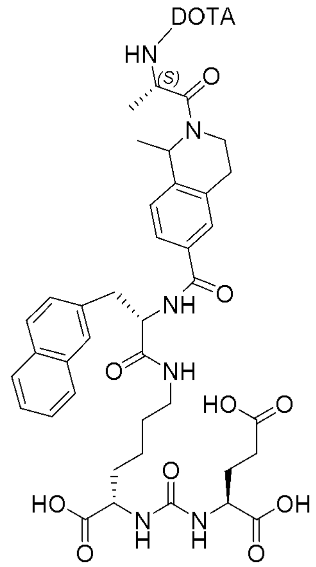

VWT021700 ,

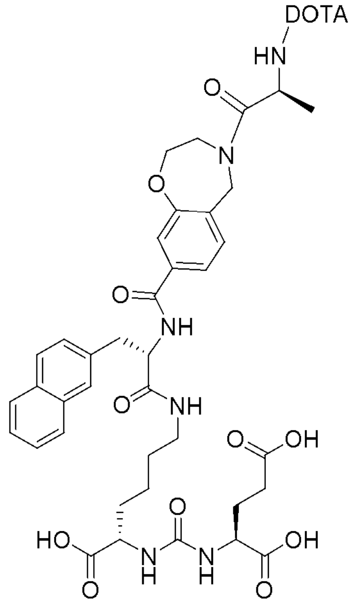

VWT022100 ,

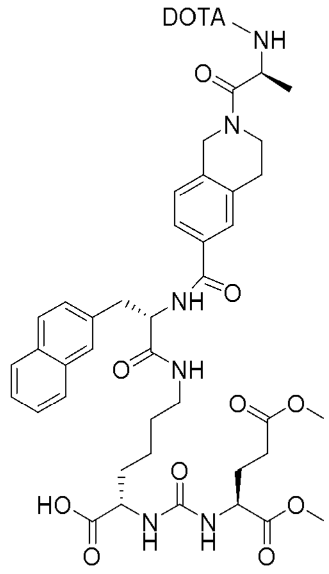

VWT023800 ,

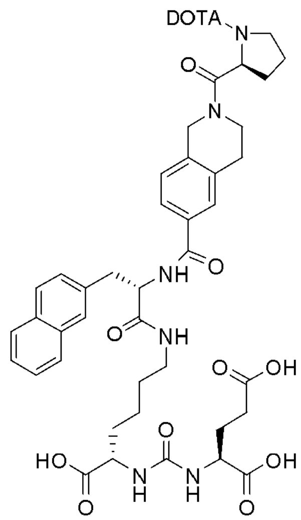

VWT032100 ,

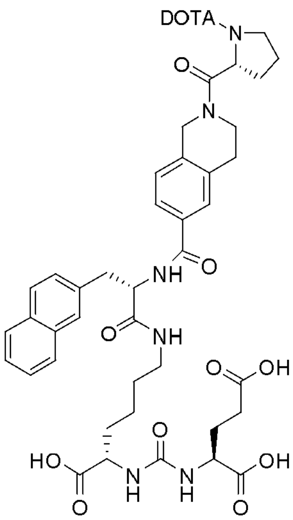

VWT032200 ,

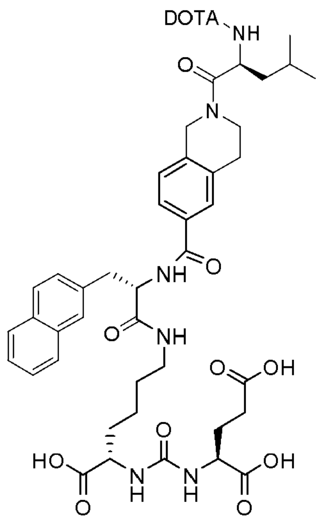

VWT044400 ,

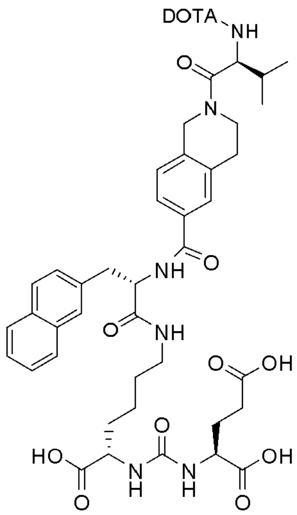
VWT044600 ,

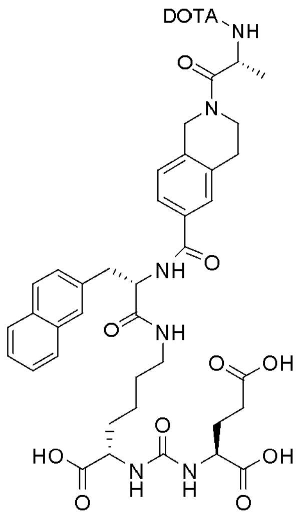
VWT044800 ,

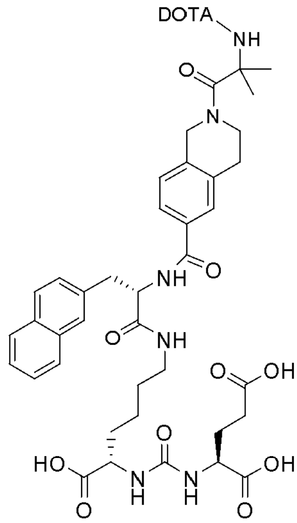
VWT044900 .

**14.** The compound or pharmaceutically acceptable salt, ester or solvate thereof according to any one of claims 1 to 11, wherein $X_b$ is a chelating agent;

$R_{y1}$ and $R_{y3}$ are each independently selected from hydrogen, deuterium, tritium, $R_Y$, hydroxyl, mercapto, cyano, amino, carboxyl, or sulfo-group; $R_Y$ is selected from a C1-C6 alkyl, a deuterated C1-C6 alkyl, a tritiated C1-C6 alkyl, a halogenated C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl or a 5-14 membered heteroaryl;

$R_{y2}$ is a C1-C16 alkyl that is optionally substituted with one or more substituents $P_1$ or unsubstituted; and the substituent $P_1$ is selected from deuterium, tritium, amino, carboxyl, sulfo-group, or $R_W$; wherein $R_W$ is selected from a C1-C6 alkoxy or a C1-C6 alkyl thioether group; and $R_W$ is optionally substituted with one or more substituents $P_2$ or unsubstituted; and the substituent $P_2$ is selected from -C(=O)$R_k$, -C(=O)O$R_k$ or $R_X$; wherein $R_X$ is selected from a C1-C6 alkyl, a C1-C6 alkoxy or a C1-C6 alkyl thioether group; $R_X$ is optionally substituted with one or more substituents $P_3$ or unsubstituted; and the substituent $P_3$ is selected from -C(=O)$R_k$ or -C(=O)O$R_k$; wherein $R_k$ is selected from hydrogen, deuterium, tritium, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

preferably, the compound of Formula I is:

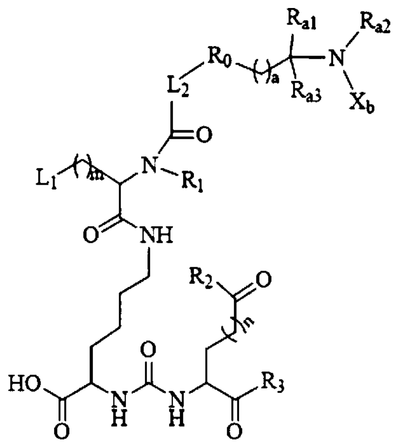

Formula Ib

wherein $X_b$ is a chelating agent; $R_{a1}$ and $R_{a3}$ are each independently selected from hydrogen, deuterium, tritium or $R_Y$; $R_Y$ is selected from a C1-C6 alkyl, a deuterated C1-C6 alkyl or a tritiated C1-C6 alkyl; $R_{a2}$ is a C1-C14 alkyl

that is optionally substituted with one or more substituents $P_1$; and the substituent $P_1$ is selected from deuterium, tritium, carboxyl, sulfo-group, or $R_W$; wherein $R_W$ is selected from a C1-C6 alkoxy; and $R_W$ is optionally substituted with one or more substituents $P_2$ or unsubstituted; and the substituent $P_2$ is selected from -C(=O)OR$_k$ or $R_X$; wherein $R_X$ is selected from a C1-C6 alkoxy or a C1-C6 alkyl thioether group; $R_X$ is optionally substituted with one or more substituents $P_3$ or unsubstituted; and the substituent $P_3$ is selected from -C(=O)OR$_k$; $R_k$ is selected from hydrogen, deuterium, tritium, a C1-C6 alkyl, a deuterated C1-C6 alkyl, or a tritiated C1-C6 alkyl; and a is 0, 1 or 2.

**15.** The compound or pharmaceutically acceptable salt, ester or solvate thereof according to claim 14, wherein the compound of Formula Ib is selected from:

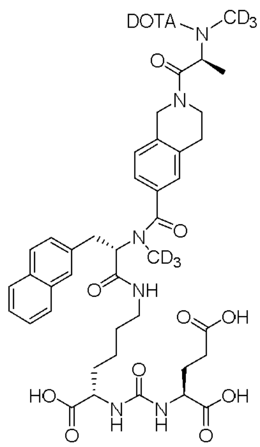
VWT023600 ,

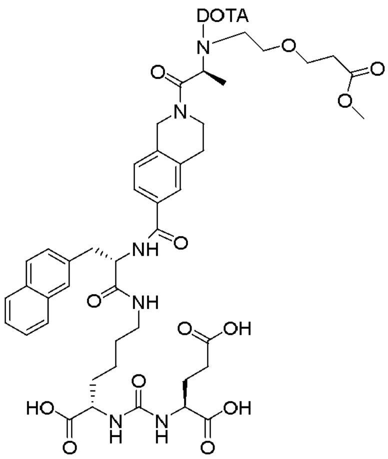
VWT026100 ,

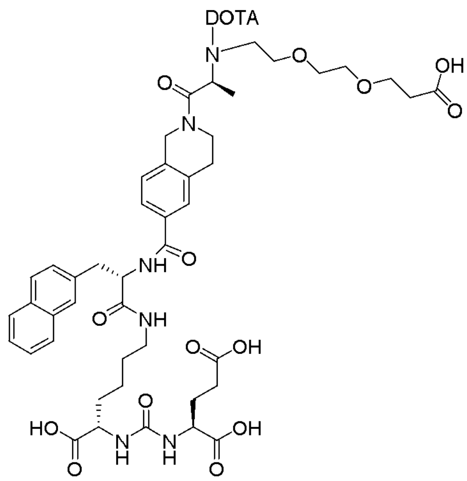
VWT026300 ,

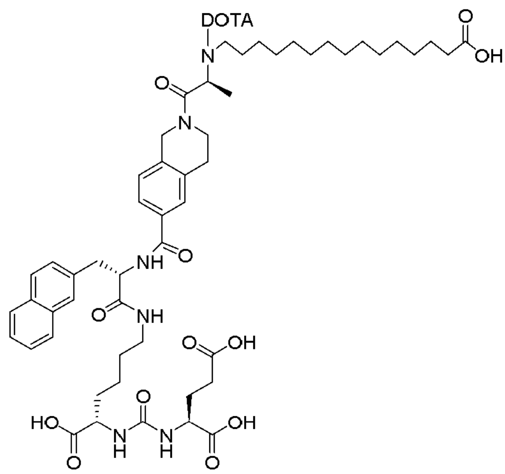
VWT026000 .

**16.** The compound or pharmaceutically acceptable salt, ester or solvate thereof according to any one of claims 1 to 11, wherein $X_b$ is a chelating agent;

$R_{y1}$ and $R_{y3}$ are each independently selected from hydrogen, deuterium, tritium, $R_Y$, hydroxyl, mercapto, nitro, cyano, oxo, thio, halogen, amino, carboxyl, sulfo-group; wherein $R_Y$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl (including but not limited to: oxadiazolidinyl), a 5-14 membered aryl (including but not limited to: phenyl) or a 5-14 membered heteroaryl (including but not limited to: imidazolyl, oxadiazolyl, benzopyrrolyl);

$R_Y$ is optionally substituted with one or more substituents $P_1$ or unsubstituted; and the substituent $P_1$ is selected from deuterium, tritium, hydroxyl, mercapto, -NR$_i$R$_j$, -C(=O)R$_k$, - C(=O)OR$_k$, -S(=O)R$_k$, -S(=O)OR$_k$, -S(=O)(=O)R$_k$, -S(=O)(=O)OR$_k$, -C(=S)R$_k$ or $R_W$;

$R_W$ is selected from a C1-C6 alkyl, a C1-C6 alkoxy, a C1-C6 alkyl thioether group, a C2-C6 alkenyl, a C2-C6

alkynyl, a 3-12 membered cycloalkyl, a 3-12 membered heterocyclic alkyl, a 5-14 membered aryl or a 5-14 membered heteroaryl; and $R_W$ is optionally substituted with one or more substituents $P_2$ or unsubstituted; and the substituent $P_2$ is selected from deuterium, tritium, hydroxyl, mercapto, cyano, oxo, thio, halogen, amino, carboxyl, sulfo-group, or $R_X$; wherein $R_X$ is selected from a C1-C6 alkyl, a C1-C6 alkoxy, or a C1-C6 alkyl thioether group; $R_k$ is selected from hydrogen, deuterium, tritium, $-NR_iR_j$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_i$ and $R_j$ are each independently selected from hydrogen, deuterium, tritium, hydroxyl, mercapto, cyano, $-S(=O)R_z$, $-S(=O)OR_z$, $-S(=O)(=O)R_z$, $-S(=O)(=O)OR_z$, a C1-C6 alkyl, a C1-C6 alkoxy, a C1-C6 alkyl thioether group, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_z$ is selected from hydrogen, deuterium, tritium, halogen, a C1-C6 alkyl, a deuterated C1-C6 alkyl, a tritiated C1-C6 alkyl, or a halogenated C1-C6 alkyl;

$R_{y2}$ is selected from hydrogen, deuterium, tritium, a C1-C6 alkyl, a deuterated C1-C6 alkyl, or a tritiated C1-C6 alkyl; or, $R_{y2}$ is connected to $R_{y1}$ (or $R_{y3}$) to form $R_{ring\ A}$, and the $R_{ring\ A}$ is a tetrahydropyrrole ring or a piperidine ring; the $R_{ring\ A}$ is optionally substituted with one or more substituents $P_{1a}$ or unsubstituted; and the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

preferably, $X_b$ is a chelating agent; $R_{y1}$ is selected from hydrogen, deuterium, tritium, a C1-C6 alkyl, or a deuterated C1-C6 alkyl; $R_{y3}$ is $R_Y$; $R_Y$ is selected from a C1-C6 alkyl; $R_Y$ is optionally substituted with one or more substituents $P_1$; and $R_{y2}$ ($R_{a2}$) is selected from hydrogen, deuterium, tritium, a C1-C6 alkyl, or a deuterated C1-C6 alkyl; or, $R_{y2}$ is connected to $R_{y1}$ (or $R_{y3}$) to form $R_{ring\ A}$, and the $R_{ring\ A}$ is a tetrahydropyrrole ring; and a is 0, 1 or 2.

**17.** The compound or pharmaceutically acceptable salt, ester or solvate thereof according to claim 16, wherein the compound of Formula I is:

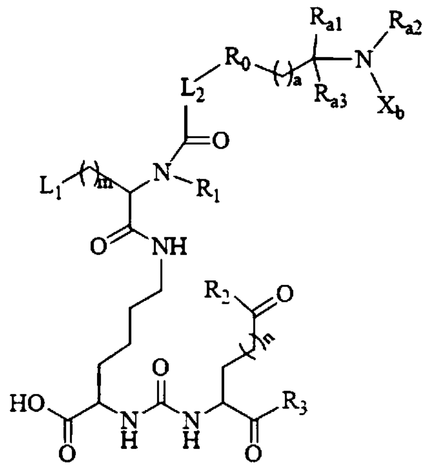

Formula Ic

wherein $X_b$ is a chelating agent;

$R_{a1}$ is selected from hydrogen, deuterium, tritium, or a C1-C6 alkyl;

$R_{a3}$ is $R_Y$; wherein $R_Y$ is selected from a C1-C6 alkyl; and $R_Y$ is optionally substituted with one or more substituents $P_1$; the substituent $P_1$ is selected from deuterium, tritium, $-S(=O)R_k$, $-S(=O)OR_k$, $-S(=O)(=O)R_k$, $-S(=O)(=O)OR_k$ or $R_W$; wherein $R_W$ is selected from oxadiazolyl, phenyl or benzopyrrolyl; and $R_W$ is optionally substituted with one or more substituents $P_2$ or unsubstituted; the substituent $P_2$ is selected from deuterium, tritium, hydroxyl, mercapto, cyano, oxo, thio, halogen or $R_X$; wherein $R_X$ is a C1-C6 alkyl; $R_k$ is selected from hydrogen, deuterium, tritium, a C1-C6 alkyl, a deuterated C1-C6 alkyl, or a tritiated C1-C6 alkyl;

$R_{a2}$ is selected from hydrogen, deuterium, tritium; or, $R_{a2}$ is connected to $R_{a1}$ (or $R_{a3}$) to form $R_{ring\ A}$, and the $R_{ring\ A}$ is a tetrahydropyrrole ring; and a is 0, 1 or 2;

preferably, the compound of Formula Ic is selected from:

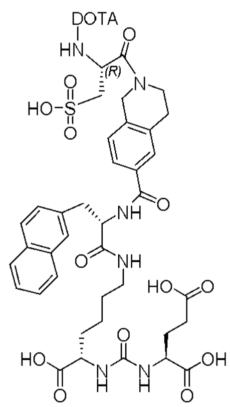
VWT022600 ,
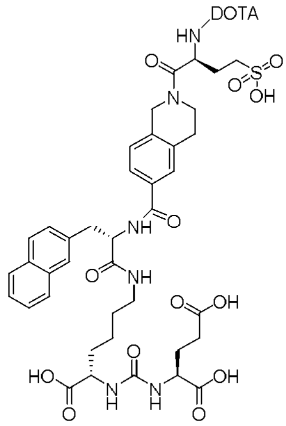
VWT032000 ,
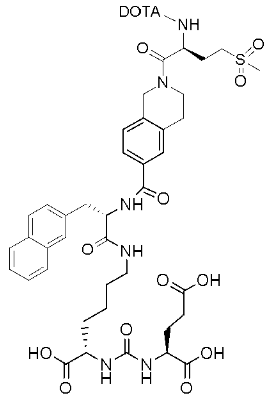
VWT032900 ,

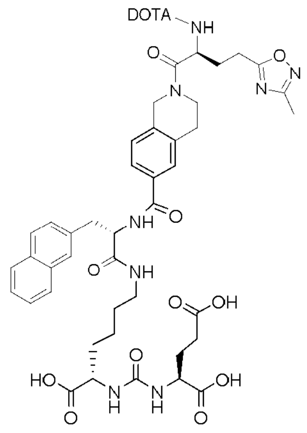
VWT033000 ,
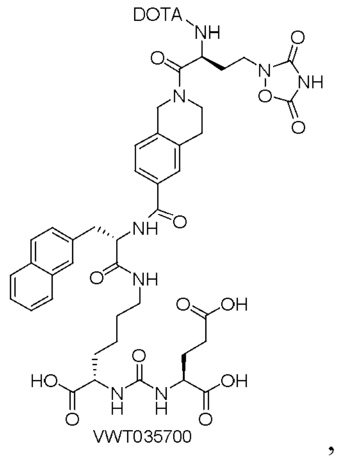
VWT035700 ,
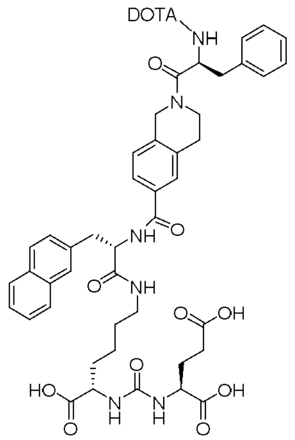
VWT044700 .

**18.** The compound or pharmaceutically acceptable salt, ester or solvate thereof according to claim 16, wherein the compound of Formula I is:

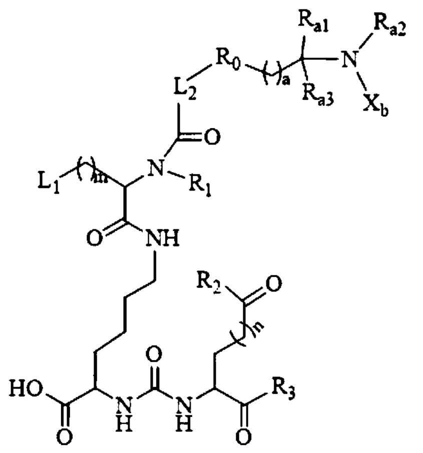

Formula Id

wherein $X_b$ is a chelating agent;
$R_{a1}$ is selected from hydrogen, deuterium, tritium, or a C1-C6 alkyl;
$R_{a3}$ is $R_Y$; wherein $R_Y$ is selected from a C1-C6 alkyl; and $R_Y$ is optionally substituted with one or more substituents

$P_1$; the substituent $P_1$ is selected from $-C(=O)R_k$, $-C(=O)OR_k$; $R_k$ is selected from hydrogen, deuterium, tritium, $-NR_iR_j$, a C1-C6 alkyl, a deuterated C1-C6 alkyl, or a tritiated C1-C6 alkyl; $R_i$ and $R_j$ are each independently selected from hydrogen, deuterium, tritium, hydroxyl, mercapto, cyano, $-S(=O)R_z$, $-S(=O)OR_z$, $-S(=O)(=O)R_z$ or $-S(=O)(=O)OR_z$; wherein $R_z$ is selected from hydrogen, deuterium, tritium, a C1-C6 alkyl, a deuterated C1-C6 alkyl, or a tritiated C1-C6 alkyl;

$R_{a2}$ is selected from hydrogen, deuterium, tritium, or a deuterated C1-C6 alkyl; or, $R_{a2}$ is connected to $R_{a1}$ (or $R_{a3}$) to form $R_{ring\ A}$, and the $R_{ring\ A}$ is a tetrahydropyrrole ring; and a is 0, 1 or 2;

preferably, the compound of Formula Id is selected from:

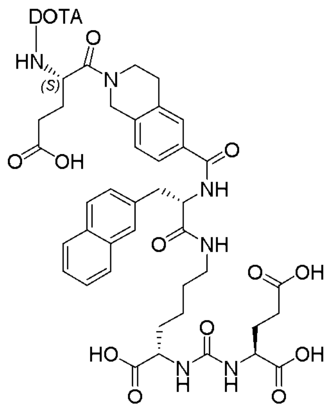
VWT024000 ,

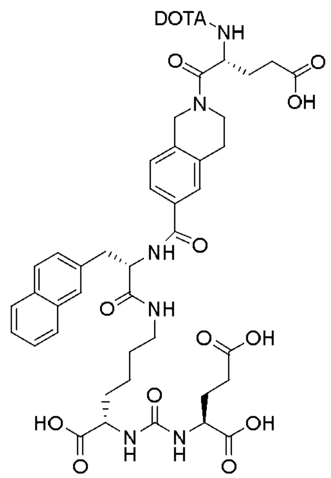
VWT031600 ,

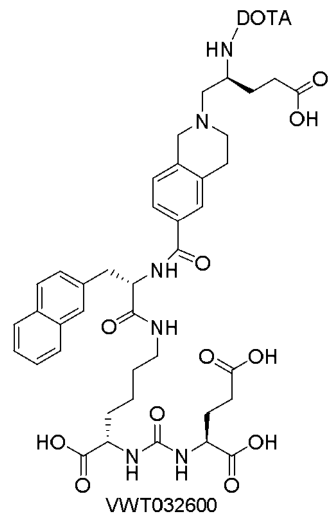
VWT032600 ,

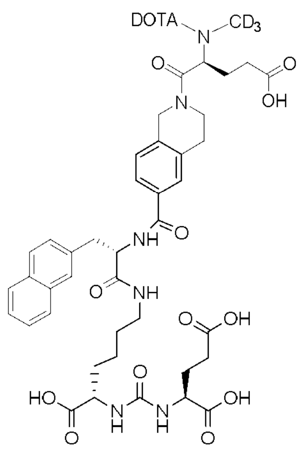
VWT032700 ,

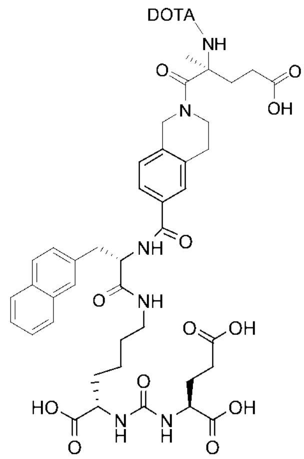
VWT032800 ,

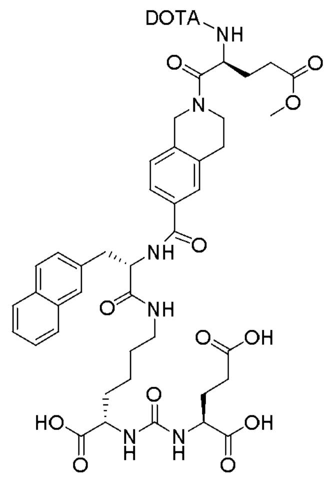
VWT024100 ,

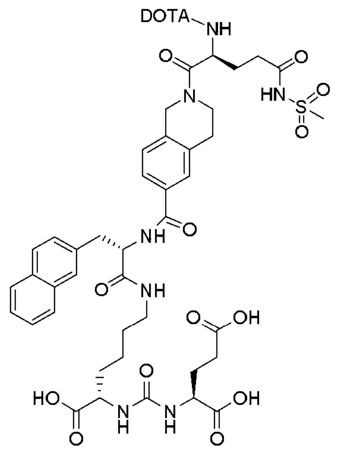

VWT031700

,

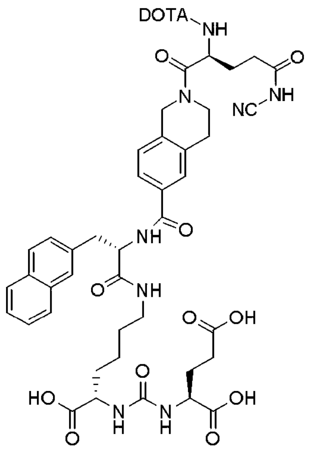

VWT031800

,

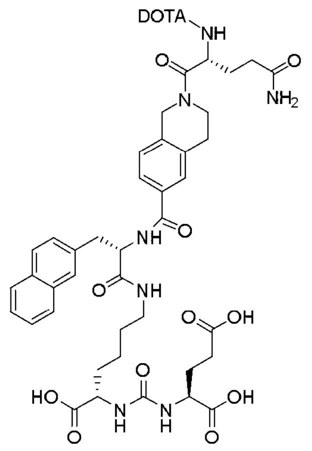

VWT032300

,

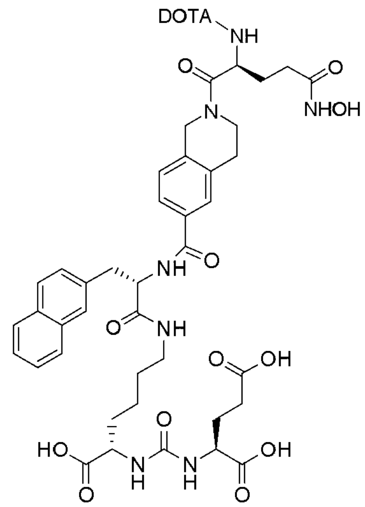

VWT035400

.

19. The compound or pharmaceutically acceptable salt, ester or solvate thereof according to claim 16, wherein the compound of Formula I is:

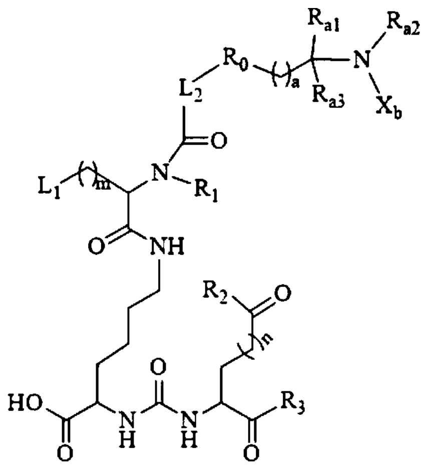

Formula Iz

wherein,

$X_b$ is a chelating agent;

$R_{a1}$ is selected from hydrogen, deuterium, tritium, or a C1-C6 alkyl;

$R_{a3}$ is $R_Y$; wherein $R_Y$ is selected from C1-C6 alkyl; and $R_Y$ is optionally substituted with one or more substituents $P_1$; the substituent $P_1$ is selected from $R_W$; $R_W$ is selected from a 5-14 membered aryl or a 5-14 membered heteroaryl; and $R_W$ is optionally substituted with one or more substituents $P_2$ or unsubstituted; the substituent $P_2$ is selected from deuterium, tritium, hydroxyl, mercapto, cyano, oxo, thio, halogen, amino, carboxyl, sulfo-group or $R_X$; wherein $R_X$ is selected from a C1-C6 alkyl, a C1-C6 alkoxy, or a C1-C6 alkyl thioether group;

$R_{a2}$ is selected from hydrogen, deuterium, tritium, or a deuterated C1-C6 alkyl; or, $R_{a2}$ is connected to $R_{a1}$ (or $R_{a3}$) to form $R_{ring\ A}$, and the $R_{ring\ A}$ is a tetrahydropyrrole ring; and a is 0, 1 or 2;

Preferably, $R_{a3}$ is Ry; wherein Ry is selected from a C1-C6 alkyl; and $R_Y$ is optionally substituted with one or more substituents $P_1$; the substituent $P_1$ is pyridyl;

more preferably, the compound of Formula Iz is:

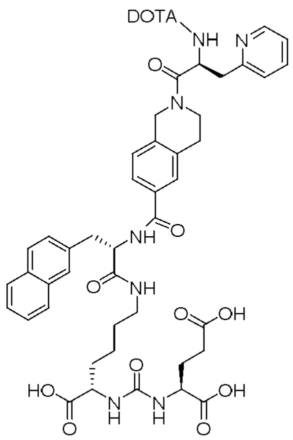

VWT045000

.

**20.** The compound or pharmaceutically acceptable salt, ester or solvate thereof according to any one of claims 1 to 11, wherein $X_b$ is

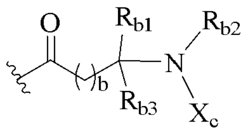

;

$X_c$ is a chelating agent or

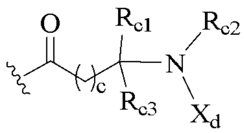

;

$X_d$ is a chelating agent or

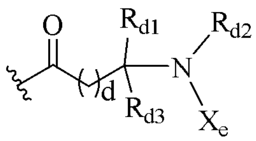

;

$X_e$ is a chelating agent;

$R_{a1}$, $R_{b1}$, $R_{c1}$ and $R_{d1}$ are all $R_{y1}$;

$R_{a2}$, $R_{b2}$, $R_{c2}$ and $R_{d2}$ are all $R_{y2}$;

$R_{a3}$, $R_{b3}$, $R_{c3}$ and $R_{d3}$ are all $R_{y3}$;

$R_{y1}$ and $R_{y3}$ are each independently selected from hydrogen, deuterium, tritium, $R_Y$, hydroxyl, mercapto, nitro, cyano, oxo, thio, halogen, amino, carboxyl, sulfo-group; $R_Y$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl (including but not limited to: oxadiazolidinyl), a 5-14 membered aryl (including but not limited to: phenyl), or a 5-14 membered heteroaryl (including but not limited to: imidazolyl, oxadiazolyl, benzopyrrolyl);

$R_Y$ is optionally substituted with one or more substituents $P_1$ or unsubstituted; the substituent $P_1$ is selected from deuterium, tritium, hydroxyl, mercapto, cyano, oxo, thio, halogen, $-NR_iR_j$, $-C(=O)R_k$, $-C(=O)OR_k$, $-S(=O)R_k$, $-S(=O)OR_k$, $-S(=O)(=O)R_k$, $- S(=O)(=O)OR_k$, $-C(=S)R_k$ or $R_W$;

$R_W$ is selected from a C1-C6 alkyl, a C1-C6 alkoxy, a C1-C6 alkyl thioether group, a C2-C6 alkenyl, a C2-C6 alkynyl, a 3-12 membered cycloalkyl, a 3-12 membered heterocyclic alkyl, a 5-14 membered aryl, or a 5-14 membered heteroaryl; and $R_W$ is optionally substituted with one or more substituents $P_2$ or unsubstituted; the substituent $P_2$ is selected from deuterium, tritium, hydroxyl, mercapto, cyano, oxo, thio, halogen, amino, carboxyl, sulfo-group or $R_X$; wherein $R_X$ is selected from a C1-C6 alkyl, a C1-C6 alkoxy, or a C1-C6 alkyl thioether group;

$R_k$ is selected from hydrogen, deuterium, tritium, $-NR_iR_j$, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_i$ and $R_j$ are each independently selected from hydrogen, deuterium, tritium, hydroxyl, mercapto, cyano, $-S(=O)R_z$, $-S(=O)OR_z$, $-S(=O)(=O)R_z$, $-S(=O)(=O)OR_z$, a C1-C6 alkyl, a C1-C6 alkoxy, a C1-C6 alkyl thioether group, a deuterated C1-C6 alkyl, a tritiated C1-C6 alkyl, a halogenated C1-C6 alkyl, an amino-substituted C1-C6 alkyl, a carboxyl-substituted C1-C6 alkyl, or a sulfo-substituted C1-C6 alkyl;

$R_z$ is selected from hydrogen, deuterium, tritium, halogen, a C1-C6 alkyl, a deuterated C1-C6 alkyl, a tritiated C1-C6 alkyl, or a halogenated C1-C6 alkyl;

$R_{y2}$ is selected from hydrogen, deuterium, tritium, a C1-C6 alkyl, a deuterated C1-C6 alkyl, or a tritiated C1-C6 alkyl; or, $R_{y2}$ is connected to $R_{y1}$ (or $R_{y3}$) to form $R_{ring A}$, the $R_{ring A}$ is a tetrahydropyrrole ring or a piperidine ring; the $R_{ring A}$ is optionally substituted with one or more substituents $P_{1a}$ or unsubstituted; the substituent $P_{1a}$ is selected from deuterium, tritium, halogen, hydroxyl, mercapto, nitro, cyano, amino, carboxyl, sulfo-group, a C1-C6 alkyl, a C1-C6 alkoxy, a C1-C6 alkyl thioether group, a halogenated C1-C6 alkyl, a deuterated C1-C6 alkyl, or a tritiated C1-C6 alkyl;

preferably,

$X_b$ is a chelating agent;

$R_{y1}$ is selected from hydrogen, deuterium, tritium, C1-C6 alkyl or deuterated C1-C6 alkyl;

$R_{y3}$ is hydrogen, deuterium, tritium, carboxyl, sulfo-group or $R_Y$;

$R_Y$ is selected from a C1-C6 alkyl; Ry is optionally substituted with one or more substituents $P_1$ or unsubstituted; and the substituent $P_1$ is selected from deuterium, tritium, hydroxyl, mercapto, cyano, oxo, thio, halogen, $-C(=O)R_k$, $-C(=O)OR_k$, $-S(=O)R_k$, $- S(=O)OR_k$, $-S(=O)(=O)R_k$, $-S(=O)(=O)OR_k$ or $R_W$; wherein $R_W$ is selected from a C1-C6 alkoxy, a C1-C6 alkyl thioether group, phenyl, benzopyrrolyl, imidazolyl, oxadiazolyl; and $R_W$ is optionally substituted with one or more substituents $P_2$ or unsubstituted; the substituent $P_2$ is selected from deuterium, tritium, hydroxyl, mercapto, cyano, oxo, thio, halogen or $R_X$; and $R_X$ is a C1-C6 alkyl;

$R_k$ is selected from hydrogen, deuterium, tritium, $-NR_iR_j$, a C1-C6 alkyl, a deuterated C1-C6 alkyl or a tritiated C1-C6 alkyl; $R_i$ and $R_j$ are each independently selected from hydrogen, deuterium, tritium, hydroxyl, mercapto, cyano, $-S(=O)R_z$, $-S(=O)OR_z$, $- S(=O)(=O)R_z$ or $-S(=O)(=O)OR_z$; wherein $R_z$ is selected from hydrogen, deuterium, tritium, halogen, a C1-C6 alkyl, a deuterated C1-C6 alkyl, or a tritiated C1-C6 alkyl;

$R_{y2}$ is selected from hydrogen, deuterium, tritium, a C1-C6 alkyl, or a deuterated C1-C6 alkyl; or, $R_{y2}$ is connected to $R_{y1}$ (or $R_{y3}$) to form $R_{ring A}$, the $R_{ring A}$ is a tetrahydropyrrole ring;

a, b, c and d are each independently selected from 0, 1 or 2.

**21.** The compound or pharmaceutically acceptable salt, ester or solvate thereof according to claim 20, wherein $R_{a1}$, $R_{b1}$, $R_{c1}$ and $R_{d1}$ are each independently selected from hydrogen, deuterium, tritium, or a C1-C6 alkyl;

$R_{a3}$, $R_{b3}$, $R_{c3}$ and $R_{d3}$ are each independently selected from hydrogen, deuterium, tritium, carboxyl, sulfo-group or $R_Y$; $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group or $R_W$; $R_W$ is selected from a C1-C6 alkoxy, a C1-C6 alkyl thioether group, phenyl, benzopyrrolyl or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;

$R_{a2}$, $R_{b2}$, $R_{c2}$ and $R_{d2}$ are each independently selected from hydrogen, deuterium, tritium or a deuterated C1-C6 alkyl; or, $R_{a2}$ is connected to $R_{a1}$ to form $R_{ring A}$, $R_{b2}$ is connected to $R_{b1}$ to form $R_{ring A}$, $R_{c2}$ is connected to $R_{c1}$ to

form $R_{ringA}$, $R_{d2}$ is connected to $R_{d1}$ to form $R_{ringA}$, the $R_{ringA}$ is a tetrahydropyrrole ring; and a, b, c and d are each independently selected from 0, 1 or 2;
preferably, the compound of Formula I is:

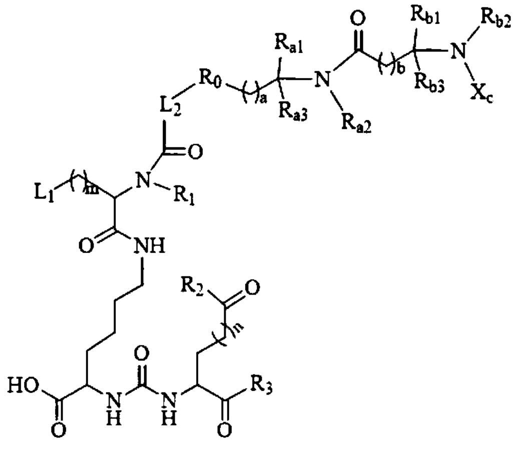

Formula Ie

wherein $X_c$ is a chelating agent;
further preferably, the compound of Formula Ie is selected from:

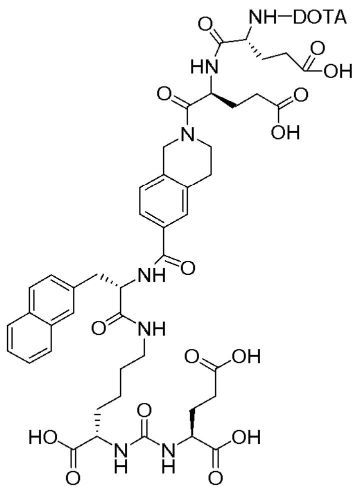

VWT023900 ,

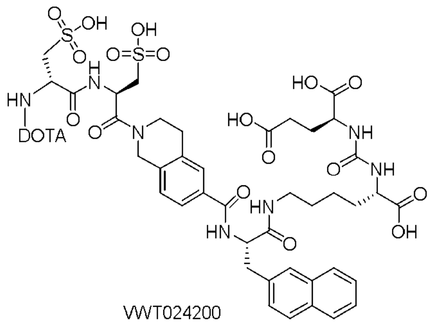

VWT024200 ,

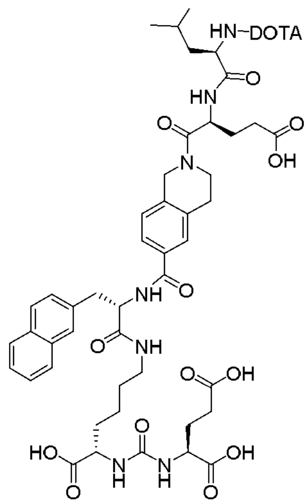

VWT037100 ,

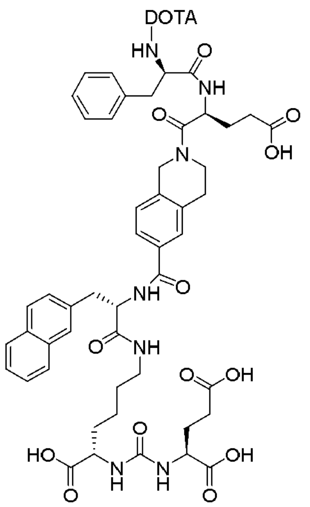
VWT037200 ,

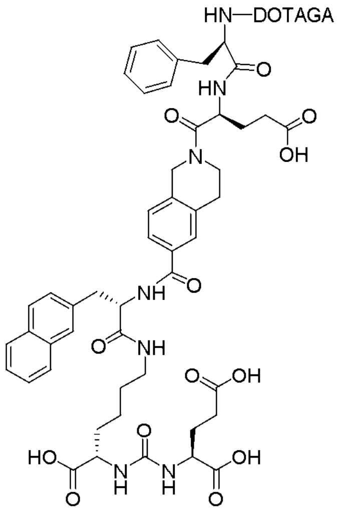
VWT038200 ,

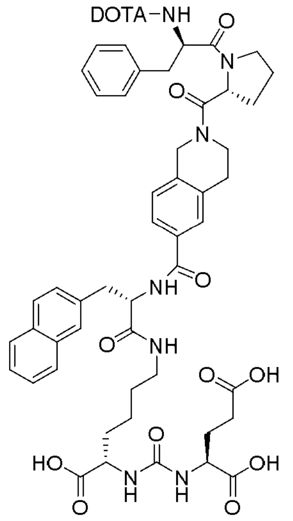
VWT038400 ,

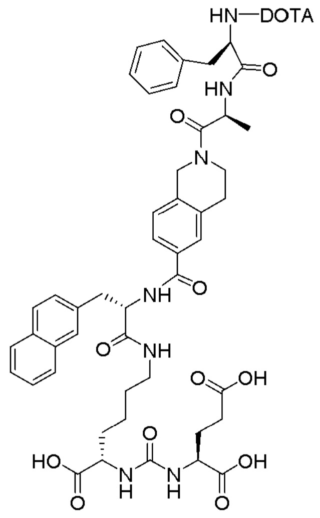
VWT039600 ,

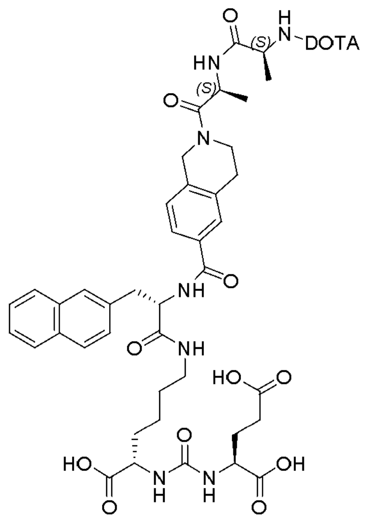
VWT043300 ,

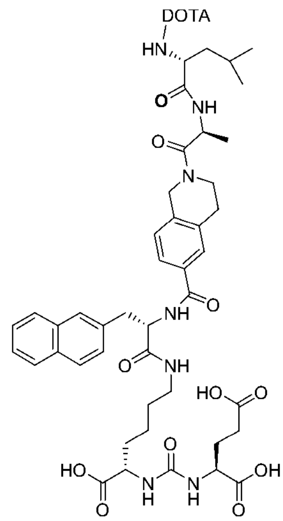
VWT044500 ,

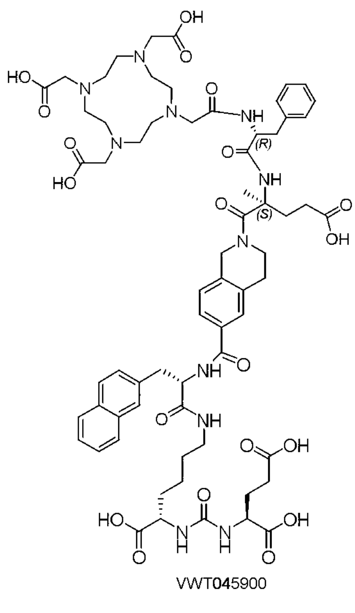
VWT045900 ,

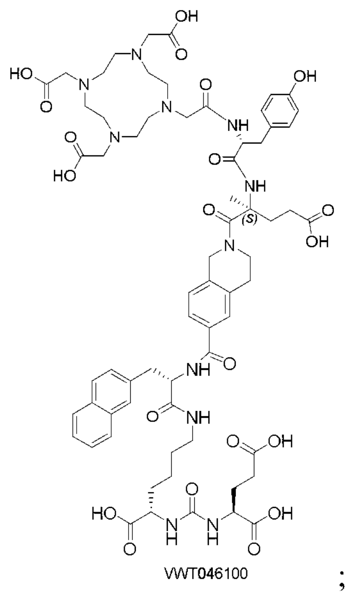
VWT046100 ;

preferably, the compound of Formula I is:

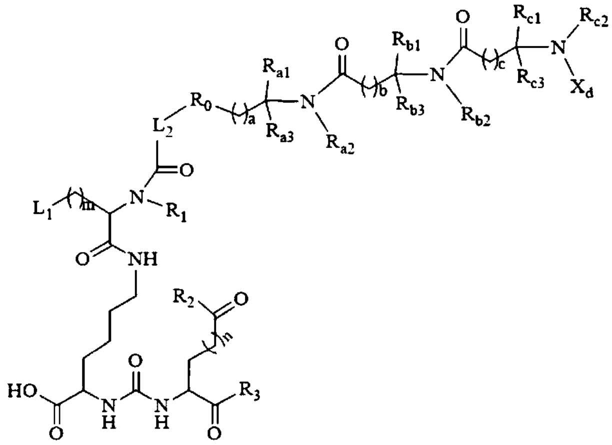

Formula If

wherein $X_d$ is a chelating agent;
further preferably, the compound of Formula If is selected from:

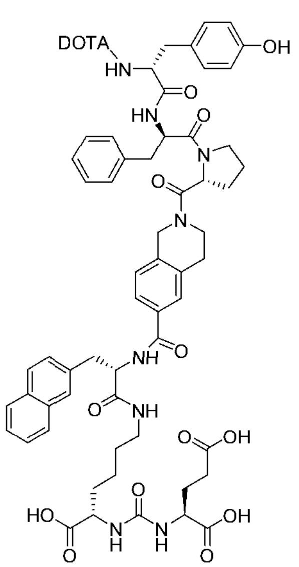

VWT038300 ,

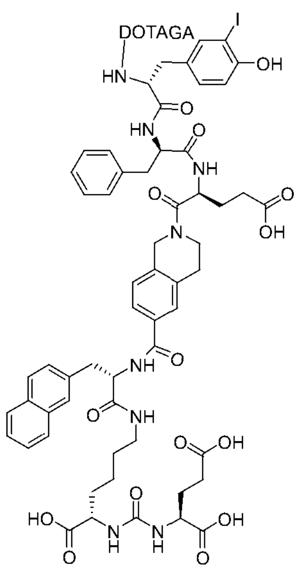

VWT038500 ,

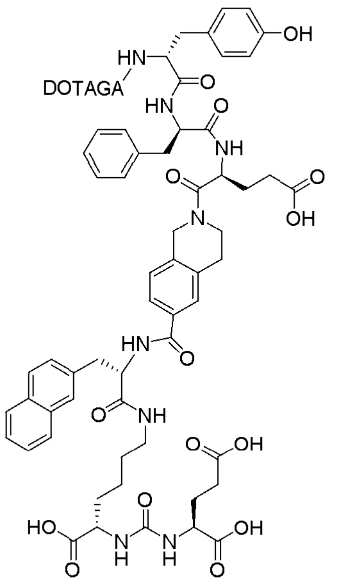

VWT038600 ,

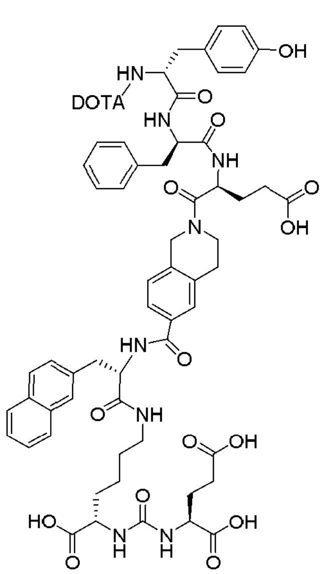
VWT038700 ,

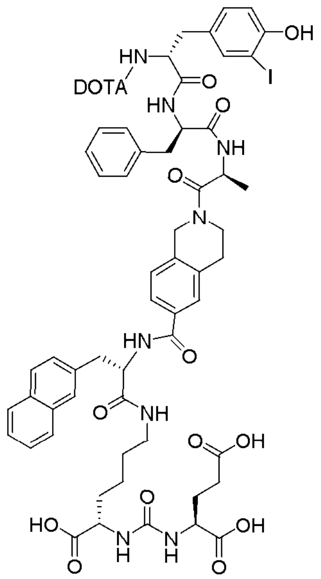
VWT039500 ,

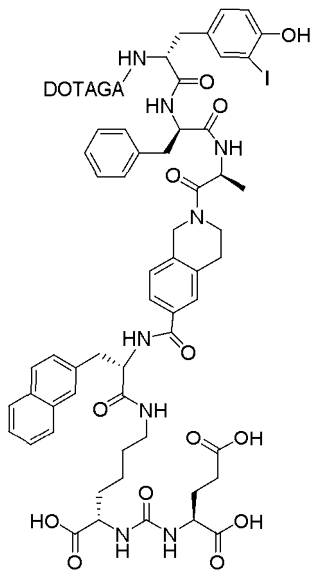
VWT039700 ,

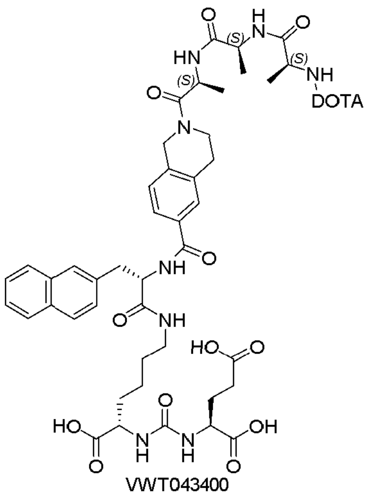
VWT043400 ,

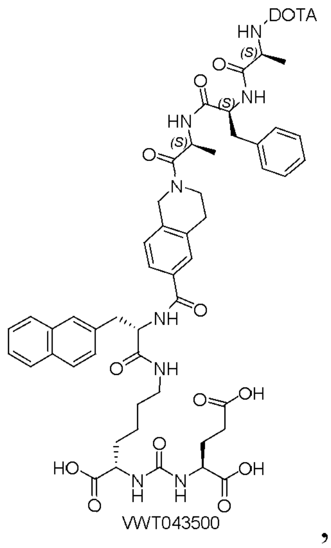
VWT043500 ,

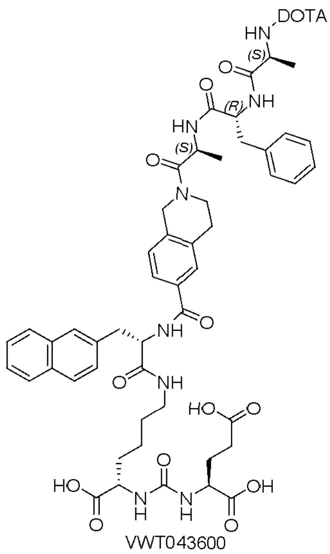
VWT043600 ;

preferably, the compound of Formula I is:

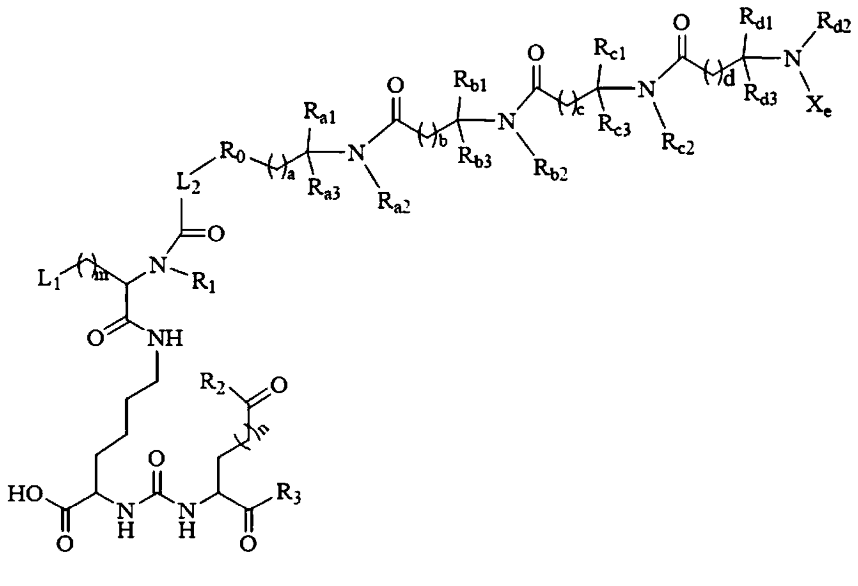

Formula Ig

wherein $X_e$ is a chelating agent;
further preferably, the compound of Formula Ig is:

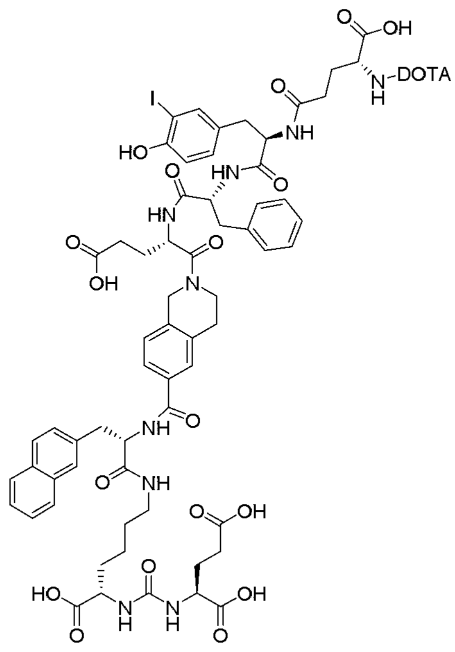

VWT037400

,

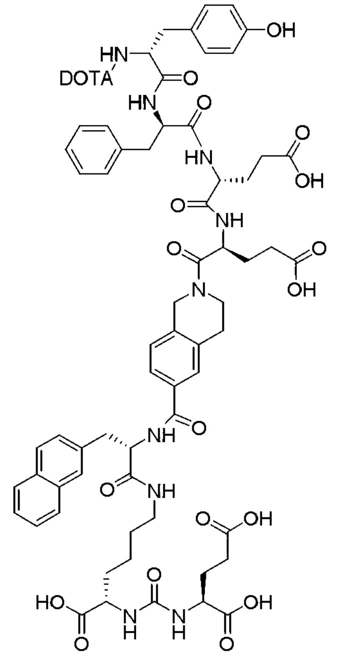

VWT039800

.

**22.** The compound or pharmaceutically acceptable salt, ester or solvate thereof according to claim 20, wherein $R_{a1}$, $R_{b1}$, $R_{c1}$ and $R_{d1}$ are each independently selected from hydrogen, deuterium, tritium, or a C1-C6 alkyl;

$R_{a3}$, $R_{b3}$, $R_{c3}$ and $R_{a3}$ are each independently selected from hydrogen, deuterium, tritium, carboxyl, sulfo-group or $R_Y$; wherein $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group, -S(=O)$R_k$, - S(=O)O$R_k$, -S(=O)(=O)$R_k$, -S(=O)(=O)O$R_k$ or $R_W$; wherein $R_k$ is selected from hydrogen, deuterium, tritium, amino, a C1-C6 alkyl, a deuterated C1-C6 alkyl, a tritiated C1-C6 alkyl, a halogenated C1-C6 alkyl, an amino-substituted C1-C6 alkyl, a carboxyl-substituted C1-C6 alkyl or a sulfo-substituted C1-C6 alkyl; $R_W$ is selected from a C1-C6 alkoxy, a C1-C6 alkyl thioether group, phenyl, benzopyrrolyl or imidazolyl; and $R_W$ is

optionally substituted with one or more substituents $P_2$ or unsubstituted; the substituent $P_2$ is selected from deuterium, tritium, hydroxyl, mercapto, halogen or a C1-C6 alkyl;

$R_{a2}$, $R_{b2}$, $R_{c2}$ and $R_{d2}$ are each independently selected from hydrogen, deuterium, tritium or a deuterated C1-C6 alkyl; or, $R_{a2}$ is connected to $R_{a1}$ to form $R_{ring A}$, $R_{b2}$ is connected to $R_{b1}$ to form $R_{ring A}$, $R_{c2}$ is connected to $R_{c1}$ to form $R_{ring A}$, $R_{d2}$ is connected to $R_{d1}$ to form $R_{ring A}$, the $R_{ring A}$ is a tetrahydropyrrole ring; and a, b, c and d are each independently selected from 0, 1 or 2;

preferably, $R_{a3}$, $R_{b3}$, $R_{c3}$ and $R_{d3}$ are each independently selected from hydrogen, deuterium, tritium, carboxyl, sulfo-group or $R_Y$; wherein Ry is a C1-C6 alkyl; and Ry is optionally substituted with one or more substituents $P_1$ or unsubstituted; the substituent $P_1$ is selected from -S(=O)(=O)$R_k$, -S(=O)(=O)O$R_k$ or $R_W$; wherein $R_k$ is selected from hydrogen, deuterium, tritium, or a C1-C6 alkyl; $R_W$ is selected from phenyl, benzopyrrolyl or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;

more preferably, the compound of Formula I is:

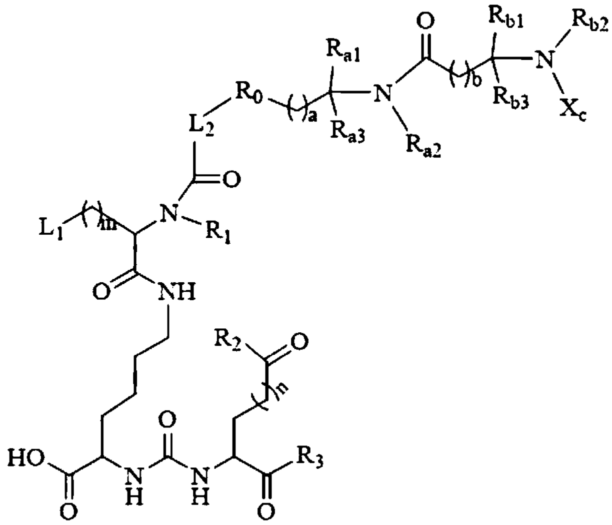

Formula Iy

wherein $X_c$ is a chelating agent;

further preferably, the compound of Formula Iy is selected from:

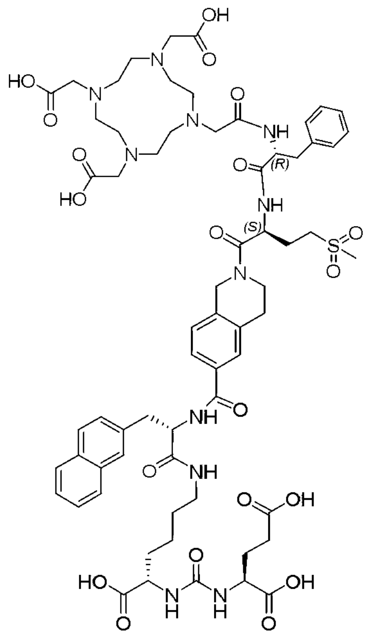

VWT045800 ,

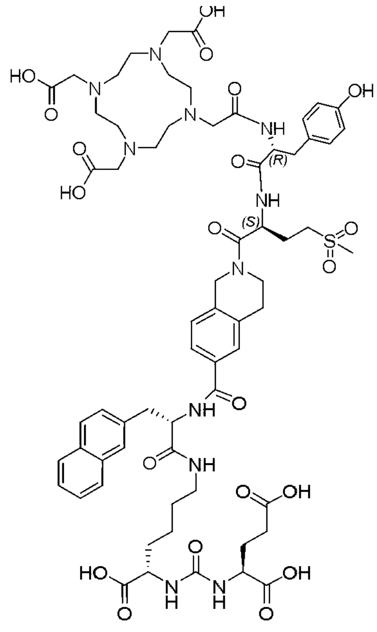

VWT046000 .

**23.** The compound or pharmaceutically acceptable salt, ester or solvate thereof according to any one of claims 1 to 11, wherein:

Q is

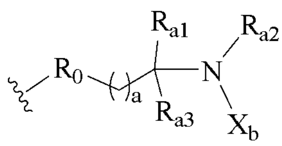

or

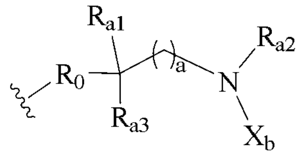

;

$R_0$ is selected from carbonyl or methylene;
$X_b$ is a chelating agent or

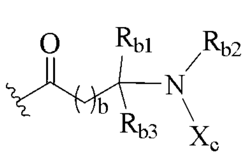

,

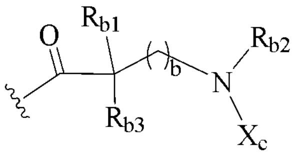

;

$X_c$ is a chelating agent or

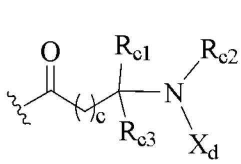

,

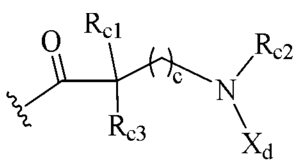

;

$X_d$ is a chelating agent or

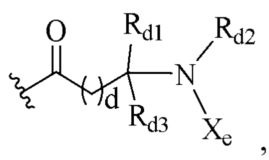 , 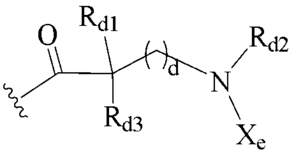 ;

$X_e$ is a chelating agent;

wherein $R_{a1}$, $R_{b1}$, $R_{c1}$ and $R_{d1}$, $R_{a2}$, $R_{b2}$, $R_{c2}$ and $R_{d2}$, $R_{a3}$, $R_{b3}$, $R_{c3}$ and $R_{d3}$, a, b, c and d are as defined in claim 20;

preferably, the compound of Formula I is:

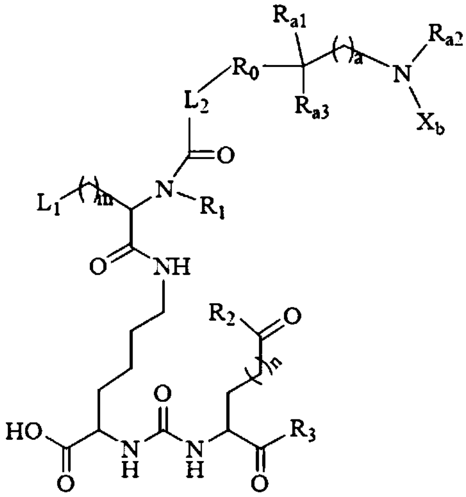

Formula Ix

wherein $X_b$ is

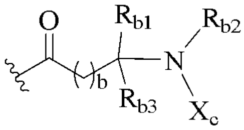 or 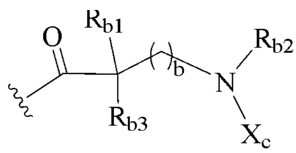 ,

$X_c$ is a chelating agent;

and/or,

$R_{a1}$ and $R_{b1}$ are each independently selected from hydrogen, deuterium, tritium or C1-C6 alkyl;

$R_{a3}$ and $R_{b3}$ are each independently selected from hydrogen, deuterium, tritium, carboxyl, sulfo-group or $R_Y$; wherein $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group or $R_W$; wherein $R_W$ is selected from a C1-C6 alkoxy, a C1-C6 alkyl thioether group, phenyl, benzopyrrolyl or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;

$R_{a2}$ and $R_{b2}$ are each independently selected from hydrogen, deuterium, tritium or a deuterated C1-C6 alkyl; or, $R_{a2}$ is connected to $R_{a1}$ to form $R_{ringA}$, $R_{b2}$ is connected to $R_{b1}$ to form $R_{ringA}$, and the $R_{ringA}$ is a tetrahydropyrrole ring; and a, b, c and d are each independently selected from 0, 1 or 2;

more preferably, the compound of Formula Ix is:

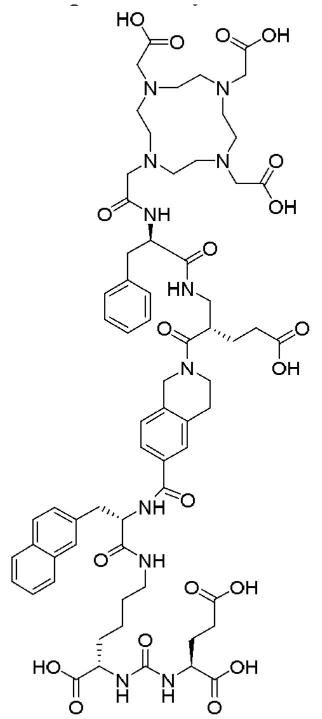

VWT037300

.

**24.** The compound or pharmaceutically acceptable salt, ester or solvate thereof according to any one of claims 1-3 or 4-8 or 9-11, wherein $L_2$ is $-NH-(CH_2)_t-L_3-$, and wherein t is 0, 1 or 2;
preferably,

$L_3$ is selected from a 3-12 membered cycloalkyl, or a 3-12 membered heterocyclic alkyl;
and/or,
Q is

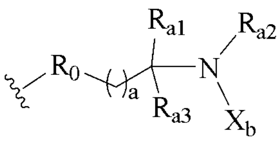 or 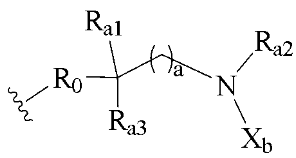 ;

$R_0$ is selected from carbonyl or methylene;
$X_b$ is a chelating agent or

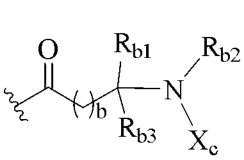 , 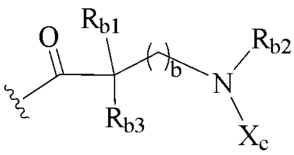 ;

$X_c$ is a chelating agent or

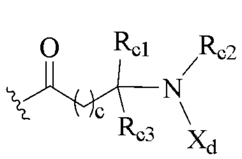 , 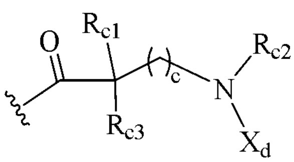 ;

$X_d$ is a chelating agent or

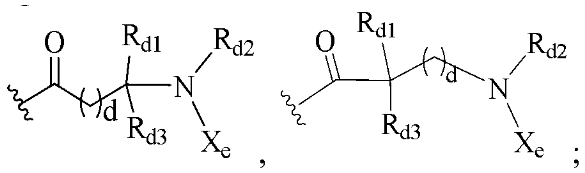

$X_e$ is a chelating agent;
wherein $R_{a1}$, $R_{b1}$, $R_{c1}$ and $R_{d1}$, $R_{a2}$, $R_{b2}$, $R_{c2}$ and $R_{d2}$, $R_{a3}$, $R_{b3}$, $R_{c3}$ and $R_{d3}$, a, b, c and d are as defined in claim 20.

**25.** The compound or pharmaceutically acceptable salt, ester or solvate thereof according to claim 24, wherein the compound of Formula I is:

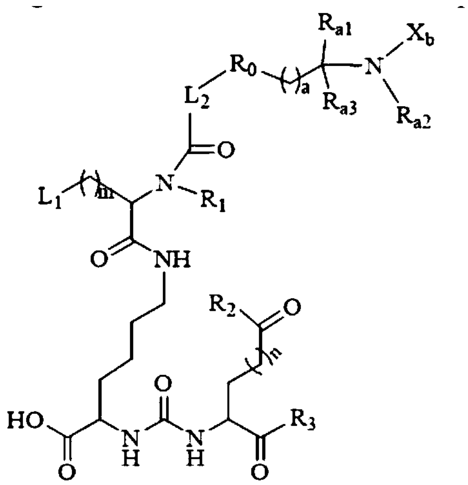

Formula Iw

wherein $X_b$ is a chelating agent or

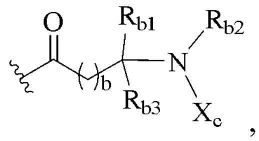

$X_c$ is a chelating agent;
and/or,
$R_{a1}$ and $R_{b1}$ are each independently selected from hydrogen, deuterium, tritium or a C1-C6 alkyl;
$R_{a3}$ and $R_{b3}$ are each independently selected from hydrogen, deuterium, tritium, carboxyl, sulfo-group or $R_Y$; wherein $R_Y$ is a C1-C6 alkyl; and $R_Y$ is optionally substituted with one or more substituents $P_1$ or unsubstituted; the substituent $P_1$ is selected from deuterium, tritium, hydroxyl, mercapto, halogen, carboxyl, sulfo-group or $R_W$; wherein $R_W$ is selected from a C1-C6 alkoxy, a C1-C6 alkyl thioether group, phenyl, benzopyrrolyl, or imidazolyl; and $R_W$ is optionally substituted with one or more substituents $P_2$ or unsubstituted; the substituent $P_2$ is selected from deuterium, tritium, hydroxyl, mercapto, halogen or a C1-C6 alkyl;
$R_{a2}$ and $R_{b2}$ are each independently selected from hydrogen, deuterium, tritium or a deuterated C1-C6 alkyl; or, $R_{a2}$ is connected to $R_{a1}$ to form $R_{ringA}$, $R_{b2}$ is connected to $R_{b1}$ to form $R_{ringA}$, and the $R_{ringA}$ is a tetrahydropyrrole ring; and a, b, c and d are each independently selected from 0, 1 or 2;
preferably, the compound of Formula Iw is selected from:

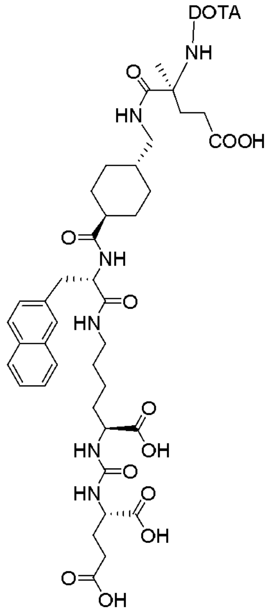

VWT049200 ,

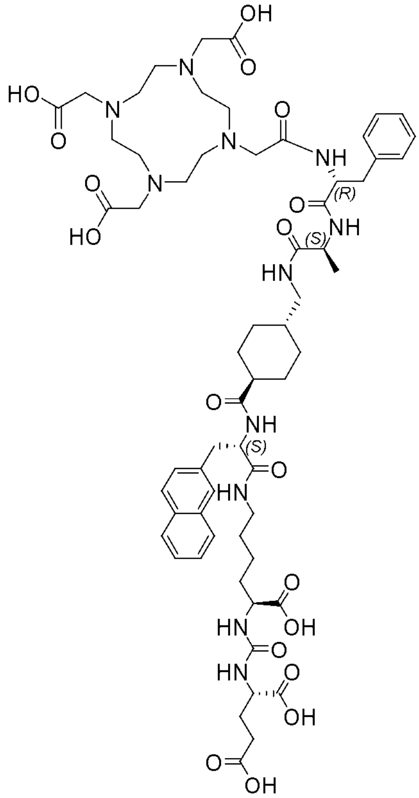

VWT049400 ,

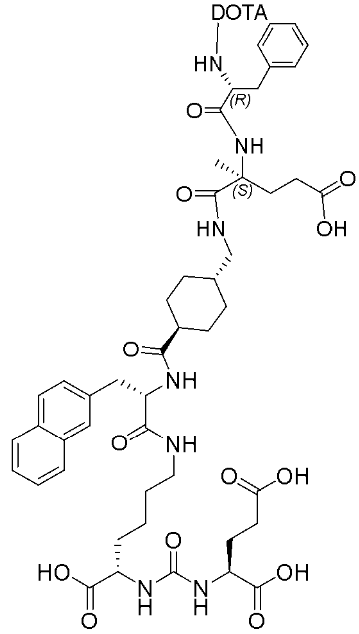

VWT0049900 ,

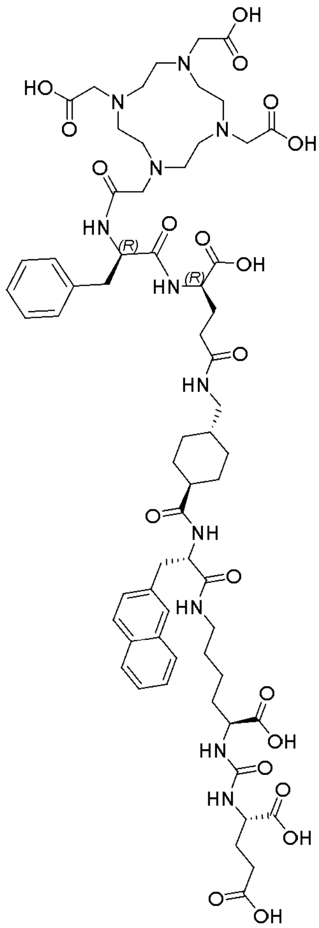

VWT050000.

**26.** The compound or pharmaceutically acceptable salt, ester or solvate thereof according to any one of claims 1 to 25, wherein the pharmaceutically acceptable salt is a base addition salt; preferably, the base addition salt is a sodium salt, potassium salt, ammonium salt, or calcium salt.

**27.** Use of the compound or pharmaceutically acceptable salt, ester, or solvate thereof according to any one of claims 1 to 26 in the preparation of a radiolabeled complex.

**28.** A radiolabeled complex comprising the compound or pharmaceutically acceptable salt, ester or solvate thereof according to any one of claims 1 to 26, and a radionuclide;

preferably, the radionuclide is selected from $^{94}$Tc, $^{99m}$Tc, $^{90}$In, $^{111}$In, $^{67}$Ga, $^{68}$Ga, $^{86}$Y, $^{90Y}$, $^{177}$Lu, $^{151}$Tb, $^{186}$Re, $^{188}$Re, $^{64}$Cu, $^{67}$Cu, $^{55}$Co, $^{57}$Co, $^{43}$Sc, $^{44}$Sc, $^{47}$Sc, $^{225}$Ac, $^{213}$Bi, $^{212}$Bi, $^{212}$Pb, $^{227}$Th, $^{153}$Sm, $^{166}$Ho, $^{152}$Gd, $^{153}$Gd, $^{157}$Gd, $^{166}$Dy, $^{55}$Fe, $^{18}$F, 11C, $^{89}$Zr, $^{123}$I, $^{124}$I, $^{125}$I, $^{131}$I or $^{211}$At;
more preferably, the radionuclide is $^{68}$Ga or $^{177}$Lu; or, the radionuclide is $^{225}$Ac.

**29.** A pharmaceutical composition comprising the radiolabeled complex according to claim 28, and one or more pharmaceutically acceptable excipients, diluents, or carriers.

**30.** Use of the radiolabeled complex according to claim 28 and/or the pharmaceutical composition according to claim 29 in the preparation of a medicament for the diagnosis and/or treatment and/or prevention of tumors; preferably, the tumor is cancer; more preferably, the cancer is prostate cancer.

**31.** Use of the radiolabeled complex according to claim 28 and/or the pharmaceutical composition according to claim 29 in the preparation of a medicament for imaging in patients; or, use of the radiolabeled complex according to claim 28 and/or the pharmaceutical composition according to claim 29 in the preparation of a contrast agent.

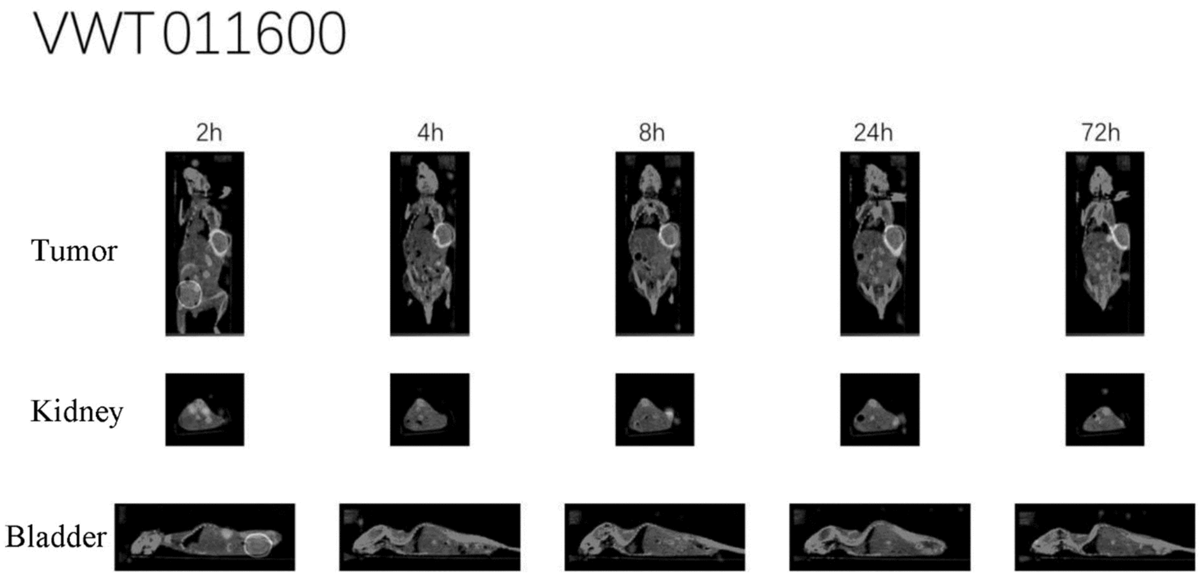
VWT011600
2h
4h
8h
24h
72h
Tumor
Kidney
Bladder

Fig. 1

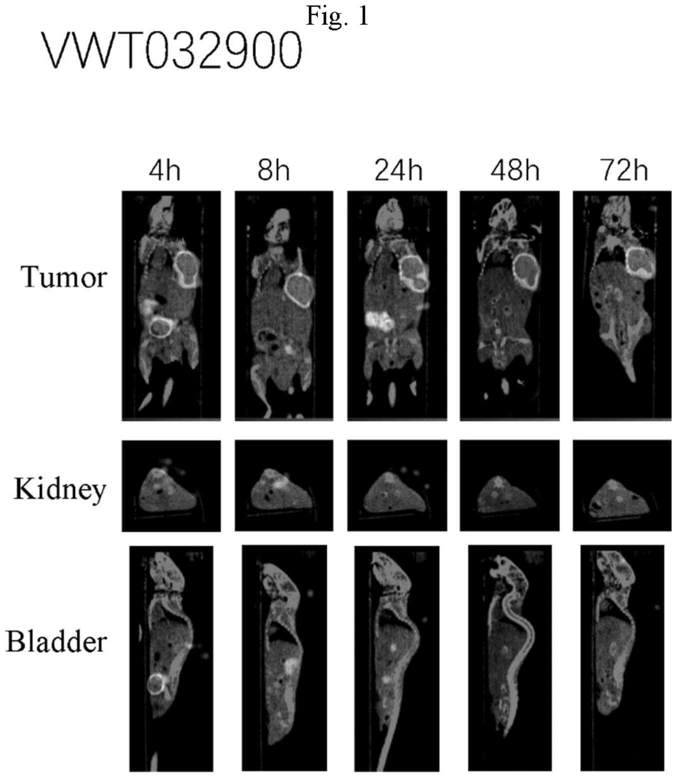
VWT032900
4h
8h
24h
48h
72h
Tumor
Kidney
Bladder

Fig. 2

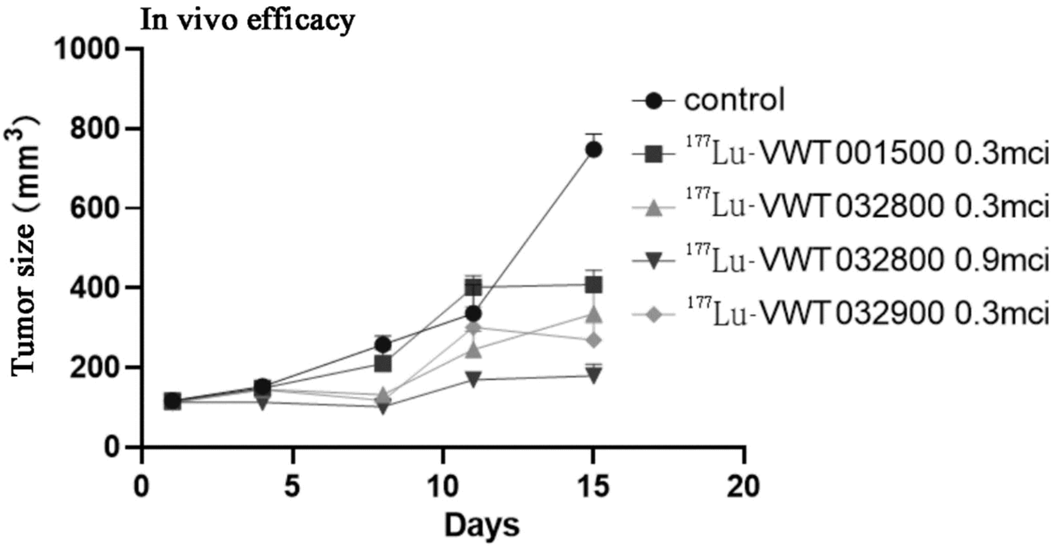
In vivo efficacy
Tumor size (mm3)
1000
800
600
400
200
0
0
5
10
15
20
Days
control
177Lu-VWT 001500 0.3mci
177Lu-VWT 032800 0.3mci
177Lu-VWT 032800 0.9mci
177Lu-VWT 032900 0.3mci

Fig. 3

## INTERNATIONAL SEARCH REPORT

International application No.
**PCT/CN2024/128619**

**A. CLASSIFICATION OF SUBJECT MATTER**

C07D401/12(2006.01)i; C07D405/12(2006.01)i; A61K51/04(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

CNTXT, VEN, ENTXT, CNKI, 万方, WANFANG, STN(Casplus, Registry): 前列腺特异性膜抗原, 异四氢喹啉, PSMA, prostate-specific membrane antigen, prostate specific membrane antigen, TETRAHYDROISOQUINOLINE, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 115745903 A (JINGHE BIO-MEDICINE TECHNOLOGY (NANJING) CO., LTD.) 07 March 2023 (2023-03-07)<br>claim 1, and compounds 3-4, 9, and 12 in claim 5 or 6. | 1-31 |
| X | WO 2023083209 A1 (SUZHOU RUIHE MEDICINE TECHNOLOGY CO., LTD.) 19 May 2023 (2023-05-19)<br>page 13, compound 3 | 1-31 |
| X | WO 2023030434 A1 (TIANJIN HENGRUI MEDICINE CO., LTD. et al.) 09 March 2023 (2023-03-09)<br>page 20, compounds 2, 6, and 10, etc. | 1-14, 16-23, 26-31 |
| PX | WO 2023222680 A1 (DEUTSCHES KREBSFORSCHUNGSZENTRUM STIFTUNG DES OEFFENTLICHEN RECHTS et al.) 23 November 2023 (2023-11-23)<br>pages 17-19 | 24-31 |
| X | US 2021121585 A1 (LI MINGXIN et al.) 29 April 2021 (2021-04-29)<br>table 1, and figure 1 | 24-31 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 December 2024** | **07 January 2025** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/128619**

**C. DOCUMENTS CONSIDERED TO BE RELEVANT**

| Category* | Citation of document, with indication, where appropriate, of the relevant passages | Relevant to claim No. |
|---|---|---|
| A | CN 112190722 A (NANFANG HOSPITAL OF SOUTHERN MEDICAL UNIVERSITY) 08 January 2021 (2021-01-08)<br>entire document | 1-31 |

Form PCT/ISA/210 (second sheet) (July 2022)

**INTERNATIONAL SEARCH REPORT**
**Information on patent family members**

International application No.
**PCT/CN2024/128619**

| Patent document cited in search report | Publication date (day/month/year) | Patent family member(s) | Publication date (day/month/year) |
|---|---|---|---|
| CN 115745903 A | 07 March 2023 | EP 4400505 A1 | 17 July 2024 |
| | | JP 2024532521 A | 05 September 2024 |
| | | WO 2023030509 A1 | 09 March 2023 |
| | | US 2024366813 A1 | 07 November 2024 |
| WO 2023083209 A1 | 19 May 2023 | CA 3237743 A1 | 19 May 2023 |
| | | EP 4431503 A1 | 18 September 2024 |
| | | TW 202321204 A | 01 June 2023 |
| | | AU 2022384375 A1 | 09 May 2024 |
| | | KR 20240105380 A | 05 July 2024 |
| WO 2023030434 A1 | 09 March 2023 | AU 2022338842 A1 | 28 March 2024 |
| | | KR 20240055014 A | 26 April 2024 |
| | | MX 2024002552 A | 10 April 2024 |
| | | US 2024366809 A1 | 07 November 2024 |
| | | JP 2024532475 A | 05 September 2024 |
| | | EP 4397322 A1 | 10 July 2024 |
| | | CA 3230165 A1 | 09 March 2023 |
| | | TW 202313000 A | 01 April 2023 |
| WO 2023222680 A1 | 23 November 2023 | None | |
| US 2021121585 A1 | 29 April 2021 | US 11850291 B2 | 26 December 2023 |
| CN 112190722 A | 08 January 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2023030509 A1


### Non-patent literature cited in the description


- *CHEMICAL ABSTRACTS*, 112883-43-9
- *CHEMICAL ABSTRACTS*, 128625-52-5
- *CHEMICAL ABSTRACTS*, 2592-95-2
- *CHEMICAL ABSTRACTS*, 148893-10-1
- *CHEMICAL ABSTRACTS*, 156311-83-0
- *CHEMICAL ABSTRACTS*, 39968-33-7
- *CHEMICAL ABSTRACTS*, 137076-54-1
- *CHEMICAL ABSTRACTS*, 873924-12-0
- *CHEMICAL ABSTRACTS*, 268734-27-6
- *CHEMICAL ABSTRACTS*, 1443238-78-5
- *CHEMICAL ABSTRACTS*, 2138226-21-6
- *CHEMICAL ABSTRACTS*, 149353-71-9
- *CHEMICAL ABSTRACTS*, 82911-69-1
- *CHEMICAL ABSTRACTS*, 6674-22-2
- *CHEMICAL ABSTRACTS*, 35661-39-3
- *CHEMICAL ABSTRACTS*, 22717-56-2
- *CHEMICAL ABSTRACTS*, 26690-80-2
- *CHEMICAL ABSTRACTS*, 71989-18-9
- *CHEMICAL ABSTRACTS*, 145038-50-2
- *CHEMICAL ABSTRACTS*, 751470-47-0
- *CHEMICAL ABSTRACTS*, 104091-08-9
- *CHEMICAL ABSTRACTS*, 51814-54-1
- *CHEMICAL ABSTRACTS*, 133803-81-3
- *CHEMICAL ABSTRACTS*, 1072845-48-7
- *CHEMICAL ABSTRACTS*, 86123-10-6
- *CHEMICAL ABSTRACTS*, 26386-88-9
- *CHEMICAL ABSTRACTS*, 3849-21-6
- *CHEMICAL ABSTRACTS*, 109745-15-5